# EUROPEAN PATENT SPECIFICATION

(11) **EP 2 566 529 B1**
(45) Date of publication and mention of the grant of the patent: **31.08.2016**
(21) Application number: 11722655.5
(22) Date of filing: 03.05.2011
(51) Int. Cl.: A61F 2/04, A61K 35/34, A61L 27/38

(54) **SMOOTH MUSCLE CELL CONSTRUCTS**
KONSTRUKTE AUS GLATTEN MUSKELZELLEN
PRODUITS DE RECOMBINAISON À BASE DE CELLULES MUSCULAIRES LISSES

(30) Priority: 12.11.2010 US 413379 P; 03.12.2010 US 419751 P; 19.08.2010 US 375106 P; 03.05.2010 US 330774 P; 28.02.2011 US 447460 P; 06.08.2010 US 371541 P; 22.11.2010 US 416267 P; 03.05.2010 US 330810 P; 12.11.2010 US 413371 P; 12.05.2010 US 334148 P
(43) Date of publication of application: 13.03.2013
(73) Proprietor: RegenMed (Cayman) Ltd., George Town Grand Cayman KY1-1104 (KY)
(72) Inventor: LUDLOW, John, W., Carrboro NC 27510 (US); JAYO, Manuel, J., Winston-Salem NC 27104 (US); BASU, Joydeep, Winston-Salem NC 27103 (US); BERTRAM, Timothy A., Statesville NC 28677 (US); GENHEIMER, Christopher W., Colfax NC 27235 (US); GUTHRIE, Kelly I., Winston-Salem NC 27104 (US); ILAGAN, Roger M., Burlington NC 27215 (US); JAIN, Deepak, Winston-Salem NC 27104 (US); KNIGHT, Oluwatoyin A., Winston-Salem NC 27127 (US); PAYNE, Richard, Winston-Salem NC 27101 (US); QUINLAN, Sarah F., Clemmons NC 27012 (US); RAPOPORT, H. Scott, 48600 Sopelana Vizkaya (ES); SANGHA, Namrata D., Winston-Salem NC 27104 (US); SHOKES, Jacob E., Winston-Salem NC 27127 (US); BURNETTE, Teresa B., High Point NC 27265 (US); BOYD, Sarah A., Mooresville NC 28117 (US); HALBERSTADT, Craig R., Clemmons NC 27012 (US); JUSTEWICZ, Dominic M., Winston-Salem NC 27101 (US); RIVERA, Elias A., Oak Ridge NC 27310 (US); SHARP, Wendy, Winston-Salem NC 27104 (US)
(74) Representative: Denison, Christopher Marcus
(86) International application number: PCT/US2011/035058
(87) International publication number: WO 2011/140137

(56) References cited:
- WO-A1-2004/103461
- WO-A2-2007/095193
- US-A- 5 855 610
- US-B1- 6 576 019
- YOSHIO HORI ET AL: "Experimental Study on Tissue Engineering of the Small Intestine by Mesenchymal Stem Cell Seeding", JOURNAL OF SURGICAL RESEARCH, vol. 102, no. 2, 1 February 2002 (2002-02-01), pages 156-160, XP55023368, ISSN: 0022-4804, DOI: 10.1006/jsre.2001.6294

## Description

### Field of the Invention

The present invention relates to the reconstruction, augmentation or replacement of luminal organs or tissue structures in a subject in need using scaffolds seeded with cells obtained from sources that are not derived from the corresponding organ or tissue structure that is the subject of the reconstruction, augmentation or replacement.

### Background of the Invention

Recent studies have demonstrated the promise *of de novo* regeneration of luminal or tubular organs in humans. In one study, pediatric patients' bladders were enlarged by implanting tubular, biodegradable scaffolds seeded with autologous urothelial and bladder smooth muscle cells. The implants initiated regeneration of full-thickness bladder wall with laminarly organized architecture and concomitant urologic functionality (Atala, A., et al. (2006) Lancet 367, 1241-1246). In another study, a functional human trachea was engineered using a scaffold of decellularized, cadaveric tracheal segment seeded with autologous respiratory epithelial cells and chondrocytes generated by directed differentiation of autologous bone marrow cells (Asnaghi, M.A., et al. (2009) Biomaterials 30, 5260-5269; Macchiarini, P., et al. (2008) Lancet 372, 2023-2030).

The use of cell-seeded scaffolds has the potential to address defects in different types of luminal or tubular organ settings. Several anomalies can cause the bladder to develop abnormally and require surgical augmentation. Urinary diversion is a way to route and excrete urine from the body when an individual is unable to urinate due to a damaged or non-functional urinary system. In general, any condition that blocks the flow of urine and increases pressure in the ureters and/or kidneys may require a urinary diversion. Some common indications for diversion include cancer of the bladder requiring a cystectomy, a neurogenic bladder that impact renal function, radiation injury to the bladder, intractable incontinence that occurs in women, and chronic pelvic pain syndromes. In general, two major strategies exist for urinary diversion: a urostomy and a continent diversion. Although small intestinal submucosa (SI) may be used for urinary diversion, it has been reported that the removal of the mucosa and submucosa may lead to retraction of the intestinal segment (see, e.g., Atala, A., J. Urol. 156:338 (1996)). Therefore, a need exists for other methods and devices of providing urinary diversion systems to patients in need.

Urinary incontinence is a prevalent problem that affects people of all ages and levels of physical health, both in the community at large and in healthcare settings. One current treatment for urge incontinence is injection of neurotoxins, such as botulinum toxin, e.g., Botox®. It is thought that botulinum toxin exerts its effect on bladder hyperactivity by paralyzing the detrusor muscle in the bladder wall or possibly impacting afferent pathways in the bladder and reducing sensory receptors in suburothelial nerves. The large size of the botulinum toxin molecule can limit its ability to diffuse, and thus prohibits it from reaching both afferent and efferent nerve fibers. However, treatment with Botox® may require many injections (typically 20 to 50) of botulinum toxin into the bladder muscle wall. There remains a need for alternative methods to treat urinary incontinence.

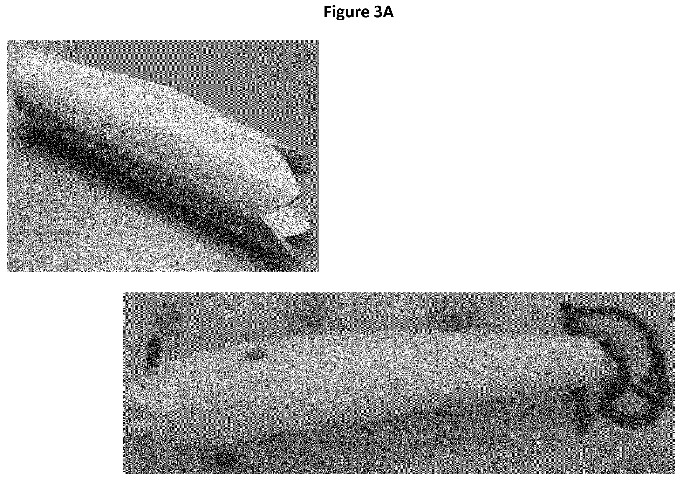

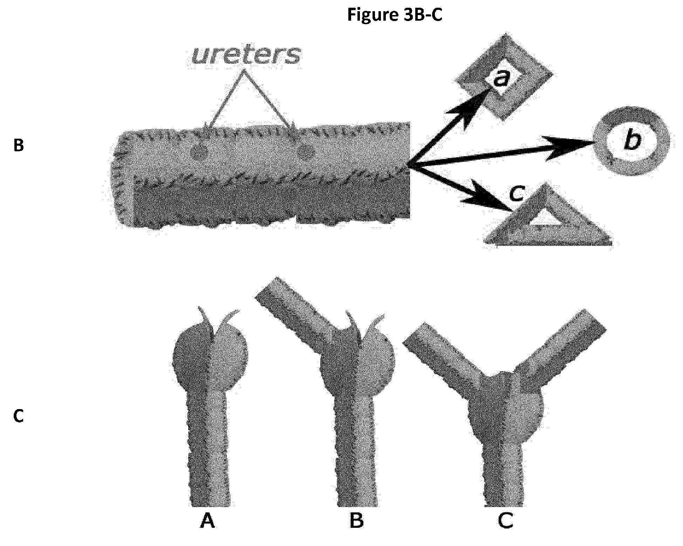

Lung may be regarded as a highly specialized tubular organ amenable to regeneration utilizing adipose-derived smooth muscle cells and a biodegradable scaffold. It has been reported that polyglycolic acid (PGA) felt sheets seeded with adipose derived "stromal cells" demonstrated pulmonary regeneration within a rat lung lobectomy model (Shigemura et al., Am J Respir Crit Care Med Vol 174. pp 1199-1205, 2006). The cell seeded PGA sheet was sealed onto the remaining lung lobe. Alveolar and vascular regeneration was observed within 1 week of implantation, with concomitant recovery of pulmonary functionality. In another study, fetal rat lung cells were seeded onto gel-foam sponge-based scaffolds and implanted into adult rat lung. Alveolar-like structures with apparent vascular networks were observed to regenerate within degrading scaffold by 4 months post-implantation (Andrade et al., Am J Physiol Lung Cell Mol Physiol 292:510-518, 2007). The results of in vivo studies using poly-lactic-co-glycolic acid/poly-L-lactic-acid (PLGA/PLLA) scaffolds seeded with fetal lung cells (Mondrinos et al. Tissue Engineering 12(4): 717-728, 2006; Mondrinos et al. Am J Physiol Lung Mol Physiolo 239: L639-L650, 2007) suggests that appropriate combinations of exogenous fibroblast growth factors chosen to target specific receptor isoforms will facilitate appropriate lung epithelial and mesenchymal cell behavior conducive to tissue regeneration. Mondrinos et al. (2006) also reported the observation that three-dimensional MATRIGEL™ constructs contained alveolar forming units (AFUs) (Abstract, Fig. 3). There remains a need for additional cell populations suitable for providing regenerative medicine-based therapeutics for the lung.

Defects in the gastrointestinal tract is another area where a need for alternative methods of treatment exists. A functional human trachea was engineered using a scaffold of decellularized, cadaveric tracheal segment seeded with autologous respiratory epithelial cells and chondrocytes generated by directed differentiation of autologous bone marrow cells (Macchiarini, P., et al. (2008) *supra*). A swine model was used for a tissue engineered trachea where both the chondrocytes derived from differentiated MSC as well as the epithelial cells were needed for host survival (Go et al. (2010) J Thorac Cardiovasc Surg 139, 437-443). Chondrocytes derived directly from autologous tracheal explants may be applicable towards de novo regeneration of trachea (Komura, M., et al. (2008) J Pediatr Surg 43, 2141-2146; Komura, M., et al. (2008) Pediatr Surg Int 24, 1117-1121).

In addition, the small intestine (SI) currently represents a pressing clinical need, with small bowel transplantation being an unsatisfactory current standard of care for pediatric small bowel syndrome. Autologous organoid units, composed of incompletely dissociated clusters of epithelial and mesenchymal cells, were derived by partial digestion of intestinal epithelium (presumably containing resident intestinal stem cells) and used to seed PLLA scaffold tubes that were subsequently matured within the peritoneal cavity of pigs. At seven weeks post-implantation, the retrieved implants were observed to contain tissue segments that recapitulated the gross overall laminar organization of native SI (Sala, F.G., et al. (2009) J Surg Res 156, 205-21225). Importantly, acellular scaffolds similarly implanted into the peritoneum did not yield gastrointestinal tissue segments. However, the effect of grafting such tissue engineered SI segments to host SI in large mammals remains to be demonstrated. Furthermore, this approach required harvesting up to 10 cm of autologous SI to derive organoids for implant. In addition, it is not certain whether *ex vivo* expansion could reduce the amount of autologous SI required, whether organoid units capable of seeding a scaffold structure can be isolated from diseased human intestine, or how much autologous tissue will be required to generate clinically-relevant implants. In another report, collagen sponge scaffolds with or without stomach-derived SMC were grafted onto 1x1 cm defects in surgically isolated ileal loops of dogs. Macroscopic analysis of tissue at the SMC+ implant sites demonstrated regeneration of native-like neo-mucosa. However, tissue at the SMC- implant sites remained ulcerated. Significantly enhanced vascularization, epithelialization, and circular muscle organization was also observed at the SMC+ implant sites relative to SMC- implant sites (Nakase, Y., et al. (2006) Tissue Eng 12, 403-412). An increase in the number of SMC seeded onto the scaffold resulted in a greater area of regenerated SI tissue, although no concomitant increase in the thickness of the smooth muscle layer was observed (Nakase, Y., et al. (2007) J Surg Res 137, 61-68).

The luminal or tubular organ regeneration approach may also be applicable to esophagus regeneration. Patch defects made in the abdominal esophagus of 27 female rats were patched with cell-free scaffolds generated from gastric acellular matrix. Of the 24 survivors, none showed evidence of lamina muscularis mucosae regeneration even at 18 months post-implantation (Urita, Y., et al. (2007) Pediatr Surg Int 23, 21-26). In contrast, in a canine model of esophageal resection and replacement, PGA tubes seeded with a mixture of keratinocytes and fibroblasts triggered regeneration of smooth muscle laminar organization similar to native esophagus within 3 weeks post-implantation, whereas acellular PGA tubes formed esophageal strictures and led to near complete obstruction within 2-3 weeks (Nakase, Y., et al. (2008) J Thorac Cardiovasc Surg 136, 850-859). Attempts to introduce an acellular SIS tubular construct into the cervical esophagus of piglets were also unsuccessful, demonstrating scarification and a minimal regenerative response (Doede, T., et al. (2009) Artif Organs 33, 328-333).

Stomach-derived organoid units, when seeded on a biopolymeric scaffold, triggered reconstitution of the gastric and muscularis mucosae in stomach tissue engineered within the peritoneal cavities of swine (Sala, F.G., et al. (2009) J Surg Res 156, 205-212). Using a canine model, circular defects were created in the stomach of 7 animals and a composite biodegradable scaffold ("New-sheet"), soaked with either autologously derived peripheral blood or bone marrow aspirate, was sutured over the defect. By 16 weeks post implantation, the defect site had formed regenerated stomach with evidence of re-epithelialization, formation of villi, vascularization and fibrosis within the submucosal layer. However, minimal regeneration of the smooth muscle layer was observed, as shown by expression of smooth muscle α-actin, though not calponin, a marker consistent with the phenotype of mature smooth muscle cells (Araki, M., et al. (2009) Artif Organs 33, 818-826).

Though strictly not a tubular organ, the anal sphincter is a component of the gastrointestinal tract and is critical in regulating patency of the large intestine. Recent efforts to engineer the anal sphincter leverage the same general platform used to catalyze bladder regeneration. To this end, smooth muscle cells isolated from human internal anal sphincter were seeded onto fibrin gels poured around a central mold. Cell mediated contraction of the gel around the mold resulted in the formation of a 3D cylindrical tube of sphincteric smooth muscle tissue. Although in vivo anastamosis remains to be demonstrated, this bio-engineered anal sphincter demonstrated contractile properties and response to defined neurotransmitters consistent with the functionality of native anal sphincter (Hashish, M., et al. (2010) J Pediatr Surg 45, 52-58; Somara, S., et al. (2009) Gastroenterology 137, 53-61). Use of alternatively sourced smooth muscle cells may facilitate the transition of engineered sphincter towards commercial production.

A major problem in blood vessel tissue engineering is the construction of vessel grafts that possess suitable, long-lasting biomechanical properties commensurate with native vessels. Arterial replacements pose special challenges due to both the cyclic loading common to all vessels, but additionally the higher operating pressure required of those vessels. Researchers have approached this problem through a variety of synthetic and organic materials, different construction modalities (e.g. electrospinning and casting) and numerous composite designs. For example, attempts have been made to create blood vessel grafts using various combinations of donor grafts, natural components, and synthetic components (see e.g. Zilla et al., U.S. Published Patent Application 2005/0131520; Flugelman, U.S. Published Patent Application 2007/0190037; Shimizu, U.S. Patent 6,136,024; Matsuda et al., U.S. Patent 5,718,723; and Rhee et al., U.S. Patent 5,292,802). Other scaffolds composed of poly (ester urethane) ureas (PEUU) (Courtney et al. (2006) Biomaterials,. 27:3631-3638), and PEUU/collagen (Guan et al. (2006) Cell Transplant. Vol. 15. Supp. I;S17-S27) have been reported as exhibiting tissue-like functional properties.

Ludlow et al. U.S. Published Application No. 20100131075 relates to the regeneration, reconstruction, augmentation or replacement of laminarily organized luminal organs or tissue structures in a subject in need using scaffolds seeded with autologous cells derived from the subject. There remains a need for additional sources of cells, such as non-autologous sources. It has been reasoned that allogeneic stem cells could provide an alternative for bladder reconstruction and treatment for bladder cancer (see Yinan and Guomin (2008) Medical Hypothesis. 70,294-297).

### Summary of the Invention

The present invention relates to the regeneration, reconstruction, augmentation or replacement of luminal or tubular organs or tissue structures in a subject in need using scaffolds seeded with cells that are derived from sources that are different from the organ or tissue structure that is the subject of the regeneration, reconstruction, augmentation or replacement described herein, methods of isolating such cells, neo-organ/tissue structure scaffolds or matrices seeded with such cells (constructs). The luminal organs may be laminarly organized. The present invention relates to methods of making such neo-organ/tissue structure constructs, and methods of treating a patient in need using the constructs. The cells may be obtained from autologous sources or non-autologous sources. If non-autologous sources are used, then the methods of treatment may be performed without the need for immunosuppressive therapy.

In one aspect, the present invention concerns an implantable cell-scaffold construct for treating a gastrointestinal (GI) disorder in a subject in need as defined in the claims. In one embodiment, the construct is made up of a) a scaffold comprising a matrix having a first surface, wherein the matrix is shaped to conform to at least a part of a native gastrointestinal organ or tissue structure in the subject; and b) a first cell population that is not derived from a gastrointestinal source which is a smooth muscle cell (SMC) population derived from adipose tissue or peripheral blood deposited on or in the first surface, said scaffold and said first cell population forming an implantable construct as defined in the claims. In another embodiment, the construct may further comprise a second cell population derived from GI tissue, wherein said matrix, said first cell population, and said second cell population form an implantable construct. The cell populations may be obtained from autologous or non-autologous sources.

In all embodiments, the first cell population is a smooth muscle cell (SMC) population. In some embodiments, the first cell population is derived from a source that is non-autologous to the subject. The non-autologous source may be an Allogeneic source. In some embodiments, the first cell population is derived from a source that is autologous to the subject. In some embodiments, the SMC population is derived from adipose. In some embodiments, the SMC population is derived from peripheral blood.

### Brief Description of the Drawings

Figure 1A-D shows examples of bladder augmentation scaffolds.
Figure 2A-D shows examples of bladder replacement scaffolds.
Figure 3A depicts examples of a urinary diversion or conduit scaffold. Figure 3B-C shows an example of a urinary diversion construct having different types of cross-sectional areas, as well as potential positions for openings that may be configured to connect to ureter(s). Figure 3C illustrates variations of a urinary diversion construct (A: open claim ovoid; B: open claim ovoid receptacle; C: closed ovoid receptacle and three tubes).
Figure 4A-D illustrates different applications of a urinary diversion or conduit construct.
Figure 5A-B show examples of a muscle equivalent scaffold.
Figure 6 depicts images of various muscle equivalent scaffolds in the form of patches or strips.
Figure 7 depicts different muscle equivalent scaffolds and representative methods of implantation.

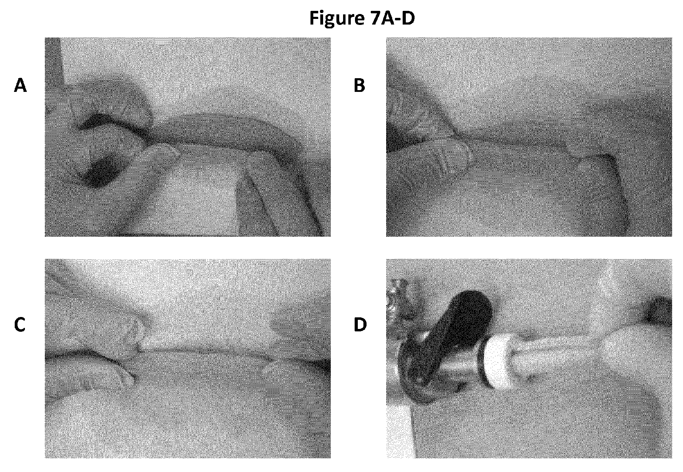

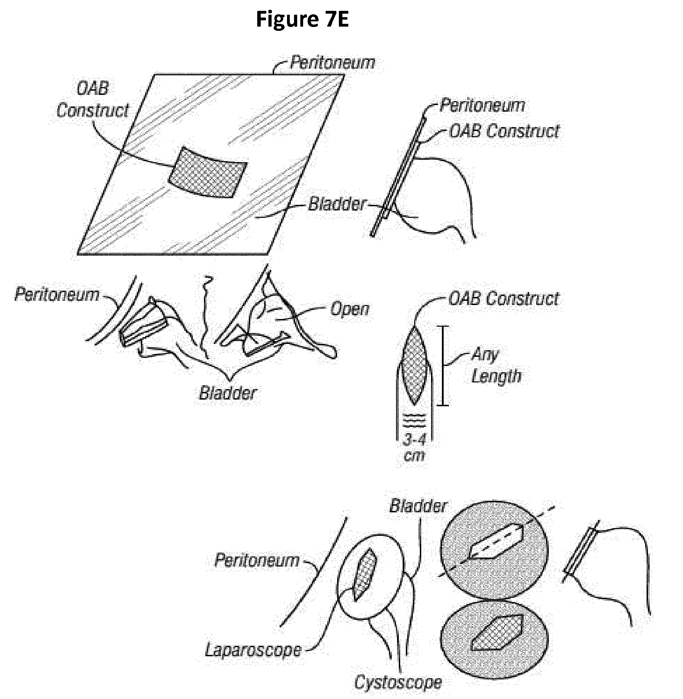

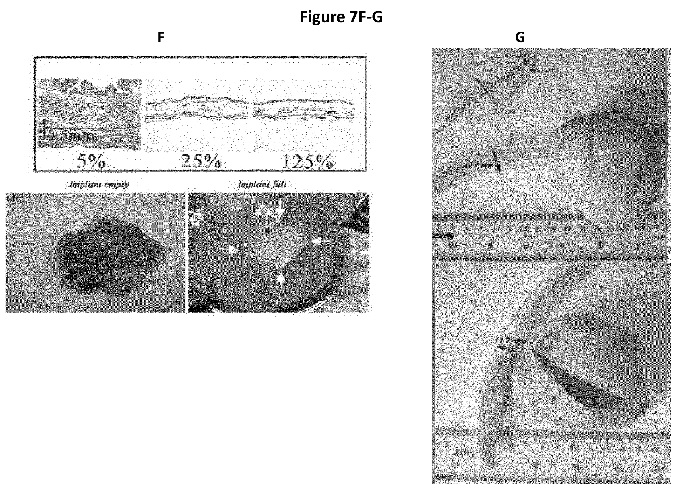

Figure 7A depicts formation of a flat sheet of scaffold. Figure 7B depicts a laparoscopically-suited scaffold which can be rolled at the time of implantation and fed through a laparoscopic tube and unrolled in the abdominal cavity. Figure 7C depicts formation of a laparoscopically-suited scaffold sheet in a rolled configuration to facilitate insertion through a laparoscopic tube, after which it is unrolled in the abdominal cavity. Figure 7D depicts formation of a laparoscopically-suited scaffold sheet in a folded configuration or accordion style to facilitate insertion through the tube, after which it is unfolded in the abdominal cavity. Figure 7E depicts possible surgical methods for the implantation of a muscle equivalent scaffold. Figure 7F depicts implantation sites on an empty and full bladder. Figure 7G depicts a urinary bladder model with surgical slit showing ellipsoid created upon sectioning of surface.
Figure 8 depicts a pre-folded accordion style scaffold sheet to facilitate insertion through a laparoscope port.
Figure 9A depicts scaffold pre-cut into strips, then sutured together to allow stacking and insertion into the laparoscope port and secured in place in the abdominal cavity. Figure 9B depicts one scaffold of 18.7 cm in length by 2.0 cm in width having 2 folds. Figure 9C depicts one scaffold of 13.3 cm in length by 2.8 cm in width having 3 folds. Figure 9D depicts one scaffold of 9.7 cm in length by 4.0 cm in width having 4 folds. Figure 9E depicts one scaffold comprised of two pieces, 2 folds each, of 9.7 cm in length and 2.0 cm in width.
Figure 10A-C depict GI tissue scaffolds.
Figure 11 A-B show examples of configurations for an implanted conduit construct.
Figure 12 depicts two alternative configurations (A and B) for an implanted Neo-Urinary Conduit scaffold.
Figure 13 shows an example of the implanted components of a permanent urinary diversion construct.
Figure 14 depicts other applications of the urinary diversion constructs.
Figure 15 shows the post-fixation tissue (longtudinally bisected) of a test animal following implantation with a urinary conduit construct.
Figure 16A-D shows expression of Clara cell secretory protein (A) and prosurfactant Protein C (B-D) from lung alveolar forming units.
Figure 17 depicts expression of Clara cell protein from lung alveolar forming units.
Figure 18 depicts expression of KRT18, SCGB1A1, and SFTPA1 from lung alveolar forming units.
Figure 19 shows confocal image of rat lung AFU stained with connexin 43.
Figure 20 depicts lung AFU on Gelfoam and PLGA scaffolds with and without pre- seeding with Ad-SMC (top left panel - Gelfoam pre-seeded with Ad-SMC, then seeded with isolated lung cells; top right panel - Gelfoam without pre-seeding with Ad-SMC, then seeded with isolated lung cells; bottom left panel - Gelfoam pre-seeded with Ad-SMC, then seeded with isolated lung cells; bottom right panel - Gelfoam without pre-seeding with Ad-SMC. then seeded with isolated lung cells; arrows depict apparent AFU formation on scaffolds pre-seeded with Ad-SMC).
Figure 21 shows Gelfoam (-) Ad-SMC stained with antibody to Clara cell protein in top left panel; top right panel shows Gelfoam (-) Ad-SMC phase image; bottom left panel shows Gelfoam (+) Ad-SMC stained with antibody to Clara cell protein; and bottom right panel shows Gelfoam (+) Ad-SMC phase image.
Figure 22 shows Gelfoam (-) Ad-SMC stained with antibody to Surfactant Protein C in top left panel; top right panel shows Gelfoam (-) Ad-SMC phase image; bottom left panel shows Gelfoam (+) Ad-SMC stained with antibody to Surfactant Protein C; and bottom right panel shows Gelfoam (+) Ad-SMC phase image (arrows in bottom panels depict apparent AFU formation).
Figure 23 depicts PLGA scaffold (+) Ad-SMC stained with antibody to Clara Cell Protein in top left panel; top right panel - PLGA scaffold (+) Ad-SMC phase image; and bottom left panel shows merging of immunofluorescent and phase images (arrows in top panel depicts apparent AFU formation).
Figure 24 shows Gelfoam scaffold (+) Ad-SMC stained with antibody to Surfactant Protein C; top right panel shows Gelfoam scaffold (+) Ad-SMC phase image; bottom left panel shows merging of immunofluorescent and phase images (arrows in panels depict hollow spaces in the Gelfoam).
Figure 25A-C shows attachment/proliferation of smooth muscle cells on various biomaterials.
Figure 26 shows the results of a live/dead assay for smooth muscle cells deposited on various biomaterials.
Figure 27A-B show scaffolds seeded with adipose-derived smooth muscle cells (Ad-SMCs) following incubation in culture medium.
Figure 27C-E show the degree of epithelial cell migration from esophageal tissue to a scaffold not pre-seeded with Ad-SMCs (C) and a scaffold pre-seeded with Ad-SMCs (D). Figure 27E shows a scaffold without esophageal tissue.
Figure 28 shows cultures of cells derived from esophagus.
Figure 29A shows gene expression for epithelial cell markers in esophageal tissue and cultured esophageal cells. Figure 29B shows cytokeratin 8,18,19 immunostaining of cultured esophageal cells.
Figure 30 shows an experimental design for assessing cell migration.
Figure 31 shows migration of esophageal cells.
Figure 32 shows migration of esophageal cells.
Figure 33A shows the surgically-created esophageal defect and subsequent construct implantation.
Figure 33B shows histology of an implanted construct at 1 day post implantation.
Figure 34 shows neo-vascularization (angiogenesis) at the site of implantation.
Figure 35 shows histology of an implanted construct at 8 days post implantation.
Figure 36 shows histology of an implanted construct at 8 days post implantation.
Figure 37A shows the incorporation of an esophagus construct at 10 weeks post implantation. Figure 37B shows Section 1 (transverse) in more detail. Figure 37C shows Section 2 (transverse) in more detail.
Figure 37D shows Section 3 (transverse) in more detail.
Figure 38 shows Ad-SMCs seeded onto woven meshes.
Figure 39A-C shows the implantation of a small intestine (SI) construct.
Figure 40A-C shows an SI patch construct at 8 weeks (A) and 16 weeks (B-C) after implantation.
Figure 41 shows an SI tubular construct at 10 weeks after implantation.
Figure 42 shows an SI tubular construct at 5 months after implantation.
Figure 43 shows live/dead staining of rat adipose-derived SMCs on scaffolds.
Figure 44 shows the cell morphology of peripheral blood cells.
Figure 45 shows RT-PCR analysis for endothelial markers on peripheral blood cells.
Figure 46 shows the cell morphology of adipose-derived cells.
Figure 47 shows RT-PCR analysis for endothelial markers on adipose derived cells.
Figure 48 shows endothelial cell gene expression analysis of adipose-derived cells cultured in DMEM containing 10% FBS.

### DETAILED DESCRIPTION OF THE INVENTION

The present invention concerns cell populations derived from sources that are different from the organ or tissue structure that is the subject of the reconstruction, augmentation or replacement described herein, methods of isolating such cells, neo-organ/tissue structure scaffolds or matrices seeded with such cells (constructs) and methods of making the same, and methods of treating a patient in need using such neo-organ/tissue structure constructs.

### 1. Definitions

Unless defined otherwise, technical and scientific terms used herein have the same meaning as commonly understood by one of ordinary skill in the art to which this invention belongs. Principles of Tissue Engineering, 3rd Ed. (Edited by R Lanza, R Langer, & J Vacanti), 2007 provides one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "smooth muscle cell" or "SMC" as used herein refers to a contractile cell that is derived from a source that is different from or the same as the native organs or tissues that are the subject of the reconstruction, augmentation or replacement using the constructs and methods as described herein. The smooth muscle cells provided by the present invention, once seeded and cultured on the scaffolds or matrices described herein, are capable of forming the non-striated muscle that is found in the walls of hollow organs (e.g. bladder, abdominal cavity, gastrointestinal tract, etc.) and characterized by the ability to contract and relax. Those of ordinary skill in the art will appreciate other attributes of smooth muscle cells.

The term "cell population" as used herein refers to a number of cells obtained by isolation directly from a suitable tissue source, usually from a mammal. The isolated cell population may be subsequently cultured *in vitro.* Those of ordinary skill in the art will appreciate various methods for isolating and culturing cell populations, as well as various numbers of cells in a cell population, that may be suitable for use in the present invention. The cell population may be derived from an autologous source or a non-autologous source. SMC populations may be derived from adipose, blood or bladder and may be characterized by the expression of markers associated with smooth muscle cells. The SMC population may also be a purified cell population.

The term "adipose-derived smooth muscle cell population" or "Ad-SMC population" as used herein refers to an adipose-derived smooth muscle cell population (SMC) that is substantially free of adipocytes or non-adherent adipose cells. The Ad-SMC population may be characterized by the expression of markers associated with smooth muscle cells. The Ad-SMC population may also be a purified cell population. The Ad-SMC population may be derived from an autologous source. The Ad-SMCs may be derived from a SVF containing vascular tissue. Thus, the Ad-SMCs may be derived from the capillaries, arterioles, and venules of the adipose-derived vascular bed, or the SMCs may be derived from the perivascular niche containing pericytes.

The term "gastro-intestinal cell population" or "GI cell population" refers to a cell population that is derived from gastro-intestinal tissue including, without limitation, esophagus, small intestine, large intestine, stomach, colon, or anal sphincter tissue. For example, the GI cell population may be a heterogeneous cell population derived from esophageal tissue.

The term "esophageal cell population" or "esophagus cell population" refers to a cell population that is derived from esophageal tissue. It may be a heterogeneous cell population that includes epithelial cells, smooth muscle cells, and any combination thereof. An esophageal cell population may be derived from an esophagus biopsy or from whole esophagus tissue. Alternatively, the esophageal cell population may be derived from *in vitro* cultures of a cell population established from an esophagus tissue biopsy or whole esophagus tissue. The esophageal cell population is characterized by the expression of markers associated with epithelial cells, smooth muscle cells, and any combination thereof. The esophageal cell population may also be a purified cell population.

The term "respiratory cell population" refers to a cell population that is a heterogeneous cell population derived from respiratory tissue (e.g. lung). The respiratory cell population may include bronchiolar cells, epithelial cells, alveolar cells, and any combination thereof. A respiratory cell population may be derived from a lung biopsy or from whole lung tissue. Alternatively, the respiratory cell population may be derived from in vitro cultures of a cell population established from a lung tissue biopsy or whole lung tissue. The respiratory cell population may be characterized by the expression of markers associated with bronchiolar cells, epithelial cells, alveolar cells, and any combination thereof. The respiratory cell population may also be a purified cell population.

The term "autologous" refers to derived or transferred from the same individual's body. Autologous cell populations described herein are derived from a subject in need of regeneration, reconstruction, augmentation or replacement of a native organ or tissue structure. An autologous cell population is derived from the subject who will be recipient of a construct, as described herein.

The term "non-autologous" refers to derived or transferred from a donor who will not be the recipient of an implantable construct as described herein. Such non-autologous sources include sources that are allogeneic, syngeneic (autogeneic or isogeneic), and any combination thereof. As used herein, a non-autologous cell population is a cell population derived from a source that is non-autologous to the subject, as described herein.

The term "marker" or "biomarker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a particular type of cell. In general, the term cell "marker" or "biomarker" refers to a molecule expressed in a cell population described herein that is typically expressed by a native cell. The marker may be a polypeptide expressed by the cell or an identifiable physical location on a chromosome, such as a gene, a restriction endonuclease recognition site or a nucleic acid encoding a polypeptide (e.g., an mRNA) expressed by the native cell. The marker may be an expressed region of a gene referred to as a "gene expression marker", or some segment of DNA with no known coding function.

The term "smooth muscle cell marker" refers to generally to a marker, the expression or presence of which in a cultured cell population can be detected by standard methods (or methods disclosed herein) and is consistent with one or more cells in the cultured cell population being a smooth muscle cell. In general, the term smooth muscle cell (SMC) "marker" or "biomarker" refers to a molecule that is typically expressed by a native smooth muscle cell. Such markers contemplated by the present invention include, but are not limited to, one or more of the following: myocardin, alpha-smooth muscle actin, calponin, myosin heavy chain, BAALC, desmin, myofibroblast antigen, SM22, and any combination thereof.

The term "respiratory cell marker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a respiratory cell. In general, the term respiratory cell "marker" or "biomarker" refers to a molecule that is typically expressed by a native respiratory cell. The marker may be a polypeptide expressed by the cell or an identifiable physical location on a chromosome, such as a gene, a restriction endonuclease recognition site or a nucleic acid encoding a polypeptide expressed by the SMC. The marker may be an expressed region of a gene referred to as a "gene expression marker", or some segment of DNA with no known coding function. Such markers contemplated by the present invention include, but are not limited to, one or more of the following: Clara Cell Secretory Protein (CCSP); Prosurfactant Protein C (PPC); KRT18; Secretoglobin, Family 1A, Member 1 (Uteroglobin or SCGB1A1); Surfactant Protein A1 (SFTPA1); and any combination thereof.

The term "gastro-intestinal cell marker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a gastro-intestinal cell. In general, the term gastro-intestinal cell "marker" or "biomarker" refers to a molecule that is typically expressed by a native gastro-intestinal cell. The marker may be a polypeptide expressed by the cell or an identifiable physical location on a chromosome, such as a gene, a restriction endonuclease recognition site or a nucleic acid encoding a polypeptide expressed by the gastro-intestinal cell. The marker may be an expressed region of a gene referred to as a "gene expression marker", or some segment of DNA with no known coding function. Those of ordinary skill in the art will appreciate suitable gastro-intestinal cell markers.

The term "esophageal cell marker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a esophageal cell. In general, the term esophageal cell "marker" or "biomarker" refers to a molecule that is typically expressed by a native esophageal cell. The marker may be a polypeptide expressed by the cell or an identifiable physical location on a chromosome, such as a gene, a restriction endonuclease recognition site or a nucleic acid encoding a polypeptide expressed by the esophageal cell. The marker may be an expressed region of a gene referred to as a "gene expression marker", or some segment of DNA with no known coding function. Such markers may be esophageal smooth muscle cell markers including, without limitation, one or more of the following: myocardin, alpha-smooth muscle actin, calponin, myosin heavy chain, BAALC, desmin, myofibroblast antigen, SM22, and any combination thereof. Such markers may be esophageal epithelial cell markers including, without limitation, one or more of the following: KRT8 (keratin 8), vWF (von Willebrand factor), cytokeratin 8, 18, 19, and any combination thereof.

The term "adipose derived smooth muscle cell marker" or "Ad-SMC marker" refers to a marker that is expressed at the gene and/or protein level in the cell population described herein. Based upon the observed protein expression, the cell population may have a particular cell surface maker profile where markers are designated positive (+) or negative (-) for protein expresion on the cell surface. For positive markers, protein expression may be observed at about 80%, about 90%, about 95%, about 96%, about 97%, about 98%, about 99%, or about 100%. For negative markers, protein expression may be observed at about 20%, about 15%, about 10%, about 5%, about 4%, about 3%, about 2%, about 1%, or about 0%.

The terms "differentially expressed gene," "differential gene expression" and their synonyms, which are used interchangeably, refer to a gene whose expression is activated to a higher or lower level in a first cell or cell population, relative to its expression in a second cell or cell population. The terms also include genes whose expression is activated to a higher or lower level at different stages over time during passage of the first or second cell in culture. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between the first cell and the second cell. Differential expression includes both quantitative, as well as qualitative, differences in the temporal or cellular expression pattern in a gene or its expression products among, for example, the first cell and the second cell. For the purpose of this invention, "differential gene expression" is considered to be present when there is an at least about one-fold, at least about 1.5-fold, at least about 2-fold, at least about 2.5-fold, at least about 3-fold, at least about 3.5 fold, at least about 4-fold, at least about 4.5-fold, at least about 5-fold, at least about 5.5-fold, at least about 6-fold, at least about 7-fold, at least about 8-fold, at least about 9-fold, at least about 10-fold, at least about 10.5-fold, at least about 11-fold, at least about 11.5-fold, at least about 12-fold, at least about 12.5-fold, at least about 13-fold, at least about 13.5-fold, at least about 14-fold, at least about 14.5-fold, or at least about 15-fold difference between the expression of a given gene in the first cell and the second cell, or at different stages over time during passage of the cells in culture. The differential expression of a marker may be in an adipose-derived cell (the first cell) relative to expression in a mesenchymal stem cell or MSC (the second cell).

The terms "inhibit", "down-regulate", "under-express" and "reduce" are used interchangeably and mean that the expression of a gene, or level of RNA molecules or equivalent RNA molecules encoding one or more proteins or protein subunits, or activity of one or more proteins or protein subunits, is reduced relative to one or more controls, such as, for example, one or more positive and/or negative controls. The under-expression may be in an adipose-derived cell relative to expression in an MSC.

The term "up-regulate" or "over-express" is used to mean that the expression of a gene, or level of RNA molecules or equivalent RNA molecules encoding one or more proteins or protein subunits, or activity of one or more proteins or protein subunits, is elevated relative to one or more controls, such as, for example, one or more positive and/or negative controls. The over-expression may be in an adipose-derived cell relative to expression in an MSC.

The term "contractile function" refers to smooth muscle contractile function involving the interaction of sliding actin and myosin filaments, which is initiated by calcium-activated phosphorylation of myosin thus making contraction dependent on intracellular calcium levels.

The term "coordinated rhythmic contractile function" or "CRCF" refers to a contractile function of a cell or cell population characterized by a pattern of periodic contractions and relaxations. This coordinated rhythmic contraction may be observed in a respiratory tissue construct described herein, e.g., following maintenance of the construct under culture conditions described herein.

The term "contact-dependent inhibition" refers to the halting of cell growth when two or more cells come into contact with each other. The absence of this property can be observed in cell culture where cells whose growth is not inhibited by contact can be observed piling on top of each other, similar to foci formation in transformed cell culture. Mesenchymal stem cells do not exhibit this property. In contrast, cells having the contact-dependent inhibition property will not be observed to pile on top of each other in culture.

The term "peripheral blood" shall generally mean blood circulating throughout the body.

The term "adipose tissue" or "fat" shall generally mean loose connective tissue made up primarily of adipocytes. Adipose tissue can be obtained from various places in the body including, without limitation, beneath the skin (subcutaneous fat) and around internal organs (visceral fat).

The term "luminal organ" or "tissue structure" shall generally relate to an organ or part thereof characterized by an outer, exterior side and an inner, luminal side. The organ or tissue structure may be laminarly organized. For example, the wall of a urinary bladder includes a number of laminarly organized layers. It has an inner lining made up of a mucous membrane of transitional epithelium, a second layer called the submucosa that supports the mucous membrane made up of connective tissue with elastic fibers, and a third layer called the muscularis made up of smooth muscle. In another example, native blood vessels have a multi-layered or laminated structure. An artery has three layers: an innermost layer called the intima that comprises vascular endothelial cells lining the luminal surface, a middle layer called the media that comprises multiple sheets of smooth muscle cells, and the outer layer called the adventia that contains loose connective tissue, smaller blood vessels, and nerves. The intima and media are separated by a basement membrane. Those of ordinary skill in the art will appreciate different luminal organs and tissue structures.

The term "construct" refers to at least one cell population deposited on or in a surface of a scaffold or matrix made up of one or more synthetic or naturally-occurring biocompatible materials. The cell population may be combined with a scaffold or matrix in vitro or in vivo.

The term "luminal organ construct" or luminal organ tissue structure construct" refers to at least one cell population deposited on or in a surface of a scaffold or matrix made up of one or more synthetic or naturally-occurring biocompatible materials. In one embodiment, the scaffold or matrix is shaped to conform to at least a part of a native luminal organ or tissue structure of a subject. The subject may be in need of reconstruction, regeneration, augmentation or replacement of a native luminal organ or tissue structure. The cell population may be a smooth muscle cell population (e.g., adipose-derived SMC population or peripheral blood derived SMC population). The cell population may be combined with a scaffold or matrix *in vitro* or *in vivo.*

The term "respiratory tissue construct" refers to a construct made up of a scaffold and one or more cell populations (e.g., an adipose-derived SMC population and/or a respiratory cell population). The construct may be cultured after the deposition of at least one cell population, and further cultured after deposition of a second cell population. The second cell population may contact the scaffold and/or the deposited first cell population.

The term "sample" or "patient sample" or "biological sample" shall generally mean any biological sample obtained from an individual, body fluid, body tissue, cell line, tissue culture, or other source. The term includes body fluids such as, for example, blood such as peripheral blood or venous blood, urine and other liquid samples of biological origin, such as lipoaspirates, and solid tissue biopsies such as a biopsy specimen (e.g., adipose tissue biopsy), or tissue cultures or cells derived therefrom, and the progeny thereof. The definition also includes samples that have been manipulated in any way after they are obtained from a source, such as by treatment with reagents, solubilization, or enrichment for certain components, such as proteins or polynucleotides. The definition also encompasses a clinical sample, and also includes cells in culture, cell supernatants, cell lysates, serum, plasma, biological fluid, and tissue samples. The source of a sample may be solid tissue, such as from fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in the development of the subject. The biological sample may contain compounds which are not naturally present with or in the tissue in nature such as preservatives, anticoagulants, buffers, fixatives, nutrients, antibiotics, or the like. The sample can be used for a diagnostic or monitoring assay. Methods for obtaining samples from mammals are well known in the art. If the term "sample" is used alone, it shall still mean that the "sample" is a "biological sample" or "patient sample", i.e., the terms are used interchangeably. A sample may also be a test sample.

The term "test sample" refers to a sample from a subject following implantation of a construct described herein. The test sample may originate from various sources in the mammalian subject including, without limitation, blood, serum, urine, semen, bone marrow, mucosa, tissue, etc.

The term "control" or "control sample" refers a negative control in which a negative result is expected to help correlate a positive result in the test sample. Alternatively, the control may be a positive control in which a positive result is expected to help correlate a negative result in the test sample. Controls that are suitable for the present invention include, without limitation, a sample known to have normal levels of a cytokine, a sample obtained from a mammalian subject known not to have been implanted with a construct described herein, and a sample obtained from a mammalian subject known to be normal. A control may also be a sample obtained from a subject prior to implantation of a construct described herein. In addition, the control may be a sample containing normal cells that have the same origin as cells contained in the test sample. Those of skill in the art will appreciate other controls suitable for use in the present invention.

The term "subject" shall mean any single human subject, including a patient, eligible for treatment, who is experiencing or has experienced one or more signs, symptoms, or other indicators of deficient organ function or failure, including a deficient, damaged or non-functional organ. Such subjects include, without limitation, subjects who are newly diagnosed or previously diagnosed and now experiencing a recurrence or relapse, or are at risk for deficient organ function or failure, no matter the cause. The subject may have been previously treated for a condition associated with deficient organ function or failure, or not so treated.

The term "patient" refers to any single animal, more preferably a mammal (including such nonhuman animals as, for example, dogs, cats, horses, rabbits, zoo animals, cows, pigs, sheep, and nonhuman primates) for which treatment is desired. Most preferably, the patient herein is a human.

The term "urinary diversion" or "conduit" refers to the resulting organ or tissue structure resulting from the subject's interaction over time with an implanted urinary diversion construct, anastomosed ureters, and optionally an adjacent atrium. The atrium is the anterior connecting chamber that allows for urine passage through the abdominal wall and may be made by the most anterior tube-like portion of a peritoneal wrap connecting the caudal end of the construct (located in the intra-abdominal cavity) to the skin.

The terms "caudal" and "cranial" are descriptive terms relating to the urinary production and flow. The term "caudal" refers to the end of the urinary diversion construct that upon implantation is closest to the stoma, while the term "cranial" refers to the end of the urinary diversion construct that upon implantation is closest to the kidneys and ureters. The caudal end may also be referred to as the "stomal" or "outflow" end of an implanted construct.

The term "detritis" refers to debris formed during the healing and regenerative process that occurs following implantation of a urinary diversion construct. Detritis can be made up of exfoliated tissue cells, inflammatory exudate and scaffold biodegradation. If the conduit is obstructed (improper outflow) by such debris, then the stagnated debris forms a detritis or semisolid bolus within the lumen of the conduit.

The term "debridement" refers to surgical or non-surgical removal of foreign matter, or lacerated, devitalized, contaminated or dead tissue from a conduit in order to prevent infection, prevent obstruction, and to promote the healing process. The debridement may involve the removal of detritis.

The term "stoma" refers to a surgically created opening used to pass urine from the draining outflow end of a urinary diversion construct to outside the body. The urine is typically collected in a reservoir outside the body.

The term "stoma port" or "stoma button" refers to means, such as a device used to maintain the integrity of the stoma opening. In one embodiment, the stoma port facilitates the passage of urine from the draining outflow end of a urinary diversion construct to outside the subject's body. In another embodiment, the lumen of the stoma port may be used to attach suture strands that are connected to stents (stent lanyards) placed in one or both ureters so as to avoid stent migration and to allow for the stents to be removed later.

The term "expanding" or "enlarging" as used herein refers to increasing the size of the existing laminarily organized luminal organ or tissue structure. For example, in one aspect of the invention, the existing laminarily organized luminal organ or tissue structure may be enlarged by 10, 11, 12, 13, 14, 15, 16, 17, 18, 19, 20, 21, 22, 23, 24, 25, 26, 27, 28, or 29 percent. In another aspect of the invention, the existing laminarily organized luminal organ or tissue structure may be enlarged such as to increase the existing volumetric capacity of the existing laminarily organized luminal organ or tissue structure.

The term "volumetric capacity" as used herein refers to the amount of liquid capable of being contained in a defined area.

"Regeneration prognosis" or "regenerative prognosis" generally refers to a forecast or prediction of the probable course or outcome of the implantation of a construct described herein. As used herein, regeneration prognosis includes the forecast or prediction of the development or improvement of a functional organ or tissue structure following implantation of a construct described herein. As used herein, "prognostic for regeneration" means providing a forecast or prediction of the probable course or outcome of the implantation of a new organ or tissue structure.

"Regenerated tissue" refers to the tissue of a new organ or tissue structure that develops after implantation of a construct as described herein.

### 2. Cell populations

The present disclosure provides populations of smooth muscle cells for use in the reconstruction, regeneration, augmentation or replacement of one or more of the following: laminarly organized luminal organs or tissue structures where the smooth muscle cells are not derived from the organ or tissue structure that is the subject of the reconstruction, regeneration, augmentation or replacement; respiratory tissue where the smooth muscle cells are not derived from respiratory tissue; gastrointestinal tissue where the smooth muscle cells are not derived from GI tissue; blood vessels where the smooth muscle cells are not derived from native blood vessels. The SMC population is characterized by contractile function and is positive for one or more smooth muscle cell markers.

As discussed herein, tissue engineering principles have been successfully applied to provide implantable cell-seeded matrices for use in the reconstruction, augmentation or replacement of laminarily organized luminal organs and tissue structures, such as a bladder or a bladder component, typically composed of urothelial and smooth muscle layers. (Becker et al. Eur. Urol. 51, 1217-1228 (2007); Frimberger et al. Regen. Med. 1, 425-435 (2006); Roth et al. Curr. Urol. Rep. 10, 119-125 (2009); Wood et al. Curr. Opin. Urol. 18, 564-569). Smooth muscle cells may be derived from the patient's own tissue or the tissue of a suitable donor. However, there are challenges associated with dependence upon the development and maintenance of cell culture systems from the primary organ site as the basic unit for developing new and healthy engineered tissues, as for example during treatment of cancerous bladder tissue. Clearly, cancerous cells from the patient are inappropriate for populating an implantable neo-bladder scaffold or matrix. In addition, the supply of cells from a donor and the ease of taking biopsies may be limiting factors.

The present invention concerns cell populations that are derived from sources that are different from the organ or tissue structure that is the subject of the reconstruction, augmentation or replacement. In one embodiment, the source is a non-autologous source. The non-autologous source may be allogeneic, syngeneic (autogeneic or isogeneic), or any combination thereof. In another embodiment, the source is an Autologous source.

In another aspect, the cell population expresses markers consistent with or typical of a smooth muscle cell population.

In one aspect, the source is peripheral blood. In one embodiment, the peripheral blood-derived smooth muscle cell population is derived from a suitable donor. The donor sample may be venous blood.

In one aspect, the source is adipose tissue. In one embodiment, the adipose tissue-derived smooth muscle cell population is derived from a suitable donor sample. The donor sample may be adipose tissue removed during an abdominalplasty procedure, or lipoaspirates.

The isolated cell populations of the present disclosure, upon culturing, can develop various smooth muscle cell characteristics including, but not limited to, hill-and valley morphology, expression of one or more smooth muscle cell markers, contractile function, filament formation, and cytokine synthesis.

In one aspect, the cultured cell population is characterized by its hill-and-valley morphology. The cells having a hill-and-valley morphology may have various characteristics including, without limitation, spindly shaped, flattened and fibroblast-like upon passage, elongated and arranged in parallel rows, a "whirled" appearance of growth, and any combination thereof. The cell population upon culturing in the appropriate media may develops a "hill-and-valley morphology," that is typical of cultured smooth muscle cells.

In another aspect, the cultured cell population is characterized by the presence of one or more smooth muscle cell markers. The cell population upon culturing in the appropriate media may develops detectable smooth muscle cell markers including, without limitation, one or more of the following myocardin, alpha-smooth muscle actin, calponin, myosin heavy chain, BAALC, desmin, myofibroblast antigen, SM22, and any combination thereof.

In another aspect, the cultured cell population is characterized by the presence of one or more cells that express one or more cell surface markers. The cell population upon culturing in the appropriate media may contains one or more cells that are positive for cell surface markers including, without limitation, one or more of the following CD73, CD90, CD105, CD166, CD31, CD54, CD56, CD117, and any combination thereof. A cell population that is positive for a cell surface marker may be positive at the level of gene expression and/or at the level of protein expression (see Example 3). For example, the cell population may demonstrate CD73 expression at the gene level but not at the protein level, while CD45 expression may be demonstrated at the gene and protein level. In another example, the cell population upon culturing in the appropriate media contains one or more cells that are CD45+, CD31+, CD54+, CD56+, CD90+, and CD105+. In another example, the cell population has a cell surface marker profile that is CD31+, CD73-, CD90+, CD105+, CD117+, CD133-. The cell surface marker profile may further include one or more of CD45+, CD166+, CD54+, and/or CD56+.

In one other aspect, the cultured cell population is characterized by the presence of one or more cells having contractile function. The cell population upon culturing in the appropriate media may develops contractile function. The contractile function may be calcium dependent. The calcium-dependent contractile function may be demonstrated by inhibition of contraction with a calcium chelator, where, the calcium chelator may be EDTA. Those of ordinary skill in the art will appreciate that other chelators known in the art may be suitable.

In yet another aspect, the cultured cell population is characterized by filament formation. The cell population upon culturing in the appropriate media may undergo filament formation.

In one aspect, the cell population includes at least one cell expressing one or more cytokines. The cytokine may be selected from the group consisting of MCP-1, oncostatin M, IL-8, and GRO.

In one aspect, the cell populations of the present disclosure have a finite proliferative lifespan in culture following isolation. For example, the cell population has a lifespan of about 1 passage, about 2 passages, about 3 passages, about 4 passages, about 5 passages, about 6 passages, about 7 passages, about 8 passages, about 9 passages, about 10 passages, about 11 passages, about 12 passages, about 13 passages, about 14 passages, about 15 passages, about 16 passages, about 17 passages, or about 18 passages. In a preferred example, the cell population has a lifespan in culture of no more than 5 passages. The adipose-derived SMCs can generally be cultured 3-5 days between passages and the blood-derived SMCs can generally be cultured 14 days before the first passage and then 3-5 days for additional passages (see Examples 1 for more details).

In one aspect, the present disclosure provides a regenerative cell population containing at least one regenerative cell that when deposited on a scaffold or matrix as described herein and implanted into a subject in need, provides a regenerative effect for the organ or tissue structure that is the subject of the reconstruction, augmentation, or replacement contemplated herein. A regenerative cell population has the ability to stimulate or initiate regeneration of laminarily organized luminal, organs or tissue structures upon implantation into a patient in need. In general, the regeneration of an organ or tissue structure is characterized by the restoration of cellular components, tissue organization and architecture, function, and regulative development In addition, a regenerative cell population minimizes the incompleteness or disorder that tends to occur at the implantation site of a cell-seeded luminal organ or tissue structure construct. Disorganization at the site of implantation can manifest itself as increased collagen deposition and/or scar tissue formation, each of which can be minimized through the use of a regenerative cell population. In addition, certain cellular events are indicative of the regenerative process. In the case of a regenerated bladder or portion of a bladder using the cell populations and scaffolds described herein, a regenerating organ or tissue structure is composed of a smooth muscle parenchyma with fibrovascular tissue radiating around numerous microvessels that extend toward the luminal, surface, as well as stromal elements having well developed blood vessels aligned to the mucosal surface (see Jayo et al. (2008) Regen Med 3, 671-682). A regenerating bladder or portion of a bladder is also characterized by the presence of spindloid/mesenchymal cells and aSMA positive muscle precursor cells. In one example, the aSMA positive spindloid cells are observed in neostromal tissues and around multiple neo-vessels (atterioles),

The regenerative cell population has the ability to stimulate or initiate regeneration of different organs or tissue structures including, without limitation, a gastrointestinal organ or tissue structure, *e.g*., esophagus, small intestine, large intestine, stomach, colon, or anal sphincter; a respiratory organ or tissue structure, *e.g*., lung, lung tissue including alveolar and bronchiolar tissue; and a blood vessel. In one embodiment, the regenerative cell is an adipose-derived smooth muscle cell, which facilitates restoration of the cellular components, tissue organization and architecture, and/or function of an organ or tissue structure. In another embodiment, the regenerative cell is not a stem cell.

In another aspect, the regenerative cell population provides a regenerative effect characterized by the adaptive regulation of the size of a restored laminarily organized luminal organ or tissue structure. In one example, the regenerative cell population's regenerative effect is the establishment of adaptive regulation that is specific to the subject that receives the scaffold or matrix seeded with the regenerative cell population. In one example, the adaptive regulation is the replacement or augmentation of a bladder in a subject using a construct described herein such that the neo-bladder grows and develops to a size that is proportional to the subject's body size.

The cell population capable of regenerative stimulation may be an MCP-1 producing cell population, which contains at least one cell that expresses the chemokine product MCP-1. The cytokines MCP-1 is a normal product of bladder detrusor cells. In aortic smooth muscle cells, it plays a role in regeneration and is well known for its ability to recruit mononuclear cells. It is however more than a chemokine; it is also a potent mitogen for vascular smooth muscle cell proliferation and recruits circulating monocytes to the area of vessel injury. Monocytes are typically transformed to macrophages which can serve as reservoirs for cytokines and growth factors. Macrophages and muscle precursor cells are both targets for MCP-1 signaling. This cytokines has been implicated in stem and progenitor cell recruiting within the body, potentially contributing to the regenerative process. In one example, the cell population capable of regenerative stimulation is an MCP-1 producing cell populations, which contains at least one cell that expresses the chemokine product MCP-1. MCP-1 regenerative stimulation is characterized by the recruitment of certain cell types to the site of implantation. In one example, MCP-1 recruits muscle progenitor cells to the site of implantation to proliferate within the neo-bladder. In another example, MCP-1 recruits monocytes to the site of implantation which in turn produce various cytokines and/or chemokines to facilitate the regenerative process. In one other embodiment, MCP-1 induces omental cells to develop into muscle cells.

In one aspect, the present disclosure provides the use of specific cytokine, such as MCP-1, as a surrogate marker for tissue regeneration. Such a marker could be used in conjunction with an assessment of regeneration based on whether function has been reconstituted. Monitoring a surrogate marker over the time course of regeneration may also serve as a prognostic indicator of regeneration.

In another embodiment, the cell population is a purified cell population. A purified cell population as described herein is characterized by a phenotype based on one or more of morphology, the expression of markers, and function. The phenotype includes without limitation, one or more of hill-and valley morphology, expression of one or more smooth muscle cell markers, expression of cytokines, a finite proliferative lifespan in culture, contractile function, and ability to induce filament formation. The phenotype may include other features described herein or known to those of ordinary skill in the art. In another embodiment, the purified populations are substantially homogeneous for a smooth muscle cell population as described herein. A purified population that is substantially homogeneous is typically at least about 90% homogeneous, as judged by one or more of morphology, the expression of markers, and function. In other embodiments, the purified populations are at least about 95% homogeneous, at least about 98% homogeneous, or at least about 99.5% homogeneous.

In another embodiment, the smooth muscle cell population is derived directly from human adipose tissue and is characterized by differential expression of one or more of the following osteopontin, Oct4B, growth differentiation factor 5 (GDF5), hepatocyte growth factor (HGF), leukemia inhibitory factor (LIF), Melanoma cell adhesion molecule (MCAM), vascular cell adhesion molecule 1 (VCAM1), PECAM, vWF, Flk-1, runt-related transcription factor 2 (RUNX2), bone morphogenetic protein 6 (BMP6), CD44, and IL-1B, relative to its level of expression in human bone marrow-derived Mesenchymal stem cells (MSCs). In one other embodiment, the SMC population (a) under-expresses one or more of GDF5, HGF, LIF, MCAM, RUNX2, VCAM1, PECAM, vWF, and Flk-1 and/or (b) overexpression one or more of Oct4B, osteopontin, BMP6, CD44, and IL-1B, relative to the expression level thereof in human bone marrow-derived MSCs. In one other embodiment, the SMC population (a) underexpresses all of GDF5, HGF, LIF, MCAM, RUNX2, VCAM1, PECAM, vWF, and Flk-1 and/or (b) overexpresses all of Oct4B, osteopontin, BMP6, CD44, and IL-1B, relative to the expression level thereof in human bone marrow-derived MSCs.

In another embodiment, the smooth muscle cell population derived directly from adipose tissue that comprises one or more cells that are CD45+ and/or one or more cells that are CD117+.

In other embodiments, the present invention provides a smooth muscle cell population derived directly from human adipose tissue having a shorter proliferative lifespan than human bone marrow-derived MSCs. In another embodiment, the SMC population exhibits contact-dependant inhibition of proliferation in culture. In one other embodiment, the SMC population derived directly from adipose tissue characterized by down-regulation of at least one smooth muscle cell (SMC) marker in response to a thromboxane A2 mimetic. In other embodiments, the SMC marker is selected from the group consisting of myocardin and myosin heavy chain - smooth muscle isoform (SMMHC). In another embodiment, the myocardin and SMMHC are down-regulated in response to a thromboxane A2 mimetic.

In all embodiments, the SMC population is derived from an autologous source or a non-autologous source.

The cell populations of the present disclosure may be administered to a subject having a disorder without the use of a scaffold, such as by engraftment. Those of ordinary skill in the art will appreciate suitable methods of engraftment.

The present disclosure provides smooth muscle cell populations isolated from sources that are different from the luminal organ or tissue structure that is the subject of the regeneration, reconstruction, augmentation or replacement. The luminal organ or tissue structure may be a bladder or portion of a bladder; a respiratory organ or tissue structure, a gastrointestinal organ or tissue structure, a vascular organ or tissue structure, e,g., a blood vessel, or an ocular tissue structure. Accordingly, the smooth muscle cell populations may be derived from non-bladder, non-respiratory, non-gastrointestinal, non-vascular, or non-ocular sources.

The present disclosure also provides GI tissue cell populations, such as esophageal cell populations derived from the esophagus. The esophageal source may be an autologous source. In one embodiment, the cell population is a heterogenous cell population. In another embodiment, the heterogenous cell population includes epithelial cells and/or smooth muscle cells. In another embodiment, the esophageal cell population is characterized by the presence of one or more biamarkers. In another embodiment, the cell population has detectable epithelial, cell markers including, without limitation, one or more of the following: KRT8 (keratin 8), vWF (von Willebrand factor), cytokeratin 8, 18, 19, and any combination thereof. In one embodiment, the cell population has detectable smooth muscle cell markers including, without limitation, one or more of the following: myocardin, alpha-smooth muscle actin, calponin, myosin heavy chain, BAALC, desmin, myofibroblast antigen, SM22, and any combination thereof.

The present disclosure also provides respiratory tissue cell populations, such as cell populations derived from the lung (e.g., whole lung or a lung biopsy). The source may be an autologous or non-autologous source. In one example, the cell population is a heterogenous cell population. In another example, the heterogenous cell population may include bronchiolar cells, epithelial cells, alveolar cells, Clara cells, or any combination thereof. The respiratory cell population may be characterized by the expression of markers associated with bronchiolar cells, epithelial cells, alveolar cells, and any combination thereof, The markers include one or more of the following: Clara cells secretory protein (CCSP), Prosurfactant Protein C (proSPC), Surfactant Protein C (SPC), KRT18, Secretoglobulin, Family 1A, Member 1 (Uteroglobulin) (SCGB1A1), and Surfactant Protein A1 (SFTPA1). (see Examples 6-9) CCSP is a marker for bronchiolar epithelial cells and proSPC is a marker for alveolar epithelial cells. KRT18 is a lung specific cytokeratin and epithelial marker. SCGB1A1 (Secretoglabin, Family 1A, Member 1 (Uteroglobin)) is a Clara Cell marker. SFTPA1 is an alveolar epithelial marker. The respiratory cell population may also be a purified cell population.

The present disclosure also provides endothelial, cell (EC) populations. Such EC populations may be used in the reconstruction, augmentation or replacement of blood vessels. Currently, cell populations are obtained directly from veins or arteries to construct blood vessel grafts (Yang et al. Annals of Plastic Surgery: March 2009 - Volume 62 - Issue 3 - pp 297-303). Endothelial cells may be obtained from the aorta (Cascade Biologicis, C-006). However, the availability of suitable blood vessel segments for isolating cell populations, the difficulty in obtaining cells from a blood vessel, and the number of cells obtained per biopsy are limiting factors. It would be advantageous to obtain cell populations from other areas of the body where cells are more plentiful and easier to obtain in greater numbers. The present invention concerns cell populations that are derived from non-vascular sources. The source may be autologous or non-autologous. The cell population may be a smooth muscle cell (SMC) population or an endothelial, cell (EC) population. The nan-vascular source may be adipose tissue or peripheral blood. The SMC population may be derived from a patient sample. The patient sample may be adipose tissue or venous blood. In one embodiment, the non-vascular source may be adipose tissue removed during an abdominalplasty procedure, or lipoaspirates. The present disclosure contemplates the use of endothelial cell (EC) populations derived from non-vascular sources, which are characterized by expression of genes consistent with or typical of an EC population. The EC population may be characterized by differential expression of one or more of the following CDH5/VECAD, vWF, PECAM1, FLT1/VEGFR, KDR/FLK1, TEK, and any combination thereof. The differentially expressed genes may be EC markers. In another embodiment, the cell population upon culturing in the appropriate media develops detectable EC markers including, without limitation, one or more of the following CDH5/VECAD, vWF, PECAM1, FLT1/VEGFR, KDR/FLK1, TEK, and any combination thereof. In one aspect, the cultured EC population is characterized by endothelial morphology. The cell exhibit a shortened, rounded or cuboidal shape. Example 17 described EC populations in more detail.

In one other aspect, the present disclosure concerns smooth muscle cell populations derived from bladder tissue for use in the reconstruction, regeneration, augmentation or replacement of laminarly organized luminal organs or tissue structures where the smooth muscle cells are derived from a non-autologous source. The non-autologous source may be allogeneic or syngeneic.

### 3. Methods of isolating cell populations

Autologous cell populations are derived directly from the subjects in need of treatment. Non-autologous cell populations may be derived from suitable donors. The source tissue is generally not the same as the organ or tissues structure that is in need of the treatment. A population of cells may be derived from the patient's own tissue or donor tissue, such as, for example, from adipose or peripheral blood. The cells may be isolated in biopsies. In addition, the cells may be frozen or expanded before use.

To prepare for construction of a cell-seeded scaffold, sample(s) obtained from a suitable donor containing smooth muscle cells are dissociated into appropriate cell suspension(s). Methods for the isolation and culture of cells were discussed in issued U.S. Pat. No. 5,567,612. Dissociation of the cells to the single cell stage is not essential for the initial primary culture because single cell suspension may be reached after a period, such as, a week, of in vitro culture. Tissue dissociation may be performed by mechanical and enzymatic disruption of the extracellular matrix and the intercellular junctions that hold the cells together. Non-autologous cells can be cultured in vitro, if desired, to increase the number of cells available for seeding on scaffold.

Cells may be transfected prior to seeding with genetic material. Smooth muscle cells could be transfected with specific genes prior to polymer seeding. The cell-polymer construct could carry genetic information required for the long term survival of the host or the tissue engineered neo-organ.

Cell cultures may be prepared with or without a cell fractionation step. Cell fractionation may be performed using techniques, which is known to those of skill in the art. Cell fractionation may be performed based on cell size, DNA content, cell surface antigens, and viability. For example, smooth muscle cells may be enriched from adipose tissue, while endothelial cells and adipocytes may be reduced for smooth muscle cell collection. While cell fractionation may be used, it is not necessary for the practice of the invention.

Another optional procedure in the methods described herein is cryopreservation. Cryogenic preservation may be useful, for example, to reduce the need for multiple invasive surgical procedures. Cells taken from a biopsy or sample from the subject may be amplified and a portion of the amplified cells may be used and another portion may be cryogenically preserved. The ability to amplify and preserve cells may minimize the number of surgical procedures required. Another example of the utility of cryogenic preservation is in tissue banks. Non-autologous cells may be stored, for example, in a donor tissue bank. As cells are needed for new organs or tissue structures, the cryopreserved supply of cells may be used as needed. Suitable donors may be initially identified and one or more biopsies may be cryogenically preserved. Later, if a recipient's organ or tissue structure fails following some manner of treatment, the cryogenically preserved non-autologous cells may be thawed and used for treatment. For example, if a cancer reappeared in a new organ or tissue structure in a subject after treatment, cryogenically preserved cells may be used for reconstruction of the organ or tissue structure without the need for additional biopsies from a donor.

Smooth muscle cells may be isolated from adipose or peripheral blood based on the following general protocols. An adipose biopsy specimen of suitable weight (e.g., in grams) and/or area (e.g., cm²) can be obtained. An appropriate volume of peripheral blood (e.g., ml) can be obtained prior to the planned implantation of a new organ or tissue structure construct.

The following is a representative example of a protocol suitable for the isolation of smooth muscle cells from the stromal vascular fraction (SVF) of adipose, which represents a heterogenous cell population composed of multiple cell types, including endothelial and smooth muscle cells as well as cells that are MSC-like as defined by the International Society for Cellular Therapy (ISCT) criteria (Domini et al. 2006 Cytotherapy 8:4, 315-317). A suitable gram weight of adipose tissue (e.g., 7-25g) can be obtained by biopsy and washed with PBS (e.g., 3 times), minced with a scalpel and scissors, transferred into a 50mL conical tube and incubated at 37°C for 60 minutes in a solution of collagenase (e.g., 0.1 to 0.3%) (Worthington) and 1% BSA in DMEM-HG. The tubes may be either continually rocked or periodically shaken to facilitate digestion. The SVF can be pelleted by centrifugation at 600 g for 10 minutes and resuspended in DMEM-HG + 10% FBS. The stromal-vascular fraction may then be used to seed passage zero.

The following is a representative example of a protocol suitable for the isolation of smooth muscle cells from peripheral blood. A suitable volume of peripheral blood (e.g. 25 ml) may be diluted 1:1 in PBS and layered with 25ml Histopaque -1077 (Sigma) in a 50mL conical tube. Following centrifugation (e.g., 800g, 30 min), the mononuclear fraction can be collected, washed once with PBS and resuspended in α-MEM/10% FBS (Invitrogen) to seed passage zero.

In another aspect, the present disclosure concerns methods for isolating smooth muscle cell populations from bladder tissue for use in the reconstruction, regeneration, augmentation or replacement of laminarly organized luminal organs or tissue structures where the smooth muscle cells (SMCs) are derived from a non-autologous source. The non-autologous source may be allogeneic or syngeneic. Those of ordinary skill in the art will appreciate protocols for isolating SMCs from bladder tissue. For example, exemplary protocols can be found in Bertram et al. U.S. Patent No. 7,918,897; Atala U.S. Patent No. 6,576,019; Kim BS and Atala A: Mesenchymal cell culture: smooth muscle. In: Methods of Tissue Engineering. Edited by A Atala and RP Lanza. San Diego: Academic Press 2002; pp 287-292; and Lai JY and Atala A: Epithelial cell culture: urothelium. In: Methods of Tissue Engineering. Edited by A Atala and RP Lanza. San Diego: Academic Press 2002; pp 243-246, each of which is incorporated herein by reference in its entirety.

Those of ordinary skill in the art will appreciate additional methods for the isolation of smooth muscle cells.

In another aspect, the present disclosure provides methods of isolating "esophageal cell populations. The following is a representative example of a protocol suitable for the isolation of esophageal cells from esophagus tissue. Esophageal tissue is obtained and placed in DMEM+5ug/mL Gentamycin (Wash Solution) and swirled frequently for 5 min. Afterwards, the tissue is placed into fresh Wash Solution. This process is repeated from 1 to 5 times before mincing the tissue to a uniform size. The minced tissue is then placed into a 50mL centrifuge tube containing Digest Solution (30OU/mL Collagenase TypeIV-Worthington/Dispase-Stem Cell in DMEM, 20mL/1g tissue). Digestion proceeds for 30 min at 37°C. Enzyme neutralization is achieved using 20%FBS. in KSFM media. The digested tissue is then mixed and filtered through a 100uM Steriflip filter to ensure that no large tissue fragments were carried over. This material is then centrifuged at 300g for 5 min to pellet the cells. The cell pellet is then washed with KSFM. The cells are then counted and plated in Growth Medium (KSFM+2%FBS or KGM (50:50 of KSFM with Supplements + DMEM 10%FBS containing 1X Anti/Anti, 1X ITS) (see Example 12).

In another aspect, the present disclosure provides methods of isolating repiratory cell populations. Example 6 provides representative example of a protocol suitable for the isolation of respiratory cells from lung tissue. Those of ordinary skill in the art will appreciate additional methods for the isolation of smooth muscle, esophageal, respiratory, and gastrointestinal cell populations.

In one aspect, the present disclosure provides methods for isolating an isolated smooth muscle cell population from SVF without the need for conditions that induce differentiation to smooth muscle cells. In one example, the method comprises a) obtaining adipose tissue, b) digesting the adipose tissue, c) centrifuging the digested adipose tissue to provide a stromal vascular fraction (SVF), d) culturing the SVF without the need for conditions that induce differentiation to smooth muscle cells, and e) isolating a smooth muscle cell population from the adipose tissue-derived SVF. In one example, the culturing step comprises washing the SVF, re-suspending the SVF in a cell culture media, and plating the resuspended SVF. In another example, the culturing step comprises providing a cell population that is adherent to the cell culture support, such as a plate or container. In another example, the method further comprises expanding the cultured cell population. In other examples, the method further comprises analysing the smooth muscle cell population for smooth muscle cell characteristics. In one embodiment, the adipose tissue is derived from a non-autologous source.

In one example, the culturing conditions do not require the use of cell culture components for inducing differentiation of the adipose tissue SVF-derived cell population to smooth muscle cells. Jack et al., J Biomaterials 30 (2009) 3529-3270 report that undifferentiated adipose stem cells derived from SVF were incubated in inductive media containing heparin for 6 weeks in order to differentiate the stem cells into smooth muscle cells (see also Rodriguez U.S. Patent No. 7,531,355). The stem cells reported by Jack et al. did not require splitting during this incubation period. In another embodiment, the culturing conditions do not require the use of inductive media, including inductive media containing heparin. In one other example, the methods of the present disclosure comprise the use of culturing conditions that do not require the use of exogenous growth factors for differentiating a cell population into smooth muscle cells or for culturing and expanding a cell population.

The advantages of the methods of the present disclosure over other reported methods include the elimination of the step of differentiating adipose derived stem cells into smooth muscle cells, which reduces the time between obtaining an adipose biopsy and isolating a smooth muscle cell population therefrom, In addition, the elimination of the need for other cell culture media components for inducing differentiation, such as exogenous growth factors, is advantageous in terms of cost.

In one other aspect, the present disclosure provides methods of isolating and culturing populations of smooth muscle cells that contain at least one cell that has contractile function and is positive for one or more smooth muscle cell markers. In one example, the method includes the step of obtaining a sample from a suitable donor, where the sample is not obtained from the luminal organ or tissue structure that is the target of the reconstruction, augmentation or replacement in the subject in need thereof. In another example, the method includes the step of deriving smooth muscle cells from the donor sample. In one other example, the luminal organ or tissue structure is a bladder or portion of a bladder. In one example, the sample is a non-autologous sample. In another embodiment, the sample is a peripheral blood sample. In yet another example, the sample is an adipose tissue sample. The adipose tissue may be tissue removed from a subject as a result of an abdominalplasty procedure.

In another example, the obtaining step is followed by a separation step. In the case of a peripheral blood sample, the separation step includes contacting the sample with a density gradient material, centrifuging the sample to define a density gradient that has a mononuclear fraction, and extracting the mononuclear fraction from the density gradient. The separation step may be followed by a culturing step in which cells from the extracted fraction are cultured.

In the case of an adipose tissue sample, the purification step includes digestion of the sample with Collagenase, centrifuging the digested sample, mixing of the centrifuged sample to separate stromal cells from primary adipocytes, centrifuging the mixed sample to obtain a stromal-vascular fraction that can be re-suspended for subsequent culturing.

In one aspect, the present disclosure provides a method of providing an isolated smooth muscle cell (SMC) population without the use of differentiation inductive cell culture media. In one example, the method includes the steps of a) obtaining an adipose tissue biopsy, b) enzymatically digesting the adipose tissue, c) centrifuging the digested adipose tissue to provide a stromal vascular fraction (SVF) that contains a heterogenous population of cells, d) washing and plating the heterogeneous population of cells; e) culturing the population of cells without the use of smooth muscle cell differentiation inductive media, f) isolating a fully differentiated SMC population from the cultured cells.

In one other example, the culturing step e) includes selecting for cells that are adherent to a cell culture support. In another embodiment, the culturing step e) does not include the use of cell culture media that contains exogenous growth factors, In one example, the culturing method includes the use of cell culture media containing minimal essential medium (e.g., DMEM or -MEM) and fetal bovine serum (e.g., 10% FBS) by standard conditions known to those of ordinary skill in the art. In another example, the smooth muscle cell population is not an adipose-derived stem cell population. In one other example, the smooth muscle cell population is not a mesenchymal stem cell population.

In one other aspect, the present disclosure provides methods of isolating and culturing populations of endothelial cells. An EC population is characterized by the presence of cells that exhibit endothelial-like morphology of a shortened, rounded or cuboidal shape. The EC population contains cells that are positive for one or more endothelial cell markers. In one example, the method includes the step of obtaining a sample from a patient in need of the reconstruction, augmentation or replacement of a blood vessel, where the sample is obtained from a non-vascular source. In another example, endothelial cells are derived from a sample obtained from the patient. In one example, the sample is autologous or non-autologous to the patient. In another example, the sample is a peripheral blood sample. In yet another example, the sample is an adipose tissue sample. The adipose tissue may be tissue removed from a subject as a result of an abdominalplasty procedure.

In another example, the obtaining step is followed by a separation step. In the case of a peripheral blood sample, the separation step includes contacting the sample with a density gradient materials, centrifuging the sample to define a density gradient that yields cells in a single isolated band, and extracting and washing the isolated band of cells from the density gradient. The separation step may be followed by a culturing step in which cells from the extracted fraction are cultured. In the case of an adipose tissue sample, the purification step includes digestion of the sample with collagenase, centrifuging the digested sample, mixing of the centrifuged sample to separate stromal cells from primary adipocytes, centrifuging the mixed sample to obtain a stromal-vascular fraction that can be re-suspended for subsequent culturing.

In one aspect, the present disclosure provides a method of providing an isolated endothelial cell (EC) population. Endothelial cells have been successfully isolated from peripheral blood and adipose sources (Daiju et al., 2005. Circulation 111: 926-931; Shepherd et al., 2006. The FASEB J. 20: E1124-E1132**;** melero-Martin JM et al., 2007. Blood 109 (11): 4761-4768; Kern at. Al., 1983. J. Clin. Invest. 71: 1822-1829; Planat-benard V et al., 2004. Circulation 109: 656-663).

In one embodiment, the present disclosure provides a method of isolating ECs from a non-vascular source. The vascular source may be peripheral blood and Example 17 provides an exemplary protocol for obtaining ECs. The non-vascular source may be adipose tissue, in which case the method may include one or more of the following steps: a) obtaining an adipose tissue biopsy, b) enzymatically digesting the adipose tissue, c) centrifuging the digested adipose tissue to provide a stromal vascular fraction (SVF) that contains a heterogenous population of cells, d) washing and plating the heterogeneous population of cells; e) culturing the population of cells with VEGF in the cell culture media, f) isolating an EC population from the cultured cells. In one other embodiment, the culturing step e) includes selecting for cells that are adherent to a cell culture support. Example 17 below provides additional information on an exemplary method of isolating ECs from adipose tissue.

In one example, the culturing method includes the use of cell culture media containing minimal essential medium (e.g., DMEM, α-MEM, or EGM-2 (Cambrex Bio Science)) and fetal bovine serum (e.g., 10% FBS) by standard conditions known to those of ordinary skill in the art. In another example, the endothelial cell population is not an adipose-derived stem cell population. In one other example, the endothelial cell population is not a mesenchymal stem cell population. Example 17 describes an exemplary protocol of obtaining EC populations.

### 4. Scaffolds

As described in Atala U.S. 6576019, scaffolds or polymeric matrices may be composed of a variety of different materials. In general, biocompatible, material and especially biodegradable material is the preferred material for the construction of the scaffolds described herein. The scaffolds are implantable, biocompatible, synthetic or natural polymeric matrices with at least two separate surfaces. The scaffolds are shaped to conform to a at least a part of the luminal organ or tissue structure in need or treatment. The biocompatible materials are biodegradeable.

Biocompatible refers to materials which do not have toxic or injurious effects on biological functions. Biodegradable refers to material that can be absorbed or degraded in a patient's body. Examples of biodegradable materials include, for example, absorbable sutures. Representative materials for forming the scaffolds include natural or synthetic polymers, such as, for example, collagen, poly(alpha hydroxy esters) such as poly(lactic acid), poly(glycolic acid), polyorthoesters and polyanhydrides and their copolymers, which degraded by hydrolysis at a controlled rate and are reabsorbed. These materials provide the maximum control of degradability, manageability, size and configuration. Preferred biodegradable polymer material include polyglycolic acid and polyglactin, developed as absorbable synthetic suture material. Polyglycolic acid and polyglactin fibers may be used as supplied by the manufacturer. Other scaffold materials include cellulose ether, cellulose, cellulosic ester, fluorinated polyethylene, poly-4-methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulfone, polyethylene, polyfluoroolefin, polyimide, polyolefin, polyoxadiazole, polyphenylene oxide, polyphenylene sulfide, polypropylene, polystyrene, polysulfide, polysulfone, polytetrafluoroethylene, polythioether, polytriazole, polyurethane, polyvinyl, polyvinylidene fluoride, regenerated cellulose, silicone, urea-formaldehyde, or copolymers or physical blends of these materials. The material may be impregnated with suitable antimicrobial agents and may be colored by a color additive to improve visibility and to aid in surgical procedures.

Other scaffold materials that are biodegradeable include synthetic suture material manufactured by Ethicon Co. (Ethicon Co., Somerville, N.J.), such as MONOCRYL™ (copolymer of glycoside and epsilon-caprolactone), VICRYL™ or Polyglactin 910 (copolymer of lactide and glycolide coated with Polyglactin 370 and calcium stearate), and PANACRYL™ (copolymer of lactide and glycolide coated with a polymer of caprolactone and glycolide). (Craig P. H., Williams J. A., Davis K. W., et al.: A Biological Comparison of Polyglactin 910 and Polyglycolic Acid Synthetic Absorbable Sutures. Surg. 141; 1010, (1975)) and polyglycolic acid. These materials can be used as supplied by the manufacturer.

In yet another embodiment, the matrix or scaffold can be created using parts of a natural decellularized organ. Biostructures, or parts of organs can be decellularized by removing the entire cellular and tissue content from the organ. The decellularization process comprises a series of sequential extractions. One key feature of this extraction process is that harsh extraction that may disturb or destroy the complex infra-structure of the biostructure, be avoided. The first step involves removal of cellular debris and solubilization of the cell membrane. This is followed by solubilization of the nuclear cytoplasmic components and the nuclear components.

Preferably, the biostructure, e.g., part of an organ is decellularized by removing the cell membrane and cellular debris surrounding the part of the organ using gentle mechanical disruption methods. The gentle mechanical disruption methods must be sufficient to disrupt the cellular membrane. However, the process of decellularization should avoid damage or disturbance of the biostructure's complex infra-structure. Gentle mechanical disruption methods include scraping the surface of the organ part, agitating the organ part, or stirring the organ in a suitable volume of fluid, e.g., distilled water. In one preferred embodiment, the gentle mechanical disruption method includes stirring the organ part in a suitable volume of distilled water until the cell membrane is disrupted and the cellular debris has been removed from the organ.

After the cell membrane has been removed, the nuclear and cytoplasmic components of the biostructure are removed. This can be performed by solubilizing the cellular and nuclear components without disrupting the infra-structure. To solubilize the nuclear components, non-ionic detergents or surfactants may be used. Examples of nonionic detergents or surfactants include, but are not limited to, the Triton series, available from Rohm and Haas of Philadelphia, Pa., which includes Triton X-100, Triton N-101, Triton X-114, Triton X-405, Triton X-705, and Triton DF-16, available commercially from many vendors; the Tween series, such as monolaurate (Tween 20), monopalmitate (Tween 40), monooleate (Tween 80), and polyoxethylene-23-lauryl ether (Brij. 35), polyoxyethylene ether W-1 (Polyox), and the like, sodium cholate, deoxycholates, CHAPS, saponin, n-Decyl-D-glucopuranoside, n-heptyl-D-glucopyranoside, n-Octyl-D-glucopyranoside and Nonidet P-40.

One skilled in the art will appreciate that a description of compounds belonging to the foregoing classifications, and vendors may be commercially obtained and may be found in "Chemical Classification, Emulsifiers and Detergents", McCutcheon's, Emulsifiers and Detergents, 1986, North American and International Editions, McCutcheon Division, MC Publishing Co., Glen Rock, N.J., U.S.A. and Judith Neugebauer, A Guide to the Properties and Uses of Detergents in Biology and Biochemistry, Calbiochem. R., Hoechst Celanese Corp., 1987. In one preferred embodiment, the non-ionic surfactant is the Triton. series, preferably, Triton X-100.

The concentration of the non-ionic detergent may be altered depending on the type of biostructure being decellularized. For example, for delicate tissues, e.g., blood vessels, the concentration of the detergent should be decreased. Preferred concentration ranges of non-ionic detergent can be from about 0.001 to about 2.0% (w/v). More preferably, about 0.05 to about 1.0% (w/v). Even more preferably, about, 0.1% (w/v) to about 0.8% (w/v). Preferred concentrations of these range from about 0.001 to about 0.2% (w/v), with about 0.05 to about 0.1% (w/v) particular preferred.

The cytoskeletal component, which includes the dense cytoplasmic filament networks, intercellular complexes and apical microcellular structures, may be solubilized using alkaline solution, such as, ammonium hydroxide. Other alkaline solution consisting of ammonium salts or their derivatives may also be used to solubilize the cytoskeletal components. Examples of other suitable ammonium solutions include ammonium sulphate, ammonium acetate and ammonium hydroxide. In a preferred embodiment, ammonium hydroxide is used.

The concentration of the alkaline solutions, e.g., ammonium hydroxide, may be altered depending on the type of biostructure being decellularized. For example, for delicate tissues, e.g., blood vessels, the concentration of the detergent should be decreased. Preferred concentrations ranges can be from about 0.001 to about 2.0% (w/v). More preferably, about 0.005 to about 0.1% (w/v). Even more preferably, about, 0.01% (w/v) to about 0.08% (w/v).

The decellularized, lyophilized structure may be stored at a suitable temperature until required for use. Prior to use, the decellularized structure can be equilibrated in suitable isotonic buffer or cell culture medium. Suitable buffers include, but are not limited to, phosphate buffered saline (PBS), saline, MOPS, HEPES, Hank's Balanced Salt Solution, and the like. Suitable cell culture medium includes, but is not limited to, RPMI 1640, Fisher's, Iscove's, McCoy's, Dulbecco's medium, and the like.

Still other biocompatible materials that may be used include stainless steel, titanium, silicone, gold and silastic.

The polymeric matrix or scaffold can be reinforced. For example, reinforcing materials may be added during the formation of a synthetic matrix or scaffold or attached to the natural or synthetic matrix prior to implantation. Representative materials for forming the reinforcement include natural or synthetic polymers, such as, for example, collagen, poly(alpha hydroxy esters) such as poly(lactic acid), poly(glycolic acid), polyorthoesters and polyanhydrides and their copolymers, which degraded by hydrolysis at a controlled rate and are reabsorbed. These materials provide the maximum control of degradability, manageability, size and configuration.

The biodegradable polymers can be characterized with respect to mechanical properties, such as tensile strength using an Instron tester, for polymer molecular weight by gel permeation chromatography (GPC), glass, transition temperature by differential scanning calorimetry (DSC) and bond structure by infrared (IR) spectroscopy; with respect to toxicology by initial screening tests involving Ames assays and in vitro teratogenicity assays and implantation studies in animals for immunogenicity, inflammation, release and degradation studies. In vitro cell attachment and viability can be assessed using scanning electron microscopy, histology and quantitative assessment with radioisotopes.

The biodegradable material may also be characterized with respect to the amount of time necessary for the material to degrade when implanted in a patient. By varying the construction, such as, for example, the thickness and mesh size, the biodegradable material may substantially biodegrade between about 2 years or about 2 months, preferably between about 18 months and about 4 months, most preferably between about 15 months and about 8 months and most preferably between about 12 months and about 10 months. In one other embodiment, the scaffold may be constructed to degrade within a shorter time frame including, without limitation, within about 1 month, within about 2 months, within about 3 months, within about 4 months, within about 5 months, within about 6 months, within about 7 months, within about 8 months, within about 9 months, within about 10 months, within about 11 months, or within about 12 months. If necessary, the biodegradable material may be constructed so as not to degrade substantially within about 3 years, or about 4 years or about five or more years. The use of a coating described herein may also be used to modulate the rate of degradation. For example, a matrix or scaffold with a coating, *e.g.,* a poly-lactide-co-glycolide copolymer, may degrade more slowly than a matrix or scaffold without a coating.

The polymeric matrix or scaffold may be fabricated with controlled pore structure as described above. The size of the pores may be used to determine the cell distribution. For example, the pores on the polymeric matrix or scaffold may be large to enable cells to migrate from one surface to the opposite surface. Alternatively, the pores may be small such that there is fluid communication between the two sides of the polymeric matrix or scaffold but cells cannot pass through. Suitable pore size to accomplish this objective may be about 0.04 micron to about 10 microns in diameter, preferably between about 0.4 micron to about 4 microns in diameter. In some embodiments, a surface of the polymeric matrix or scaffold may comprise pores sufficiently large to allow attachment and migration of a cell population into the pores. The pore size may be reduced in the interior of the polymeric matrix or scaffold to prevent cells from migrating from one side of the polymeric matrix or scaffold to the opposite side. One embodiment of a polymeric matrix or scaffold with reduced pore size is a laminated structure of a small pore material sandwiched between two large pore material. Polycarbonate membranes are especially suitable because they can be fabricated in very controlled pore sizes such as, for example, about 0.01 microns, about 0.05 micron, about 0.1 micron, about 0.2 micron, about 0.45 micron, about 0.6 micron, about 1.0 micron, about 2.0 microns and about 4.0 microns. At the submicron level the polymeric matrix or scaffold may be impermeable to bacteria, viruses and other microbes.

The following characteristics or criteria, among others, are taken into account in the design of each discrete matrix, or part thereof: (i) shape, (ii) strength, (iii) stiffness and rigidity, and (iv) suturability (the degree to which the matrix, or part thereof, is readily sutured or otherwise attached to adjacent tissue). As used herein, the stiffness of a given matrix or scaffold is defined by the modulus of elasticity, a coefficient expressing the ratio between stress per unit area acting to deform the scaffold and the amount of deformation that results from it. (See e.g., Handbook of Biomaterials evaluation, Scientific, Technical, and Clinical Testing of Implant Materials, 2nd edition, edited by Andreas F. von Recum, (1999); Ratner, et al., Biomaterials Science: An Introduction to Materials in Medicine, Academic Press (1996)). The rigidity of a scaffold refers to the degree of flexibility (or lack thereof) exhibited by a given scaffold.

Each of these criteria is a variable that can be changed (through, among other things, the choice of material and the manufacturing process) to allow the matrix, or part thereof to best placed and modified to address the medical indication and the physiological function for which it is intended. For example, the material comprising the matrix or scaffold for bladder replacement, reconstruction and/or augmentation must be sufficiently strong to support sutures without tearing, while being sufficient compliant so as to accommodate fluctuating volumes of urine.

Optimally, the matrix or scaffold should be shaped such that after its biodegradation, the resulting reconstructed bladder is collapsible when empty in a fashion similar to a natural bladder and the ureters will not be obstructed while the urinary catheter has been removed from the new organ or tissue structure without leaving a leak point. The bioengineered bladder construct can be produced as one piece or each part can be individually produced or combinations of the sections can be produced as specific parts. Each specific matrix or scaffold part may be produced to have a specific function. Otherwise specific parts may be produced for manufacturing ease. Specific parts may be constructed of specific materials and may be designed to deliver specific properties. Specific part properties may include tensile strength similar to the native tissue (e.g. ureters) of 0.5 to 1.5 MPa.sup.2 and an ultimate elongation of 30 to 100% or the tensile strength may range from 0.5 to 28 MPa.sup.2, ultimate elongations may range from 10-200% and compression strength may be <12.

The polymeric matrix or scaffold may have a three-dimensional (3-D) shape. The 3-D shape may be a tubular, half-tubular, or half-cylindrical shape. The 3-D shape may be a concave shape. The polymeric matrix or scaffold may have a flat shape. The flat-shaped polymeric matrix or scaffold may have pre-treated areas to allow more flexibility. In certain embodiments, the pre-treated areas are coated in the areas to be creased. In one embodiment, the polymeric matrix or scaffold is sufficiently malleable to be rolled, folded, or otherwise shaped for implantation through a laparoscope tube and/or port. In such embodiments, the polymeric matrix or scaffold is sufficiently malleable to be unrolled, unfolded, or otherwise returned to shape following insertion through the laparoscope tube and/or port. Those of ordinary skill in the art will appreciate that other shaped scaffolds may be suitable for use in the present invention.

A mesh-like structure formed of fibers, which may be round, scalloped, flattened, star shaped, solitary or entwined with other fibers is preferred. The use of branching fibers is based upon the same principles which nature has used to solve the problem of increasing surface area proportionate to volume increases. All multicellular organisms utilize this repeating branching structure. Branching systems represent communication networks between organs, as well as the functional units of individual organs. Seeding and implanting this configuration with cells allows implantation of large numbers of cells, each of which is exposed to the environment of the host, providing for free exchange of nutrients and waste while neovascularization is achieved. The polymeric matrix or scaffold may be made flexible or rigid, depending on the desired final form, structure and function.

In one preferred embodiment, the polymeric matrix or scaffold is formed with a polyglycolic acid with an average fiber diameter of 15 µm and configured into a bladder shaped mold using 4-0 polyglactin 910 sutures. The resulting structure is coated with a liquefied copolymer, such as, for example, poly-DL-lactide-co-glycolide 50:50, 80 milligram per milliliter methylene chloride, in order to provide certain benefits including, without limitation, to delay degradation of the coated matrix or scaffold, to achieve adequate mechanical characteristics and to set its shape. In one embodiment, the scaffold comprises a coating is provided such that it settles or accumulates at the junction between fibers of the scaffold.

In a further embodiment, the scaffolds of the present invention are coated with a biocompatible and biodegradable shape-setting material. In one embodiment, the shape-setting material contains a poly-lactide-co-glycolide copolymer. In another embodiment, the shape setting material is liquefied. In other embodiments, the poly-lactide-co-glycolide comprises lactide portions derived from the group consisting of L-lactide, D-lactide, DL-lactide, and both D-lactide and L-lactide. In another embodiment, the poly-lactide-co-glycolide comprises lactide portions derived from the group consisting of L-lactide (to form poly-L-lactide-co-glycolide), D-lactide (to form poly-D-lactide-co-glycolide), or DL-lactide or both D-lactide and L-lactide (to form poly-DL-lactide-co-glycolide). The coating may be poly-lactide-co-glycolide (PLGA) 50:50 in about 20, about 25, about 30, about 35, about 40, about 45, about 50, about 55, about 60, about 65, about 70, about 75, or about 80 mg per mL of methylene chloride. In one preferred embodiment, the PLGA 50:50 may be in about 42.5 mg/mL methylene chloride. In an exemplary protocol, PLGA is dissolved in a solvent, *e.g*., methylene chloride, to form the coating as a liquid solution, which is applied to the scaffold.

In another embodiment, the coating is provided at about 40% w/w to about 60% w/w, about 41% w/w to about 59% w/w, about 42% w/w to about 58% w/w, about 43% w/w to about 57% w/w, about 44% w/w to about 56% w/w, about 45% w/w to about 55% w/w, about 46% w/w to about 54% w/w, about 47% w/w to about 53% w/w, about 48% w/w to about 52% w/w, or about 49% w/w to about 51% w/w of the matrix or scaffold. In other embodiments, the coating is provided at about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 59% w/w, or about 60% w/w, of the matrix or scaffold.

In one other aspect, the scaffolds of the present invention may be treated with additives or drugs prior to implantation (before or after the polymeric matrix or scaffold is seeded with cells), e.g., to promote the regeneration of new tissue after implantation. Thus, for example, growth factors, cytokines, extracellular matrix or scaffold components, and other bioactive materials can be added to the polymeric matrix or scaffold to promote graft healing and regeneration of new tissue. Such additives will in general be selected according to the tissue or organ being reconstructed, replaced or augmented, to ensure that appropriate new tissue is formed in the engrafted organ or tissue (for examples of such additives for use in promoting bone healing, see, e.g., Kirker-Head, C. A. Vet. Surg. 24 (5): 408-19 (1995)). For example, when polymeric matrices (optionally seeded with endothelial cells) are used to augment vascular tissue, vascular endothelial growth factor (VEGF), (see, e.g., U.S. Pat. No. 5,654,273) can be employed to promote the regeneration of new vascular tissue. Growth factors and other additives (e.g., epidermal growth factor (EGF), heparin-binding epidermal-like growth factor (HBGF), fibroblast growth factor (FGF), cytokines, genes, proteins, and the like) can be added in amounts in excess of any amount of such growth factors (if any) which may be produced by the cells seeded on the polymeric matrix, if added cells are employed. Such additives are preferably provided in an amount sufficient to promote the regeneration of new tissue of a type appropriate to the tissue or organ, which is to be reconstructed, replaced or augmented (e.g., by causing or accelerating infiltration of host cells into the graft). Other useful additives include antibacterial agents such as antibiotics.

One preferred supporting matrix or scaffold is composed of crossing filaments which can allow cell survival by diffusion of nutrients across short distances once the cell support is implanted. The cell support matrix or scaffold becomes vascularized in concert with expansion of the cell mass following implantation.

The building of three-dimensional structure constructs in vitro, prior to implantation, may facilitate regenerative events after implantation in vivo, and may minimize the risk of an inflammatory response towards the matrix, thus avoiding graft contracture and shrinkage.

The polymeric matrix or scaffold may be sterilized using any known method before use. The method used depend on the material used in the polymeric matrix. Examples of sterilization methods include steam, dry heat, radiation, gases such as ethylene oxide, gas and boiling.

The synthetic materials that make up the scaffolds may be shaped using methods such as, for example, solvent casting, compression molding, filament drawing, meshing, leaching, weaving and coating. In solvent casting, a solution of one or more polymers in an appropriate solvent, such as methylene chloride, is cast as a branching pattern relief structure. After solvent evaporation, a thin film is obtained. In compression molding, a polymer is pressed at pressures up to 30,000 pounds per square inch into an appropriate pattern. Filament drawing involves drawing from the molten polymer and meshing involves forming a mesh by compressing fibers into a felt-like material. In leaching, a solution containing two materials is spread into a shape close to the final form of the construct. Next a solvent is used to dissolve away one of the components, resulting in pore formation. (See Mikos, U.S. Pat. No. 5,514,378, hereby incorporated by reference.) In nucleation, thin films in the shape of a scaffold are exposed to radioactive fission products that create tracks of radiation damaged material. Next the polycarbonate sheets are etched with acid or base, turning the tracks of radiation-damaged material into pores. Finally, a laser may be used to shape and bum individual holes through many materials to form a structure with uniform pore sizes. Coating refers to coating or permeating a polymeric structure with a material such as, for example liquefied copolymers (poly-DL-lactide co-glycolide 50:50 80 mg/ml methylene chloride) to alter its mechanical properties. Coating may be performed in one layer, or multiple layers until the desired mechanical properties are achieved. These shaping techniques may be employed in combination, for example, a polymeric matrix or scaffold may be weaved, compression molded and glued together. Furthermore different polymeric materials shaped by different processes may be joined together to form a composite shape. The composite shape may be a laminar structure. For example, a polymeric matrix or scaffold may be attached to one or more polymeric matrixes to form a multilayer polymeric matrix or scaffold structure. The attachment may be performed by gluing with a liquid polymer or by suturing. In addition, the polymeric matrix or scaffold may be formed as a solid block and shaped by laser or other standard machining techniques to its desired final form. Laser shaping refers to the process of removing materials using a laser.

In a preferred embodiment, the scaffolds are formed from nonwoven polygycolic acid (PGA) felts and poly(lactic-co-glycolic acid) polymers (PLGA).

As described in Bertram et al. U.S. Published Application 20070276507, the polymeric matrix or scaffold of the present invention may be shaped into any number of desirable configurations to satisfy any number of overall system, geometry or space restrictions. The matrices may be three-dimensional matrices shaped to conform to the dimensions and shapes of a laminarily organized luminal organ or tissue structure. For example, in the use of the polymeric matrix for bladder reconstruction, a three-dimensional matrix may be used that has been shaped to conform to the dimensions and shapes of the whole or a part of a bladder. Naturally, the polymeric matrix may be shaped in different sizes and shapes to conform to the bladders of differently sized patients. Optionally, the polymeric matrix should be shaped such that after its biodegradation, the resulting reconstructed bladder may be collapsible when empty in a fashion similar to a natural bladder. The polymeric matrix may also be shaped in other fashions to accommodate the special needs of the patient. For example, a previously injured or disabled patient, may have a different abdominal cavity and may require a bladder replacement scaffold, a bladder augmentation scaffold, a bladder conduit scaffold, and a detrusor muscle equivalent scaffold adapted to fit. In one aspect, the present invention contemplates additional scaffolds suitable for use with the smooth muscle cell populations described herein. For example, scaffolds suitable for implantation into the eye may be provided.

### A. Augmentation or replacement scaffolds

In one other aspect of the present disclosure, the polymeric matrix or scaffold is shaped to conform to part of a bladder. In one embodiment, the shaped matrix is conformed to replace at least about 50%, at least about 60%, at least about 70%, at least about 80%, at least about 90%, or at least about 95% of the existing bladder of a recipient. In one other aspect, the polymeric matrix or scaffold is shaped to conform to 100% or all of a bladder.

In one embodiment, the polymeric matrix comprises a first implantable, biocompatible, synthetic or natural polymeric matrix or scaffold having at least two separate surfaces, and a second implantable, biocompatible, synthetic or natural polymeric matrix or scaffold having at least two separate surfaces, which are adapted to mate to each other and shaped to conform to at least a part of the luminal organ or tissue structure in need of the treatment when mated. The first and second polymeric matrices may be formed from one integral unit subdivided into two or more distinct parts, or from two or more distinct parts, adapted to mate. In some embodiments, the first and second polymeric matrices once mated may be used for reconstruction, augmentation, or replacement of a luminal organ or tissue structure.

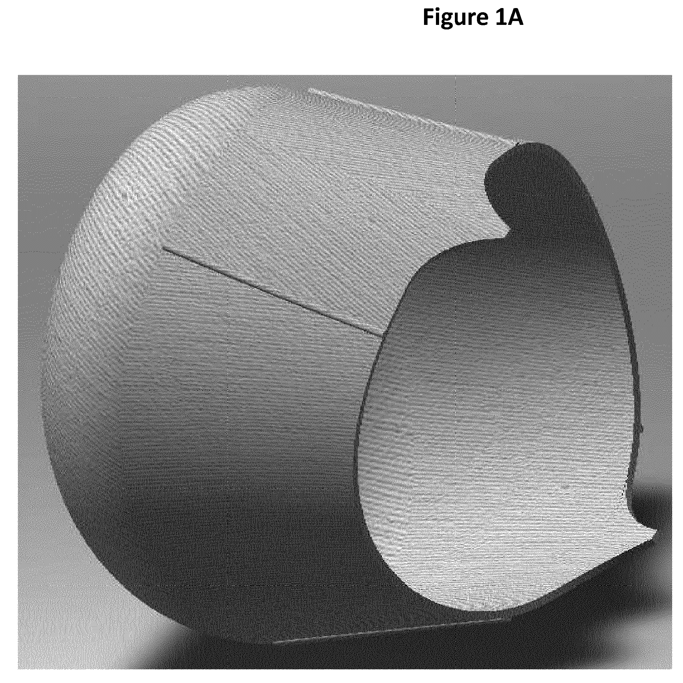

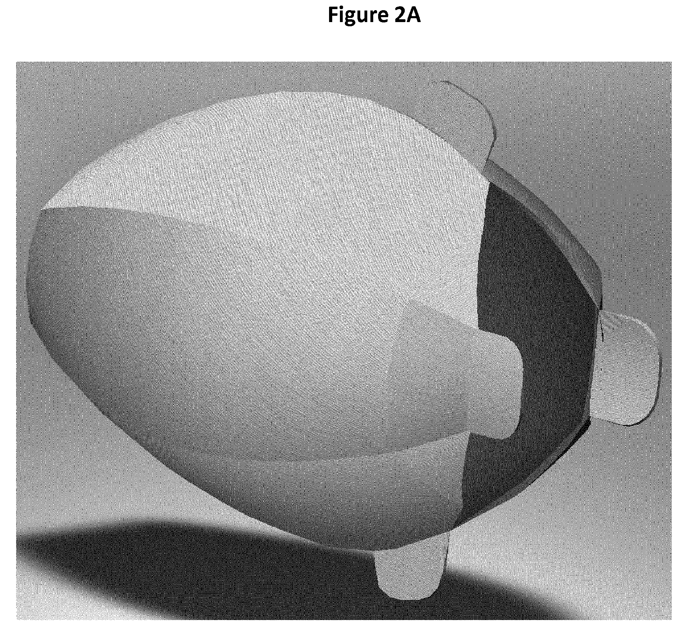

In some embodiments, the first and second polymeric matrices are symmetrical, while in other embodiments, the first and second polymeric matrices are asymmetrical. In one embodiment, the first polymeric matrix or scaffold has a hemispherical or quasi-hemispherical shape having a closed, domed end and an open, equatorial border, and the second polymeric matrix or scaffold is a collar adapted to mate with the equatorial border of the first polymeric matrix. In another embodiment, the first and second polymeric matrices are each hemispherical or quasi-hemispherical in shape, having a closed, domed end and an open, equatorial border. In yet another embodiment, the first and second polymeric matrices each comprise a circular or semi-circular base and at least 2 petals radially extending from each base. In this embodiment, the bases and petal shaped portions of the first and the second polymeric matrices are mated to create a hollow spherical or quasi-spherical matrix or scaffold such that a flanged longitudinal, elliptical opening is created on one side of the mated polymeric matrices, and a circular opening is created on the side opposite the longitudinal opening. In another embodiment, the first and second polymeric matrices are made from 3 parts comprising a top, a front and a sidepiece, adapted to mate. In this embodiment, the 3 distinct parts are mated using at least 3, preferably four vertical seams, thereby forming a crown shaped neo-bladder construct. The crown shaped constructs are preferably used alone as a device for luminal organ reconstruction, augmentation, or replacement. In one embodiment, the construct is a bladder augmentation scaffold. One example of a bladder augmentation scaffold is depicted in Figure 1A-D. In another embodiment, the construct is a bladder replacement scaffold. One example of a bladder replacement scaffold is depicted in Figure 2A-D.

Additionally, the first polymeric matrix, the second polymeric matrix, or both, may contain at least one receptacle or port adapted to receive a tubular vessel or insert where the connection of the construct to a native vessel or tube is necessary. The vessels or inserts are themselves, for example, cylindrical or tubular shaped polymer matrices, each having at least one flange located at a first end of the cylindrical polymer. The vessels or inserts are, preferably, composed of the same biocompatible material as the first or second polymeric matrices described above. In some embodiments, the vessel or insert also contains a washer adapted to fit around the cylindrical or tubular vessel or insert polymer matrix. For example, the washer is a hydrogel. The cylindrical or tubular vessel or insert may optionally contain a washer. The washer may be hydrogel. Additionally, the cylindrical or tubular insert may be self-stabilizing.

In another embodiment, the receptacles or ports adapted to receive tubular vessels or inserts where the connection of the scaffold or matrix (once seeded with cells) to a native vessel or tube is necessary also applies to other the matrices discussed below.

In one aspect, the scaffold is an organ or tissue structure replacement scaffold that includes at least two matrices. In one embodiment, the scaffold comprises a first matrix having a first surface and a second matrix having a first surface. The first matrix and the second matrix may be configured or adapted to mate. In another embodiment, the first matrix and the second matrix may be shaped to conform to at least a part of a luminal organ when mated. The first and second matrix may comprise a biocompatible material. The biocompatible material may comprise a biodegradable material.

In one embodiment, the first matrix may have a hemispherical shape with a closed end and an open, equatorial border, and the second matrix may have a collar configured or adapted to mate with the equatorial border of the first matrix. The closed end may be domed. In another embodiment, the first matrix and the second matrix may each have a hemispherical shape having a closed end and an open, equatorial border. The closed end may be domed. In yet another embodiment, the first matrix may further comprises a flanged region along at least one border of the first matrix. The second matrix further may comprise a flanged region along at least one border of the second matrix, and wherein the flanged region of the second matrix is adapted to mate with the flanged region of the first matrix.

In one embodiment, the scaffold comprises a first, biocompatible matrix and a second, biocompatible matrix, where the first and second matrix may each comprise a base and may be configured or adapted to mate. In one embodiment, the first and second matrices may be shaped to conform to at least a part of a luminal organ when mated. In another embodiment, the first and second matrix may further comprise at least two petals radially extending from each base.

In one other embodiment, each of the first and second matrices may be originally derived from a template comprising a base and at least four petals. In one configuration, a pair of opposing petals may be shorter in length than the other petals. In another embodiment, the first and second matrices may be two distinct units adapted to mate.

In one embodiment, the bases of the first and second matrixes are adapted to mate. In some embodiments, the first and second matrices are mated via the petal shaped portions of the first and second matrixes.

In other embodiments, the first and second matrices may be configured or adapted to form a hollow spherical or quasi-spherical shape with a longitudinal opening at a first mating point between the first and second matrices and a circular opening at a second mating point between the first and second matrices that is opposite the longitudinal opening. The scaffold may further include at least one flap incorporated into the base of the first or second matrix. In another embodiment, the longitudinal opening has a lip and at least one flap is disposed at the lip of the longitudinal opening.

In another aspect, the matrix or matrices may be connectable to a native vessel. In one embodiment, the first matrix, the second matrix, or both, are each configured or adapted to receive a native vessel. In another embodiment, the first matrix, the second matrix, or both, further comprise at least one receptacle. The at least one receptacle may be configured or adapted to receive a tubular insert. The tubular insert may be disposed within the receptacle. In some embodiments, the tubular insert has an end. The insert may have at least one flange located at this end. In another embodiment, the tubular insert may be configured or adapted to connect to a native vessel. In a further embodiment, the scaffold has a surface and a washer disposed around the tubular insert. The washer may be configured or adapted to form a watertight seal between the flange and the surface of the construct. In some embodiments, the washer comprises a hydrogel.

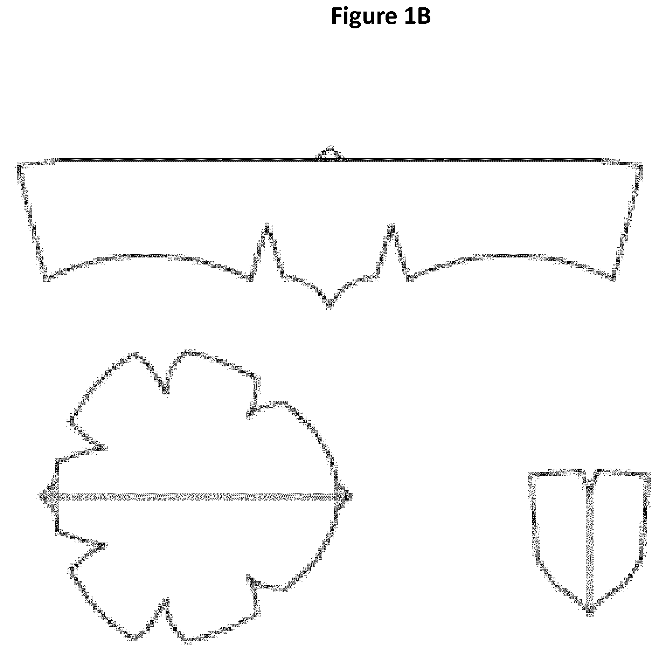

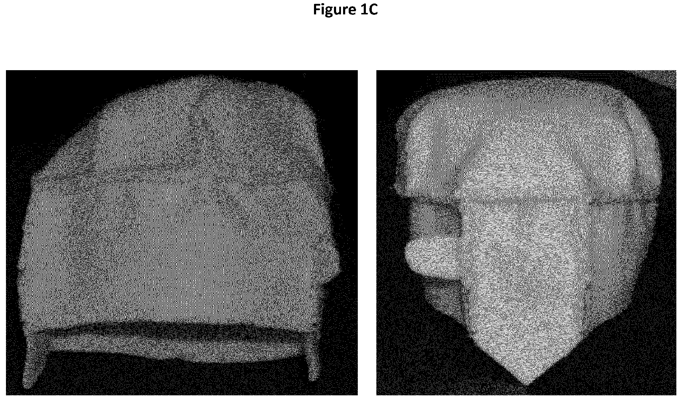

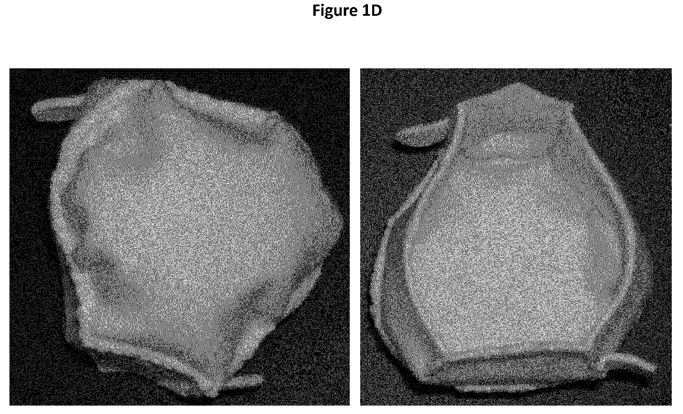

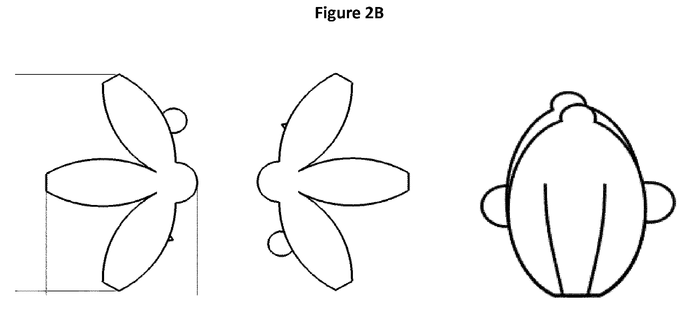

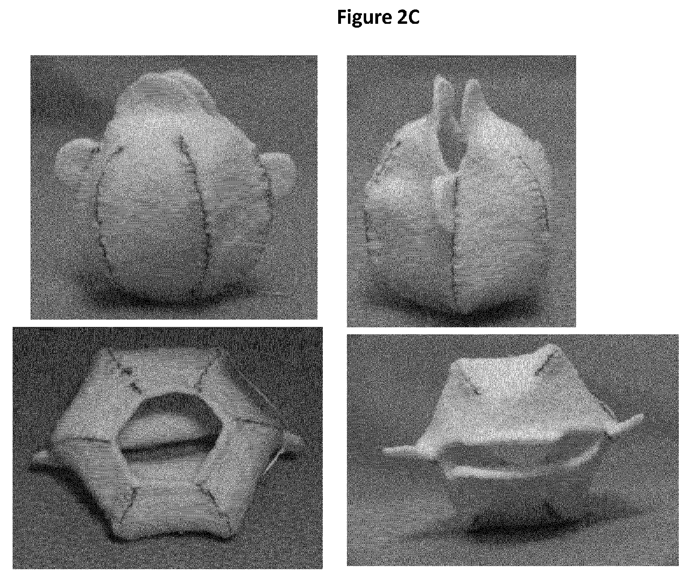

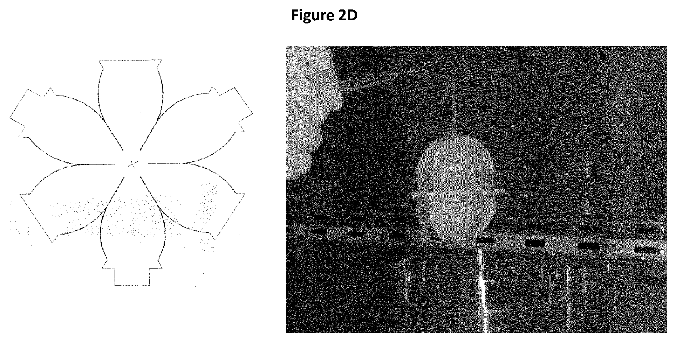

Figures 1 and 2 provide representative depictions of scaffold configurations that include at least two matrices.

In one aspect, the scaffold is an organ or tissue structure augmentation scaffold that includes one or more matrices. In one embodiment, the scaffold includes a first matrix having a base and a plurality of notches, wherein the first matrix is adapted to form a hemi-shape that conforms to at least a part of a luminal organ when assembled. In another embodiment, the scaffold includes a second and a third matrix, wherein the first, second and third matrices may be configured or adapted to mate and are shaped to conform to at least part of the luminal organ when mated. The first, second and third polymeric matrices may be derived from a template comprising three subdivided parts. In another embodiment, the first, second and third matrices are derived from three distinct templates and may are configured or adapted to mate. In one other embodiment, the first, second, and/or third matrix comprise a biocompatible material. The biocompatible material may comprise a biodegradable material.

In one other aspect, the scaffold is made up of parts having different shapes or configurations. In one embodiment, the scaffold may include a first, second and third polymeric matrices that are correspond to a top piece, a front piece, and a side piece, respectively, that when mated together form a first crown shape. In another embodiment, the front piece and the side piece may each comprise a first edge and a second edge. The first edge of the front piece may be joined to the first edge of the side piece. The second edge of the front piece may be joined to the second edge of the side piece. In one other embodiment, the first edges may be joined by a seam and/or the second edges may be joined by a seam. In other embodiments, the front piece may include a notch having a first edge and a second edge. The first and second edges may be joined, such as, for example by a seam. In another embodiment, the top piece may have a first edge, the side piece has a third edge, and the front piece has a third edge. The first first edge of the top piece may be joined to the third edge of the side piece and/or the first edge of the top piece may be joined to the third edge of the front piece. The first and third edges may be joined by a seam. In another embodiment, each notch may have a first edge and a second edge. These edges may be joined, such as, for example by a seam. In other embodiments, the side piece may include at least one flap.

In all embodiments, each individual matrix or all matrices in a scaffold may comprise a biodegradable material. The material may be selected from the group consisting of polyglycolic acid, polylactic acid and a copolymer of glycolic acid and lactic acid. In other embodiments, the matrix or matrices comprise polyglycolic acid and a copolymer of glycolic acid and lactic acid.

In one embodiment, the luminal organ is a tubular or hollow organ. The organ may be a genitourinary organ. In another embodiment, the genitourinary organ is selected from the group consisting of bladder, ureters and urethra. In one other embodiment, the genitourinary organ is a bladder or a bladder segment. In some embodiments, the scaffolds used are configured or adapted to form regenerated bladder tissue in vivo that exhibits the compliance of natural bladder tissue.

In one embodiment, the mated matrices with deposited cells form an implantable construct. In another embodiment, the at least first cell population comprises a muscle cell population as described herein. The muscle population may be a smooth muscle cell population.

In another embodiment, the scaffold may have at least a first cell population deposited on or in a first surface of the first matrix, a first surface of the second matrix, or both. In one other embodiment, the scaffold may further include a second population of cells deposited on or in a second surface of the first matrix, a second surface of the second matrix, or both. The second population of cells comprises urothelial cells.

The augmentation and replacement scaffolds described herein, as well as methods of making and using the same, are further described in Bertram et al. U.S. Published Patent Application No. 20070276507 (incorporated herein by reference in its entirety).

### B. Urinary conduit scaffolds

The present disclosure provides neo-urinary diversion or conduit scaffolds that can be seeded with cells and used as a replacement for gastrointestinal tissue in the construction of a urinary diversion in a subject. For example, the neo-urinary diversions described herein may have application after radical cystectomy for the treatment of patients who would otherwise undergo an ileal loop diversion. In one aspect, the present disclosure contemplates conduit scaffolds or matrices suitable for use as urinary diversions in a subject in need formed from the methods described herein. One end of the conduit scaffold may be connected to one or more ureters and the other end may be connected to a urine reservoir that is external to the subject's body. In one embodiment, the conduit may exit the subject's body via a stoma. In another embodiment, the polymeric matrix comprises a first implantable, biocompatible, synthetic polymeric matrix or scaffold provided in a tubular form. In some embodiments, the tubular scaffold comprises a first end configured to connect to a ureter of the subject. In another embodiment, the first scaffold further includes a second end configured to form a stoma or sphincter in the subject. In another embodiment, the first scaffolds further includes at least one side opening configured to connect to a least one ureter. In some embodiments, the first scaffold includes a first side opening configured to attach to a first ureter and a second side opening configured to attach to a second ureter.

In one aspect, the scaffold is designed to be flexible as to the attachment of one or both ureters in the subject. In one embodiment, the scaffold may have one or more openings for attachment of a ureter on the side of the tubular structure. In another embodiment, the scaffold may have an opening at one end of the tubular structure for attachment of a ureter. The attachment of a ureter to one end of the structure rather than the side may present less strain on the ureter if the distance between the end of the ureter to be attached and the scaffold end is less than the distance between the end of the ureter and the side of the scaffold. In general, the scaffold, or parts thereof, is configured to be attached to parts of the subject, e.g., ureters, abdominal wall, skin, etc., and such configurations include, without limitation, open or closed ends of the tubular matrix, ends or side openings configured to be sutured or otherwise connected to the subject's ureters, abdominal wall, skin, etc. Those of ordinary skill in the art will appreciate that the different configurations will depend upon the particular dimensions of the abdominal cavity of the recipient.

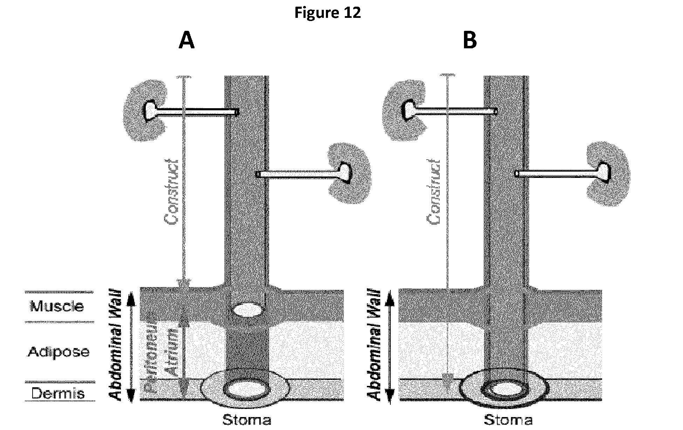

In one aspect, the tubular conduit scaffold comprises one end of the tube that serves as the outflow end for urine that passes from one or both ureters through the tubular scaffold and ultimately out of the recipient. In one embodiment, the outflow end of the scaffold is configured to terminate at the wall of the abdominal cavity of the recipient. Figure 12 (panel A) illustrates an exemplary configuration for the scaffold.

In another embodiment, the outflow end of the scaffold is configured to extend through the abdominal wall, i.e., transabdominal, and connect directly to the subcutaenous layer of the skin stoma, i.e., percutaneous. Figure 12 (panel B) illustrates an exemplary configuration for the scaffold.

In one other embodiment, the tubular structure comprises a first end comprising an even edge and a second end comprising a non-uniform or uneven edge. The non-uniform edge may include a circular base with a number of petals radially extending from the base. The number of petals may be 1, 2, 3, 4, 5, or 6. The uneven edge may comprise a series of petals such as, for example, those shown in Figure 3A. In one embodiment, the tubular structure has a form suitable for use as a urinary diversion system or a conduit in a patient in need. In another embodiment, the system diverts urine from one or more ureters to an abdominal wall section such as, for example, in the case of a ureterostomy. In other embodiments, the system diverts urine from the bladder to an abdominal wall section such as, for example, in the case of a cystostomy. In one other embodiment, the system connects the bladder to the urethra. In yet another embodiment, a first system may divert urine from one or more ureters to an abdominal wall section and a second system may divert urine from the bladder to an abdominal wall section. In all embodiments, the system may divert urine from one or more ureters to an abdominal wall section such as, for example, in the formation of a stoma.

In another embodiment, the tubular matrix or scaffold is a urinary diversion or conduit scaffold. In one embodiment, the tubular structure of the urinary diversion system is of rectangular, circular, or triangular cross sectional area. Figure 3B illustrates some of the different cross sectional configurations contemplated herein.

In another embodiment, tubular structure retains sufficient rigidity to remain patent following implantation. In one other embodiment, the tubular structure's rigidity is retained with or without the use of a catheter in its lumen. Where a catheter is used, it can be placed into the luminal space of the tubular structure to provide additional patency.

In one other embodiment, the conduit scaffold may further include a second scaffold in the form of a round or ovoid connector configured to connect the first end of the first scaffold to a ureter. In yet another embodiment, the conduit scaffold may further include a third scaffold in the form of a washer-ring configured to form a stoma or sphincter with the second end of the first tubular scaffold to create a stoma in a subject. Figure 3C illustrates variations of a urinary diversion construct (A - open claim ovoid; B - open claim ovoid receptacle; C - closed ovoid receptacle and three tubes).

In some embodiments, the tubular structure may include a washer structure for connection to a tissue, organ or body part to achieve anastomosis for the creation of a continent stoma or sphincter. In another embodiment, the washer is provided with a thickness of about less than 1 mm, about less than 1.5 mm, about less than 2 mm, about less than 2.5 mm, about less than 3 mm, about less than 3.5 mm, about less than 4 mm, about less than 4.5 mm, or about less than 5 mm.

In one embodiment, the urinary diversion or conduit scaffold is shaped into the configuration shown in Figure 3A. In one other embodiment, the tubular structure comprises a first end comprising an even edge and a second end comprising a non-uniform or uneven edge. The non-uniform edge may include one or more fasteners configured for attachment to an external region of the subject, such as in the formation of a stoma external to the subject. In one embodiment, the first and second ends of the tubular structure may be in the form illustrated in Figure 3A. The number of fasteners may be 1, 2, 3, 4, 5, or 6.

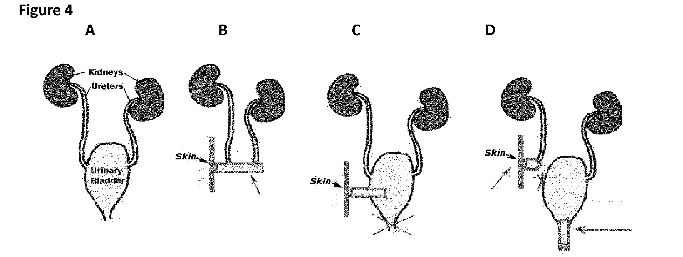

Figure 4A depicts a part of the normal anatomy for the human urinary system.

In one embodiment, the tubular structure has a form suitable for use as a urinary diversion or a conduit in a patient in need. In another embodiment, the conduit diverts urine from one or more ureters to an abdominal wall section such as, for example, in the case of a ureterostomy (Figure 4B, 4D). In other embodiments, the conduit diverts urine from the bladder to an abdominal wall section such as, for example, in the case of a cystostomy (Figure 4C). In one other embodiment, the conduit connects the bladder to the urethra (Figure 4D). In yet another embodiment, a first conduit may divert urine from one or more ureters to an abdominal wall section and a second conduit may divert urine from the bladder to an abdominal wall section. In all embodiments, the conduit may divert urine from one or more ureters to an abdominal wall section (Figure 4B). In all embodiments, the conduit may be configured to form a stoma.

In one embodiment, the tubular structure of the urinary diversion or conduit scaffold is of rectangular, circular, or triangular cross sectional area. In another embodiment, the tubular structure retains sufficient rigidity to remain patent following implantation. In one other embodiment, the tubular structure's rigidity is retained with or without the use of a catheter in its lumen. In some embodiments, a urinary diversion scaffolds further include a catheter configured to be placed in the luminal space of tubular structure upon implantation. In one embodiment, the catheter is a Foley-like balloon catheter. Where a catheter is used, it can be placed into the luminal space of the tubular structure to provide additional patency. Those of ordinary skill in the art will appreciate that other catheters known in the art may be suitable for use according to the present disclosure.

In another embodiment, the thickness of the tubular wall of the scaffolds will be less than about 2 mm, less than about 2.5 mm, less than about 3.5 mm, less than about 4 mm, less than about 4.5 mm, less than about 5 mm, less than about 5.5 mm, or less than about 6 mm.

In some embodiments, the scaffolds may have variable outer and inner diameters. In one embodiment, the ends of the scaffold may be flared, non-flared, sealed, or rounded.

In other embodiments, the scaffold is permeable to urine. In one embodiment, the scaffold's pore size is about greater than about 0 microns to about 500 microns. In another embodiment, the pore size is from about 100 microns to about 200 microns. In another embodiment, the pore size is from about 150 microns to about 200 microns. In other embodiments, the pore size is about 100 microns, about 110 microns, about 120 microns, about 130 microns, about 140 microns, about 150 microns, about 160 microns, about 170 microns, about 180 microns, about 190 microns, or about 200 microns. In some embodiments, the pore size is about 100 microns, about 200 microns, about 300 microns, about 400 microns, about 500 microns, or about 600 microns. In other embodiments, the scaffold includes a pore architecture that is a single pore size distribution, multiple pore size distribution, or a pore gradient distribution.

In another embodiment, the scaffold material is suturable and may form connections with tissue that are resistant to leakage.

In other embodiments, the tubular scaffold material is selected to maintain patency throughout the duration of implantation use, support cell attachment and the in-growth of host tissue, and retain flexibility. In another embodiment, the material will have a burst strength that exceeds the pressures to which it will be exposed during normal in vivo fluid cycling. In other embodiments, the material will have a degradation time commensurate with host tissue in-growth.

### C. Muscle equivalent scaffolds

In one aspect, the polymeric matrix or scaffold of the present disclosure is a muscle equivalent scaffold. In one embodiment, the muscle equivalent scaffold is a detrusor muscle equivalent scaffold. In another embodiment, the scaffold is suitable for laparoscopic implantation.

In one aspect, the polymeric matrix comprises a polymeric matrix or scaffold shaped to conform to at least a part of the organ or tissue structure in need of said treatment and of a sufficient size to be laparoscopically implanted. In certain embodiments, the polymeric matrix or scaffold of the invention is between about 3 and about 20 cm in length. In one embodiment the polymeric matrix or scaffold is about 20 cm in maximal length. In another embodiment, the polymeric matrix or scaffold is about 15 cm in maximal length. In another embodiment, the polymeric matrix or scaffold is about 10 cm in maximal length. In another embodiment, the polymeric matrix or scaffold is about 8 cm in maximal length. In another embodiment, the polymeric matrix or scaffold is about 4 cm in maximal length. In yet another embodiment, the polymeric matrix or scaffold is about 3 cm in maximal length. In certain embodiments, the polymeric matrix or scaffold of the invention is between about 1 and about 8 cm in width. In some embodiments, the polymeric matrix or scaffold is about 4 cm in maximal width. In other embodiments, the polymeric matrix or scaffold is about 3 cm in maximal width. In yet other embodiments, the polymeric matrix or scaffold is about 5 cm in maximal width.

In one embodiment, the polymeric matrix or scaffold has a three-dimensional (3-D) shape. In another embodiment, the polymeric matrix or scaffold has a flat shape. In one embodiment, the flat-shaped polymeric matrix or scaffold comprises pre-treated areas to allow more flexibility. In certain embodiments, the pre-treated areas are coated in the areas to be creased. In one embodiment, the polymeric matrix or scaffold is sufficiently malleable to be rolled, folded, or otherwise shaped for implantation through a laparoscope tube and/or port. In such embodiments, the polymeric matrix or scaffold is sufficiently malleable to be unrolled, unfolded, or otherwise returned to shape following insertion through the laparoscope tube and/or port. In one embodiment, the polymeric matrix or scaffold is cut into 2, 3, 4, 5, 6, 7, 8, 9 or 10 strips prior to implantation through a laparoscope tube and/or port. In certain embodiments, the 2, 3, 4, 5, 6, 7, 8, 9 or 10 strips are mated prior to implantation through a laparoscope tube and/or port. The 2, 3, 4, 5, 6, 7, 8, 9 or 10 strips may be mated using glue, staples, sutures, or other technique known to one of ordinary skill in the art. In such embodiments the 2, 3, 4, 5, 6, 7, 8, 9 or 10 mated strips are folded and/or stacked to pass through a laparoscope tube and/or port. In such embodiments, the 2, 3, 4, 5, 6, 7, 8, 9 or 10 strips are unfolded and/or unstacked following insertion through the laparoscope tube and/or port. In some embodiments, the previously placed mating means are tightened as appropriate following insertion through the laparoscope tube and/or port.

In one embodiment, the polymeric matrix comprises a first implantable, biocompatible, synthetic or natural polymeric matrix or scaffold provided in the form of a patch or in the form of a strip. In one embodiment, the patch has a form suitable for use as a detrusor muscle equivalent in the bladder of a patient in need. In one other embodiment, the patch has a form suitable for increasing the volume capacity of the existing bladder of a patient in need. In certain embodiments, the patch increases the bladder size between about 50 mL and about 500 mL. In some embodiments, the patch would increase bladder size in increments of 50 mL. In some embodiments, the patch increases the bladder size about 450 mL. In one embodiment, a surface area increase of 30 cm² increases the volume of a 200 mL bladder to 250 mL. In another embodiment, an increase of 25 cm² increases the volume of a 350 mL bladder to 400 mL. In one embodiment, the scaffold has a two-dimensional surface area of about 30 cm². In another embodiment, the scaffold has a two-dimensional surface area of about 25 cm². In one embodiment, the patch is in the form of a strip, disc, square, ellipsoid, or any other appropriate configuration. In other embodiments, the patch is provide in a pre-folded form, e.g., like an accordion.

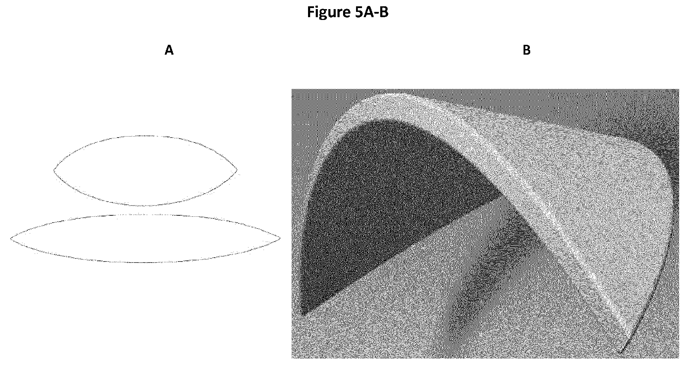

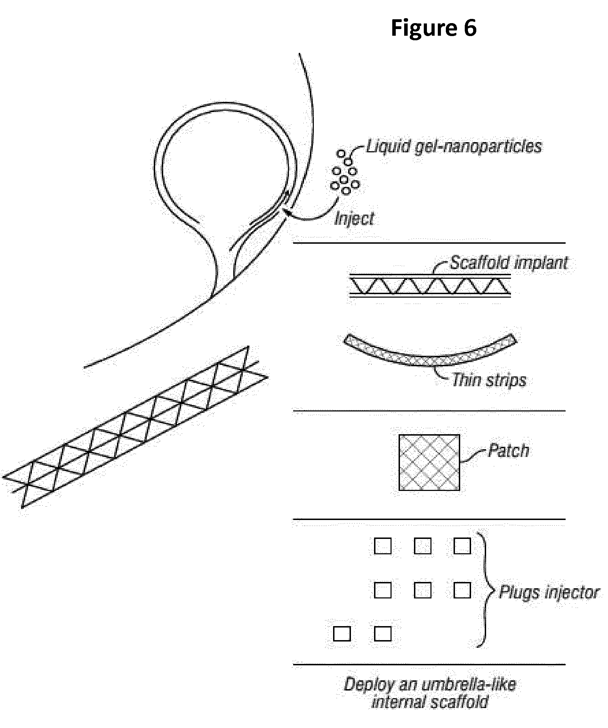

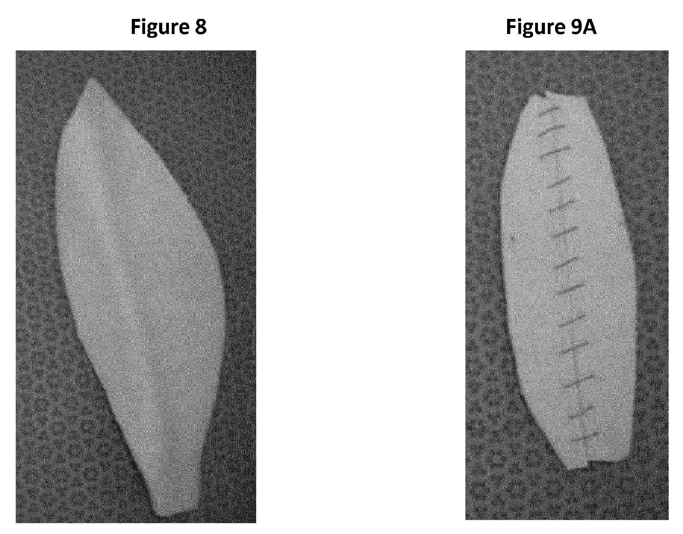

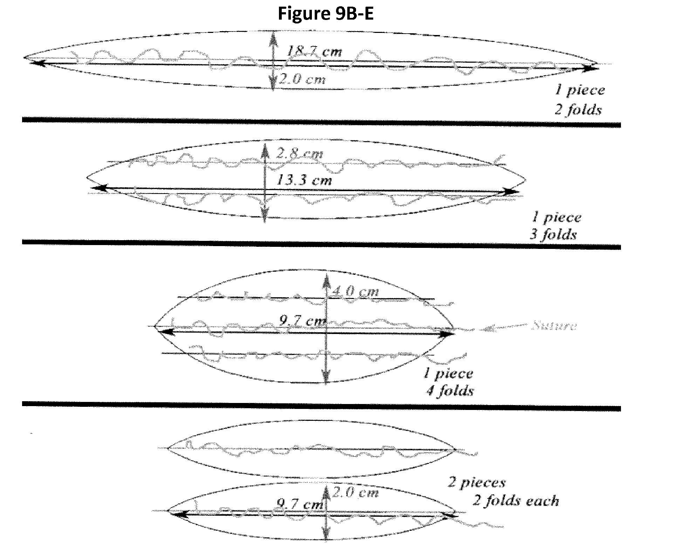

Figure 5A-B show examples of a muscle equivalent scaffold or polymeric matrix. In one embodiment, the polymeric matrix or scaffold is in the shape of a double wedge, e.g., the shape shown in Figure 5A. In another embodiment, the polymeric matrix is shaped into one of the configurations shown in Figures 6-9.

In all embodiments, the polymeric matrix or scaffold is shaped so as to minimize the strain on both the bladder and matrix or scaffold.

In another embodiment, the polymeric matrix comprises a first implantable, biocompatible, synthetic or natural polymeric matrix or scaffold provided in the form of a patch or in the form of a strip. In one embodiment, the patch has a form suitable for use as a detrusor muscle equivalent in the bladder of a patient in need. In one other embodiment, the patch has a form suitable for increasing the volume capacity of the existing bladder of a patient in need. In some embodiments, the patch would increase bladder size in increments of 50 mL. In one embodiment, the patch is in the form of a strip, disc, square, ellipsoid, or any other appropriate configuration. In other embodiments, the patch is provide in a pre-folded form, e.g., like an accordion.

In one embodiment, the polymeric matrix is shaped to conform to at least a part of a luminal organ or tissue structure of the urinary system, such as for example in one of the configurations shown in Figures 1-9. In all embodiments, the biocompatible material used for these matrices or scaffolds is, for example, biodegradable. In all the embodiments, the biocompatible material may be polyglycolic acid.

In all embodiments, the polymeric matrix or scaffold is coated with a biocompatible and biodegradable shaped setting material. In one embodiment, the shape setting material may comprise a liquid copolymer. In another embodiment, the liquid co-polymer may comprise a liquefied lactide/glycolide copolymer. In one embodiment, the liquid co-polymer may comprise poly-lactide-co-glycolide. The liquid co-polymer may comprise poly-D-lactide-co-glycolide, poly-L-lactide-co-glycolide, or poly-DL-lactide-co-glycolide.

### D. Gastro-intestinal tissue scaffolds

The present invention provides scaffolds suitable for the formation of a gastro-intestinal tissue construct, e.g., an esophageal tissue or intestine scaffold. In one aspect, the polymeric matrix or scaffold of the present invention is suitable for implantation at, on, or into a part of the gastrointestinal tract. In one aspect, the polymeric matrix comprises a polymeric matrix or scaffold shaped to conform to at least a part of the gastro-intestinal (GI) tract in need of treatment. Suitable parts of the GI tract include, without limitation, esophagus, small intestine, large intestine, stomach, colon, or anal sphincter tissue.

In a preferred embodiment, the GI scaffolds are formed from a nonwoven polygycolic acid (PGA)/poly(lactic-co-glycolic acid) (PLGA) polymer, a VICRYL™ woven mesh, and a woven PGA. In one preferred embodiment, the PLGA/PGA polymer scaffold is regular (e.g., 3 mm thick) or thin (e.g., 0.5 mm thick).

In one embodiment, the GI polymeric matrix comprises a first implantable, biocompatible, synthetic or natural polymeric matrix or scaffold provided in the form of a patch or in the form of a strip. In one embodiment, the patch has a form suitable for use as a GI tissue scaffold in the GI tissue of a patient in need. In one other embodiment, the patch has a form suitable for regeneration, replacement, augmentation, or reconstruction of the existing GI tissue of a patient in need. In one embodiment, the patch is in the form of a strip, disc, square, ellipsoid, or any other appropriate configuration. In other embodiments, the patch is provide in a pre-folded form, e.g., like an accordion.

In all embodiments, the biocompatible material used for these GI matrices or scaffolds is, for example, biodegradable. In all the embodiments, the biocompatible material may be polyglycolic acid. In some embodiments, the polymeric GI matrix or scaffold is coated with a biocompatible and biodegradable shaped setting material, as described herein.

The present invention contemplates the application of electrospun tubular composites with surface corrugations/ruffling for the regeneration, reconstruction, augmentation or replacement of GI tissue or organs. The composites may be electrospun such that they mimic the topography of GI tissue. Where the GI tissue is luminal in nature, the topography may be mimic the luminal surface or the external, non-luminal surface. A tubular composite is initially electrospun with corrugations on the external surface, which may be suitable for use as a GI tissue scaffold. If the composite is inverted inside out, this provides for the placement of the corrugations on the internal or luminal side. This orientation mimics organization of the villi characteristics of the lumen of the small intestine. Providing a single tubular composite whose topographical features recapitulate GI tissue, e.g., esophagus or small intestine, provides a strategy to facilitate the application of the GI constructs described herein to GI-related disorders. The GI represents a concentrically organized tubular composite with histological substructure and topographical features vary by spatial location. Tissue engineering approaches for regeneration of components of the GI have generally focused on replacement or augmentation using materials such as SIS, silicone, or PVDF that are typically not seeded with SMCs, epithelial, or other cell types. Such approaches have had limited or variable success as measured by regeneration of concentrically organized musculature and epithelial layers (Jwo et al. 2008, Brit. J. Surgery 95:657-663; Jansen PL et al., 2004, Eur Surg Res 36: 104-111; Ueno T et al., 2007, J Gastrointest Surg 11: 918-922; Hoeppner J et al., 2009, J Gastrointest Surg 13:113-119; Kaihara Set al" 2000, 69(9): 1927-1932; Nakase Y et al., 2008 *supra;* Takimoto Yet al., 1993, ASAIO J 39: M736-739; Lopes MF et al., 2006, Dis Esophagus 19: 254-259; Gonzalez-Saez LA et al., 2003, Eur Surg Res 35: 372-376; Ansaloni L et al., 2006, Trans. Proc 38: 1844-1848; Pekmezci Set al., 1997, Turk J Med Res 15; Saxena AK et al., 2009, J Ped Surg 44: 896-901; Greikscheit TC et al., 2004, Ann Surg 240). In contrast, the present invention contemplates an approach for regeneration of the gastrointestinal tract that leverages the foundational platform of smooth muscle cell seeded biomaterial that has demonstrated success in regeneration of the bladder and bladder derivatives (Jayo MJ et al., 2008: Regen Med 3:671-682). Electrospinning is a process for the creation of biomaterials using an electrically charged jet of polymer solution (Ramakrishna S, et al., 2005: An introduction to electrospinning and nanofibers. World Scientific Publishing Co.). Numerous biodegradable and non-biodegradable polymers may be electrospun into tubular composites suitable for application in the regeneration of tubular organ systems including components of the GI tract, e.g., esophagus, intestine, colon, or anal sphincter. Examples of such polymers include, but are not limited to, PGA, PLCL and polyurethane. Such materials are well-established to support cell migration and proliferation (Ramakrishna S, et al., 2005 *supra).*

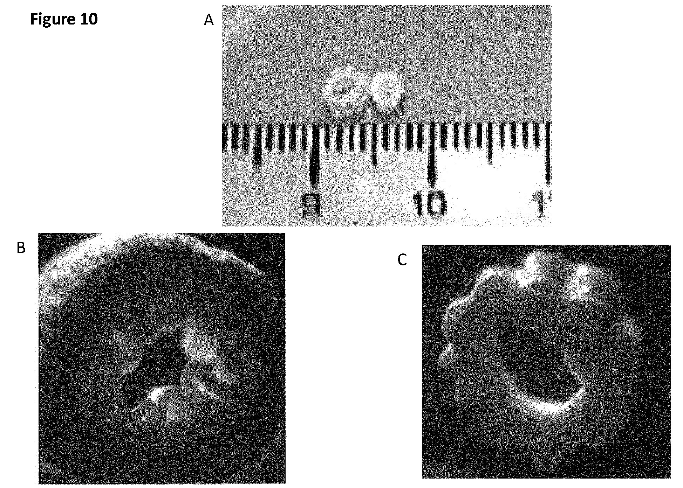

Strategies have been developed for the reconstitution of topographical features characteristic of certain organ systems based on iterative electrospinning protocols (see Rapoport et al. U.S. Published Patent Application No. 20090227026, incorporated herein by reference in its entirety). Briefly, a binary electrospinning methodology may be used wherein a first round of electrospinning is used to create a tube around a mandrel of defined diameter. This tubular construct is then forcibly expanded by insertion of multiple additional mandrels to increase its working diameter several-fold. The expanded tubular construct is then used as a template for a second round of electrospinning. Removal of all mandrels from the lumen of the tubular composite then results in reversion of the tubular construct to its original diameter. Consequently, excess electrospun material from the second layer is folded into a series of ruffles or corrugations protruding from the external, non-luminal surface of the tubular composite. Such a tubular construct may be appropriate for application in the regeneration of the esophagus or small intestine, such as for example, as shown in Figure 10A-C.

Inversion of the tubular construct reorients the surface corrugations towards the internal, luminal surface to create a luminai topography recapitulating the villi characteristic of the luminal surface of the small intestine. In this way, a single electrospun tubular composite may have dual application for regeneration of either esophagus or small intestine based on whether its topographical features are oriented towards the lumen or the external surface. The tube with external corrugations may be turned inside-out to create a luminal surface ruffling resembling the villi of the small intestine. Thus, a single tubular composite with surface corrugations may at least be applied towards regeneration of either esophagus (corrugations on external surface as illustrated in Figure 10C) or small intestine (corrugations on internal, luminal surface as illustrated in Figure 10B).

### E. Respiratory tissue scaffolds

The present disclosure provides scaffolds suitable for the formation of a respiratory tissue construct, e.g., a lung tissue scaffold. In one aspect, the polymeric matrix or scaffold of the present invention is suitable for implantation at, on, or into a part of the lung. In one aspect, the polymeric matrix comprises a polymeric matrix or scaffold shaped to conform to at least a part of the native respiratory tissue in need of treatment. Suitable native respiratory tissue include, without limitation, lung, alveolar tissue, and bronchiolar tissue.

In all embodiments, the respiratory tissue scaffold is implantable, e.g., into the lung. In all embodiments, the scaffold is biodegradable. In all embodiments, the scaffold is biocompatible. The scaffolds are suitable for seeding of a first cell population and/or a second cell population. In all embodiments, the first cell population is an adipose-derived smooth muscle cell population. In all embodiments, the second cell population is a respiratory cell population. In all embodiments, the second cell population is derived from lung. In all embodiments, the construct is a respiratory tissue construct. In all embodiments, the construct is positive for at least one smooth muscle cell marker. In all embodiments, the construct is positive for at least one respiratory tissue marker. In all embodiments, the respiratory tissue marker is one or more of the following: a bronchiolar marker, an alveolar marker, and an epithelial marker. In all embodiments, the construct includes one or more alveolar forming units (AFUs). In all embodiments, the construct includes cells having coordinated rhythmic contractile function. In all embodiments, the construct is adapted to form respiratory tissue following implantation. In all embodiments, the respiratory tissue is lung tissue. In all embodiments, the lung tissue includes alveolar tissue and/or bronchiolar tissue.

### F. Blood vessel scaffolds

As described in Rapoprt et al. U.S. Published Application No. 20090227026, native blood vessels have a multi-layered of laminated structure and specialized architectural features (undulations, corrugations, kinks) facilitate parallel arrangements of collagen and elastin lamina being mechanically engaged to differing degrees at differing strains. The typical observation in native arteries are corrugations in elastic laminae but no corrugations in surrounding collagen layers. An exception to this is an unusual architecture documented in fin whales, where a novel connective tissue design is present in which the collagenous component, which happens to be the tensile element, is highly corrugated (Gosline & Shadwick (1998) American Scientist. 86:535-541)). As described in Rapoport et al., tissue engineering scaffolds and methods of making the same can take a reverse approach to what is typically seen in native arteries, that is, the tensile layer of the scaffold has corrugations but not the elastic layer. This approach is advantageous because it is easier to impart corrugations within a tensile layer than it is to impart them in an elastic layer.

Blood vessel scaffolds suitable for use according to the present disclosure may have a mutli-layered or laminated structure. In one embodiment, the scaffold includes (a) a first tubular element that contains an elastomeric element, an exterior surface and an interior luminal surface; and (b) a second tubular element that contains a tensile element, an exterior surface and an interior luminal surface in contact with the exterior surface of the first tubular element.

In another embodiment, the second tubular element is corrugated. The corrugations present in the tissue engineering scaffolds described herein are exemplified in Rapoport et al. U.S. Published Application No. 20090227026 showing their appearance on the outer surface of the scaffolds.

In other embodiments, the corrugated second tubular element has a fibrous network in which the fiber direction is oriented circumferentially.

Additional tubular elements may be added over the first and second tubular elements.

The interior luminal surface of the first tubular element and the exterior surface of the second tubular element are both accessible for further manipulation, such as, for example in the formation of a tissue engineered blood vessel (TEBV). As described herein, the blood vessel scaffolds of the present disclosure may be used to make TEBVs by incorporating one or more cell populations into the scaffold. The laminated construction of the scaffolds provides a more natural vessel morphology which might facilitate the expected partitioning of cell populations, such as smooth muscle cells, endothelial cells, and fibroblasts.

The elastomeric element of the scaffolds described herein confers to the scaffold an ability to respond to stress with large-scale deformations that are fully recoverable and repeatable. The elastomeric elements have an elastomeric component that may be a natural component, a synthetic component, a mixture of more than one natural component, a mixture of more than one synthetic component, a mixture of natural and synthetic components, or any combination thereof. In general, an organic or natural component is a protein that is normally present in native tissue structures, or can be derived from native tissue structures, or can be produced recombinantly or synthetically based on the known nucleic acid sequence encoding the protein and/or its amino acid sequence. For example, elastin is naturally present in arteries and may be utilized as a natural component in the blood vessel scaffolds of the present invention. A natural component may be part of a blood vessel scaffold and/or a TEBV, as described herein, that also includes or does not include a synthetic component.

In some embodiments, the elastomeric element of the first tubular element includes an organic or natural component, such as an elastic protein, including without limitation, elastin, gluten, gliadin, abduction, spider silks, and resilin or pro-resilin (Elvin et al. (2005) Nature. Oct 12:437(7061):999-1002). Those of ordinary skill in the art will appreciate other natural elastic proteins that may be suitable for use in the scaffolds of the present invention.

The use of natural materials provides an advantage when the intact blood vessel scaffold is subjected to further manipulation for the purpose of constructing a tissue engineered blood vessel. For example, when a particular cell population is cultured on or seeded on the scaffold, the natural elastin protein present in the scaffold encourages proper cell interaction with the scaffold.

In other embodiments, the elastomeric element includes a synthetic component. Examples of synthetic elastomeric components, include without limitation, latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU).

In one embodiment, the present disclosure contemplates first tubular elements in which the elastomeric element includes a natural elastic component and a synthetic elastic component.

The tensile element of the scaffolds described herein confers to the scaffold rigidity or tensility that allows the scaffold to resist elongation in response to stress. The tensile elements have a tensile component that may be a natural component, a synthetic component, a mixture of more than one natural component, a mixture of more than one synthetic component, a mixture of natural and synthetic components, or any combination thereof.

In another embodiment, the tensile element of the second tubular element comprises an organic or natural component, such as a fibrous protein, including without limitation, collagen, cellulose, silk, and keratin. Those of ordinary skill in the art will appreciate other natural fibrous proteins that may be suitable for use according to the scaffolds of the present disclosure. In other embodiments, the tensile element is a synthetic component. Examples of synthetic tensile components, include without limitation, nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU). In one disclosure embodiment, the present disclosure contemplates second tubular elements in which the tensile element includes a natural tensile component and a synthetic tensile component.

The elastomeric and tensile elements of the scaffolds may contain different combinations of natural and synthetic components. For example, a scaffold may contain a natural elastic component and/or a natural tensile component, and a synthetic elastic component and/or a synthetic tensile component.

In one aspect of the present disclosure, the TE scaffolds are not limited to a two layer structure having a second tubular element over a first tubular element, as described above. In some embodiments, the scaffolds include additional tubular elements, such as a third tubular element over the second tubular element, a fourth tubular element over the third tubular element, a fifth tubular element over the fourth tubular element, etc. In addition, as described herein, the additional tubular elements may contain an elastomeric element(s) (e.g. natural and/or synthetic) or a tensile element(s) (e.g. natural and/or synthetic). The additional tubular elements may be bonded by the techniques described herein.

In one aspect, the elastomeric component contained in the elastomeric element and the tensile component contained in the tensile element each have a different elastic modulus. In one embodiment, the elastic modulus of the elastomeric component of the elastomeric element has a first elastic modulus and the tensile component of the tensile element has a second elastic modulus. In a preferred embodiment, the second elastic modulus is greater than the first elastic modulus by at least about one order of magnitude. In one embodiment, the second elastic modulus is greater than the first elastic modules by about one order of magnitude, about two orders of magnitude, about three orders of magnitude, about four orders of magnitude, or additional orders of magnitude. For instance, Example 1 of Rapoport et al. U.S. Published Application No. 20090227026 shows the tensile components PDO and Vicryl to have elastic moduli of 3 GPa and 9-18 GPa, respectively, as compared to the 0.3 MPa to 0.5 MPa elastic modulus of the elastomeric component latex.

In another aspect, the TE scaffolds of the present disclosure exhibit structural and functional properties substantially similar to those found in native blood vessels. Those of ordinary skill in the art will appreciate the numerous parameters that can be used to demonstrate that the scaffolds of the present invention mimic or closely resemble native blood vessels, including without limitation, a response to stress and strain, compliance, Young's modulus, porosity, strength, etc. In one embodiment, the scaffolds of the present invention are characterized by having the ability to respond mechanically to stress and strain in an anisotropic manner. Those of ordinary skill in the art will appreciate that there are a number of well-recognized parameters in the art are useful for characterizing the behavior of tissue engineering scaffolds (Rapoport et al. U.S. Published Application No. 20090227026). Such parameters are useful in characterizing the mechanical behavior of a tissue engineering scaffold of the present invention, and in particular, in determining whether the scaffold will exhibit properties substantially similar to that of a native blood vessel.

Table 1 provides characterization specifications based upon the literature that project to provide mechanical properties to a scaffold or TEBV that are substantially similar to a native blood vessel. The present disclosure is directed to tissue engineering scaffolds that are characterized by the values of Table 1 and that exhibit mechanical properties substantially similar to those of a native blood vessel, preferably (i) a mechanical response to stress and strain characterized by a J-shaped stress/strain curve; (ii) resistance to fracturing; (iii) viscoelasticity; or (iv) any combination of (i)-(iii). In addition, the scaffolds are characterized by accessibility to various cell types for the purpose of cell seeding to form a TEBV.

**Table 1**

| **Test** | **Parameter** | **Value** |
|---|---|---|
| **Material** | Wall Thickness (µm) | 600 - 1200 |
| | Porosity (%) | 90 - 99 |
| | Pore Diameter (µm) | 5 - 100 |
| | | Adventitial side pore size |
| | | ∼100µm to luminal pore size of |
| | Pore Gradient (µm) | ∼5µm to ∼15µm. |
| | Fiber diameter (µm) | 0.05 - 20 |
| **Tube-Circumferential** | Breaking Strain (1/1) | 1.1 - 1.5 |
| | Breaking Stress MPa) | 1.5 - 3.5 |
| | Elastic Modulus 1 (MPa) | 0.1 - 0.5 |
| | Elastic Modulus 2 (MPa) | 3.0 - 6.0 |
| | Modulus 1 to Modulus 2 Transition | 0.57 - 1.12 |
| | Toughness (MJ/m³) | 0.45 - 1.0 |
| **Tube-Axial** | Breaking Strain (1/1) | >0.8 |
| | Breaking Stress (MPa) | >0.75 |
| | Elastic Modulus 1 (MPa) | 0.1 - 0.3 |
| | Elastic Modulus 2 (MPa) | 1.0 - 6.0 |
| | Modulus 1 to Modulus 2 Transition | 0.64 - 0.80 |
| | Toughness (MJ/m³) | 0.1 - 0.5 |
| **Tube-Viscoelastic Properties** | Tan Delta | 0.05 - 0.3 |
| | Storage Modulus (MPa) | 400 - 0.12 |
| **Vessel** | Burst Pressure (mm-Hg) | 1300-2000 |
| | Compliance (%/100 mm-Hg) | 2.5 - 5.0 |
| | Kink Radius (mm) | 5-12 |

In one embodiment, the characteristic of a J-shaped stress/strain curve exhibited by the tissue engineering scaffolds of the present invention is attributable to (i) a circumferential tube elastic modulus 1 of about 0.1 MPa to about 0.5 MPa, (ii) a circumferential tube elastic modulus 2 of about 3.0 MPa to about 6.0 MPa; and (iii) a circumferential modulus transition of about 0.57 to about 1.12, and any combination thereof. In another embodiment, the circumferential tube elastic modulus 1 is about 0.1 MPa, 0.13 MPa, about 0.15 MPa, about 0.17 MPa, about 0.2 MPa, about 0.22 MPa, about 0.25 MPa, about 0.27 MPa, about 0.3 MPa, about 0.32 MPa, about 0.35 MPa, about 0.37 MPa, about 0.4 MPa, about 0.42 MPa, about 0.45 MPa, about 0.47 MPa, or about 0.5 MPa. In another embodiment, the circumferential tube elastic modulus 2 is about 3.0 MPa, about 3.2 MPa, about 3.5 MPa, about 3.7 MPa, about 4.0 MPa, about 4.2 MPa, about 4.5 MPa, about 4.7 MPa, about 5.0 MPa, about 5.2 MPa, about 5.5 MPa, about 5.7 MPa, or about 6.0 MPa. In another embodiment, the circumferential modulus transition is about 0.57, about 0.59, about 0.61, about 0.63, about 0.65, about 0.67, about 0.69, about 0.71, about 0.73, about 0.75, about 0.77, about 0.79, about 0.81, about 0.83, about 0.85, about 0.87, about 0.89, about 0.91, about 0.93, about 0.95, about 0.97, about 0.99, about 1.01, about 1.03, about 1.05, about 1.07, about 1.09, about 1.11, or about 1.12.

In another embodiment, the property favoring resistance to fracture is (i) a circumferential tube toughness of about 0.45 MJ/m³ to about 1.0 MJ/m³; (ii) an axial tube toughness of about 0.1 MJ/m³ to about 0.5 MJ/m³; or (iii) a combination of (i) and (ii). The toughness of a biomaterial is one parameter that helps determine its resistance to fracture. Clearly, the resistance to fracturing or tearing is a desired feature in a TE scaffold because it helps ensure the patency of any TEBV or vascular graft derived therefrom. Native blood vessels are subject to deformation in response to the stress and strain of cyclic loading of fluid. As such, they are at risk for a split or fracture in a longitudinal or axial manner and/or a circumferential manner. Similar to native blood vessels, the vascular grafts derived from the TE scaffolds and TEBVs of the present disclosure are also at risk for a fracture. The present disclosure concerns the discovery that a particular axial toughness and/or a particular circumferential toughness contributes to a TE scaffold that is resistant to fracture or tearing. In one embodiment, the circumferential tube toughness is about 0.45 MJ/m³, about 0.50 MJ/m³, about 0.55 MJ/m³ , about 0.60 MJ/m³, about 0.65 MJ/m³, about 0.70 MJ/m³, about 0.75 MJ/m³, about 0.80 MJ/m³, about 0.85 MJ/m³, about 0.90 MJ/m³, about 0.95 MJ/m³, about 1.0 MJ/m³. In another embodiment, the axial tube toughness is about 0.1 MJ/m³ about 0.15 MJ/m³, about 0.20 MJ/m³, about 0.25 MJ/m³, about 0.30 MJ/m³, about 0.35 MJ/m³, about 0.40 MJ/m³, about 0.45 MJ/m³, or about 0.50 MJ/m³. In another embodiment, the TE scaffolds of the present invention are characterized by one or more of: i) a scaffold which has a mechanical response to stress and strain characterized by a J-shaped stress/strain curve; ii) a fracture-resistant scaffold; and iii) a viscoelastic scaffold.

In another embodiment, the viscoelastic properties of a TE scaffold are characterized by (i) a tangent delta of about 0.05 to about 0.3; (ii) a storage modulus of about 400 MPa to about 0.12 MPa; or (iii) a combination of (i) and (ii). Viscoelastic materials exhibit both viscous and elastic characteristics in response to deformation. While viscous materials resist strain linearly with time when stress is applied, elastic materials strain instantly in response to stress and rapidly return to their original state once the stress is removed. A viscoelastic material exhibits a time-dependent strain in response to stress, which typically involves the diffusion of atoms or molecules within an amorphous material. As native blood vessels display viscoelasticity to cope with the cyclic loading of fluid, this trait is desirable for the TE scaffolds of the present invention that will be used to create a TEBV or vascular graft. The present invention concerns the discovery that the viscoelasticity of a TE scaffold of the present invention is characterized by a particular tangent delta value and/or a particular storage modulus value. In one embodiment, the tangent delta is about 0.05, about 0.06, about 0.07, about 0.08, about 0.09, about 0.10, about 0.11, about 0.12, about 0.13, about 0.14, about 0.15, about 0.16, about 0.17, about 0.18, about 0.19, about 0.20, about 0.21, about 0.22, about 0.23, about 0.24, about 0.25, about 0.26, about 0.27, about 0.28, about 0.29, or about 0.30. In other embodiments, the storage modulus is about 400 MPa, about 350 MPa, about 300 MPa, about 250 MPa, about 200 MPa, about 150 MPa, about 100 MPa, about 90 MPa, about 80 MPa, about 70 MPa, about 60 MPa, about 50 MPa, about 40 MPa, about 30 MPa, about 20 MPa, about 10 MPa, about 9 MPa, about 8 MPa, about 7 MPa, about 6 MPa, about 5 MPa, about 4 MPa, about 3 MPa, about 2 MPa, about 1 MPa, about 0.9 MPa, about 0.8 MPa, about 0.7 MPa, about 0.6 MPa, about 0.5 MPa, about 0.4 MPa, about 0.3 MPa, about 0.2 MPa, about 0.19 MPa, about 0.18 MPa, about 0.17 MPa, about 0.16 MPa, about 0.15 MPa, about 0.14 MPa, about 0.13 MPa, or about 0.12 MPa.

There are several techniques well-known to those of ordinary skill in the art that are suitable for identifying and characterizing the desirable properties for the scaffolds of the present disclosure. These techniques include, without limitation, burst pressure testing; quasi-static mechanical testing (a.k.a. tensile testing) in the circumferential direction (results provided in a stress/strain diagram); determining porosity and pore size (e.g. by mercury intrusion porosimetry); cell attachment assays; and degradation rate; pressure/volume curves for measurement of graft compliance.

### 5. Constructs

In one aspect, the invention provides one or more polymeric scaffolds or matrices that are seeded with at least one cell population. Such scaffolds that have been seeded with a cell population and may be referred to herein as "constructs". In one embodiment, the cell-seeded polymeric matrix or matrices form a construct that is shaped or adapted to conform to at least a part of a native luminal organ or tissue structure in a subject in need of such a construct. The native luminal organ or tissue structure may be laminarly organized.

Cell-seeded polymeric matrix or matrices can form a neo-bladder construct selected from the group consisting of a bladder replacement construct, a bladder augmentation construct, a bladder conduit construct, and a detrusor muscle equivalent construct. In addition, other cell-seeded polymeric matrices are provided that form a construct selected from the group consisting of a respiratory tissue construct, a gastrointestinal tissue construct, a neo-blood vessel construct, and an ocular tissue construct.

Those of skill in the art will appreciate that the seeding or deposition of one or more cell populations described herein may be achieved by various methods known in the art. For example, bioreactor incubation and culturing, (Bertram et al. U.S. Published Application 20070276507; McAllister et al. U.S. Pat. No. 7,112,218; Auger et al. U.S. Pat. No. 5,618,718; Niklason et al. U.S. Pat. No. 6,537,567); pressure-induced seeding (Torigoe et al. (2007) Cell Transplant., 16(7):729-39; Wang et al. (2006) Biomaterials. May; 27(13):2738-46); and electrostatic seeding (Bowlin et al. U.S. Pat. No. 5,723,324, each of which is incorporate herein by reference in its entirety) may be used. In addition, a recent technique that simultaneously coats electrospun fibers with an aerosol of cells may be suitable for seeding or deposition (Stankus et al. (2007) Biomaterials, 28:2738-2746).

In one other aspect, the invention provides scaffolds seeded with cells at particular cell densities for any of the constructs described herein. In one embodiment, a scaffold is seeded with a smooth muscle cell population at a cell density of about 20 x 10⁶ to about 30 x 10⁶ cells. In another embodiment, the cell density is about 1 x 10⁶ to about 40 x 10⁶, about 1 x 10⁶ to about 30 x 10⁶, about 1 x 10⁶ to about 20 x 10⁶, about 1 x 10⁶ to about 10 x 10⁶, or about 1 x 10⁶ to about 5 x 10⁶. In a further embodiment, the density is about 20 x 10⁶ to about 98 x 10⁶ cells. In yet further embodiments, the density is about 21 x 10⁶ to about 97 x 10⁶, about 22 x 10⁶ to about 95 x 10⁶, about 23 x 10⁶ to about 93 x 10⁶, about 24 x 10⁶ to about 91 x 10⁶, about 25 x 10⁶ to about 89 x 10⁶, about 26 x 10⁶ to about 87 x 10⁶, about 28 x 10⁶ to about 85 x 10⁶, about 29 x 10⁶ to about 83 x 10⁶, about 30 x 10⁶ to about 80 x 10⁶, about 35 x 10⁶ to about 75 x 10⁶, about 40 x 10⁶ to about 70 x 10⁶, about 45 x 10⁶ to about 65 x 10⁶, or about 50 x 10⁶ to about 60 x 10⁶. In a preferred embodiment, the density is about 24 x 10⁶ to about 91 x 10⁶ cells. In another embodiment, the density is about 2.5 x 10⁶ to about 40 x 10⁶, about 5 x 10⁶ to about 40 x 10⁶, about 7.5 x 10⁶ to about 35 x 10⁶, about 10 x 10⁶ to about 30 x 10⁶, about 15 x 10⁶ to about 25 x 10⁶, and about 17.5 x 10⁶ to about 22.5 x 10⁶. In another embodiment, the cell density is about 1 x 10⁶, about 2 x 10⁶, about 3 x 10⁶, about 4 x 10⁶, about 5 x 10⁶, about 6 x 10⁶, about 7 x 10⁶, about 8 x 10⁶, about 9 x 10⁶, about 10 x 10⁶, about 11 x 10⁶, about 12 x 10⁶, about 13 x 10⁶, about 14 x 10⁶, about 15 x 10⁶, about 16 x 10⁶, about 17 x 10⁶, about 18 x 10⁶, about 19 x 10⁶, about 20 x 10⁶, about 21 x 10⁶, about 22 x 10⁶, about 23 x 10⁶, about 24 x 10⁶, about 25 x 10⁶, about 26 x 10⁶, about 27 x 10⁶, about 28 x 10⁶, about 29 x 10⁶, about 30 x 10⁶, about 31 x 10⁶, about 32 x 10⁶, about 33 x 10⁶, about 34 x 10⁶, about 35 x 10⁶, about 36 x 10⁶, about 37 x 10⁶, about 38 x 10⁶, about 39 x 10⁶, about 40 x 10⁶, about 41 x 10⁶, about 42 x 10⁶, about 43 x 10⁶, about 44 x 10⁶, about 45 x 10⁶, about 46 x 10⁶, about 47 x 10⁶, about 48 x 10⁶, about 49 x 10⁶, about 50 x 10⁶, about 51 x 10⁶, about 52 x 10⁶, about 53 x 10⁶, about 54 x 10⁶, about 55 x 10⁶, about 56 x 10⁶, about 57 x 10⁶, about 58 x 10⁶, about 59 x 10⁶, about 60 x 10⁶, about 61 x 10⁶, about 62 x 10⁶, about 63 x 10⁶, about 64 x 10⁶, about 65 x 10⁶, about 66 x 10⁶, about 67 x 10⁶, about 68 x 10⁶, about 69 x 10⁶, about 70 x 10⁶, about 71 x 10⁶, about 72 x 10⁶, about 73 x 10⁶, about 74 x 10⁶, about 75 x 10⁶, about 76 x 10⁶, about 77 x 10⁶, about 78 x 10⁶, about 79 x 10⁶, about 80 x 10⁶, about 81 x 10⁶ about 82 x 10⁶, about 83 x 10⁶, about 84 x 10⁶, about 85 x 10⁶, about 86 x 10⁶, about 87 x 10⁶, about 88 x 10⁶, about 89 x 10⁶, about 90 x 10⁶, about 91 x 10⁶, about 92 x 10⁶, about93 x 10⁶, about 94 x 10⁶, about 95 x 10⁶, about 96 x 10⁶, about 97 x 10⁶, about 98 x 10⁶, or about 99 x 10⁶.

In a further aspect, the invention provides scaffolds seeded with cells at particular cell densities per cm² of a scaffold. In one embodiment, the density is about 3,000 cells/cm² to about 15,000 cells/cm², about 3,500 cells/cm² to about 14,500 cells/cm², about 4,000 cells/cm² to about 14,000 cells/cm², about 4,500 cells/cm² to about 13,500 cells/cm², about 5,000 cells/cm² to about 13,000 cells/cm², about 4,500 cells/cm² to about 13,500 cells/cm², about 5,000 cells/cm² to about 13,000 cells/cm², about 5,500 cells/cm² to about 12,500 cells/cm², about 6,000 cells/cm² to about 12,000 cells/cm², about 6,500 cells/cm² to about 11,500 cells/cm², about 7,000 cells/cm² to about 11,000 cells/cm², about 7,500 cells/cm² to about 10,500 cells/cm², about 8,000 cells/cm² to about 10,000 cells/cm², about 7,500 cells/cm² to about 9,500 cells/cm², or about 8,000 cells/cm² to about 9,000 cells/cm². In a preferred embodiment, the density is about 3,000 cells/cm² to about 7,000 cells/cm², or about 9,000 cells/cm² to about 15,000 cells/cm². In a preferred embodiment, the density is about 9.5 x 10⁴ cells/cm², about 10 x 10⁴ cells/cm², about 10.5 x 10⁴ cells/cm², about 11 x 10⁴ cells/cm², about 11.5 x 10⁴ cells/cm², about 12 x 10⁴ cells/cm², about 12.5 x 10⁴ cells/cm², about 13 x 10⁴ cells/cm², about 13.5 x 10⁴ cells/cm², about 14 x 10⁴ cells/cm², about 14.5 x 10⁴ cells/cm², or about 15 x 10⁴ cells/cm².

In one embodiment, the deposition of cells includes the step of contacting a scaffold with a cell attachment enhancing protein. In another embodiment, the enhancing protein is one or more of the following: fibronection, collagen, and MATRIGEL™. In one other embodiment, the scaffold is free of a cell attachment enhancing protein. In another embodiment, the deposition of cells includes the step of culturing after contacting a scaffold with a cell population. In yet another embodiment, the culturing may include conditioning by pulsatile and/or steady flow in a bioreactor.

Smooth muscle cell populations isolated from adipose or peripheral blood as described herein may then be seeded on a scaffold described herein to form a construct.

The following is a representative example of a protocol for seeding cells on a scaffold. Adipose- or peripheral blood-derived smooth muscle cells may be expanded for up to 7 weeks to generate the quantity of cells required for seeding a scaffold. The density of cells suitable for seeding a scaffold is described below. Adipose-derived smooth muscle cells may be expanded for 2 passages before harvesting of cells for seeding of scaffolds to produce a construct. Peripheral blood-derived smooth muscle cell cultures may be expanded to P3-4 before harvesting for scaffold seeding. To prepare a scaffold for cell seeding, a suitable material (e.g., PGA felt) may be cut cut to size, sutured into the appropriate shape, and coated with material (e.g., PLGA). The scaffold may then be sterilized using a suitable method (e.g., ethylene oxide). On the day prior to cell seeding, the sterilized scaffold may be serially pre-wetted by saturation with 60% methanol/40% D-PBS, 100% D-PBS, D-MEM/10% FBS or α-MEM/10% FBS followed by incubation in D-MEM/10% FBS or α-MEM/10% FBS at room temperature overnight. The scaffold can then be seeded with adipose-, or peripheral blood-derived smooth muscle cells and the seeded construct matured in a humidified 37oC incubator at 5% CO2 until implantation in a subject (e.g., by day 7). Those of ordinary skill in the art will appreciate additional methods for preparing scaffolds for seeding of cells and seeding of cells onto scaffolds.

In one aspect, the present invention provides methods of preparing a construct in a reduced time frame, which is advantageous to the subject awaiting implantation of a construct. It has been reported that undifferentiated adipose stem cells derived from SVF must be incubated in inductive media for 6 weeks prior to differentiation into smooth muscle cells (Jack et al. 2009 supra). In one embodiment, the method includes the steps of a) obtaining a human adipose tissue sample; b) isolating a fully differentiated smooth muscle cell population from the sample; c) culturing the cell population; and d) contacting the cell population with a shaped polymeric matrix cell construct, wherein steps a), b), c) and d) are performed in about 45 days or less. In another embodiment, the isolating step is performed without cell selection. In another embodiment, the isolating step b) is performed about 72 hours or less after obtaining step a). In yet another embodiment, the culturing step c) is performed in about 4 weeks or less. In other embodiments, the contacting step d) is performed in about 10 days or less. In another embodiment, steps a), b), c) and d) are performed in about 28 days or less. In one other embodiment, the isolating step b) is performed about 48 hours or less after obtaining step a). In one embodiment, the culturing step c) is performed in about 2 weeks or less. In another embodiment, the contacting step d) is performed in about 5 days or less. In all embodiments, the human adipose tissue sample is obtained from a non-autologous source. In one other embodiment, the method further includes the step of detecting expression of a smooth muscle cell marker. In another embodiment, expression is mRNA expression. In a further embodiment, the expression is polypeptide expression. In one embodiment, the polypeptide expression is detected by intracellular immunoflourescence.

In one embodiment, the scaffold comprises a cell population as described herein. In another embodiment, the scaffold consists essentially of a cell population as described herein. In one other embodiment, the scaffold consists of a cell population as described herein.

### A. Urinary system constructs

The present disclosure provides constructs for use in the reconstruction, replacement, augmentation, or regeneration of native luminal organs or tissue structures of the urinary system. The organs or tissue structures of the urinary system may also be referred to as genitourinary or urogenital organs or tissue structures. The native organs or tissue structures may be laminarly organized.

In another embodiment, the construct containing the matrix and cells is free of any other cell populations. In a preferred embodiment, the construct is free of urothelial cells.

These constructs are used to provide a luminal organ or tissue structures such as genitourinary organs, including for example, the urinary bladder, ureters and urethra, to a subject in need. The subject may require the reconstruction, augmentation or replacement of such organs or tissues. In one embodiment, the luminal organ or tissue structure is a bladder or portion thereof, and the polymeric matrix or scaffold has smooth muscle cells deposited on a surface of the matrix. The constructs may also be used to provide a urinary diversion or conduit, or a detrusor muscle equivalent.

In one aspect, the disclosure provides scaffolds or matrices that are seeded with a cell population described herein.

As described herein, the bladder augmentation or replacement scaffolds described herein may include at least one, or at least two matrices. The matrices may be polymeric and/or biocompatible. The first polymeric matrix or the second polymeric matrix, if any, or both, will have at least one cell population deposited on or in a first surface off the first polymeric matrix, a first surface of the second polymeric matrix, or both, to form a construct of matrix or scaffold plus cells, wherein at least one cell population comprises substantially a muscle cell population. The muscle cell population is, e.g., a smooth muscle cell population. In another embodiment, the first surface and the second surface are each the outer surface of the first and second polymeric matrices.

The scaffolds or matrices for forming bladder conduit, urinary diversion or urinary conduit construct will be seeded with a cell population described herein. Such scaffolds that have been seeded with a cell population and may be referred to herein as "constructs". In one embodiment, the urinary diversion or bladder conduit construct is made up of one or more scaffolds as described herein and a cell population deposited on one or more surfaces of the one or more scaffolds as described herein.

Scaffolds or matrices for forming muscle equivalent constructs may be used to enhance an existing luminal organ or tissue structures such as genitourinary organs, including for example, the urinary bladder, to a subject in need. The subject may require expansion or treatment of such organs or tissues. In one embodiment, the luminal organ or tissue structure is a bladder or portion thereof, and the polymeric matrix or scaffold has smooth muscle cells deposited on a surface of the matrix. In one embodiment, the constructs are used to provide a detrusor muscle equivalent.

Those of ordinary skill in the art will appreciate there are several suitable methods for depositing cell populations upon matrices or scaffolds.

In one aspect, the constructs are suitable for implantation into a subject in need of a new organ or tissue structure. In one embodiment, the construct comprises a population of cells that produce the cytokine MCP-1. In another embodiment, the MCP-1 elicits the migration of the subject's or recipient's native mesenchymal stem cells to the site of implantation. In one embodiment, the migrating recipient native mesenchymal stem cells assist in the regeneration of the new organ or tissue structure.

In one aspect, the constructs of the present invention are adapted to provide particular features to the subject following implantation. In one embodiment, the constructs are adapted to provide regeneration to the subject following implantation. In another embodiment, the constructs are adapted to promote regeneration in a subject at the site of implantation. For example, following implantation, regenerated tissue may form from the construct itself at the site of implantation. In another embodiment, the construct may impart functional attributes to the subject following implantation. For example, a urinary diversion construct may be adapted to allow the passage of a subject's urine from a first ureter (e.g., first side opening) to the interior of the tubular scaffold, and/or adapted to provide temporary storage and passage of urine (e.g., tubular scaffold) out of a subject. In one embodiment, a urinary diversion construct may be adapted to provide an epithelialized mucosa upon implantation. In another embodiment, a construct may be adapted to provide homeostatic regulative development of a new organ or tissue structure in a subject.

In one aspect, the present invention provides a mesh structure adapted to be implanted at the site of connection between the first end of the tubular scaffold of the urinary diversion construct and the abdominal wall section, as described herein. In one embodiment, the mesh structure is adapted to faciliate formation of a neo-urinary conduit following implantation of a urinary diversion construct described herein. In another embodiment, the mesh structure is adapted to be implanted between subcutaneous fat and skeletal muscle. In one other embodiment, the mesh structure is adapted to provide stomal patency. In another embodiment, the mesh structure is located in a position adjunct to an opening in an abdominal wall (e.g., location of a stoma). In a preferred embodiment, the mesh structure is a hernia patch, more preferably a subcutaneous hernia patch.

The constructs for use with organs or tissue structures of the urinary system may be seeded with smooth muscle cell populations described herein. The SMC populations may be obtained from sources, e.g., adipose or peripheral blood, that are not the organ or tissue structure that is the subject of the reconstruction, replacement, augmentation, or regeneration, where the source is autologous or non-autologous, e.g., Allogeneic or syngeneic, to the subject in need of the construct.

In one other aspect, the present disclosure concerns constructs for use in the reconstruction, replacement, augmentation, or regeneration of native luminal organs or tissue structures of the urinary system that contain smooth muscle cells (SMCs) derived from a bladder source that is a non-autologous source. The non-autologous source may be an allogeneic source or a syngeneic source. The non-autologous bladder-derived SMCs may be seeded on a suitable scaffold according to the protocols described herein. In one preferred embodiment, the construct is formed from a scaffold and bladder-derived SMCs but is free of urothelial cells. The bladder-derived SMCs may be seeded onto a bladder augmentation, a bladder replacement, a urinary conduit, or a muscle equivalent scaffold to form a construct.

### B. Gastrointestinal tissue constructs

In one aspect, the invention provides one or more gastrointestinal (GI)-related polymeric scaffolds or matrices that are seeded with a cell population. Such GI scaffolds that have been seeded with at least one cell population and may be referred to herein as "constructs". The scaffold is seeded with a cell population that is not derived from gastrointestinal (GI) tissue source. The non-GI source may be adipose or peripheral blood. In another embodiment, the cell population is an SMC population. The scaffold may be substantially free of other cell populations including, for example, gastro-intestinal cell populations. The scaffold may comprise, consist of, or consist essentially of one cell population, *e.g*., an SMC population that is not derived from GI tissue. In another embodiment, the scaffold may be a GI tissue scaffold such as, for example, an esophageal tissue or an intestine scaffold.

In one embodiment, an autologous SMC cell population could be isolated from the adipose tissue or peripheral blood of a subject in need. The cell population could be seeded onto a scaffold suitable for implantation at a site within the GI system of the subject. An advantage of the cell populations of the present invention is that suitable SMCs may not be available for sourcing from the subject's GI system if the subject has a defective GI system, e.g., cancer of the GI system. The cell populations may be used in cases where part or all of a subject's GI system is removed, such as in the case of esophageal cancer. Upon removal of an esophagus or a part of an esophagus in a subject, an autologous SMC population could be isolated from an adipose biopsy, cultured, seeded on a suitable scaffold, and implanted into the subject to provide a new esophagus or new esophagus tissue structure.

In one other aspect, the invention provides one or more polymeric scaffolds or matrices that are seeded with an SMC population and a GI cell population. In one embodiment, the cell-seeded polymeric matrix or matrices form a gastro-intestinal (GI) tissue construct or a neo-GI construct.
In one other aspect, the present invention concerns constructs that are adapted to facilitate the deposition or seeding of GI cell populations through the use of an SMC population. It has been discovered that depositing or seeding an adipose-derived SMC population on to or in a GI tissue scaffold potentially enhances the subsequent seeding or deposition of a GI cell population. In one embodiment, the construct having a previously deposited SMC population facilitates the migration or seeding of a GI cell population on or in a surface of the scaffold. In another embodiment, construct with previously deposited SMC population facilitates the migration of a GI cell population to contact the scaffold and/or the deposited SMC population. The migration of the GI cell population may be *in vitro* or *in vivo.* In one other embodiment, the GI cell population is derived from one of: esophagus, small intestine, large intestine, stomach, colon, or anal sphincter.

Cells may be seeded on GI scaffolds according to the protocols described herein. Examples 11 and 13 provides exemplary methods of seeding cell populations on GI scaffolds. Those of ordinary skill in the art will appreciate additional methods for preparing scaffolds for seeding of cells and seeding of cells onto scaffolds.

In one embodiment, the GI scaffold comprises a cell population as described herein. In another embodiment, the GI scaffold comprises an adipose-derived SMC population and a GI cell population. The GI cell population may be an esophageal cell population or a small intestinal cell population. In another embodiment, the scaffold consists essentially of an adipose-derived SMC population and a GI cell population, as described herein. In one other embodiment, the scaffold consists of an adipose-derived SMC population and a GI cell population, as described herein.

In another aspect, the present invention provides constructs that are only seeded with a smooth muscle cell population. In one embodiment, the construct is only seeded with an adipose- or blood-derived SMC population only. Such constructs are free of any other gastro-intestinal cell population including, without limitation, an esophageal cell population (e.g., an esophageal epithelial cell population), a small intestinal cell population, a large intestinal cell population, a stomach cell population, a colon cell population, or anal sphincter cell population. In another embodiment, the construct is free of a fibroblast cell population, e.g., a dermal fibroblast cell population.

In another embodiment, the SMC-only construct is selected from an esophageal, small intestine, large intestine, stomach, colon, or anal sphincter construct. In one other embodiment, the construct comprises, consists of, or consists essentially of a scaffold and a smooth muscle cell population, e.g., an adipose- or blood-derived SMC population.

In one other aspect, the constructs of the invention may be provided with certain structural and functional features that make them particularly advantageous for the uses and methods of treatment described herein. In one embodiment, the constructs are made up of a scaffold provided as a matrix with a first surface and a cell population directly seeded on, or in the first surface. The construct may have only one cell population. In one embodiment, the one cell population is a smooth muscle cell population. The scaffold may be provided with corrugations on the external surface or the luminal surface, as described above. In another embodiment, the construct is made up of a scaffold seeded with a smooth muscle cell (SMC) population, where the construct has the functional ability to attract or facilitate the migration of another cell population onto the SMC-seeded construct. Example 13 provides a demonstration of this functional ability. The migratory cell population may be an esophageal cell population, a small intestinal cell population, a large intestinal cell population, a stomach cell population, a colon cell population, or anal sphincter cell population.

In one other embodiment, the migratory cell population may be an esophageal epithelial cell population. An intact, native esophagus consists of an inner luminal layer of epithelial cells. It is advantageous for an SMC-seeded construct that is not seeded with epithelial cells to have the functional ability to attract or facilitate the migration of epithelial cells from the native esophageal tissue onto or in the construct, prior to implantation into a subject.

In another embodiment, a first scaffold or polymeric matrix (and/or second scaffold or polymeric matrix, if any, or both) comprise at least one SMC population deposited on or in a first surface of the first polymeric matrix (and/or a first surface of the second polymeric matrix, or both) to form a construct of matrix or scaffold plus cells, wherein at least one cell population comprises an SMC population. The SMC is an adipose- or blood-derived smooth muscle cell population.

In one aspect, the constructs described herein are adapted to or capable of provide a regenerated GI organ or GI tissue to a subject following implantation of the construct. Aspects of the regenerating/regenerated GI organ or GI tissue are described in Examples 8 (esophagus) and 9 (small intestine).

In one aspect, the present invention provides constructs for treating gastro-intestinal (GI) disorders in a subject in need, and methods of treatment using the same. In one embodiment, the construct includes (a) a scaffold; (b) a first cell population that is not derived from gastro-intestinal tissue deposited on or in a first surface of the scaffold; and (c) a second cell population derived from gastro-intestinal tissue. The first cell population may derived from adipose. The first cell population may be a smooth muscle cell population. In another embodiment, the SMC population may be positive for at least one smooth muscle cell marker. In one other embodiment, the second cell population is derived from esophagus, small intestine, large intestine, stomach, colon, or anal sphincter.

In yet another embodiment, the construct is positive for at least one GI marker. The GI marker may be an epithelial cell marker.

In one embodiment, the second cell population is deposited on or in a second surface of the scaffold. In another embodiment, the second cell population is in contact with the deposited first cell population.

In one other embodiment, the construct includes cells having coordinated rhythmic contractile function. In another embodiment, the construct is adapted to form gastro-intestinal tissue following implantation. The GI tissue may be esophagus, small intestine, large intestine, stomach, colon, or anal sphincter.

In another embodiment, the construct forms regenerated GI tissue upon implantation. In one other embodiment, the regenerated GI tissue is esophagus, small intestine, large intestine, stomach, colon, or anal sphincter tissue. In another embodiment, the regenerated tissue is esophagus tissue. The regenerated esophagus tissue may have luminal mucosal surface and/or lamina propria and muscularis mucosa. In a preferred embodiment, the regenerated GI tissue is esophagus tissue. In another embodiment, the construct is adapted to form an epithelialized luminal mucosal surface upon implantation.

In all embodiments, the scaffold is a GI tissue scaffold. In all embodiments, the GI tissue scaffold is an esophagus tissue scaffold, a small intestine tissue scaffold, a large intestine tissue scaffold, a stomach tissue scaffold, a colon tissue scaffold, or an anal sphincter tissue scaffold.

In all embodiments, the construct is a GI tissue construct. In all embodiments, the GI tissue construct is an esophagus tissue construct, a small intestine tissue construct, a large intestine tissue construct, a stomach tissue construct, a colon tissue construct, or an anal sphincter tissue construct.

### C. Respiratory tissue constructs

In one aspect, the disclosure provides respiratory tissue constructs that are seeded with a cell population described herein. Such scaffolds that have been seeded with a cell population and may be referred to herein as "constructs". In one embodiment, the respiratory tissue construct is made up of one or more scaffolds as described herein and a cell population deposited on one or more surfaces of the one or more scaffolds as described herein. The scaffolds are seeded with a smooth muscle cell (SMC) population derived from an autologous or non-autologous source. In one embodiment, an Autologous SMC cell population could be isolated from the adipose tissue or peripheral blood of a subject in need. The cell population could be seeded onto a scaffold suitable for implantation at a site within the lung(s) of the subject. An advantage of the cell populations of the present invention is that suitable SMCs may not be available for sourcing from the subject's lung if the subject has a defective respiratory system, e.g., lung cancer. The cell populations may be used in cases where part or all of a subject's lung is removed, such as in the case of lung cancer. Upon removal of a lung or a part of a lung in a subject, an autologous SMC population could be isolated from a biopsy, cultured, seeded on a suitable scaffold, and implanted into the subject to provide a new lung or new lung tissue structure.

The present disclosure also provides respiratory tissue constructs that include a scaffold, a first cell population that is not derived from respiratory tissue deposited on or in a first surface of the scaffold; and a second cell population derived from respiratory tissue. In one embodiment, the first cell population is derived from adipose. In another embodiment, the first cell population is a smooth muscle cell population (SMC). The SMC population may be seeded on the scaffold first, as per the procotols described herein, after which the respiratory cell population could be seeded on the scaffold pre-seeded with SMCs. Those of ordinary skill in the art will appreciate that different methods may be suitable for seeding of cell populations.

In one other embodiment, the construct is positive for at least one smooth muscle cell marker. In yet another embodiment, the construct is positive for one or more of the following SMC markers: myocardin, alpha-smooth muscle actin, calponin, myosin heavy chain, BAALC, desmin, myofibroblast antigen, SM22, and any combination thereof. In one embodiment, the second cell population is derived from lung. In another embodiment, the construct is positive for at least one respiratory tissue marker. In yet another embodiment, the respiratory marker is one or more of the following: a bronchiolar marker, an alveolar marker, and an epithelial marker. In one other embodiment, the construct is positive for one or more of the following a respiratory tissue markers: Clara Cell Secretory Protein (CCSP); Prosurfactant Protein C (ProSP-C); KRT18; Secretoglobin, Family 1A, Member 1 (Uteroglobin or SCGB1A1); Surfactant Protein A1 (SFTPA1); and any combination thereof. In one embodiment, the bronchiolar marker is CCSP and/or SCGB1A1, the alveolar marker is proSP-C and/or SFTPA1, and the epithelial marker is KRT18. In another embodiment, the second cell population is deposited on or in a second surface of the scaffold. In one other embodiment, the second cell population is in contact with the deposited first cell population. In yet another embodiment, the construct includes one or more alveolar forming units (AFUs). In one embodiment, the construct includes cells having coordinated rhythmic contractile function. In another embodiment, the construct is adapted to form respiratory tissue following implantation. In other embodiments, the respiratory tissue is lung tissue. The lung tissue may include alveolar tissue and/or bronchiolar tissue.

These constructs are used to provide a tissue structure such as a lung tissue structure to a subject in need. The subject may require the reconstruction, augmentation or replacement of respiratory tissue. In one embodiment, the respiratory tissue is a lung or a portion thereof, and the polymeric matrix or scaffold has smooth muscle cells deposited on a surface of the matrix.

In one aspect, the respiratory tissue constructs of the present disclosure are adapted to provide particular features to the subject following implantation. In one embodiment, the constructs are adapted to provide regeneration to the subject following implantation. In another embodiment, the constructs are adapted to promote regeneration in a subject at the site of implantation. For example, following implantation, regenerated tissue may form from the construct itself at the site of implantation. In another embodiment, the construct may impart functional attributes to the subject following implantation. In one embodiment, the construct may be adapted to form alveolar forming units (AFUs) at the site of implantation.

In one aspect, the present disclosure concerns the use of adipose-derived smooth muscle cell-seeded scaffolds as a substrate for seeding with lung cell suspensions to provide an increase in the number of alveolar forming units (AFUs) compared relative to a scaffold that has not been seeded with adipose-derived smooth muscle cell. This has a potentially profound improvement over the current state of the art, described herein, regarding lung tissue regeneration. A regenerative medicine-based approach using a fully characterized autologous differentiated cell type is advantageous over a non-autologous cell type, e.g., Allogeneic fetal-derived (Andrade et al. 2007 *supra)* or an undifferentiated, pluripotent, stem/progenitor cells (Shigemura et al. 2006 *supra*). Adipose-derived smooth muscle cell have certain advantages over other cells that have been described in the literature (Basu and Ludlow, Trends Biotechnol. 2010 Oct;28(10):526-33. Epub 2010 Aug 25). The present disclosure concerns respiratory tissue constructs that are adapted to facilitate the deposition or seeding of respiratory cell populations through the use of an SMC population. It has been discovered that depositing or seeding an adipose-derived SMC population on to or in a respiratory tissue scaffold potentially enhances the subsequent seeding or deposition of a respiratory cell population. In one embodiment, the construct having a previously deposited SMC population facilitates the migration or seeding of a respiratory cell population on or in a surface of the scaffold. In another embodiment, construct with previously deposited SMC population facilitates the migration of a respiratory cell population to contact the scaffold and/or the deposited SMC population. The migration of the respiratory cell population may be *in vitro* or *in vivo.*

In one other aspect, the disclosure provides one or more polymeric scaffolds or matrices that are seeded with an SMC population and a respiratory cell population. In one embodiment, the cell-seeded polymeric matrix or matrices form a respiratory tissue construct.

In one aspect, the present disclosure provides muscle equivalent constructs that may be used to enhance an existing respiratory organ or tissue structure such as a lung, to a subject in need. The subject may require expansion or treatment of such organs or tissues. In one embodiment, the respiratory tissue is a lung or portion thereof, and the polymeric matrix or scaffold has smooth muscle cells deposited on a surface of the matrix.

### D. Blood vessel constructs

The present disclosure relates to blood vessel scaffolds and methods of making the same. The scaffolds are manipulated to form blood vessel constructs. In one aspect, the present disclosure concerns blood vessel constructs. In one embodiment, the construct includes a) a biocompatible tubular scaffold having a first and a second surface; and b) a first cell population derived from a non-vascular source deposited on or in the first surface of the scaffold. In another embodiment, the first cell population is a smooth muscle cell population. In one other embodiment, the second cell population may be derived from a non-vascular source. The second cell population may be deposited on or in the second surface of the scaffold. In another embodiment, the second cell population is an endothelial cell population. In all embodiments, the non-vascular source is adipose tissue. In all embodiments, the non-vascular source is peripheral blood. In another embodiment, the first surface of the tubular scaffold is an exterior surface. In a preferred embodiment, the exterior surface is corrugated. In one other embodiment, the second surface of the tubular scaffold is an interior, luminal surface. In all embodiments, the scaffold may include a synthetic material. In all embodiments, the scaffold may include a natural material.

In another embodiment, the first cell population is a smooth muscle cell population. In one other embodiment, the second cell population may be derived from a non-vascular source. The second cell population may be deposited on or in the second surface of the scaffold. In another embodiment, the second cell population is an endothelial cell population. In all embodiments, the non-vascular source is adipose tissue. In all embodiments, the non-vascular source is peripheral blood. In another embodiment, the first surface of the tubular scaffold is an exterior surface. In a preferred embodiment, the exterior surface is corrugated. In one other embodiment, the second surface of the tubular scaffold is an interior, luminal surface. In all embodiments, the scaffold may include a synthetic material. In all embodiments, the scaffold may include a natural material. In one embodiment, the non-vascular source used to obtain the first or second cell population is autologous to the subject. In another embodiment, the non-vascular source used to obtain the first or second cell population is non-autologous to the subject.

In another aspect, the present disclosure provides blood vessel constructs that are derived from the scaffolds of the present invention. Given their substantial similarity to native blood vessels, the scaffolds are particularly amenable to modification to create the constructs that in turn can be used as vascular bypass grafts for the treatment of cardiovascular disorders. Vascular bypass grafts include arteriovenous (AV) shunts. In a preferred embodiment, the scaffolds of the present invention can be used to create bloof vessel constructs having a small diameter, typically less than 6 mm, for use in treating cardiovascular disorders.

As discussed herein, certain embodiments of the scaffolds have been shown to exhibit a mechanical response to stress and strain characterized by a J-shaped stress/strain curve that is attributable to a range of elastic moduli and modulus transition, and any combination thereof. In addition to the moduli parameters, there are other properties exhibited by the scaffolds that make them attractive for use in making vascular grafts. In one aspect, the scaffolds of the present disclosure exhibit certain properties which render them particularly suitable for making a blood vessel construct for use as a vascular graft in the first place, and for ensuring that the vascular graft will retain patency once implanted. Such properties include, without limitation, those that allow the seeding of cells on a scaffold, those that provide resistance to fracture of the scaffold, and those that provide viscoelasticity to a scaffold.

In one embodiment, the property favoring the seeding of cells on the scaffolds is attributable to a pore gradient where the pore diameter gradually decreases from about 100 microns at the Adventitial or exterior side to about 5 to about 15 microns at the luminal or interior side of a tubular element. It is well known in the art that pore diameter is an important factor for the successful seeding of cells on and within a scaffold. For example, the pore diameter must be large enough for various cell types to migrate to the surface of a scaffold and through a scaffold, such that they can interact with other migrating cells in a manner similar to that observed *in vivo.* The present disclosure concerns the discovery that a particular pore gradient contributes to the successful seeding of cells. In one embodiment, the pore gradient renders the scaffold accessible to cells and thereby enhances its capacity for cell seeding. In another embodiment, the pore gradient is about 100 microns (exterior side) to about 5 microns (interior side), about 100 microns (exterior side) to about 6 microns (interior side), about 100 microns (exterior side) to about 7 microns (interior side), about 100 microns (exterior side) to about 8 microns (interior side), about 100 microns (exterior side) to about 9 microns (interior side), or about 100 microns (exterior side) to about 10 microns (interior side).

In one aspect, the pore gradient provides architecture that is advantageous for the seeding of cells on the luminal, interior side of a TE scaffold and for the seeding of cells on the exterior, adventitial side of a TE scaffold. In one embodiment, the smaller pore size on the luminal, interior surface is suited for seeding of endothelial cells on and within the interior surface, and the larger pore size on the exterior, adventitial side is suited for seeding of smooth muscle cells on and within the exterior surface. In another embodiment, the endothelial cells are seeded to form a monolayer or flat sheet-like structure on and within the interior, luminal surface of the TE scaffold and/or the smooth muscle cells are seeded on and/or within the exterior, adventitial surface of the TE scaffold.

In some embodiments, the endothelial cells seeded on and throughout the interior, luminal surface of the TE scaffold are unable to migrate towards the exterior, adventitial surface beyond certain pore size. In a preferred embodiment, the pore size is about 15 to about 20 microns. In another preferred embodiment, the pore size is about 15 microns, about 16 microns, about 17 microns, about 18 microns, about 19 microns, or about 20 microns.

In another aspect, the present disclosure provides blood vessel constructs that are derived from the blood vessel scaffolds described herein. As a result, the constructs exhibit structural and functional properties substantially similar to those found in native blood vessels. In one embodiment, the TEBVs of the present disclosure are characterized by having the ability to respond mechanically to stress and strain in an anisotropic manner. In another embodiment, the TEBVs have (i) properties favoring resistance to fracture of the scaffold; and/or (ii) properties favoring the viscoelasticity of a scaffold.

In another aspect, the tissue engineered blood vessels (TEBVs) of the present disclosure can modulate certain complications associated with vascular grafts that have been observed following implantation. In one embodiment, the TEBVs modulate compliance mismatch after implantation. In another embodiment, the modulation comprises one or more of the following: resistance to aneurysm formation, resistance to dilatation, resistance to fracture, resistance to thrombosis, resistance to anastomotic hyperplasia, and resistance to intimal hyperplasia. Those of skill in the art will appreciate additional factors subject to modulation by the TEBVs.

In one aspect, the present disclosure provide blood vessel constructs that contain a scaffold and a cell population, as described herein. In one embodiment, a scaffold may be manipulated to form a blood vessel construct that it is suitable for transplantation into a mammal in need. For example, a scaffold may be manipulated by adding one or more cell populations by the methods described herein. Those of ordinary skill in the art will appreciate that the blood vessel constructs of the present invention may be applicable to many types of blood vessels, including without limitation, the carotid artery, the subclavian artery, the celiac trunk, the mesenteric artery, the renal artery, the iliac artery, arterioles, capillaries, venules, the subclavian vein, the jugular vein, the renal vein, the iliac vein, the venae cavae.

The blood vessel construct may have a first cell population within the second tubular element and/or on the exterior surface of second tubular element of the construct. In a preferred embodiment, the first cell population is a smooth muscle cell population. Those of skill in the art will appreciate that various types of smooth muscle cells (SMCs) may be suitable for use in the present invention (see Bertram et al. U.S. Published Application 20070190037 and Ludlow et al. U.S. Published Application No. 20100131075, both of which are incorporated herein by reference in their entirety), including without limitation, human aortic smooth muscle cells, human umbilical artery smooth muscle cells , human pulmonary artery smooth muscle cells, human coronary artery smooth muscle cells , human bronchial smooth muscle cells, human radial artery smooth muscle cells, and human saphenous or jugular vein smooth muscle cells. As described in Bertram *et* a/. *supra,* the SMCs may be isolated from a variety of sources, including, for example, biopsies from living subjects and whole-organ recover from cadavers. As described in Ludlow *et al. supra,* the SMCs may be obtained from adipose tissue or peripheral blood. The SMCs may be Autologous cells to a subject in need of treatment with the construct and isolated from a biopsy isolated from the subject intended to be the recipient of the construct. The SMCs may be non-autologous to a subject in need of treatment and isolated from a biopsy obtained from a suitable donor.

In one embodiment, the smooth muscle cell population seeded on a scaffold is derived from a non-vascular source. In one other embodiment, the constructs of the present invention are free of cells derived from a vascular source. The blood vessel constructs of the present invention may comprise, consist essentially of, or consist of a smooth muscle cell population that is derived from a non-vascular source. The SMC population may be derived from adipose tissue or peripheral blood.

In another embodiment, the construct comprises a second cell population on the interior or luminal surface of the construct. In a preferred embodiment, the second cell population is an endothelial cell population. Those of skill in the art will appreciate that various types of endothelial cells (ECs) may be suitable for use in the present invention (see U.S. Published Application 20070190037 incorporated herein by reference in its entirety), including without limitation, arterial and venous ECs such as human coronary artery endothelial cells, human aortic endothelial cells, human pulmonary artery endothelial cells, dermal microvascular endothelial cells, human umbilical vein endothelial cells, human umbilical artery endothelial cells, human saphenous vein endothelial cells, human jugular vein endothelial cells, human radial artery endothelial cells, and human internal mammary artery endothelial cells. ECs may be isolated from a variety of sources including, without limitation, adipose tissue, peripheral blood, the vascular parenchyma, circulating endothelial cells and endothelial cell precursors such as bone marrow progenitor cells, peripheral blood stem cells and embryonic stem cells (see Bischoff et al. U.S. Published Application 20040044403 and Raffi et al. U.S. Patent 6,852,533, each of which are incorporated by reference in their entirety).

Those of skill in the art will appreciate that the seeding or deposition of one or more cell populations described herein may be achieved by various methods known in the art. For example, bioreactor incubation and culturing, (Bertram et al. U.S. Published Application 20070276507; McAllister et al. U.S. Patent 7,112,218; Auger et al. U.S. Patent 5,618,718; Niklason et al. U.S. Patent 6,537,567); pressure-induced seeding (Torigoe et al. (2007) Cell Transplant., 16(7):729-39; Wang et al. (2006) Biomaterials. May;27(13):2738-46); and electrostatic seeding (Bowlin et al. U.S. Patent No. 5,723,324) may be used. In addition, a recent technique that simultaneously coats electrospun fibers with an aerosol of cells may be suitable for seeding or deposition (Stankus et al. (2007) Biomaterials, 28:2738-2746).

In one embodiment, the deposition of cells includes the step of contacting a tubular scaffold with a cell attachment enhancing protein. In another embodiment, the enhancing protein is one or more of the following: fibronection, collagen, and MATRIGEL™. In one other embodiment, the tubular scaffold is free of a cell attachment enhancing protein. In another embodiment, the deposition of cells includes the step of culturing after contacting a tubular scaffold with one or more cell populations. In yet another embodiment, the culturing may include conditioning by pulsatile and/or steady flow in a bioreactor.

### E. Ocular tissue constructs

In another aspect, the present disclosure contemplates the application of the SMC populations described herein for treating ocular disorders. An ocular disorder is one in which the subject has a defective eye due to improper function of the muscles of the eye. Smooth muscle is present as ciliary muscle in the eye and controls the eye's accommodation for viewing objects at varying distances and regulates the flow of aqueous humour through Schlemm's canal. Smooth muscle is also present in then iris of the eye. Individuals with ocular disorders such as presbyopia and hyperopia could benefit from these SMC populations. In one embodiment, a non-autologous SMC cell population could be isolated from the adipose tissue or peripheral blood of a subject in need. The cell population could be seeded onto a scaffold suitable for implantation at a site within the eye of the subject. An advantage of the cell populations of the present invention is that suitable SMCs may not be available for sourcing from the subject's eye if the subject has a defective eye or due to the limited availability of eye tissue or due to difficulties in taking biopsies. A non-autologous SMC population could be isolated from a biopsy, cultured, seeded on a suitable scaffold, and implanted into the subject to provide new eye tissue. In another embodiment, the smooth muscle cell populations of the present disclosure may be obtained from non-ocular sources and administered to a subject having an ocular disorder without the use of a scaffold, such as by engraftment. Those of ordinary skill in the art will appreciate suitable methods of engraftment. The non-ocular source may be autologous or nan-autologous.

### 6. Methods of use

### A. Use of Luminal organ and tissue structure constructs

In one aspect, the present invention concerns methods for providing a laminarily organized luminal organ or tissue structure to a subject in need of such treatment. In one embodiment, the subject may be in need of reconstruction, regeneration, augmentation, or replacement of an organ or tissue. In one embodiment, the method includes the step of providing a biocompatible synthetic or natural polymeric matrix shaped to conform to at least a part of the organ or tissue structure in need of an organ or tissue structure. The providing step may be followed by depositing at least one cell population that is non-autologous to the subject and is not derived from the corresponding organ or tissue structure that is the subject of the reconstruction, regeneration, augmentation or replacement. The depositing step may include culturing the cell population on the polymeric matrix. After depositing the cell population on the matrix to provide a construct, it can be implanted into a patient at the site of treatment for the formation of the desired laminarily organized luminal organ or tissue structure.

In one other aspect, the present invention concerns methods for providing a laminarily organized luminal organ or tissue structure to a subject in need. In one embodiment, the method includes the steps of a) providing a biocompatible synthetic or natural polymeric matrix shaped to conform to at least a part of the organ or tissue structure in need of said treatment; b) depositing on or in a first area of the polymeric matrix a cell population that is non-autologous to the subject and is not derived from an organ or tissue corresponding to the new organ or tissue structure; and c) implanting the shaped polymeric matrix cell construct into said the subject for the formation of laminarily organized luminal organ or tissue structure.

The present invention also concerns methods for replacing a native luminal organ or tissue structure with a non-native organ or artificial organ. The replacement organ is non-native or artificial in the sense that it is not in the original form of the native organ but nonetheless is capable of funtioning in the same or a substantially similar way. The native organ or tissue structure of a subject may be replaced with a non-native organ or artificial organ by implanting a construct described herein to form the non-native organ or artificial organ. The non-native organ or artificial organ comprises cells that are autologous or non-autologous to the subject receiving the construct. At the initial stage of implantation and for a finite period thereafter, the construct is composed of a biodegradeable scaffold seeded with cells but following degradation of the scaffold, the non-native organ or artificial organ will not contain synthetic components such as the scaffold. The non-native organ or artificial organ will have been formed from the implanted construct. In one other embodiment, the non-native organ or artificial organ is formed from an implanted construct including, without limitation, a bladder augmentation construct, a bladder replacement construct, a urinary conduit construct, a muscle equivalent construct, a respiratory tissue construct, a gastrointestinal tissue construct, or a blood vessel construct.

In all embodiments, the methods further include the step of wrapping the implanted luminal organ or tissue structure construct with the subject's omentum, mesentery, muscle fascia, and/or peritoneum to allow for vascularization.

In one embodiment, the scaffolds, cell populations, and methods described herein may further be used for the preparation of a medicament useful in the treatment of a disorder described herein. The disorders include any condition in a subject that requires the regeneration, reconstruction, augmentation or replacement of laminarly organized luminal organs or tissue structures.

In another embodiment, the cells deposited on the implanted construct produce MCP-1 and release it at the site of implantation, which stimulates native mesenchymal stem cells (MSCs) to migrate to the site of implantation. In one other embodiment, the native MSCs facilitate and/or enhance regeneration of the implanted construct at the site of implantation.

As described above, the constructs of the present invention may contain cell populations that are non-autologous to the subject in need of treatment. In addition, the cell populations of the present invention may be administered to a subject having a disorder without the use of a scaffold, such as by engraftment. Those of ordinary skill in the art will appreciate suitable methods of engraftment. The non-autologous cell populations may be allogeneic or syngeneic.

In one embodiment, any of the constructs containing non-autologous cells and/or the cells themselves may be administered to a subject in need thereof without the need for an immunosuppressant agent. The immunosuppressant agents that are not required include, without limitation, azathioprine, cyclophosphamide, mizoribine, ciclosporin, tacrolimus hydrate, chlorambucil, lobenzarit disodium, auranofin, alprostadil, gusperimus hydrochloride, biosynsorb, muromonab, alefacept, pentostatin, daclizumab, sirolimus, mycophenolate mofetil, leflonomide, basiliximab, dornase α, bindarid, cladribine, pimecrolimus, ilodecakin, cedelizumab, efalizumab, everolimus, anisperimus, gavilimomab, faralimomab, clofarabine, rapamycin, siplizumab, saireito, LDP-03, CD4, SR-43551, SK&F-106615, IDEC-114, IDEC-131, FTY-720, TSK-204, LF-080299, A-86281, A-802715, GVH-313, HMR-1279, ZD-7349, IPL-423323, CBP-1011, MT-1345, CNI-1493, CBP-2011, J-695, LJP-920, L-732531, ABX-RB2, AP-1903, IDPS, BMS-205820, BMS-224818, CTLA4-1g, ER-49890, ER-38925, ER-38925, RDP-58, PNU-156804, LJP-1082, TMC-95A, TV-4710, PTR-262-MG, and AGI-1096 (see U.S. Patent No. 7,563,822). Those of ordinary skill in the art will appreciate other immunosuppressant agents that are not required.

In another aspect, the disclosure provides methods for prognostic evaluation of a patient following implantation of a new organ or tissue structure. In one embodiment, the method includes the step of (a) detecting the level of MCP-1 expression in a test sample obtained from said subject; (b) determining the expression level in the test sample to the level of MCP-1 expression relative to a control sample (or a control reference value); and (c) predicting regenerative prognosis of the patient based on the determination of MCP-1. expression levels, wherein a higher level of expression of MCP-1 in the test sample, as compared to the control sample (or a control reference value), is prognostic for regeneration in the subject.

In another aspect, the disclosure provides methods for prognostic evaluation of a patient following implantation of a new organ or tissue structure in the patient, the methods comprising: (a) obtaining a patient biological sample; and (b) detecting MCP-1 expression in the biological sample, wherein MCP-1 expression is prognostic for regeneration in the patient. In some embodiments, increased MCP-1 expression in the patient biological sample relative to a control sample (or a control reference value) is prognostic for regeneration in the subject. In some embodiments, decreased MCP-1 expression in the patient sample relative to the control sample (or control reference value) is not prognostic for regeneration in the subject. The patient sample may be a test sample comprising a bodily fluid, such as blood or urine.

In some embodiments, the determining step comprises the use of a software program executed by a suitable processor for the purpose of (i) measuring the differential level of MCP-1 expression in a test sample and a control; and/or (ii) analyzing the data obtained from measuring differential level of MCP-1 expression in a test sample and a control. Suitable software and processors are well known in the art and are commercially available. The program may be embodied in software stored on a tangible medium such as CD-R171VI, a floppy disk, a hard drive, a DVD, or a memory associated with the processor, but persons of ordinary skill in the art will readily appreciate that the entire program or parts thereof could alternatively be executed by a device other than a processor, and/or embodied in firmware and/or dedicated hardware in a well known manner.

Following the determining step, the measurement results, findings, diagnoses, predictions and/or treatment recommendations are typically recorded and communicated to technicians, physicians and/or patients, for example. In certain embodiments, computers will be used to communicate such information to interested parties, such as, patients and/or the attending physicians. In some embodiments, the assays will be performed or the assay results analyzed in a country or jurisdiction which differs from the country or jurisdiction to which the results or diagnoses are communicated.

In a preferred embodiment, a prognosis, prediction and/or treatment recommendation based on the level of MCP-1 expression measured in a test subject having a differential level of MCP-1 expression is communicated to the subject as soon as possible after the assay is completed and the prognosis and/or prediction is generated. The results and/or related information may be communicated to the subject by the subject's treating physician. Alternatively, the results may be communicated directly to a test subject by any means of communication, including writing, electronic forms of communication, such as email, or telephone. Communication may be facilitated by use of a computer, such as in case of email communications. In certain embodiments, the communication containing results of a prognostic test and/or conclusions drawn from and/or treatment recommendations based on the test, may be generated and delivered automatically to the subject using a combination of computer hardware and software which will be familiar to artisans skilled in telecommunications. One example of a healthcare-oriented communications system is described in U.S. Pat. No. 6,283,761; however, the present invention is not limited to methods which utilize this particular communications system. In certain embodiments of the methods of the invention, all or some of the method steps, including the assaying of samples, prognosis and/or prediction of regeneration, and communicating of assay results or prognoses, may be carried out in diverse (e.g., foreign) jurisdictions.

In another aspect, the prognostic methods described herein provide information to an interested party concerning the success of the implantation, and the rehabilitation/treatment protocol for regeneration. In one embodiment, the methods include the steps of detecting the level of MCP-1 expression in a test sample obtained from said subject; (b) determining the expression level in the test sample to the level of MCP-1 expression relative to a control sample (or a control reference value); and (c) predicting regenerative prognosis of the patient based on the determination of MCP-1 expression levels, wherein a higher level of expression of MCP-1 in the test sample, as compared to the control sample (or a control reference value), is indicative of the state of regeneration of new respiratory tissue.

As described herein, "regeneration prognosis" or "regenerative prognosis" generally refers to a forecast or prediction of the probable course or outcome of the implantation of a construct described herein. In one embodiment, a regeneration prognosis encompasses the forecast or prediction of any one or more of the following: development or improvement of a functional bladder after bladder replacement or augmentation through implantation of a construct described herein, development of a functional urinary diversion after implantation of a construct described herein, development of bladder capacity or improved bladder capacity after implantation of a construct described herein, or development of bladder compliance or improved bladder compliance after implantation of a construct described herein, development or improvement of gastro-intestinal (GI) function or GI capacity after reconstruction, augmentation or replacement of gastro-intestinal tissue following implantation of a tissue construct described herein, development or improvement of a functional lung after respiratory tissue replacement or augmentation through implantation of a construct described herein, or development of lung capacity or improved lung capacity after implantation of a construct described herein.

As described herein, "regenerated tissue" refers to the tissue of a new tissue structure that develops after implantation of a construct as described herein. The regenerated structure may be a bladder or part of a bladder, a urinary conduit, gastrointestinal tissue, or a lung or part of a lung. The regenerated tissue may include a continuous urothelium with underlying smooth muscle.

In all embodiments, the present invention relates to methods for providing a new organ or tissue structure to a subject in need that include certain post-implantation monitoring steps. In one embodiment, the methods of providing an organ or tissue structure to a subject in need of such treatment as described herein may include the post-implantation step of prognostic evaluation of regeneration as described above. In another embodiment, the effect and performance of an implanted constructs is monitored, such as through ultrasound imaging, pyelogram, as well as urine and blood analysis at different time-points after implantation.

In one embodiment, the scaffolds, cell populations, constructs, and methods described herein may further be used for the preparation of a medicament useful in the treatment of a disorder described herein. The disorders include any condition in a subject that requires the regeneration, reconstruction, augmentation or replacement of a native luminal organ or tissue structure. The organ or tissue structure may be laminarily organized.

### B_{.} Use of Urinary system organ and tissue structure constructs

The present disclosure provides methods for the use of constructs for use in the reconstruction, replacement, augmentation, or regeneration of native luminal organs or tissue structures of the urinary system. The organs or tissue structures of the urinary system may also be referred to as genitourinary or urogenital organs or tissue structures. The native organs or tissue structures may be laminarily organized.

In one other aspect, the present disclosure provides methods for providing a neo-bladder or portion thereof to a subject in need. In one embodiment, the method includes a) providing a biocompatible synthetic or natural polymeric matrix shaped to conform to a bladder or portion thereof; b) depositing a cell population that is non-autologous to the subject and is not derived from the subject's bladder on or in a first area of the polymeric matrix; and c) implanting the shaped polymeric matrix cell construct into the subject for the formation of the neo-bladder or portion thereof. In another embodiment, the cell population of step b) of the methods described herein contains one or more peripheral blood-derived smooth muscle cells having contractile function that are positive for a smooth muscle cell marker, or the cell population of step b) contains one or more adipose tissue-derived smooth muscle cells having contractile function that are positive for a smooth muscle cell marker. In one other embodiment, the contractile function of the cell population is calcium-dependent.

In all embodiments, the methods of the present disclosure utilize a construct for implantation that is based upon a bladder replacement scaffold, a bladder augmentation scaffold, a bladder conduit scaffold, or a detrusor muscle equivalent scaffold that has been seeded with a cell population as described herein.

In all embodiments, the methods of the present disclosure further include the step of wrapping the implanted construct with the subject's omentum, mesentery, muscle fascia, and/or peritoneum to allow for vascularization. The wrap step may be used when implanting any of the urinary system constructs described herein.

The organ or tissue structure is a bladder or a part of the bladder. In one other embodiment, the laminarily organized luminal organ or tissue structure formed in vivo exhibits the compliance of natural bladder tissue.

In one other aspect, the present disclosure provides methods for providing a urinary diversion or conduit for a defective bladder in a subject in need. In one embodiment, the method for providing a urinary diversion to a subject in need includes the steps of (a) providing a biocompatible conduit scaffold; (b) depositing a first cell population that is non-autologous to the subject on or in a first area of said scaffold, said first cell population being substantially a muscle cell population; and (c) implanting the scaffold of step (b) into said subject to form a conduit that allows urine to exit the subject. In another embodiment, the biocompatible material is biodegradeable. In other embodiments, the biocompatible material is polyglycolic acid, In yet another embodiment, the first cell population is substantially a smooth muscle cell population.

In one embodiment, the method includes the step of providing a urinary diversion or conduit scaffold as described herein. In other additional embodiments, the urinary diversion or conduit scaffold is provided in multiple parts, such as a first, second, and third scaffold, as described herein. In another embodiment, the method further includes the step of depositing a cell population that is not derived from the defective bladder to form a urinary diversion or conduit construct. In one other embodiment, the depositing step may include culturing the cell population on the scaffold. In some embodiments, the methods further includes the step of implanting the urinary diversion construct into a patient in need. In another embodiment, the implantation is at the site of the defective bladder.

In one embodiment, an open end of the construct (e.g., a first end configured to connect to the abdominal wall) is anastomosed to the skin (ostomy) throught the abdominal or suprapubic wall to form a stoma or sphincter. In another embodiment, a catheter is inserted through stoma opening and into the lumen of the construct to provide urine outflow.

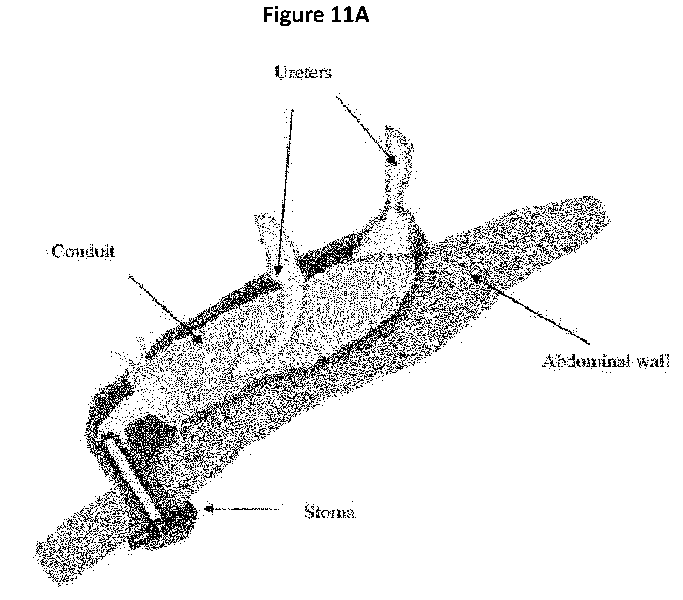

Figure 11A-B illustrates configurations for an implanted conduit construct.

In another embodiment, the methods of the present invention further include the step of monitoring the conduit for the presence of an obstruction following implantation of the urinary diversion construct. The obstruction may be caused by the build-up of detritis. The method may further include the step of removing detritis from the lumen of the conduit if an obstruction is detected (e.g., debridement).

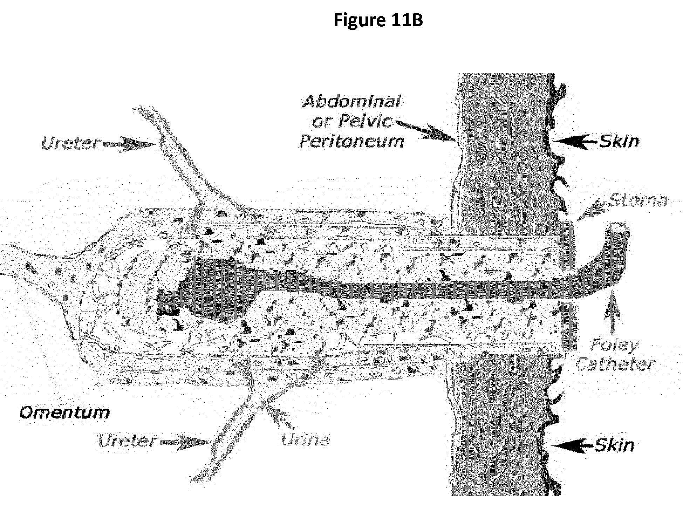

In one aspect, the present disclosure provides a urinary diversion to a subject in need on a temporary basis. In one embodiment, a temporary urinary diversion or conduit construct is implanted into a subject to form a stoma opening, and a catheter or other device is temporarily inserted through the stoma to the lumen of the conduit construct. A temporary conduit provides the advantage of allowing urine to exit the subject while a permanent solution to the defective bladder is attempted. For example, the implantation of a conduit construct could be performed prior to, following, or simultaneous with the implantation of a neo-bladder construct seeded with a cell population (see for example Bertram et al. supra). Figure 11B shows an example of the implanted components of a temporary urinary diversion construct.

In one embodiment, the methods of the present disclosure further include the step of wrapping the implanted urinary diversion or conduit construct with the subject's omentum, mesentery, muscle fascia, and/or peritoneum to allow for vascularization.

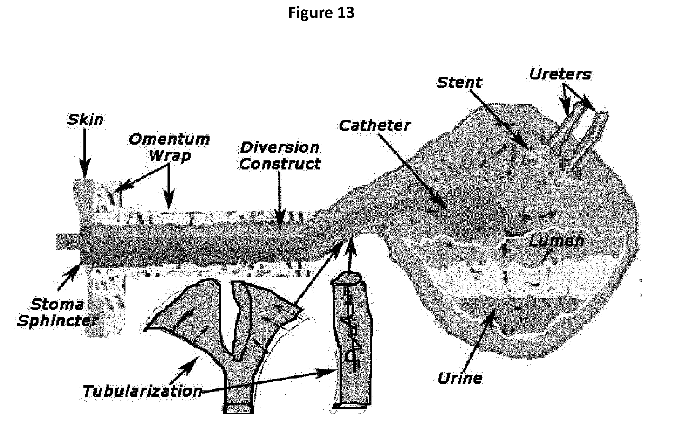

In one aspect, the present disclosure provides a urinary diversion to a subject in need on a permanent basis. Figure 13 shows an example of the implanted components of a permanent urinary diversion construct.

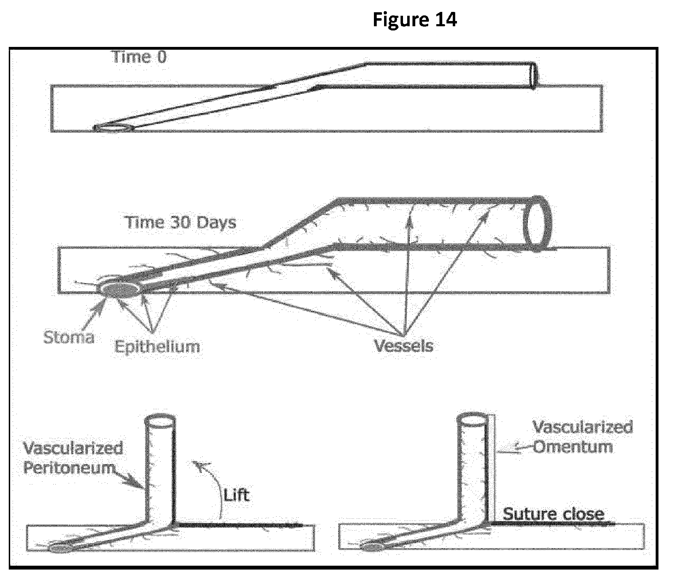

In one embodiment, the constructs described herein may be used for a prostatic urethra replacement and urinary diversion. Such a procedure is necessary for subjects requiring a radical prostatectomy to remove the prostatic urethra. In other embodiments, the constructs may be used for a percutaneous diversion tube to form a continent tube with a valve-like kink. In an additional embodiment, the constructs may be used as a bladder neck sling and wrapping materials used in bladder neck surgery and urinary outlets with continent channels or catherizable openings. Examples of such embodiments are depicted in Figure 14.

Urine exits the body via the urethral meatus, a distinct structure incorporating features that defend the opening against local and/or ascending infections, and emptying in the vaginal vestibule in females and fossa navicularis in males. Specifically, the mucocutaneous in this region is a non-keratinized stratified squamous epithelium composed of glycogen-rich cells that provide substrate for a protective endogenous lactobacteria flora. Also, as the epithelium nears the skin it is associated with acid-phosphatase activity and lysozyme-like immunoreactivity indicative of the presence of macrophages that secrete bactericidal compounds (Holstein AF et al. (1991) Cell Tissue Res 264: 23).

In one aspect, the urinary diversion or neo-urinary conduit (NUC) constructs described herein may lead to the formation of a native-like transition between urinary mucosa and skin epithelium that has the structural features of mucocutaneous regions observed in native urethras. The transition region may be referred to as an epithelialized mucosa. In one embodiment, the construct is adapted to form an epithelialized mucosa upon implantation. In one embodiment, the epithelialized mucosa comprises a vestibular region and a mucocutaneous region. In another embodiment, the vestibular region is adjacent to the mucocutaneous region. In another embodiment, the mucocutaneous region is located at the stromal end of the construct connected to the abdominal wall and skin of the subject. In general, naturally-occuring mucocutaneous regions are characterized by the presence of mucosa and cutaneous skin and typically exist near the orifices of the body where the external skin ends and the mucosa that covers the inside of the body starts. The epithelialized mucosa provided by the constructs and methods of the present invention develops at the first end of the urinary diversion construct following implantation into the subject. In a further embodiment, the epithelialized mucosa is characterized by the presence of an epithelium that first appears in the vestibular region and gradually expands or increases through the mucocutaneous region towards the stomal end of the construct. In another embodiment, the epithelium is characterized by expression of an epithelial cell marker. In a further embodiment, the epithelial cell marker is cytokeratin. The cytokeratin may be one or more of the cytokeratins known in the art including, without limitation, cytokeratins 1 through 19. In one other embodiment, the cytokeratin is detectable with an anti-pancytokeratin (AE-1/AE3) antibody and/or a cytokeratin 7 (CK-7) antibody.

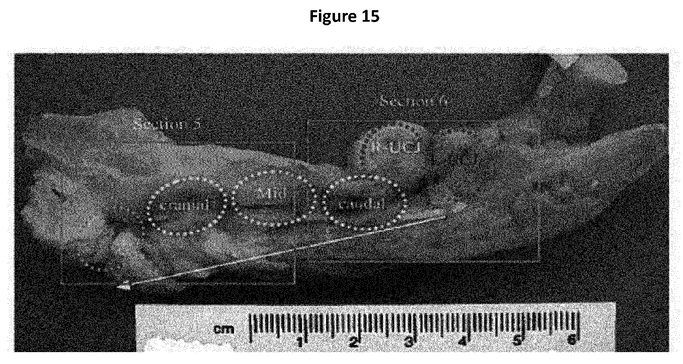

Table 5.4 and Figure 15 indicate different regions of an exemplary implanted urinary diversion or conduit. Sections 5 and 6 correspond to (i) the cranial end: the stoma, cranial, and mid-portion of the conduit, and (ii) the caudal end: the remaining mid-portion of the conduit and the left/right ureteral-conduit junctions, respectively. In yet another embodiment, epithelium covering a luminal surface of a caudal section of the conduit is positive for CK-7, as detectable by an anti-CK-7 antibody (e.g., section 6). In one other embodiment, epithelium covering a luminal surface of a cranial and mid-aspect of the conduit is negative for CK-7, as detectable by an anti-CK-7 antibody (e.g., section 5). In one embodiment, epithelium covering a luminal surface of a caudal section of the conduit is positive for AE1/AE3 (e.g., section 6). In another embodiment, epithelium covering a luminal surface of a cranial and mid-aspect of the conduit is negative for AE1/AE3, as detectable by an anti- AE1/AE3 antibody (e.g., section 5). In another aspect, the urinary diversion is characterized by expressions of a smooth muscle cell (SMC) maker. In one embodiment, the SMC marker is α-Smooth Muscle Actin (SMA) and/or calponin. In another embodiment, the SMC marker is detectable with an anti-α-Smooth Muscle Actin (SMA) antibody and/or an anti-calponin antibody. In one other embodiment, the conduit wall or outer surface components at the caudal region of the conduit are positive for SMA, as detectable by an anti-SMA antibody (e.g., section 6). In another embodiment, the conduit wall or outer surface components at the cranial and mid-conduit sections are negative for SMA, as detectable by an anti-SMA antibody (e.g., section 5). In one other embodiment, the conduit wall or outer surface components at the caudal region of the conduit are positive for calponin, as detectable by an anti-calponin antibody (e.g., section 6). In one other embodiment, the conduit wall or outer surface components at the cranial and mid-conduit sections are negative for calponin, as detectable by an anti-calponin antibody (e.g., section 5).

The ability of the constructs described herein to form an epithelialized mucosa provides a solution to the major challenge of achieving urinary diversion via an abdominal stoma. It is accepted that the longevity of percutaneous devices is often hampered by exit-site infection (Knabe C et al. (1999) Biomaterials 20: 503). Percutaneous devices such as catheters, cannulas, prosthetic attachments, and glucose sensors, regardless of their intended medical goal, penetrate the skin, disrupt its protective barrier, and create a sinus tract for bacterial invasion (Isenhath SN et al. (2007) J Biomed Mater Res A 83: 915). Breakdown of the product-skin interface due to improper epidermal healing, lack of biocompatibility, or mechanical stresses can cause additional failure risks (von Recum AF and Park JB. (1981) Crit Rev Bioeng 5:37).

In another aspect, the urinary diversion constructs through interaction with the tissue of a recipient regenerate a tubular organoid. In one embodiment, the interaction of the construct with the recipient tissue is by transabdominal-percutaenous placement. In one other embodiment, the tubular organoid allows the flow of urine from the ureters to outside of the recipient. Urine flows out of the recipient while maintaining native-like functional properties found in bladders, urethras, and stomas (i.e., a meatus or opening). The muco-cutaneous junction resembles a junction found at the anterior urethra's openings; at the vaginal vestibule and fossa navicularis, of the human female and male, respectively. These natural junctions are covered by mucosal zones critical to wet-dry surfaces that may provide protection against ascending infections. The squamous epithelium of these mucosal zones is 1) glycogen-rich, 2) secretory (able to release enzymes and bactericidal agents), and 3) phagocytic; and can rapidly migrate to injured surfaces.

In one aspect, the present disclosure provides methods of providing a mucocutaneous junction (MCJ) to a subject in need. The MCJ resembles a naturally-occuring mucocutaneous region characterized by the presence of mucosa and cutaneous skin, which typically exist near the orifices of the body where the external skin ends and the mucosa that covers the inside of the body starts. In one embodiment, the method includes the step of providing a construct made up of a scaffold described herein and a cell population described herein. In another embodiment, the method further includes the step of administering to a subject in need the construct with a first part (e.g., a first end of a tubular shaped construct) adapted to be exposed to air and a second part (e.g., a second end of a tubular shaped construct) that is not exposed to air, such that an MCJ forms following implantation of the construct.

Grafting of scaffolds to an organ or tissue to be enlarged can be performed according to the methods described in the Examples or according to art-recognized methods. The matrix or scaffold can be grafted to an organ or tissue of the subject by suturing the graft material to the target organ.

In all embodiments, the method of providing a urinary diversion or conduit construct further comprises administering a mesh structure. In another embodiment, the administering step comprises inserting a mesh structure between a subcutaneous fat layer and skeletal muscle. In one embodiment, the mesh structure is subcutaneously implantated. In another embodiment, the mesh structure is implanted at the site of connection between the first end and the abdominal wall section. In another embodiment, the mesh structure faciliates formation of a neo-urinary conduit following implantation of a urinary diversion construct described herein. In one other embodiment, the mesh structure provides stomal patency. In a preferred embodiment, the mesh structure is a hernia patch, preferably a subcutaneous hernia patch. In one other embodiment, the mesh structure is adapted for administration to a subject at risk for intestinal herniation. For example, if a part of the intestine is located above a selected abdominal wall opening, then the intestine may protrude towards or through the opening due to peristalsis associated with food passage through the intestine. A person of ordinary skill in the art can assess whether the subject is at risk for intestinal protrusion based upon an examination of the selected location of the abdominal wall opening and the subject's intestine. After such an assessment, the person of ordinary skill in the art can determine whether the subject should be administered a urinary diversion construct described herein and a mesh structure.

In one aspect, the present disclosure provides methods to treat subjects in need of treatment for some defect in the urinary system. Suitable subjects include any single human subject, such as a patient, eligible for treatment, who is experiencing or has experienced one or more signs, symptoms, or other indicators of a deficient organ function or failure, including a deficient, damaged or non-functional urinary system. In general, the subject is a subject in need of the regeneration of, the reconstruction of, the augmentation of, or the replacement of a laminarily organized luminal organ or tissue structure. Such subjects include, without limitation, subjects who are newly diagnosed or previously diagnosed and now experiencing a recurrence or relapse, or are at risk for deficient organ function or failure, no matter the cause. The subject may have been previously treated for a condition associate with deficient organ function or failure, or not so treated. Subjects may be candidates for a urinary diversion including, without limitation, subjects having cancer of the bladder requiring a cystectomy, subjects having a neurogenic bladder that impacts renal function, subjects having radiation injury to the bladder, and subjects having intractable incontinence. The subject may be newly diagnosed as requiring a urinary diversion, or previously diagnosed as requiring a urinary diversion and now experiencing complications, or at risk for a deficient, damaged or non-functional urinary system, no matter the cause. The subject may have been previously treated for a condition associated with a deficient, damaged or non-functional urinary system, or not so treated. The cell populations described herein are non-autologous to the subject.

The described techniques may be used to expand an existing laminarily organized luminal organ or tissue structure in a patient in need of such treatment. For example, an existing laminarily organized luminal organ or tissue structure may be enlarged by providing a polymeric matrix or scaffold shaped to conform to at least a part of the organ or tissue structure in need of said treatment and of a sufficient size to be laparoscopically implanted, depositing a cell population that non-autologous to the subject and is not derived from the organ or tissue structure on or in a first area of said polymeric matrix; and laparoscopically implanting the shaped polymeric matrix construct into said patient at the site of said treatment such that the existing laminarily organized luminal organ or tissue structure is expanded.

Figure 7e depicts possible surgical methods for the implantation of a muscle equivalent scaffold described herein. Figure 7f depicts implantation sites on an empty and full bladder. Figure 7g depicts a urinary bladder model with surgical slit showing ellipsoid created upon sectioning of surface. A plastic tube may be used as a model of the limited space available in order to pass the folded or rolled polymeric matrices or scaffolds.

The described techniques may also be used to increase bladder volumetric capacity in a patient in need of such treatment. For example, bladder volumetric capacity may be increased by providing a biocompatible synthetic or natural polymeric matrix shaped to conform to at least a part of the organ or tissue structure in need of said treatment and of a sufficient size to be laparoscopically implanted; depositing a cell population that is non-autologous to the subject and is not derived from the corresponding organ or tissue structure that is the subject of increase in capacity on or in a first area of said polymeric matrix; and laparoscopically implanting the shaped polymeric matrix construct laparoscopically into said patient at the site of said treatment such that bladder volume capacity is increased. In one embodiment, the matrix or scaffold of the instant invention is suitable for increasing bladder volume capacity about 50 mL. In other embodiments, the matrix or scaffold of the instant invention is suitable for increasing bladder volume capacity about 100 mL. In other embodiments, the matrix or scaffold of the instant invention is suitable for increasing bladder volume capacity about 60, about 70, about 80, or about 90 mL.

The described techniques may further be used to expand a bladder incision site in a patient in need of such treatment. For example, a bladder incision site may be expanded by providing a biocompatible synthetic or natural polymeric matrix shaped to conform to at least a part of the organ or tissue structure in need of said treatment and of a sufficient size to be laparoscopically implanted; b) depositing a cell population that is non-autologous to the subject and is not derived from the corresponding organ or tissue structure that is the subject of increase in capacity on or in a first area of said polymeric matrix; and c) laparoscopically implanting the shaped polymeric matrix construct laparoscopically into said patient at the site of said treatment such that the bladder incision site is expanded.

Another use includes methods for the treatment of urinary incontinence in a patient in need of such treatment. For example, urinary incontinence may be treated by providing a biocompatible synthetic or natural polymeric matrix shaped to conform to at least a part of the organ or tissue structure in need of said treatment and of a sufficient size to be laparoscopically implanted; depositing a cell population that is non-autologous to the subject and is not derived from the corresponding organ or tissue structure that is the subject of treatment on or in a first area of said polymeric matrix; and laparoscopically implanting the shaped polymeric matrix construct laparoscopically into said patient at the site of said treatment such that bladder volume capacity is increased.

In one other aspect, the present disclosure concerns methods for the use of constructs for the reconstruction, replacement, augmentation, or regeneration of native luminal organs or tissue structures of the urinary system that contain smooth muscle cells (SMCs) derived from a bladder source that is a non-autologous source. The native organs or tissue structures may be laminarly organized. The non-autologous source may be an Allogeneic source or a syngeneic source. In one preferred embodiment, the methods of treatment include the use of a construct formed from a scaffold and bladder-derived SMCs but is free of urothelial cells. The bladder-derived SMCs may be seeded onto a bladder augmentation, a bladder replacement, a urinary conduit, or a muscle equivalent scaffold to form a construct. The construct containing non-autologous bladder-derived SMCs may used in the methods of treatment described herein.

In one other aspect, the present disclosure provides methods for the regeneration of a neo-bladder following implantation into a subject in need thereof based upon biomechanical stimulation or cycling. In one aspect, the methods are suitable for use in promoting the regeneration of an implanted neo-bladder construct that has been implanted for the augmentation or replacement of a bladder or a portion of a bladder. In one embodiment, the neo-bladder construct is formed from seeding cells on a neo-bladder matrix or scaffold. In another embodiment, the neo-bladder scaffold is a bladder replacement scaffold, a bladder augmentation scaffold, a bladder conduit scaffold, or a detrusor muscle equivalent scaffold.

In one aspect, the method of the present disclosure applies to implanted neo-bladder constructs formed from seeding neo-bladder scaffolds with at least one cell population. In one embodiment, the cell-seeded polymeric matrix (or matrices) is a bladder replacement scaffold, a bladder augmentation scaffold, a bladder conduit scaffold, or a detrusor muscle equivalent scaffold. In one embodiment, the at least one cell population comprises substantially a muscle cell population. In another embodiment, the muscle cell population may be a smooth muscle cell population. Different densities of cells for seeding may be appropriate as described herein.

In one aspect, the methods of the present disclosure are performed at different times and for different durations following the implantation of the neo-bladder. In one embodiment, the cycling is performed on a daily basis over a period of time, on a weekly basis over a period of time, or every other week. In another embodiment, the duration of the daily cycling regimen is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, or longer than 14 weeks. An exemplary protocol for cycling is described in Example 18.

In one embodiment, a daily cycling protocol for a subject may include the steps of filling the neo-bladder for about an hour, draining the filled neo-bladder for about an hour, and allowing the neo-bladder to drain freely, typically overnight. This protocol can be performed on day one of the cycling regimen in the subject. This daily sequence can be performed for a number of consecutive days after the first day. In one embodiment, the cycling protocol may be performed on a day after day one in which the duration of the filling step is increased to about two hours, about three hours, about four hours, or more than about four hours. In another embodiment, the filling and draining steps may be repeated more than once daily before allowing the neo-bladder to drain freely.

In another embodiment, the subjects are catheterized post-implantation, and the cycling time is controlled by clamping and unclamping the subject's catheter.

Those of ordinary skill in the art will appreciate that additional cycling regimens are contemplated herein.

An example of a cycling protocol is as follows. Following implantation of a neo-bladder construct formed by seeding a neo-bladder matrix or scaffold with cells as described herein, cycling will be performed every 2 weeks (14 ± 2 day intervals) starting approximately 1 month after implantation and continuing until approximately Day 90. Cycling will be completed after certain types of assessment, such as compliance measurement of the implanted neo-bladder, but before other types of assessment such as fluoroscopic imaging. Cycling will be performed by re-inflating the bladder with sterile saline (warmed by incubator) after the completion of compliance measurement at a rate of 10-25 mL/min. The cycling will be repeated at least 5-10 times. The starting pressure of 0-10 mmHg will be achieved and recorded along with the start time. Time, volume of isotonic solution delivered, and the pressure obtained will be recorded for each cycle at the time leakage is observed around the catheter (a.k.a. leak point), or when the volume delivered is equal to that of the Compliance measurement just performed, whichever comes first.

In one embodiment, the present disclosure provides a method of promoting regeneration of a neo-bladder implanted in a subject that includes the steps of (a) filling the implanted neo-bladder with a fluid; (b) emptying the filled neo-bladder of step (a). In another embodiment, the method includes step (c) repeating steps (a) and (b). In one other embodiment, the method is commenced within the first 2 weeks post-implantation. In one embodiment, the steps (a) and (b) are performed once daily, once weekly, or once every other week. In some other embodiments, the filling step (a) is performed for about one hour and the emptying step (b) is performed for about one hour. In yet another embodiment, steps a) and b) are performed at least until about six weeks post-implantation. In one other embodiment, steps a) and b) are not performed for more than about ten weeks post-implantation. In another embodiment, steps a) and b) are performed for more than about ten weeks post-implantation. In other embodiments, the filling comprises expanding the neo-bladder. In another embodiment, the regeneration comprises an increase in the capacity of the neo-bladder as compared to a neo-bladder in a subject that has not undergone cycling. In one other embodiment, the regeneration comprises an increase in compliance of the neo-bladder as compared to a neo-bladder in a subject that has not undergone cycling. In other embodiments, the regeneration comprises an increase in extracellular matrix development in the neo-bladder as compared to a neo-bladder in a subject that has not undergone cycling. In one embodiment, the increase in extracellular matrix development comprises the development of elastin fibers.

In one other aspect, the present disclosure concerns methods for providing homeostatic regulative development of neo-bladders in mammals such that implanted neo-bladders are responsive to the needs of the recipient. In one embodiments, the implanted neo-bladder grows to a size proportionate to the recipient. In another embodiment, the methods for providing homeostatic regulative development of a neo-bladder in a subject include the steps of (a) providing a biocompatible polymeric scaffold; (b) depositing an a first cell population on or in a first area of said scaffold, said first cell population being substantially a muscle cell population; and (c) implanting the scaffold of step (b) into said subject to establish homeostatic regulative development. In one other embodiment, the homeostatic regulative development comprises restoration of organ size and structure. In another embodiment, the homeostatic regulative development comprises neo-bladder capacities proportionate to body weight. In one embodiment, the proportionate neo-bladder capacity is achieved at about four months post-implantation. In another embodiment, the method for providing homeostatic regulative development of a neo-bladder, in a subject includes the step of monitoring the state of homeostatic regulative development or progress of the implanted neo-bladder. The monitoring may include a cystogram procedure to show the position and shape of the implanted neo-bladder, and/or a measurement of urodynamic compliance and capacity.

The methods of the present disclosure have application for the treatment of subjects with disorders related to the urinary system. Such subjects include any single human subject, including a patient, eligible for treatment, who is experiencing or has experienced one or more signs, symptoms, or other indicators of a a deficient organ function or failure, including a deficient, damaged or non-functional urinary system. Such subjects include, without limitation, subjects who are newly diagnosed or previously diagnosed and now experiencing a recurrence or relapse, or are at risk for deficient organ function or failure, no matter the cause. The subject may have been previously treated for a condition associate with deficient organ function or failure, or not so treated. Subjects may be candidates for a urinary diversion including, without limitation, subjects having cancer of the bladder requiring a cystectomy, subjects having a neurogenic bladder that impacts renal function, subjects having radiation injury to the bladder, and subjects having intractable incontinence. The subject may be newly diagnosed as requiring a urinary diversion, or previously diagnosed as requiring a urinary diversion and now experiencing complications, or at risk for a deficient, damaged or non-functional urinary system, no matter the cause. The subject may have been previously treated for a condition associated with a deficient, damaged or non-functional urinary system, or not so treated.

### C. Use of Gastrointestinal tissue constructs

In one aspect, the present invention contemplates methods for providing a GI organ or GI tissue structure to a subject in need of such treatment. In one embodiment, the GI organ or tissue structure may be a laminarily organized luminal organ or tissue structure. In another embodiment, the subject may be in need of reconstruction, augmentation, or replacement of an organ or tissue. In one embodiment, the method includes the step of providing a biocompatible synthetic or natural polymeric matrix shaped to conform to at least a part of the GI organ or GI tissue structure in need of an organ or tissue structure. The providing step may be followed by depositing at least one cell population that is autologous or non-autologous to the subject and is not derived from the corresponding GI organ or GI tissue structure that is the subject of the reconstruction, augmentation or replacement. The depositing step may include culturing the cell population on the polymeric matrix. After depositing the cell population on the matrix to provide a construct, it can be implanted into a patient at the site of treatment for the formation of the desired laminarily organized luminal GI organ or GI tissue structure. In one embodiment, the laminarly organized luminal GI organ or GI tissue structure is a esophagus or a part of a esophagus; or a small intestine or a part of a small intestine.

In one other aspect, the present invention relates to methods for providing a GI organ or GI tissue structure to a subject in need. In one embodiment, the method includes the steps of a) providing a biocompatible synthetic or natural polymeric matrix shaped to conform to at least a part of the GI organ or GI tissue structure in need of said treatment; b) depositing on or in a first area of the polymeric matrix a cell population that is not derived from a GI organ or GI tissue; and c) implanting the shaped polymeric matrix cell construct into the subject for the formation of a GI organ or tissue structure. In one other aspect, the present invention provides methods for providing a GI organ or GI tissue structure to a subject in need. In one embodiment, the method includes a) providing a biocompatible synthetic or natural polymeric matrix shaped to conform to a GI organ or GI tissue structure; b) depositing a cell population that is not derived from a GI organ or GI tissue on or in a first area of the polymeric matrix; and c) implanting the shaped polymeric matrix cell construct into the subject for the formation of the GI organ or tissue structure. In another embodiment, the cell population of step b) of the methods described herein contains one or more peripheral blood-derived smooth muscle cells having contractile function that are positive for a smooth muscle cell marker, or the cell population of step b) contains one or more adipose tissue-derived smooth muscle cells having contractile function that are positive for a smooth muscle cell marker. In one other embodiment, the contractile function of the cell population is calcium-dependent.

In one other aspect, the present invention provides methods for providing a GI construct for a defective GI system in a subject in need. In one embodiment, the method for providing a GI construct to a subject in need includes the steps of (a) providing a biocompatible GI tissue scaffold; (b) depositing a first cell population on or in a first area of said scaffold, said first cell population being substantially a muscle cell population; (c) implanting the scaffold of step (b) into said subject to form GI tissue in the subject. The method may further include the step of depositing a second cell population on or in a first area of said scaffold and/or contacting the second cell population with the deposited first cell population after step (b). In another embodiment, the biocompatible material is biodegradeable. In other embodiments, the biocompatible material is polyglycolic acid. In yet another embodiment, the first cell population is substantially a smooth muscle cell population. In another embodiment, the second cell population is a GI cell population, e.g., an esophageal cell population, a small intestinal cell population, etc.

In one embodiment, the methods of the present invention further include the step of wrapping the implanted GI tissue construct with the subject's omentum, mesentery, muscle fascia, and/or peritoneum to allow for vascularization.

In one aspect, the present invention contemplates the application of the smooth muscle cell populations described herein for GI-related disorders. Methods to treat esophageal-related disorders are also contemplated herein. The esophagus contains smooth muscle and an esophagus-related disorder is one in which the subject has a defective esophagus due to improper function, *e.g.,* dysfunctional esophageal muscles. It has been reported that certain cell populations may provide beneficial effects to the esophagus when administered. (e.g., Nakase (2008) *supra*).

In another aspect, the present invention provides methods for the treatment of GI-related disorders. The term "gastrointestinal disorder", "GI disorder", "gastrointestinal-related disorder" or "GI-related disorder" shall refer to any defect within the GI tract, which is made up of the esophagus, stomach, small and large intestines, anal sphincter, and anus. The defect may be a structural defect occurring at any point along the gastrointestinal tract, and may cause obstructions or blockages that can lead to vomiting, as well as swallowing problems and bowel movement problems. The defect may also include an interruption or gap along the GI tract. Those of ordinary skill in the art will appreciate that various GI disorders may be suitable for treatment with the constructs and methods described herein. In one embodiment, the GI-related disorder suitable for treatment is an esophageal-related disorder including, without limitation, Barrett's esophagus, esophageal atresia, long-gap esophageal atresia, tracheoesophageal fistula, atresia with tracheoesophageal distal fistula, atresia with tracheoesophageal proximal fistula, and atresia with tracheoesophageal double fistula. In another embodiment, the GI-related disorder is a small intestine-related disorder resulting from small bowel resection performed when essential for patients that present certain indications. For example, massive resection may be performed for individuals with inflammatory bowel disease, trauma, mesenteric vascular disease, volvulus, congenital atresias, and neonatal necrotizing enterocolitis. A common consequence of such resections is Short Bowel Syndrome (SBS), which results from disruption of normal nutrient and fluid absorption, including deficiencies in calcium, magnesium, zinc, iron, B12, and fat soluble vitamins, and is characterized by diarrhea, dehydration, malabsorption of nutrients and concomitant progressive malnutrition.

In another embodiment, the GI-related disorder is cancerous in nature including, without limitation, esophageal cancer, stomach cancer, intestinal cancer, cancer of the sphincter, or colon cancer.

In one aspect, the present invention provides methods for the regeneration, reconstruction, augmentation or replacement of gastro-intestinal tissue in a subject in need. In one embodiment, the method includes the step of administering a gastro-intestinal tissue construct that includes (a) a scaffold; (b) a first cell population that is not derived from gastro-intestinal tissue deposited on or in a first surface of the scaffold; and (c) a second cell population derived from gastro-intestinal tissue. In one other embodiment, the first cell population is derived from adipose. In another embodiment, the first cell population is a smooth muscle cell (SMC) population. The SMC population may be positive for at least one smooth muscle cell marker. In yet another embodiment, the second cell population is derived from esophagus, small intestine, large intestine, stomach, colon, or anal sphincter. In one embodiment, the construct is positive for at least one gastro-intestinal (GI) tissue marker. The GI tissue marker may be an epithelial cell marker. In another embodiment, the construct includes cells having coordinated rhythmic contractile function. In one other embodiment, the construct is adapted to form gastro-intestinal tissue following implantation. In yet another embodiment, the GI tissue is esophagus, small intestine, large intestine, stomach, colon, or anal sphincter tissue.

In another embodiment, the method includes the step of obtaining one or more samples form the subject prior to the administering step. In one other embodiment, the method includes the step of isolating one or more cell populations from the samples and culturing them as described herein. In yet another embodiment, the method includes the step of contacting one or more cell populations with a GI tissue scaffold described herein to form a GI tissue construct.

In one aspect, the present invention provides the use of a construct described herein for the preparation of a medicament useful in the treatment of a GI-related disorder in a subject in need. In one embodiment, the construct is an esophageal tissue construct that includes an esophageal tissue scaffold seeded with a smooth muscle cell population and an esophageal cell population. In another embodiment, the GI-related disorder is an esophageal-related disorder. In one embodiment, the construct is an intestinal tissue construct that includes an intestinal tissue scaffold seeded with a smooth muscle cell population and an intestinal cell population. The intestinal cell population may be derived from small intestine. In another embodiment, the GI-related disorder is an intestinal-related disorder.

The methods of the present invention have application for the treatment of subjects with gastrointestinal disorders. Such subjects include any single human subject, including a patient, eligible for treatment, who is experiencing or has experienced one or more signs, symptoms, or other indicators of deficient gastro-intestinal (GI) function or failure, including a deficient, damaged or non-functional gastro-intestinal system. Such subjects include, without limitation, subjects who are newly diagnosed or previously diagnosed and now experiencing a recurrence or relapse, or are at risk for deficient GI function or failure, no matter the cause. The subject may have been previously treated for a condition associated with deficient GI function or failure, or not so treated. Subjects may be candidates having a GI-related disease including, without limitation, subjects having an esophageal-related disease, a stomach-related disease, an intestinal-related disease, or a disease related to the anal sphincter. The subject may be newly diagnosed as requiring treatment for such a disease, or previously diagnosed as requiring treatment for such a disease and now experiencing complications, or at risk for a deficient, damaged or non-functional esophagus, stomach, intestine, or anal sphincter, no matter the cause. The subject may have been previously treated for a condition associated with a deficient, damaged or non-functional esophagus, stomach, intestine, or anal sphincter, or not so treated.

### D. Use of Respiratory tissue constructs

In one aspect, the present disclosure contemplates methods for providing a respiratory tissue structure to a subject in need of such treatment. In one embodiment, the subject may be in need of reconstruction, regeneration, augmentation, or replacement of respiratory tissue, such as a lung, lung tissue, alveolar tissue, and bronchiolar tissue. In one embodiment, the method includes the step of providing a biocompatible synthetic or natural polymeric matrix shaped to conform to at least a part of a respiratory tissue in need of an organ or tissue structure. The providing step may be followed by depositing on or in the matrix a first cell population that is not derived from respiratory tissue. The first cell population may be an SMC population. The depositing step may include culturing the first cell population on the polymeric matrix. After depositing the first cell population on the matrix, a second cell population may be deposited on the matrix such that it contacts the matrix and/or the deposited first cell population to form a construct. The second cell population may be a respiratory cell population. The construct may be a respiratory tissue construct that can be implanted into a patient at the site of treatment for the formation of the desired respiratory tissue structure. In one embodiment, the respiratory tissue structure is part of a lung. In one embodiment, the first and/or the second cell populations are autologous to the subject in need of treatment. In another embodiment, the first and/or the second cell populations are non-autologous to the subject in need of treatment.

In another aspect, the present disclosure provides methods for the regeneration, reconstruction, augmentation or replacement of respiratory tissue in a subject in need. In one embodiment, the method includes the step of administering a respiratory tissue construct that includes (a) a scaffold; (b) a first cell population that is not derived from respiratory tissue deposited on or in a first surface of the scaffold; and (c) a second cell population derived from respiratory tissue. The present invention provides scaffolds suitable for the formation of a respiratory tissue construct, e.g., a respiratory tissue scaffold seeded with cells. In one embodiment, the scaffold comprises a cell population as described herein. In another embodiment, the scaffold comprises an adipose-derived SMC population and a respiratory cell population. In another embodiment, the scaffold consists essentially of an adipose-derived SMC population and a respiratory cell population, as described herein. In one other embodiment, the scaffold consists of an adipose-derived SMC population and a respiratory cell population, as described herein. The cell populations may be autologous or non-autologous to the subject.

In another embodiment, the cells deposited on the implanted construct produce MCP-1 and release it at the site of implantation. MCP-1 may stimulate native mesenchymal stem cells (MSCs) to migrate to the site of implantation. In one other embodiment, the native MSCs may facilitate and/or enhance regeneration of the implanted construct at the site of implantation.

In one embodiment, the cell population deposited is a smooth muscle cell (SMC) population derived from adipose tissue as described herein. In another embodiment, the SMC population includes at least one cell that has contractile function and is positive for a smooth muscle cell marker, such as myocardin, alpha-smooth muscle actin, calponin, myosin heavy chain, BAALC, desmin, myofibroblast antigen, SM22, and any combination thereof. In other embodiments, the SMC population includes at least one cell that demonstrates myocardin (MYOCD) expression. The MYOCD expression may be expression of a nucleic acid encoding a MYCOD polypeptide or a MYOCD polypeptide. In another embodiment, the contractile function of the SMC is calcium-dependent. In another embodiment, the polymeric matrix is seeded with an adipose-derived SMC population and a respiratory cell population.

In all embodiments, the methods of the present disclosure utilize a construct for implantation that is based upon a respiratory tissue scaffold that has been seeded with a cell population as described herein.

In another embodiment, the methods for the regeneration, reconstruction, augmentation or replacement of respiratory tissue described herein include the steps of a) providing a biocompatible synthetic or natural polymeric matrix shaped to conform to at least a part of the respiratory tissue in the subject in need of treatment; b) depositing a first cell population on or in a first area of said polymeric matrix at a cell density described herein, said first cell population being substantially an SMC population; c) depositing a second cell population on or in a second area of the polymeric matrix (and/or to contact the deposited first cell population) at a cell density described herein; and d) implanting the shaped polymeric matrix cell construct into a subject at the site of said treatment for the formation of the respiratory tissue structure. In one other embodiment, the respiratory tissue structure formed in vivo exhibits the behavior of natural respiratory tissue. In one embodiment, the respiratory tissue structure exhibits coordinated rhythmic contractile function.

In one aspect, the method of the present disclosure applies to implanted respiratory tissue constructs formed from seeding respiratory tissue scaffolds with a first and a second cell population. In one embodiment, the first cell population is an adipose-derived smooth muscle cell population and the second cell population is a respiratory cell population. Different densities of cells for seeding may be appropriate as described herein.

In another embodiment, the smooth muscle cell populations of the present disclosure may be administered to a subject having a respiratory disorder without the use of a scaffold, such as by engraftment. Those of ordinary skill in the art will appreciate suitable methods of engraftment.

The methods of the present disclosure have application for the treatment of subjects with respiratory disorders. Airway smooth muscle is present in the bronchial tree of most vertebrates. A respiratory disorder is one in which the subject has a defective respiratory system due to improper function of the muscles of the lung. It has been reported that certain cell populations may provide beneficial effects when administered to the lung (e.g., Ohnishi et al. Int J Chron Obstruct Pulmon Dis. 2008 December; 3(4): 509-514). Individuals with lung cancer could also benefit. Subjects that might benefit from treatment by the methods described herein include any single human subject, including a patient, eligible for treatment, who is experiencing or has experienced one or more signs, symptoms, or other indicators of deficient respiratory function or failure, including a deficient, damaged or non-functional respiratory system. Such subjects include, without limitation, subjects who are newly diagnosed or previously diagnosed and now experiencing a recurrence or relapse, or are at risk for deficient respiratory function or failure, no matter the cause. The subject may have been previously treated for a condition associated with deficient respiratory function or failure, or not so treated. Subjects may be candidates having a lung-related disease including, without limitation, subjects having chronic obstructive pulmonary disease (COPD) (i.e., chronic bronchitis, emphysema), lung cancer, idiopathic pulmonary fibrosis (IPF), asthma, obstructive and restrictive airway diseases, pulmonary hypoplasia (e.g., from pre-mature birth). The subject may be newly diagnosed as requiring treatment for a lung-related disease, or previously diagnosed as requiring treatment for a lung-related disease and now experiencing complications, or at risk for a deficient, damaged or non-functional lung, no matter the cause. The subject may have been previously treated for a condition associated with a deficient, damaged or non-functional lung, or not so treated.

### E. Use of Blood vessel constructs

The blood vessel scaffolds and constructs may be used in methods for treating a cardiovascular disorder in a subject in need thereof. In one embodiment, the method includes the step of implanting a blood vessel construct. In another embodiment, the blood vessel construct may include a) a biocompatible tubular scaffold having a first and a second surface; and b) a first cell population derived from a non-vascular source deposited on or in the first surface of the scaffold. In one embodiment, the first cell population is a smooth muscle cell population.

The method of treating a cardiovascular disorder may include the step of identifying a subject in need. One or more biopsies or samples may be obtained from the subject. In one other embodiment, the method includes the step of isolating one or more cell populations from the sample(s) and culturing the one or more cell populations on a scaffold to provide a construct or TEBV. In another embodiment, the culturing includes conditioning of a cell-seeded scaffold in a bioreactor. In one embodiment, the conditioning comprises steady and/or pulsatile flow in a bioreactor. In another embodiment, the method includes the implantation of the cell-seeded, conditioned TEBV into the subject in need to treat the cardiovascular disease or disorder.

Those of ordinary skill in the art will appreciate the various cardiovascular disorders that are suitable for treatment by the methods of the present invention. In general, the cardiovascular disorder is a disorder that would benefit from the formation of a passage or anastomosis between two native vessels where blood is diverted from a first native blood vessel directly to a second native blood vessel. For example, the first native vessel may be an artery and the second native vessel may be a vein, such as in the case of an arteriovenous (AV) shunt. The methods and constructs of the present disclosure may also be suitable for a variety of other shunts including, without limitation, a Blalock-Taussig shunt, a cardiovascular shunt, a left-to-right shunt, a right-to-left shunt, a LaVeen peritoneovenous shunt, a portacaval shunt, and a splenorenal shunt.

In another embodiments, the present disclosure provides the use of the TE scaffolds and/or TEBVs described herein for the preparation of a medicament useful in the treatment of a cardiovascular disorder in a subject in need. The scaffolds are also provided for use in making a blood vessel construct. The blood vessel constructs are provided for use in treating a cardiovascular disorder.

The methods of the present disclosure have application for the treatment of subjects with cardiovascular disorders. Such subjects include any single human subject, including a patient, eligible for treatment who is experiencing or has experienced one or more signs, symptoms, or other indicators of a deficient cardiovascular function or failure. Such subjects include without limitation subjects who are newly diagnosed or previously diagnosed and now experiencing a recurrence or relapse, or are at risk for deficient cardiovascular function or failure, no matter the cause. The subject may have been previously treated for a condition associated with deficient cardiovascular function or failure, or not so treated.

The following examples are offered for illustrative purposes only, and are not intended to limit the scope of the present invention in any way.

### EXAMPLES

### EXAMPLE 1 - Peripheral blood and adipose tissue as a source of SMCs

### Blood-derived cells

As described in Ludlow et al. U.S. Published Patent Application No. 201 00131075, smooth muscle cells have been successfully isolated from canine, porcine, and human peripheral blood. All the results described in Example 1 can also be found in Ludlow *et al.* Briefly, a dilution of 50 ml of peripheral blood 1:1 with phosphate buffered saline (PBS; 100 mL final volume) was prepared and layered onto Histopaque, a density gradient material, and centrifuged at 1,354 xg for 20 minutes at room temperature. After centrifugation, four layers will be clearly defined in the density gradient (from top to bottom): serum, buffy coat, Histopaque, red blood cells. The mononuclear cells are located in the buffy coat, which appears as an opaque white/gray band. The buffy coat was withdrawn and transfered into a separate 50ml conical tube. Dilute to 50 mL with PBS. Centrifuge the samples at 711 xg for 10 minutes at room temperature to pellet cells. Resuspend pellet and culture the cells. When appropriate cell numbers are reached by subsequent cell passaging, an aliquote is fixed and processed for end-point analysis including immunodetection of expressed smooth muscle cell proteins, nucleic acid detection of smooth muscle cell mRNA transcripts, cellular contraction, cytokine and enzyme synthesis.

### RESULTS

Media selection. The mononuclear fraction of a single 40-50 mL canine peripheral blood sample was resuspended in six different media formulations and seeded into 6-well Primaria or collagen-coated plates. After one week of culture, small adherent colonies and small cell aggregates were observed in all conditions (DMEM media isolations are not shown) but the identity of the cell types were indiscernible. Small clusters and cell aggregates were observed on Primaria culture dishes when grown in alpha-MEM + 10% FBS, EGM-2 medium with all of the accompanying supplements, and EGM-2 with selected accompanying supplements (minus VEGF and FGF2) and collagen type I coated on tissue culture plastic 85 plates grown in the same medias. Similar results were seen in peripheral blood cultures grown in DMEM formulations.

After two weeks of culture, outgrowth colonies and small monolayers were observed in α-MEM on Primaria and collagen-coated plates. Morphologically, these colonies appeared smooth muscle or endothelial. Outgrowth colonies of smooth muscle or endothelial morphology also formed in other media/substrate conditions. Some macrophages were initially maintained in alpha-MEM, but did not carry over into subsequent passages. Cells isolated in αMEM with 10% FBS on Primaria plates were of smooth muscle or macrophage morphology. No endothelial cells were isolated under these conditions. Cells isolated in αMEM/10% FBS on collagen I plates were of smooth muscle, endothelial and macrophage morphology. Other media/substrate formulations such as EGM-2 and DMEM supplemented with 20% FBS also permitted outgrowth of mescenchymal- and endothelial-like cells.

Of the twelve media/substrate conditions, Primaria plates with alpha-MEM /10% FBS contained the most homogeneous isolation of smooth muscle cells without outgrowth colonies of endothelial cells. Cells isolated on Primaria plates and expanded on Nunclon surfaces (in alpha-MEM/10% FBS) exhibited the classical 'hill and valley' morphology that is typical of smooth muscle cells (SMC), and is consistent with descriptions in other studies (Kassis et al. (2006); Koerner et al. (2006); Simper et al. (2002), *supra).*

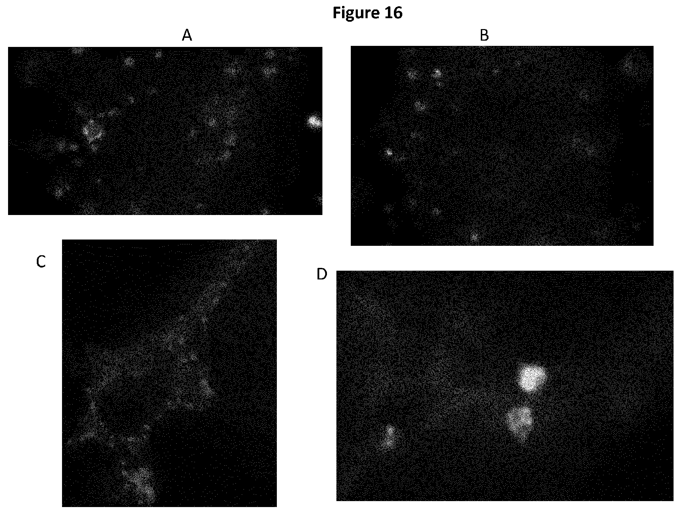

These cells also maintained this morphology for several passages. As shown in Figure 16 of Ludlow et al. U.S. Patent Application No. 20100131075, images of porcine carotid artery SMC (H) and dog bladder SMC (I) are shown for comparison. The smooth muscle cells at later passages (F, G) became larger and more spread out, appearing more like mature SMC. Early passages (A-E) resemble smooth muscle cells (SMC) isolated from porcine carotid artery (H) and dog bladder (I). Later passages of smooth muscle cells (F, G) are larger and more spread out, suggesting a smooth muscle phenotype.

### Adipose-derived cells

Smooth muscle cells have been isolated from porcine adipose tissue according the following procedure. All procedures are performed in the biosafety hoods.

Obtain adipose sample. Store at room temperature or 4° C for no more than 24 hours prior to use in biosafety container. Prepare collagenase solution by adding 1gm of BSA and 0.1gm of collagenase per 100ml of PBS. Filter the solution through a 0.2µm filter unit. Warm to 37° C. Add equivalent volume of Collagenase solution per adipose volume to each centrifuge bottle. One tissue volume of collagenase solution is required (i.e. 10ml of collagenase solution per 10ml adipose tissue). Wipe the tubes with disinfectant, cap, wrap with parafilm and place in a 37°C incubator on a rocker for 60 minutes. Centrifuge at 300 xg at Room Temperature for 5 minutes. Take the tubes out of the centrifuge and shake them vigorously for 10 seconds to thoroughly mix the cells. This is to complete the separation of stromal cells from the primary adipocytes. Centrifuge again at 300 xg for 5 minutes. Carefully aspirate off the oil on top, the primary adipocytes (yellow layer of floating cells), and the collagenase solution. Leave behind approximately 10ml of the brown collagenase solution above the pellet so that the stromal-vascular fraction (dark red cells on bottom) is not disturbed. Resuspend the pellet of cells in PBS with 1%BSA and filter using Steri-Flip. Centrifuge the cells at 300 xg for 5 minutes and aspirate the remaining collagenase solution. When aspirating, the tip of the pipette should aspirate from the top so that the oil is removed as thoroughly as possible. The cell pellet should be tightly packed at the bottom. Add 10ml of tissue culture medium to each centrifuge tube and resuspend the cells. Pool the cells to one tube and centrifuge again. Aspirate supernatant. Suspend the cells in 10 ml of medium. Divide the cells equally and accordingly to the appropriate number of flasks. 24-72 hours after plating, aspirate medium from flask. Wash with PBS and aspirate. Add the original volume per flask of fresh medium. Cells will be grown to 80-90% confluence and then either passaged, frozen down as P1 (Passage One) cells or differentiated. When appropriate cell numbers are reached by subsequent cell passaging, an aliquot is fixed and processed for immunodetection of expressed smooth muscle cell proteins.

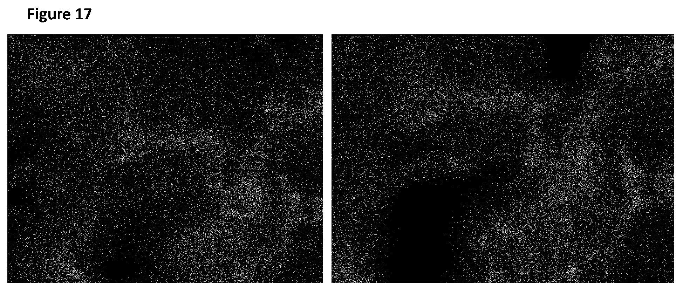

The morphology of the cultures was assessed after 3 to 5 days in culture. Human and porcine cells derived from adipose tissue exhibit smooth muscle cell morphological characteristics (Figure 17 of Ludlow et al. U.S. Patent Application No. 20100131075). The cells demonstrate a hill-and-valley morphology and exhibit additional characteristics such as spindly shaped, flattened and fibroblast-like upon passage, elongated and arranged in parallel rows, and a "whirled" appearance of growth, all of which are typical of cultured smooth muscle cells.

Smooth muscle markers. Increased expression of contractile genes (and the proteins they encode) is associated with SMC maturation. We determined if the smooth muscle cells isolated from blood or adipose tissue expressed the smooth muscle cell markers myocardin, smooth muscle alpha actin, SM22, myosin heavy chain, and calponin by isolating total RNA and performing semi-quantitative RT-PCR. The results indicate that these cells express all of these smooth muscle cell markers at the gene level, consistent with the smooth muscle cell markers found in bladder smooth muscle cells. These data support the notion that these smooth muscle cells isolated from peripheral blood or adipose tissue have properties of smooth muscle cells.

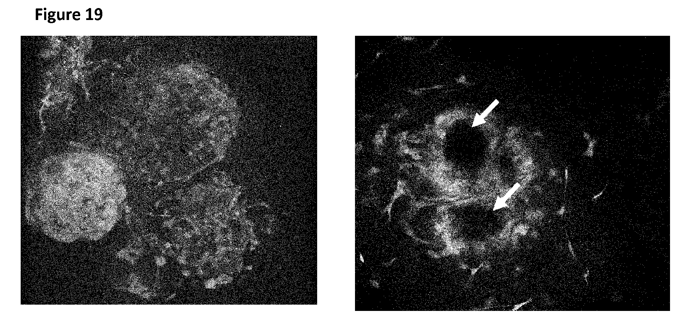

Phenotypic characterization. We have already shown that these peripheral blood isolated smooth muscle cells express a transcriptional regulator of smooth muscle gene expression as well as specific smooth muscle contractile proteins. RT-PCR analysis was conducted for gene expression of SMC markers myocardin, smooth muscle alpha-actin, SM22, smooth muscle myosin heavy chain, and calponin (see Figure 19 of Ludlow et al. U.S. Patent Application No. 20100131075). Samples were from smooth muscle cells isolated from porcine adipose, peripheral blood, and bladder (passage 4). The SMCs isolated from adipose tissue can be cultured 3-5 days between each passage, while the SMCs isolated from blood can be cultured for 14 days before the first passage and then 3-5 days for additional passages. Gene expression for beta-actin was used as an internal loading control for the gel. Expression profiles for adipose and peripheral blood cell isolates are comparable to that of the bladder SMC.

Immunofluorescence staining was performed utilizing a variety of antibodies directed towards smooth muscle cell expressed protein markers. The markers alpha-actin, SM22, calponin, and smooth muscle myosin heavy chain were examined in smooth muscle cells isolated from porcine adipose, peripheral blood, and bladder. These proteins are all involved in the contractile function of smooth muscle cells. Smooth muscle cells at multiple passages stained positively for smooth muscle alpha actin, SM22, calponin, and smooth muscle myosin heavy chain. Subcellular localization of these proteins was virtually identical in smooth muscle cells compared to bladder SMC. Detailed staining of these proteins in the stress fibers of the cells was noted. This pattern of staining is typical and expected for smooth muscle cells.

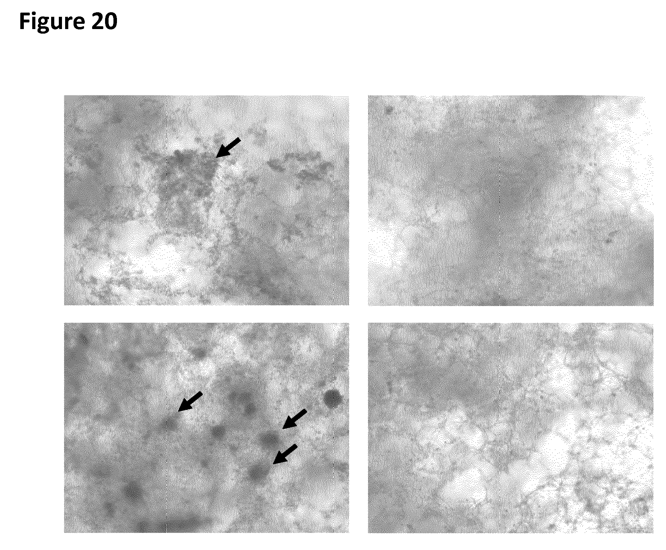

Immunostaining of smooth muscle cells isolated from human peripheral blood was observed at passage 5 (see Figure 20 of Ludlow et al. U.S. Patent Application No. 20100131075). Probes for smooth muscle alpha actin, SM22, and calponin were used. Dual staining for smooth muscle alpha actin and calponin (top right panel) reveals co-expression of these two proteins within the same cells. This simultaneous expression of more than one smooth muscle cell marker in a single cell further supports the notion that these smooth muscle cells..

Contractility. Since the peripheral blood derived smooth muscle cells express smooth muscle contractile proteins, we performed a three-dimensional gel contraction assay to assess their capacity to function like SMC. SMC have been shown to spontaneously induce contraction of a collagen matrix when embedded in a three-dimensional gel (Travis et al. (2001) Circ Res 88:77-83). Adipose tissue-derived smooth muscle cells were also tested for contractility.

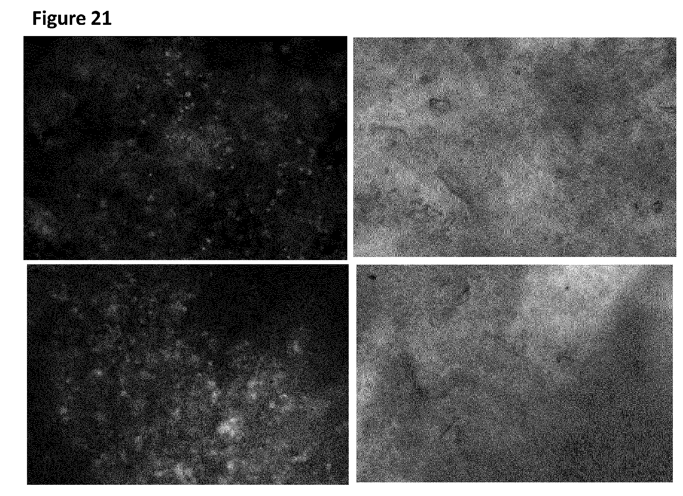

As shown in Figure 21 of Ludlow et al. U.S. Patent Application No. 20100131075, porcine blood-derived (A) and porcine adipose tissue-derived (B) cells contract to a degree comparable to that of bladder smooth muscle cells (C). The addition of EDTA to the mixture inhibits contraction, thus supporting the idea that the contraction is calcium dependent, another characteristic of smooth muscle cells. These data indicate that diameter reduction is dependent on contractile cells, and that the cells function in this capacity. The cells were seeded at 500,000 cells/ml and found to be capable of contraction as demonstrated by a reduction of collagen gel diameter after two days. Porcine bladder smooth muscle cells were used as a positive control. To demonstrate the calcium dependence of this contraction, the calcium chelator EDTA was added to separate samples to inhibit contraction. These results confirm the ability of the cells to contract in a calcium-dependent manner similarly to bladder-derived smooth muscle cells.

Growth kinetics. In order to utilize smooth muscle cells in cell therapy applications, it is important to determine if the required cell numbers can be achieved in an acceptable time frame. The results from canine and porcine studies indicate that smooth muscle colonies (from a 40ml sample of peripheral blood) can be observed as early as 7 days post seeding, and can readily be passed within 14 days. In one study, 1.2 million cells were obtained after 18 days of culture (end of passage 2), at which time they were cryopreserved. These particular cells were thawed ∼50 days later, and routinely passed when ∼80% confluent to determine growth kinetics. Six days after thawing, the cell population expanded to 16.7 million cells (end of passage 3). After another 7 days of culture, the cell population reached 31.7 million cells (end of passage 4). This initial study indicates that 30 million cells can be achieved in roughly 30 days of culture.

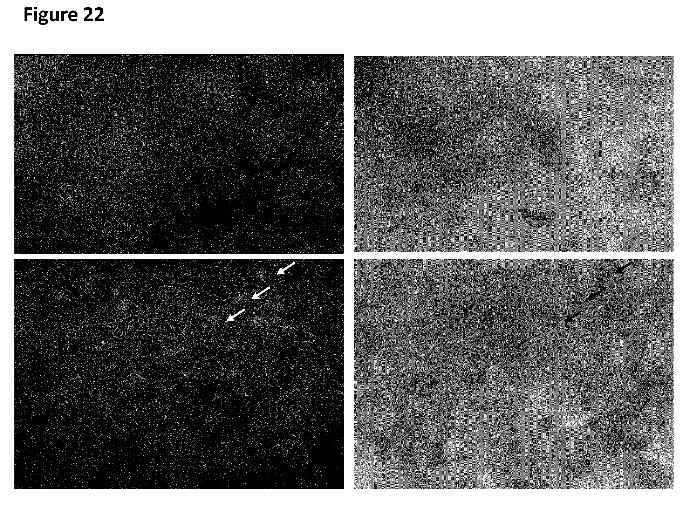

The cells were found to have limited proliferation potential. Figure 22 of Ludlow et al. U.S. Patent Application No. 20100131075 shows the growth of smooth muscle cells isolated from human adipose tissue as a function of the numbers of cells recovered per unit area. These data indicate that between passages 4 and 5, the number of recovered cells begins to decline, supporting the contention that these cells have a limited and finite proliferative capacity, which is characteristic of progenitor cells, but not true stem cells.

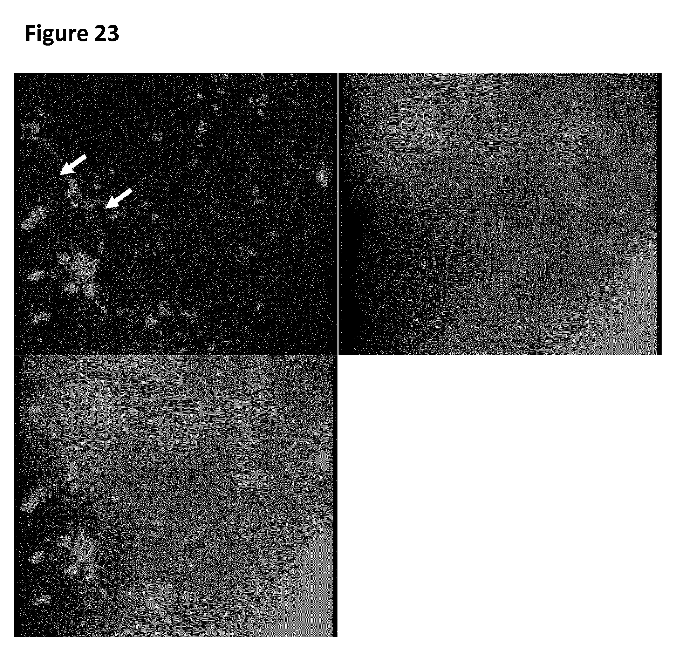

Figure 23 of Ludlow et al. U.S. Patent Application No. 20100131075 shows the growth of smooth muscle cells isolated from porcine adipose, peripheral blood, and bladder smooth muscle as a function of the number of recovered cells per passage. As illustrated, dramatic expansion in cell numbers is achieved between passages 2 and 3, over a time frame of 2-4 weeks, enabling recovery of tens of millions of cells. This demonstrates the limited or finite proliferation potential of the adipose-derived cells.

Contact inhibition of proliferation. The smooth muscle cells isolated from peripheral blood and adipose tissue exhibit contact inhibition of proliferation. For example, the morphological assessment of these cells demonstrates the presence of contact inhibition of proliferation over several passages. The cells do not continue proliferating upon contact with each other. In contrast, MSCs do not exhibit contact inhibition of proliferation and they can be observed piling on top of each other, similar to foci formation in transformed cell cultures. For example, Zhou *et al.* report on the isolation and culturing of MSCs from the mononuclear cell fraction of mouse bone marrow, and observe that after three passages the cultured MSCs demonstrated a loss of contact inhibition (see page 10850 and Figure 1A) (Cancer Res. 2006; 66(22):10849-10854).

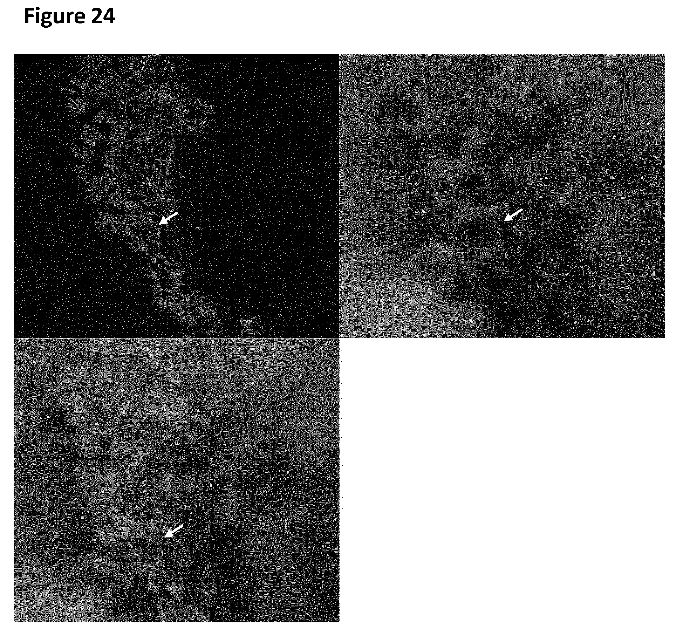

Cytokine MCP-1 production. MCP-1 is a normal product of bladder detrusor cells. In aortic smooth muscle cells, MCP-1 plays a role in regeneration. In order to quantitate MCP-1 produced by human peripheral blood smooth muscle cells, an ELISA based assay system from R&D Systems was employed. Medium samples were assayed in duplicate and compared to a standard curve to provide estimated MCP-1 levels and reported as ug/24 hr/one million cells. Expression of the cytokine MCP-1 for cells isolated from human bladder smooth muscle, adipose, peripheral blood, and bladder urothelium (negative control) was determined. Figure 24 of Ludlow et al. U.S. Patent Application No. 20100131075 shows the results from this analysis indicates that human peripheral blood-derived and human adipose tissue-derived smooth muscle cells produce MCP-1 at levels comparable to that of human bladder smooth muscle cells. These data support the conclusion that, just like bladder SMC, MCP-1 is expressed by the smooth muscle cells isolated from adipose and peripheral blood. In addition, these data suggest that the production of MCP-1 may play a critical role in regeneration by directly or indirectly causing muscle progenitor cells to be recruited/migrate or to proliferate within the construct.

Isolated smooth muscle cells from adipose demonstrate several smooth muscle cell characteristics. Our studies have indicated that the cells can readily be isolated from adipose using standard enzymatic digestion and low-speed centrifugation protocols. Cells can be expanded very rapidly, perhaps reaching ~30 million cells within a month's time. Our studies have further demonstrated that these cells may, in fact, represent a smooth muscle cell population rather than a true stem cell population, as smooth muscle markers are present as early as passage 3. Furthermore, the smooth muscle cells isolated from are capable of contractile function as demonstrated by standard collagen gel contraction assays. Characterization of smooth muscle cells. We have already shown that during subsequent passages, the smooth muscle cell cellular morphology is retained. There is also good correlation of smooth muscle markers at both the gene and protein levels. Cytokine induction. Expression of MCP-1 by adipose smooth muscle cells has lead us to hypothesize that the production of MCP-1 may play a critical role in neo-organ or tissue structure regeneration by directly or indirectly causing native mesenchymal stem cells to be recruited/migrate or to proliferate within the construct.

### EXAMPLE 2 - MCP-1 production and cell density

Conditioned medium from cultures of bladder smooth muscle cells were analyzed using commercially available kits for the detection and quantitation of MCP-1. Conditioned media samples from 9 constructs (3 from each of 3 seeding levels) and the paired SMC cells used for seeding the constructs were tested for MCP-1 levels. The results are shown in Table 2.1.

**Table 2.1**

| | | **cIL2** | **cIL6** | **cIL10** | **cMCP-1** | **cIFNg** | **cTNFa** | **cTGFb** |
|---|---|---|---|---|---|---|---|---|
| **Test ID** | **Sample ID** | pg/ml | pg/ml | pg/ml | pg/ml | pg/ml | pg/ml | pg/ml |
| 1 | **TT1** | <1.0 | <9.8 | 1.0 | <3.7 | <2.4 | <0.2 | |
| 2 | **TT2** | <1.0 | 8.8 | <2.0 | 39.6 | <2.4 | <0.2 | |

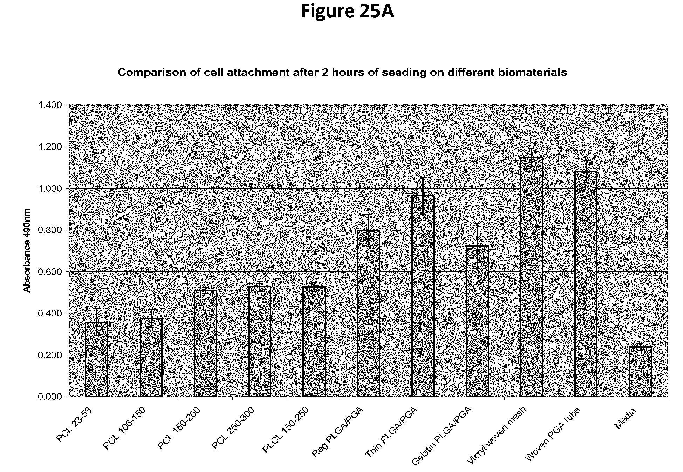

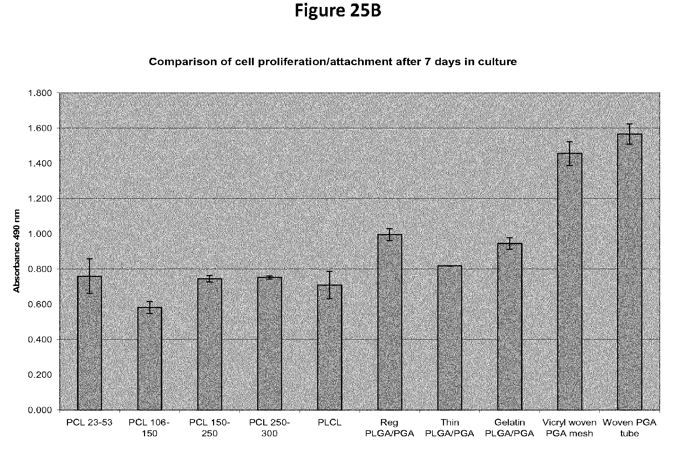

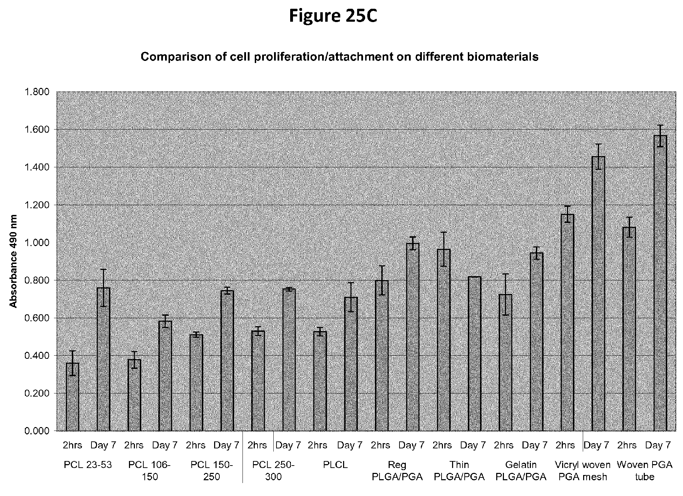

In order to quantitate MCP-1 present in the construct medium, an ELISA based assay system specific for Canine MCP-1 from R&D Systems was employed. Samples were assayed in duplicate and compared to a standard curve to provide estimated MCP-1 levels in construct medium. As shown in Figure 25 of Ludlow et al. U.S. Patent Application No. 20100131075, the results from this analysis show a positive correlation between MCP-1 production and the density of cells seeded. Table 2.2 shows MCP-1 quantitation of construct medium as determined by R&D Systems ELISA. Table 2.3 shows a comparison of the average MCP-1 levels from each group in which it can been seen that the resulting ratios parallel the differences in seeding densities.

**Table 2.2**

| **Group** | **Construct** | **MCP-1 pg/ml** | **Group Average** | **Std Dev** |
|---|---|---|---|---|
| | 1151 | 71 | | |
| | 1152 | 102 | | |
| | 1153 | 59 | | |
| **4 million** | 1154 | 80 | 65 | 24 |
| | 1155 | 74 | | |
| | 1156 | 24 | | |
| | 1157 | 70 | | |
| | 1158 | 39 | | |
| | 1159 | 253 | | |
| | 1160 | 85 | | |
| | 1161 | 412 | | |
| **12 million** | 1162 | 167 | 188 | 135 |
| | 1163 | 69 | | |
| | 1164 | 349 | | |
| | 1165 | 91 | | |
| | 1166 | 78 | | |
| | 1167 | 183 | | |
| | 1168 | 307 | | |
| | 1169 | 181 | | |
| **25 million** | 1170 | 527 | 385 | 207 |
| | 1171 | 771 | | |
| | 1172 | 534 | | |
| | 1173 | 260 | | |
| | 1174 | 321 | | |

Results indicated that there was a positive correlation between cell number and MCP-1 levels detected in the media. It had been previously noted that some tissue from a regenerated canine bladder (approximately 9 million cells seeded) processed for SMC explantation contained more fat than is typically observed in native and regenerated canine tissue. The tissue when explanted was very soft and the explants when viewed contained fatty tissue in greater proportion to that observed with native tissue.

**Table 2.3**

| Group | Average MCP-1 | 4 million MCP-1 | Cell # | 12 million MCP-1 | Cell # | 25 million MCP-1 | Cell # |
|---|---|---|---|---|---|---|---|
| 4 million | 65 | | | 0.35 | 0.33 | 0.17 | 0.16 |
| 12 million | 188 | 2.89 | 3.00 | | | 0.49 | 0.48 |
| 25 million | 385 | 5.92 | 6.25 | 2.05 | 2.08 | | |

The media on these explant plates also exhibited a "sheen" on the surface typically observed when fatty tissue is present. These observations suggest a role for MCP-1/CCR-2 interaction in fat deposition/adipogenesis of regenerated bladder tissue.

### Example 3 - Adipose-derived smooth muscle cells versus Mesenchymal stem cells (MSCs)

Adipose tissue represents a heterogenous cell population composed of endothelial cells, adipocytes, smooth muscle cells and progenitor cells with limited mesenchymal differentiation potential. As described in Ludlow et al. U.S. Published Patent Application No. 20100131075 (incorporated herein by reference in its entirety) and Basu et al. Tissue Eng Part C Methods. 2011 Apr 2. [Epub ahead of print], quantitative RT-PCR, antigen expression, protein fingerprinting, growth kinetics and functional analysis, to quantitatively evaluate the cellular composition of the adherent, stromal vascular fraction (SVF) derived from human adipose. It was found that media formulation influences enrichment for the smooth muscle cell compartment of adipose SVF. These human adipose-derived smooth muscle cells (Ad-SMC) are phenotypically and functionally distinct from mesenchymal stem cells (MSC) or other adipose-derived progenitor populations.

The cellular composition of the initial "passage zero" adherent human SVF-derived cell population was investigated using quantitative real-time PCR methods (TAQMAN™). It was found that from the starting adherent SVF-derived cell population (composed of cells expressing endothelial, smooth muscle, and adipocyte-associated markers), it is possible to identify and culture a cell population with markedly distinctive biological properties through the expansion of SVF-derived cells under defined media conditions that select against the growth of MSC (Gong et al. 2009. Tissue Engineering part A 15: 1-11; Lund et al. Cytotherapy 11, 189-197 (2009)). Despite partial overlap in the expression of markers historically associated with MSC, this cell population clearly has a pronounced smooth muscle cell phenotype relative to MSC based on FACs and RT-PCR (reverse transcription PCR) analysis of the expression of key nuclear and cell surface markers. This population is also noticeably less endothelial when compared to MSC. Manifestation of a smooth muscle cell phenotype is independent of passage number, adipose donor source or the requirement for directed differentiation with recombinant cytokines and growth factors. Additionally, this smooth muscle cell enriched population has a distinctive proteomic signature which unambiguously discriminates it from MSC. Finally, we have leveraged the diametrically opposing responses of this smooth muscle cell like population and MSC towards the thromboxane A2 mimetic U46619 to document a clear functional dichotomy between the two cell types. Taken together, these data support the conclusion that this population is more accurately described as an adipose-derived smooth muscle cells (Ad-SMC) population, and represents a separate and distinct cellular species compared to other classes of adipose-derived cells including endothelial cells and MSC.

**Methods and materials.** Preparation of Adipose Tissue. Human adipose samples were obtained either subcutaneously or through lipoaspiration (Zen-Bio, Research Triangle Park, NC), and washed 3-5 times with an equalvolume of PBS/gentamycin (Gibco) (5µg/ml). Adipose was digested with filter-sterilized collagenase I (Worthington)(0.1%, 1% BSA in DMEM-HG(Gibco)) at 37°C for 1 hour, then centrifuged for 5 minutes at 300g in 50 ml conical tubes. The stromal vascular fraction was resuspended in PBS/1% BSA and filtered through a 100 5µm Steriflip vacuum filter. The cell population was pelleted again at 300g for 5 minutes and resuspended in DMEM-HG + 10% FBS + gentamycin 5µg/ml. Bone marrow derived MSC at the end of passage two was obtained from a commercial supplier (Lonza). For studies on the effect of media type on expression of smooth muscle cell markers, the SVF-derived cells were alternatively resuspended in α-MEM (Gibco) + 10% FBS, SMCM (ScienCell) or L15 (Sigma).

Taq-Man qRT-PCR. RNA was purified from MSC or Ad-SMC using the RNeasy Plus Mini Kit (Qiagen) according to the manufacturer's instructions. cDNA was generated from 2 µg of RNA using the SuperScript VILO cDNA Synthesis Kit (Invitrogen) according to the manufacturer's instructions. Following cDNA synthesis, each sample was diluted 1:10. qRT-PCR was setup as follows using the TaqMan primers and probes listed below: 10 µl master mix (2X), 1 µl primer/probe, 9 µl cDNA (diluted 1:10).

The following TaqMan primers were used for evaluation of smooth muscle, endothelial and adipogenic gene expression: SmαA (smooth muscle alpha actin): Hs00909449_ml, SM22: Hs00162558_ml, myocardin: Hs00538076_ml, SMMHC (smooth muscle myosin heavy chain): Hs00224610_ml, calponin: Hs00154543_ml, adiponectin: Hs00605917_ml, FABP-4 (fatty acid binding protein #4): Hs1086177_ml, CDH5/VECAD (vascular endothelial cadherin): Hs00174344_ml, vWF (von Willebrand factor): Hs00169795_ml, PECAM1 (platelet endothelial cell adhesion molecule #1): Hs00169777_ml, FLT1/VEGFR (VEGF receptor): Hs01052936_ml, KDR/FLK1 (fetal liver kinase #1): Hs00176676_ml, TEK (tyrosine kinase, endothelial): Hs00945155_ml. 18s rRNA was used as endogenous control and all samples were calibrated against bladder smooth muscle cell cDNA. All primer/probes were secured from Applied Biosystems. All reactions were carried out in an ABI 7300 real time thermal cycler using default cycling parameters. Analysis of PCR data was performed using the method of Relative Quantitation (RQ) by Comparative Ct.

Array-RT-PCR. Real time array-based qRT-PCR analysis was performed for 35 cycles using the SABiosciences MSC (PAHS-082A) and Cell Surface Marker PCR array platform (PAHS-055A) according to the manufacturer's instructions.

FACs analysis. 0.5 x 10⁶ - 1 x 10⁶ cells per data point were fixed in 2% paraformaldehyde and Fc receptors blocked to prevent non-specific binding. Cells were then incubated with a directly conjugated antibody for the cell surface markers CD31, CD45, CD54, CD56, CD73, CD90, CD105, CD117 or CD133 (BD Biosciences) as recommended by the manufacturer. Subsequent to final washing (PBS, 0.1% Triton X-100), antigen detection was performed utilizing the BD FACS Aria 1 or Guava EasyCyte Mini Express Assay system using the appropriate fluorescent channel. A minimum of 5000-10,000 events were acquired from each sample.

2D Proteomic analysis. Passage controlled (end of P2) bone marrow derived MSC and Ad-SMC were lysed in Lysis Buffer (50mM Tris pH 8; 150mM NaCl; 0.5% NP40 and protease inhibitors, Roche) and 40 µg of protein lysate from each cell type was run out on a pH 4.0-7.0 Zoom IEF strip (Invitrogen) according to the manufacturer's instructions. Each strip was then loaded onto a 4-12% Bis/Tris acrylamide gel and run out on the 2nd dimension. The gels were stained with SYPRO Ruby stain (Invitrogen) according to the manufacturer's instructions.

Passage controlled (end of P2) bone marrow derived MSC and Ad-SMC were lysed in Lysis Buffer (50mM Tris pH 8; 150mM NaCl; 0.5% NP40 and protease inhibitors, Roche) and 40 µg of protein lysate from each cell type was run out on a pH 4.0-7.0 Zoom IEF strip (Invitrogen) according to the manufacturer's instructions. Each strip was then loaded onto a 4-12% Bis/Tris acrylamide gel and run out on the 2nd dimension. The gels were stained with SYPRO Ruby stain (Invitrogen) according to the manufacturer's instructions.

**Results.** Expression markers in Ad-SVF (Figure 91 of Ludlow et al. U.S. Published Patent Application No. 20100131075). We performed a quantitative TaqMan RT-PCR analysis of thecell population derived from the stromal-vascular fraction of adipose tissue adherent on the tissue culture flask within the initial 24-48 hours subsequent to plating, using a panel of defined endothelial, adipocytic and smooth muscle cell specific TaqMan primers. This served to analyze expression markers in the initial adherent cell population as well as establishing a baseline for subsequent analysis of the effects of passage, time and media formulation upon expression of smooth muscle cell specific genes. Low but detectable levels of FABP-4 and adiponectin were observed in the adherent cell population within the first 24 hours, consistent with the presence of residual adipocytes. Similarly, an endothelial population defined by expression of VECAD, vWF, PECAM, FLT1, FLK and TEK was present at this time point. A smooth muscle cell population defined by expression of SM_A, SM22, myocardin, SMMHC and calponin was also observed within the earliest adherent cell population. We were able to detect all three cell populations at comparable levels within 24-48 hrs of plating. As discussed below, smooth muscle cells were isolated from this mixture of cell populations.

Expression of smooth muscle markers is dependent on media type. As adipose is a heterogenous tissue composed of multiple cell types, it is reasonable to expect that enrichment for smooth muscle cells over endothelial cells or MSCs may be affected by media formulation. Isolation of undifferentiated MSCs from bone-marrow and adipose is closely dependant on media composition (Gong et al. 2009 *supra).* In particular, the presence of elevated levels of glucose in the media or growth at high density appears to select *against* the expansion of MSC (Lund et al. 2009 *supra;* Stolzing et al. Rejuv Res 2006;9:31-35). We reasoned that modulation of media formulation may be useful in enrichment for smooth muscle cells at the expense of MSC and other cell populations. As shown in Figure 92 of Ludlow et al. U.S. Published Patent Application No. 20100131075 (Taqman analysis of SMC marker expression by media type), the expansion of a smooth muscle cell enriched population from adipose-SVF is tightly dependent upon growth in DMEM-HG media. Expansion in α-MEM, SMCM or L15 is associated with a markedly reduced smooth muscle cell phenotype as shown by decreased expression of SMαA, SM22, myocardin, SMMHC and calponin.

Ad-SMC more closely resemble smooth muscle cells than MSC. We used semi-quantitative RT-PCR to assess the smooth muscle cell associated gene expression signatures of Ad-SMC and MSC. As shown in Figure 93 of Ludlow et al. U.S. Published Patent Application No. 20100131075, the expression of the key smooth muscle markers calponin, myocardin and SMMHC is noticeably more pronounced in Ad-SMC when compared to MSC, supporting our hypothesis that this cell population is more similar to smooth muscle cells than to MSC. We then evaluated the stability of expression of SMC specific markers across multiple independent adipose preparations (*n*=4) and over 5 passages in culture. As shown in Figure 94 of Ludlow et al. U.S. Published Patent Application No. 20100131075 (RT-PCR of Ads across passage), the expression of SMαA, SM22, SMMHC, myocardin and calponin is remarkably constant across passage and is independent of donor, demonstrating that expression of a smooth muscle cell phenotype is stable over time. These observations are consistent with Ad-SMC being a more fully differentiated, phenotypically stable cell population.

Array-based RT-PCR analysis demonstrates significant differences in gene expression of key markers between Ad-SMC and MSC. We have used the SABiosciences MSC Marker Array panel to systematically identify differences in gene expression between passage controlled (P2) Ad-SMC and MSC. This panel profiles the expression status of 84 genes involved in MSC pluripotency and self-renewal. A summary of the key markers identified as distinct between Ad-SMC and MSC is shown in Table 3.1. Significant (at least ten fold) down-regulation in Ad-SMC relative to MSC was observed for GDF5, HGF, LIF, MCAM, RUNX2 and VCAM1. Significant (at least ten fold) up-regulation in Ad-SMC compared to MSC was observed for BMP6, CD44, and IL1β. These key differences in gene expression were observed to remain consistent independent of passage or cell sample (*n*=6, data not shown). Gene expression analysis was continued using the SABiosciences Surface Marker Array.

**Table 3.1**

| **Symbol** | **Description** | **MSC Ct** | **Hu Ad-SMC P4 Ct** | **Fold Regulation Ad-SMC vs MSC** |
|---|---|---|---|---|
| BMP6 | Bone morphogenetic protein 6 | 34.09 | 28.57 | 35.5555 |
| CD44 | CD44 molecule (Indian blood group) | 31.87 | 23.06 | 347.7725 |
| IL1B | Interleukin 1, beta | 35 | 29.62 | 32.2673 |
| | | | | |
| GDF5 | Growth differentiation factor 5 | 25.34 | 31.89 | -120.9276 |
| HGF | Hepatocyte growth factor (hepapoietin A; scatter factor) | 27.95 | 32.77 | -36.4539 |
| LIF | Leukemia inhibitory factor (cholinergic differentiation factor) | 29.37 | 35 | -63.9113 |
| MCAM | Melanoma cell adhesion molecule | 27.99 | 33.67 | -66.1652 |
| RUNX2 | Runt related transcription factor 2 | 27.69 | 30.58 | -12.1426 |
| VCAM1 | Vascular cell adhesion molecule 1 | 24.36 | 34.1 | -1103.5987 |

| **Historically Defined Cell Surface Markers** | | | | |
|---|---|---|---|---|
| **Symbol** | **Description** | **MSC Ct** | **Hu Ads P4 Ct** | **Fold Regulation MSC vs. Ads** |
| ALCAM | Activated leukocyte cell adhesion molecule (CD166) | 24.88 | 24.44 | 1.0512 |
| ENG | Endoglin (CD105) | 23.06 | 22.57 | 1.0882 |
| NT5E | 5'-nucleotidase, ecto (CD73) | 25.2 | 24.38 | 1.3679 |
| THY1 | Thy-1 cell surface antigen (CD90) | 29.54 | 29.68 | -1.4221 |

Summary of the key results is presented in **Table 3.2,** where we have examined Ad-SMC at P0 & P4.

**Table 3.2**

| **Cell Type** | **Symbol** | **Description** | **Ct P0** | **Ct P4** | **Fold Regulation P0 to P4** |
|---|---|---|---|---|---|
| SMC | MYH9 | Myosin, heavy chain 9, non-muscle | 23.59 | 24.2 | -2.5245 |
| | MYH10 | Myosin, heavy chain 10, non-muscle | 25.94 | 26.05 | -1.7851 |
| | MYOCD | Mvocardin | 35 | 33.09 | 2.2721 |
| | | | | | |
| Endothelial | ENG | Endoglin (Osler-Rendu-Weber syndrome 1) | 23.84 | 22.73 | 1.305 |
| | ICAM2 | Intercellular adhesion molecule 2 | 27.35 | 29.02 | -5.2634 |
| | NOS3 | Nitric oxide synthase 3 (endothelial cell) | 30.01 | 31.66 | -5.191 |
| | PECAM1* | Platelet/endothelial cell adhesion molecule (CD31 antigen) | 29.07 | 35 | -100.8453 |
| | SELP | Selectin P (granule membrane protein 140kDa, antigen CD62) | 35 | 35 | N/A |
| | TEK* | TEK tyrosine kinase, endothelial (venous malformations, multiple cutaneous and mucosal) | 31.27 | 25.68 | 29.1212 |
| | VCAM1* | Vascular cell adhesion molecule 1 | 25.88 | 34.16 | -514.1338 |
| | VWF* | Von Willebrand factor | 27.2 | 31.49 | -32.3569 |
| | | | | | |
| Adipocyte | RETN | Resistin | 35 | 35 | N/A |
| | | | | | |
| Fibroblast | ALCAM | Activated leukocyte cell adhesion molecule | 28.6 | 24.98 | 7.4333 |
| | COL1A1 | Collagen, type I, alpha 1 | 20.26 | 20.65 | -2.1675 |
| | COL1A2 | Collagen, type I, alpha 2 | 19.36 | 18.2 | 1.351 |
| | | | | | |
| HLA | HLA-A | Major histocompatibility complex, class I, A | 24.53 | 24.7 | -1.8609 |
| | HLA-DRA* | Major histocompatibility complex, class II, DR alpha | 26.57 | 34.12 | -309.9733 |
| | CD74* | CD74 molecule, major histocompatibility complex, class II invariant chain | 28.13 | 35 | -193.4746 |
| | | | | | |
| Other | NT5E | 5'-nucleotidase, ecto (CD73) | 27.43 | 24.24 | 5.5174 |
| | NCAM1 | Neural cell adhesion molecule 1 | 35 | 35 | N/A |

| | | | | | |
|---|---|---|---|---|---|
| * = Change in Fold Regulation > \|10.0\| | | | | | |

Expression of the fibroblastic/stromal markers ALCAM, COL1A1 and COL1A2 is maintained across passage, as are the smooth muscle cell specific markers MYH10, MYH9 and MYOCD. The population is negative for the adipocyte marker RETN, indicating that there is minimal contamination with adherent adipocytes. Importantly, although Ad-SMC acquire an HLA MHC II negative status within 4 passages they are initially HLA MHC II positive, a key distinction with MSC which are MHC II negative. Another interesting observation is that Ad-SMC becomes progressively *less* endothelial with passage, as judged by the general trend in down-regulation of the endothelial markers ENG, ICAM2, NOS3, PECAM1, SELP, TEK, VECAM and VWF. This data is independently confirmed by the RTPCR analysis in Figure 95 of Ludlow et al. U.S. Published Patent Application No. 20100131075.

To further compare the gene expression profiles between the adipose-derived smooth muscle cells (Ad-SMC) and mesenchymal stem cells (MSC), a PCR-based gene array analysis was performed for human mesenchymal stem cell markers (SABiosciences; PCR Array Catalog # PAHS-082A) (data not shown). The results illustrated the extent of homologous gene expression among Ad-SMC, MSC, and a well characterized non-MSC cell type, human aortic endothelial cells (HuAEC). Of the 84 human MSC genes analyzed, human Ad-SMC share only 27% homology (23 of 84 genes) with human MSC at initial isolation (data not shown). In contrast, the well characterized, non-MSC, HuAEC share 49% homology (41 of 84 genes) with MSC (data not shown). This supports the conclusion that the Ad-SMC share significantly less homology with MSC than HuAEC, which is a well-known non-MSC cell type. Thus, the Ad-SMC are even less like MSCs than HuAEC are, further supporting the conclusion that the Ad-SMC cells isolated from adipose tissue are Ad-SMC and not MSC.

**The cell surface profile of Ad-SMC is significantly different from that defined for MSC.** We observed that both MSC and Ad-SMC share expression of the surface markers CD73, CD90, CD105 and CD166 which are traditionally associated with MSC **(Table 3.1).** However, as discussed below, these markers have no intrinsic biological significance beyond their historical association with MSC. The gene expression results from the cell surface marker RT-PCR analysis were generally reflected in the comparative FACs analysis shown in Ludlow et al. U.S. Published Patent Application No. 20100131075 (Fig. 96A-C (Ad-SMCs) and Figure 97A-B (MSCs)), which shows that Ad-SMC are CD31+, CD45+, CD54+, CD56+, CD90+, CD105+. Importantly, Ad-SMC was CD45+ and CD117+, a clear distinction from MSC, which are CD45- CD117-. Expression of CD73 is consistent with that previously reported for adipose stromal vascular fraction (da Silva Meirelles et al. J Cell Sci., 119:2204 (2006)), but differs from that reported for bone-marrow derived MSC.

**Passage controlled MSC and Ad-SMC have unique proteomic signatures.**

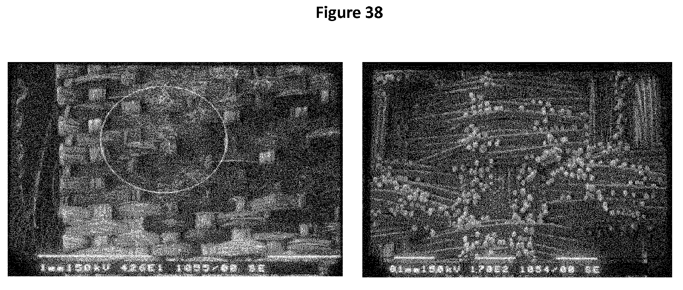

Figure 38 of Ludlow et al. U.S. Published Patent Application No. 20100131075 shows a comparative analysis of the whole proteomic signatures of MSC, bladder-derived SMC, Ad-SMC, and human aortic smooth muscle cells. The top two panels demonstrate that Ad-SMCs are distinct from MSC and are also clearly different from MSC isolated from adipose tissue as well as other classes of stem and progenitor cells (Roche et al; Proteomics 2009;9:223-232; Noel et al. Exp Cell Res 2008;314:1575-1584). The arrows on both gels highlight one difference between MSCs and AdSMCs; concentration of proteins at different and distinct locations within the pH gradient and molecular weight range. MSC have this protein concentration closer to a pH of 7.0, and greater than or equal to 60,000 molecular weight. In contrast, AdSMC have this protein concentration closer to a pH of 4.0, and less than 60,000 in molecular weight. AdSMC also had more protein present with pI above 7 than MSC, as indicated by the smear along the right outside edge of the gel at pH 7.0. Bladder smooth muscle cells were analyzed as a control. The boxes indicate areas of similarity among all samples. It is clear that the AdSMC protein profile is most like the profile for bladder-derived SMC (lower left panel), which is distinct from the pattern observed for MSC. Aortic smooth muscle cells were also analyzed as an additional smooth muscle cell control (lower right panel). The proteomic signature of the aortic smooth muscle cells and bladder smooth muscle cells are almost identical. Taken together, the high degree of similarity among the profiles for AdSMC, bladder and aortic smooth muscle cells, which are distinctly different from the profile of the MSC, supports the conclusion that SMCs, not MSCs, are being isolated from adipose tissue. All gels were stained with SPRYO Ruby stain to visualize the protein pattern.

**Growth kinetics of Ad-SMC differ markedly from MSC.** The proliferative potential of Ad-SMC differs markedly from MSC which have been successfully expanded to up to 40 passages (Bruder et al., J Cell Biochem., 64:278-294 (1997)). As shown in Figure 99 of Ludlow et al. U.S. Published Patent Application No. 20100131075, Ad-SMC show a marked decline in proliferative capacity after the 4th-5th day in culture. We have also observed that unlike MSC, Ad-SMC exhibit contact dependant inhibition of proliferation. These observations demonstrate that Ad-SMC have no capacity for self-renewal and therefore by definition are not stem or progenitor cells. MSCs do not exhibit contact inhibition of proliferation and they can be observed piling on top of each other, similar to foci formation in transformed cell cultures. This is consistent with previous observations (Zhou *et al.* 2006 *supra*).

**Ad-SMC and MSC have distinctly opposing responses to treatment with U46619.** As part of our efforts to evaluate the effects of small molecules targeting signaling cascades involved in the activation of smooth muscle cell related developmental pathways, we have focused on U46619, a thromboxane A2 mimetic whose effects include increasing intracellular Ca²⁺ levels and activation of RhoA, CaM and MLC kinase signaling cascades. As reported previously (Kim et al. 2009, Stem Cells. 27(1):191 -199), we have confirmed that treatment with U46619 (1 µM) led to up-regulation of the key smooth muscle cell markers myocardin and SMMHC in MSC. However, Ad-SMC responded to the same treatment by unambiguous *downregulation* of myocardin and SMMHC expression as shown in Figure 100 of Ludlow et al. U.S. Published Patent Application No. 20100131075 (Lanes: 1-MSC control; 2-MSC+U46619; 3-Ad-SMC control; 4-Ad-SMC+U46619; 5-H20; and 6-SMC). These results provide clear evidence for a functional dichotomy between Ad-SMC and MSC.

**Expression of functional markers.** Figure 101 of Ludlow et al. U.S. Published Patent Application No. 20100131075 provides results of RT-PCR analysis of mesodermal differentiation markers. Lane contents: 1: MSC control; 2:MSC experimental; 3: AdSMC control; 4: AdMSC experimental; 5: Peripheral blood control; 6: Peripheral blood experimental; and 7: H₂O The expression of markers of mesodermal differentiation in MSC and AdSMCs undergoing adipogenic differentiation. AdSMC shows significantly greater expression of ostepontin relative to MSC during growth under standard conditions (n=1). Expression of Oct4B, a splice-variant of Oct4A, an established marker for pluripotentiality (Kotoula et al., 2008, Stem Cells 26(1): 290-1), is significantly upregulated in adipose-derived cells relative to MSC. Neither MSC nor adipose-derived cells show expression of Oct4A.

Figure 102 of Ludlow et al. U.S. Published Patent Application No. 20100131075 shows the results of RT-PCR analysis of Oct4A/Oct4B expression in MSC/AdSMC. Lane contents: 1: Bladder SMC; 2: HFF-1 (human fibroblast); 3: MSC; 4: AdSMC; 5: peripheral blood; 6: H₂O Expression of the closely related transcriptional isoforms Oct4A and Oct4B was evaluated in MSC, AdSMC, fibroblast and SMC lines. No expression of the pluripotency marker Oct4A (Gong et al. 2009 *supra)* was observed, though all cell lines evaluated expressed Oct4B (n=1).

This study demonstrates that the isolation of Ad-SMCs directly from the P0 adherent stromal vascular fraction of adipose depends upon the media formulation. Expression of smooth muscle cell markers is robust and consistent and is independent of donor source and across passage. We have shown that Ad-SMCs are phenotypically distinct from MSC as demonstrated by gene expression, proteomic and surface marker analysis, and are functionally distinct from MSC as evaluated by their response to pharmacologic agents targeting smooth muscle cell associated signaling pathways. In contrast to other published reports, isolation of these smooth muscle cells does not require directed differentiation with TGF-β or related small molecules. Ad-SMC may be expanded to up to 107 cells within 4-5 passages, express the full range of smooth muscle cell associated markers and are functionally comparable to bladder-derived SMC both in vitro (Ca2+-dependant contractility) and in vivo (regeneration of neo-urinary conduit in swine cystectomy model) (Basu et al. International Society for Stem Cell Research, 7th Annual Meeting , July 8-11, 2009). These data support the conclusion that this population is more accurately described as an adipose-derived smooth muscle cell (Ad-SMC) population, and represents a separate and distinct population compared to other classes of adipose-derived cells including endothelial cells and MSC.

### Example 4 - Construction of a neo-urinary conduit from non-bladder cell sources

Ludlow et al. U.S. Published Patent Application No. 20100131075 and Ludlow et al. U.S. Provisional Application No. 61/330,774 (the contents of which are incorported herein by reference in their entirety) describes the isolation and characterization of smooth-muscle cells from porcine peripheral blood and adipose. The peripheral blood- and adipose-derived smooth-muscle cells may be used to seed synthetic, biodegradeable tubular scaffold structures and that implantation of these seeded scaffolds into a porcine cystectomy model leads to successful regeneration of a neo-urinary conduit. Smooth muscle cells were obtained from from porcine bladder, adipose and peripheral blood according to the protocols described in Example 1 (also see Example 3 of Ludlow et al. U.S. Provisional Application No. 61/330,774).

Direct plating of the peripheral blood-derived mononuclear fraction from swine resulted in outgrowth of colonies with typical smooth muscle cell morphology. All (100%) animals screened (*n*=24) generated smooth muscle cell colonies, with 2.44 x 10³-2.37 x 10⁶ smooth muscle cells recovered at passage zero from 50ml of peripheral blood. Recovery of smooth muscle cells was unaffected by changes in media formulation, cell density or surface coatings (data not shown). A similar approach was used to investigate the potential application of subcutaneous or lipoaspirate-derived adipose as a source of smooth muscle cells. We were able to generate colonies (expandable into monolayers) of smooth muscle cells from porcine adipose with 100% efficiency (n=24), with a cell recovery rate of 1.37 x 10⁵-4.36 x 10⁵ cells/g adipose tissue. In comparison, smooth muscle cells could be isolated from bladder tissue with a recovery rate of 1.29 x 10⁶-9.3 x 10⁶ cells/g bladder tissue. Expansion of smooth muscle cell colonies from peripheral blood or adipose resulted in the formation of a cell monolayer with a typical whirled, "hill-and-valley" organization characteristic of cultured bladder-derived smooth muscle cells.

Increased expression of proteins associated with smooth muscle contractility (myocardin, SM22, α-smooth muscle actin (SMαA), smooth muscle myosin heavy chain (SMMHC) and calponin (CNN)) was confirmed by semi-quantitative RT-PCR and immuno-fluorescence analysis. Contractility of each of porcine bladder-, adipose-, and peripheral blood- derived smooth muscle cells was confirmed by a Ca²⁺ dependent contractility assay in a collagen gel matrix and a three dimensional Ca²⁺-dependant contractility assay. Contractility was inhibited by EDTA, a known Ca²⁺ chelator. Growth kinetics of porcine (A) bladder-, (B) adipose-, and (C) peripheral blood- derived smooth muscle cells were also evaluated. It was observed that smooth muscle cell colonies (from a 50ml sample of porcine peripheral blood or 7-25g porcine adipose) are identifiable within 7 days post seeding, and may be passaged within 14 days. One million to tens of millions of smooth muscle cells were recovered from bladder, peripheral blood or adipose within 2- 4 weeks (*n*=24). Bladder and adipose-derived smooth muscle cells were expanded for 2 passages prior to harvesting of cells for seeding a synthetic, neo-urinary conduit scaffold. Peripheral blood-derived smooth muscle cells were expanded for 3-4 passages to generate equivalent cell numbers. On average, 30-40 x 10⁶ smooth muscle cells were used to seed a neo-urinary conduit scaffold.

Materials and Methods. Generation of smooth muscle cells from porcine bladder, adipose and peripheral blood. Smooth muscle cells were isolated from bladder & adipose biopsies as well as peripheral blood draws for use in generation of a Neo-Urinary Conduit construct. A 1cm² bladder biopsy specimen, 2cm² adipose biopsy specimen, and 50mL of peripheral blood was obtained from each of 24 Gottingen swine approximately 8 weeks prior to the planned implantation of the final Neo-Urinary Conduit. For isolation of bladder-derived smooth muscle cells, the urothelial cell layer was dissected away from the bladder biopsy and the remaining smooth muscle layer cut into 1mm² pieces and arranged onto the surface of a tissue culture plate. Biopsy pieces were dried in a biosafety cabinet for 10-30 minutes. DMEM-HG (Gibco) + 10% FBS was added to the biopsy samples and the plates incubated in a humidified 37°C incubator at 5% CO₂.

Adipose tissue (7-25g) was washed 3 times with PBS, minced with a scalpel and scissors, transferred into a 50mL conical tube and incubated at 37°C for 60 minutes in a solution of 0.3% collagenase (Worthington) and 1% BSA in DMEM-HG. The tubes were either continually rocked or periodically shaken to facilitate digestion. The stromal-vascular fraction was pelleted by centrifugation at 600g for 10 minutes and resuspended in DMEM-HG + 10% FBS. The stromal-vascular fraction was then used to seed passage zero. 25 ml of porcine peripheral blood was diluted 1:1 in PBS and layered with 25ml Histopaque -1077 (Sigma) in a 50mL conical tube. Following centrifugation (800g, 30 min), the mononuclear fraction was collected, washed once with PBS and resuspended in α-MEM/10% FBS (Invitrogen) to seed passage zero.

**Assembly of a Neo-Urinary Conduit cell/scaffold composite.** Bladder, adipose and peripheral blood-derived smooth muscle cells were expanded separately for up to 7 weeks to generate the 10⁷ cells required for seeding a NUC scaffold. Bladder and adipose-derived smooth muscle cells were expanded for 2 passages before harvesting of cells for seeding of scaffolds to produce the final construct. Peripheral blood-derived smooth muscle cell cultures were expanded to P3-4 before harvesting for scaffold seeding. To make the NUC scaffold, PGA felt was cut to size, sutured into the shape of a NUC, and coated with PLGA. This construct was then sterilized using ethylene oxide. On the day prior to cell seeding, the NUC scaffold was serially pre-wetted by saturation with 60% ethanol/40% D-PBS, 100% D-PBS, D-MEM/10% FBS or α-MEM/10% FBS followed by incubation in D-MEM/10% FBS or α-MEM/10% FBS at room temperature overnight. The NUC scaffold was then seeded with bladder-, adipose-, or peripheral blood-derived smooth muscle cells and the seeded construct matured in a humidified 37°C incubator at 5% CO₂ until readyt for implantation by day 7.

Isolation of RNA and semi-quantitative RT-PCR analysis. RNA was isolated from porcine bladder, adipose and peripheral-blood derived smooth muscle cells using the RNeasy Plus RNA Mini isolation kit (Qiagen). 1 µg of RNA from each sample was reverse-transcribed using the Quantitect cDNA synthesis kit (Invitrogen). The following smooth muscle cell specific primers were used to set up RT-PCR reactions (5'-3'): **β*-actin* (F:** TTC TAC AAT GAG CTG CGT GTG (SEQ ID NO:1 ), **R:** CGT TCA CAC TTC ATG ATG GAG T) (SEQ ID NO:2), ***SM22 (transgelin)* (F:** GAT CCA ACT GGT TTA TGA AGA AAG C, (SEQ ID NO:3) **R:** TCT AAC TGA TGA TCT GCC GAG GTC(SEQ ID NO:4)), ***SMαA* (F:** CCA GCA GAT GTG GAT CAG CA(SEQ ID NO:5), **R:** AAG CAT TTG CGG TGG ACA AT(SEQ ID NO:6)), ***SMMHC* (F:** GCT CAG AAA GTT TGC CAC CTC, (SEQ ID NO:7) **R:** TCC TGC TCC AGG ATG AAC AT (SEQ ID NO:8)), ***CNN (calponin)*** (F: CAT GTC CTC TGC TCA CTT CAA C (SEQ ID NO:9), **R:** CCC CTC GAT CCA CTC TCT CA (SEQ ID NO:10)), *MYOCD* **(F:** AAG AGC ACA GGG TCT CCT CA (SEQ ID NO:11), **R:** ACT CCG AGT CAT TTG CTG CT (SEQ ID NO:12)). Cycling conditions: denature 95° (2 min), denature 95° (45s), anneal (45s), extension 72° (45s), final extension 72° (5 min). 35 cycles (myocardin 40 cycles). Annealing temps: β-actin=58°, SM22=56°, SMαA=55°, SMMHC=60°, CNN=51°, MYOCD=52°. PCR reactions were carried out using GoTaq Green PCR mix (Promega) and cycled on an iQcycler (Bio-Rad). Immuno-fluorescence analysis. The following antibodies were used for immuno-fluorescence analysis: SMαA (Dako #M0851), CNN (Dako #M3556), SM-MHC (Sigma #M7786), myocardin (Santa Cruz #SC3428), SM22 (Abcam #ab28811-100), anti-msIgG1/Alexafluor 488 (Invitrogen #A21121), anti-msIgG2a/Alexafluor 488 (Invitrogen #A21131), anti-gtIgG/Alexafuor 488 (Invitrogen #A11055). All primary antibodies were used at a final concentration of 5 µg/ml, except SMMHC which was used at 10 µg/ml. Contractility assay. Contractility assays were performed as described previously (Travis et al., 2001 *supra).* Growth kinetics. Expansion of smooth muscle cells from tissue isolation to seeding of the Neo-Urinary Conduit scaffold was by serial passaging at a confluence ≥70%.

### Example 5 - Study in Yorkshire Swine to Assess Neo-Urinary Conduit Implantation

The study will assess neo-urinary conduit implantation in a female Yorkshire pig model over an 8 week time period. The objective of this study is to determine the safety and functionality of skin stomal creation methodologies at the use of a Neo-Bladder Conduit Construct seeded with allogeneic smooth muscle cells for tissue regeneration after surgical removal of the bladder (radical cystectomy) and diversion of the ureters to the inflow end of the Neo-Bladder Conduit Construct implant system. Peritoneum will be used to wrap the whole construct. The draining outflow end of the construct will be directed and attached towards the surgically created stoma in order to pass urine. At the completion of the recovery period (Day 56 +/-5), the animals shall be euthanized and a necropsy performed for harvesting the kidneys, conduits, and associated organs and tissues for histological preparation and pathological examination. Three animals will be subjected to one major procedure. A neo-Bladder Conduit construct seeded with allogeneic SMCs will be implanted. Device implantation includes a skin stomal creation for the voiding of urine. In this study a stoma will be created which will address the requirements for a human abdominal stoma in combination with a Neo-Bladder Conduit Construct.

**Study Design.** Six (6) York Shire Swine underwent a surgical removal of urinary bladder (total cystectomy). Hernia patches were evaluated to avoid potential bowel hernia/evisceration in quadrupedal animal with a ventral abdominal stoma. Stoma formation methods were evaluated in two phases, A and B (Table 5.1). Briefly, methods of everted stoma formation were evaluated in Phase A and methods of forming a flat stoma were evaluated in Phase B. Table 5.2 describes the four phases of the study and Table 5.3 summarizes the study plan.

**Test Article.** The test article was a PGA/PLGA Neo-Urinary Conduit Construct with allogeneic adipose-derived SMCs. The scaffold is composed of synthetic lactide/co-glycolide acid polymers that is seeded with allogeneic adipose-derived SMCs. The test articles were stored at 22°C± 5. The test articles were implanted into the animals via the surgical procedure described below.

Smooth muscle cells were obtained from an allogeneic adipose source but no immunosuppressive therapy was used during or after the surgical procedure.

**Table 5.1**

| **Animal No.** | **Phase** | **SMC** | **Surgical** | **Right Ureter** | **Left Ureter** | **"Nipple" Stoma** | **Hernia Patch** | **Survival Days*** |
|---|---|---|---|---|---|---|---|---|
| | | **Source** | **Methodology** | | | | | |
| 1 | A | | Total cystectomy; | Reimplanted to lateral side | Reimplanted to lateral side | Autologous Adipose | NA | 63 |
| 2 | | | NUC placement parallel to linea alba with raised skin stoma; | Transected and anastomosed to lateral side | Reimplanted to lateral side | Silicone Ring | NA | 61 |
| 3 | | | Ureter stents <14 days | Transected and anastomosed to lateral side | Reimplanted to lateral side | Shutter Skin Eversion | NA | 32 |
| 4 | B | Adipose-derived | Total cystectomy; | Reimplanted to lateral side | Reimplanted to lateral side | NA | Dual Hernia Patch (Intra-abdominal and | 63 |
| 5 | | | NUC placement angled to linea alba with flat stoma; Ureter | Reimplanted to lateral side | Reimplanted to lateral side | NA | Dual Hernia Patch (Intra-abdominal and | 20 |
| 6 | | | stents <14 days | Reimplanted to lateral side | Reimplanted to lateral side | NA | Single Hernia Patch (Intra-abdominal) | 21 |

| | | | | | | | | |
|---|---|---|---|---|---|---|---|---|
| No. = Number; SMC = smooth muscle cells; * Electively Euthanized at 20-63 days post-implantation | | | | | | | | |

**Table 5.2**

| Phase | Summary |
|---|---|
| A | Generate test article construct (seeded with allogeneic smooth muscle cells) |
| B | Surgical implantation procedure of 3 animals |
| C | Survival: Post operative care and monitoring, Observation, Data Collection |
| D | Pre-Necropsy follow up & Necropsy with tissue harvest and histology |

**Table 5.3**

| **Group ID** | Allogeneic Adipose SMCs |
|---|---|
| **Number of animals** | 3 female |
| **Test Device** | ◆Transpose 2 ureters to be attached to inflow end of neo-bladder conduit construct |
| **Implantation Surgery** | |
| **Day 0** | ◆The ureter is stented with a DaVINCI Stent for ~7 days ◆Peritoneum is used to wrap and cover the whole construct. |
| | ◆The draining outflow end of the construct is attached through the abdominal wall and exiting to the skin without a continent stoma. |
| | ◆DaVinci made Stoma button is placed permanently to retain patency |
| **Bodv Weight (Kg)** | ◆Pre-Sx ◆Pre-Nx |
| **Stoma Button & Incision Site Assessment** | ◆Incision site: assessed daily for 14 days or until healed |
| | ◆ Stoma Button: |
| | •Daily maintenance for duration of study as needed. |
| | •Debridement on per animal basis as needed |
| **Cystoscope** | ◆Pre-Nx |
| **Nx (56 ± 5 days)** | Harvest: ◆kidneys ◆ ureters ◆ neo-conduit ◆stoma ◆Gross lesions (discretionary) |

Each animal was sedated and then anesthetized prior to surgery preparation. Each animal was then intubated to receive inhalant for induction and maintenance of anesthesia. The operative area(s) was then cleaned and draped for aseptic surgery. The preparation was be performed prior to surgery. Vital signs were monitored during implant surgery.

**Ureteral transposition through conduit with cystectomy.** The ureteral transposition procedure was performed via laparotomy. A midline incision was be made in the abdomen beginning 5cm cranial to the umbilicus extending approximately 15cm caudal. The peritoneum was identified, carefully separated from the abdominal space until the tissue is long enough to cover the Neo-Urinary Conduit Construct and form a conduit that can exit through the body wall. The peritoneum was measured and cut in order to wrap the construct and form a conduit that will extend out of the body wall .The peritoneum was sutured around the construct with 3-0 Vicryl. Care was taken to ensure the tissue remains intact and vascularized. The urinary bladder was then exposed and emptied of urine taking care to avoid urine from entering into the abdominal cavity. The arteries and veins supplying the bladder were identified and ligated. The ureters were identified, two 7Fr 14cm non-absorbable ureteral stents were inserted in ascending fashion and the ureters were carefully transected from the bladder. The urethra was over sewn as it is transected. The bladder was then removed. The left ureter was carefully freed from the surrounding retroperitoneal fascia extending cranially until there is enough mobility to reach the right side. The right ureter was dissected free to reach the end of the construct. The ureters were sutured on to the construct with 3-0 Vicryl in a simple continuous pattern. A stoma was created on the ventral abdominal wall lateral to the mammary glands. Varied skin stomal creation methodologies may be developed during the surgical procedure. The peritoneal conduit was exteriorized and sutured to the skin. Surgical adhesive was placed along the suture line and where the peritoneum exits the body wall. The suture strands that are connected to the stents were exteriorized through the stoma for future removal, and a stoma button/catheter of appropriate length was inserted into the stoma allowing adequate drainage for a period of 7-21 days (upon completion of approximately one week, the stents will be removed consciously or under anesthesia if needed). Once secured, the abdominal incision was closed with non-absorbable Prolene suture. The skin was closed in a routine fashion. The animal was allowed to recover. The peritoneum was handled and manipulated with great care to prevent staunching blood flow through the vasculature. The ureteral non-degradable stents were left in place for approximately 7 days unless diagnostic evaluations revealed a need to remove them prematurely (e.g., renal obstruction). The surgery was performed once on each animal on Day 0.

### Stoma Button Care and Maintenance & Incision site Assessment.

*Stoma Button:* After the definitive surgery, the stoma catheter (DaVINCI generated stoma button or equivalent: 3-10 cm based on need at various time points) will be reinserted and secured to the animal with sutures. Stoma button will be kept in place based on per case need and potentially for the duration of the study. The stoma button will be flushed with sterile saline when it is not dripping to assure patency. Between days 7 and 21, scaffold material undergoes degradation and particulates (protein-associated) start to be shed in the urine. This may cause obstruction of the stoma button and retention of urine volume above or beyond the construct's capacity. Therefore, debridement of the stoma and or neo-conduit will be conducted as necessary.

*Frequency*/*Duration:* Daily observations followed by maintenance as needed when catheter is observed not dripping. Time required for approximately 15 minutes.

*Incision Site Assessment:* The incision site will be evaluated daily for the initial 14 days or until healed. The stoma area and surrounding tissue will be cleaned as needed. Stoma will be observed for urine drainage, incision site will be evaluated for dehiscence, abnormal discharge, odor, irritation or any abnormalities. *FrequencylDuration:* Daily for the initial 14 days or until healed and/or at the discretion of the Facility Veterinarian. Time required for approximately 15 minutes.

*Stagnant stoma tissue debridement procedure:* Animals with stagnant tissue within the stoma/conduit will undergo a debridement procedure. Animal will be sedated according to protocol. A small incision may be made on the stoma to facilitate insertion of forceps for debridement. The stagnant issue will be visually/tactilely identified and grasped with forceps and gently tugged. Once all stagnant tissue is removed, the stoma/conduit may be flushed with saline solution. If incision is required in debridement procedure, it will be closed with a suture(s). A stoma button will be reinserted and secured to the animal with sutures. Animal will be recovered in individual cage. *Frequency*/*duration:* Time required for approximately 45 minutes.

*Recovery:* Immediately following completion of each surgery, animal will be allowed to recover from anesthesia and transferred to the home cage. This period will occur at the end of any surgical procedure for approximately 1 hour.

*Clinical Observations Post-Implantation post-Surgery for Duration of the Study:* Post-implantation, individual animal evaluations of food intake and fecal/urine output will be conducted daily post implantation for 8 weeks. Observations will be made 5 days post-implantation/reimplant surgery: Clinical observations will be conducted daily for 8 weeks.

*Survival:* Post-implantation/re-implant surgery recovery animals will be survived for a period of 56 +/- 5 days. During this period health assessments will be conducted on animals.

### Animal Sacrifice and Necropsy.

*Physical Examination -* All animals will be evaluated prior to euthanasia. The examination will include recording the general condition of the animal: rectal body temperature, respiratory rate, heart rate, and capillary refill time.

*Necropsy* -All animals will be subjected to necropsy. There will be a specific focus on the kidneys, conduit, ureters, and stoma. Gross evaluation will be performed on the kidneys, ureters, conduit, stoma, thoracic, abdominal & pelvic cavities and their organs and tissues. If any gross lesions, adhesions and/or organ changes (including reproductive) are observed, they will be evaluated, photographed and collected for histopathological assessment. The complete neo-bladder conduit area will be visualized and photographed *in situ.* Additional photographs and/or gross lesion may be taken at the discretion of the prosector. Fixation of conduit will be done with formalin by infusion of formalin into the stoma and inflating the conduit and ureters. This will be done with Foley (or equivalent) catheter while the stoma is tied off to hold pressure. Necropsies will be performed in approximately 1/2 hr per animal on Day 56 (±5 days).

RESULTS. Urinary diversions were successfully established by surgical implantation of Neo-Urinary Conduit (NUC) from ureters to a stoma in the abdomen. It was possible to collect urine via the stoma indicating the success of NUC implantation. Hernia patches were evaluated to avoid potential bowel hernia/evisceration in quadrupedal animal with a ventral abdominal stoma.

The fixed urinary organs were collected, trimmed, examined, embedded in paraffin, and sectioned. Slides were stained with hematoxylin and eosin (H & E) and Masson's Trichrome (elastin). The abdominal cavity was opened and the outcome of the implanted test article and surgical methodology was visualized and digitally photographed *in situ.* The conduit was removed *en bloc* with the kidneys and ureters. The ureters were measured, and then detached from the conduit by transverse sectioning 3-4 cm away from the anastomotic site. Representative sections of the kidneys, ureters, lymph nodes, and any other lesions observed grossly were obtained. All tissue samples were placed in 10% Neutral Buffered Formalin (NBF) for 24 - 48 hours prior to histological processing.

After fixation, depending on the size and shape of the regenerated implant, the conduit was either opened longitudinally (parallel to the flow), or transverse (perpendicular to the flow). Figure 15 shows a trimming scheme. Post-fixation conduit tissue (longitudinally bisected), showing the various regions of interest (dashed/highlighted circles) for histological assessment. Tissue sampling (number of cassettes) submitted for histology processing varied (4 - 7 cassettes) depending on size of conduit. The direction of urine flow is indicated by arrow. The tissue sampling is outlined in Table 5.4. Some conduits were too short in length to accommodate the trimming scheme, so the available conduit was divided into fewer sections.

**Table 5.4**

| Cassette or section number | Tissue Sample |
|---|---|
| 1 | Left Kidney |
| 2 | Right Kidney |
| 3 | Lumbar Lymph Node |
| 4 | Mesenteric Lymph Nodes |
| 5 | Macro-cassette -the stoma, cranial and mid portion of the conduit |
| 6 | Macro-cassette - remaining mid portion of the conduit and left/right UCJs |
| 7 | Additional sections of NUC (if needed) |
| 8 | Left Ureter-Conduit Junction |
| 9 | Right Ureter-Conduit Junction |
| 10 | Left Ureter |
| 11 | Right Ureter |
| 12 | Stoma - skin Junction (if needed) |
| 13, 14 | 13, 14, etc Gross Lesions, as applicable |

| | |
|---|---|
| *UCJ - ureteral-conduit junction | |

During trimming of tissues, digital photographs were taken for illustration purposes. Post fixation, tissues were processed routinely to macroslides or microslides and stained with hematoxylin and eosin (H&E) and Masson's trichrome. In addition, four immunohistochemistry stains were performed on animal no. 3; anti-alpha smooth muscle actin and calponin for smooth muscle, anti-pancytokeratin (AE-1/AE3) and anticytokeratin 7 (CK-7) for epithelium/urothelium. All slides were evaluated microscopically. Where appropriate, microscopic observations for Individual Animal Data were given a score of "0" through "4" based upon the criteria listed in Text Table 5.5.

**Macroscopic and Microscopic Findings.** A comprehensive list of macroscopic findings and microscopic correlates for all animals was generated. The individual animal microscopic data for the Neo-Urinary Conduit was also collected. Upper urinary tracts of each animal were evaluated to assess the impact of stomal stenosis on the incidence of intermittent partial obstruction. Findings (e.g., hydroureter, hydronephrosis, pyelonephritis) were consistent with the observations of stomal stenosis as outlined below.

**Table 5.5**

| Grade | Interpretation |
|---|---|
| 0 (Not Present) | This score corresponded to an absence of histologic change. Normal; no apparent histological change. For the Smooth Muscle Score, indicated no smooth muscle layers in neo-urinary conduit wall (only connective tissue and possibly isolated myocytes present). |
| 1 (Minimal) | This score corresponded to a small histological change. The tissue involvement was considered minor, small or infrequent. The score reflected a focal, multifocal or diffuse distribution, in which approximately <10% of the tissue was involved. |
| 2 (Mild) | This score corresponded to a noticeable, but not prominent histological change. The tissue involvement was considered small, but consistently present. The score reflected a focal, multifocal or diffuse distribution in which approximately 10-25% of the tissue was involved. |
| 3 (Moderate) | This score corresponded to a histological change that was a prominent feature of the tissue. The tissue involvement was consistently present. The score reflected a focal, multifocal or diffuse distribution, in which approximately 26-50% of the tissue is involved. |
| 4 (Marked) | This score corresponded to a histological change that was overwhelming and persistent. The change may or may not a have adversely affect organ function, depending on the nature of the finding. The score reflected a focal, multifocal, or diffuse distribution, in which approximately > 50% of the tissue is involved. |

### Phase A animals (nos. 1, 2, and 3).

*Autologous Adipose Stoma (Animal 1):* This surgical approach resulted in the construction of a flush stoma, which remained patent until schedule necropsy time point (63-days). Patency of stoma was primarily achieved by placement of a stoma (tubing) port at the time of initial surgery that remained in place until subsequent reconstruction surgery. The reconstruction surgery consisted of a mucosal inversion, which was found to be ineffective in preventing stomal-skin strictures leading to stenosis and subsequent obstruction.

*Silicone Ring Stoma (Animal 2):* This surgical approach resulted in the construction of a raised stoma, which remained patent for the first 30 days. The placement of a stoma (tubing) port and subsequent stoma repair/reconstruction with dermal patch was made to prevent stoma narrowing from 30 days post-implant until the scheduled necropsy at 60 days post-implant. The initial silicone stoma construction and the dermal patch reconstruction methodologies did not prevent stomal stenosis.

*Shutter Skin Eversion Stoma (Animal 3):* This surgical approach resulted in the construction of a flush stoma, which remained patent until schedule necropsy time point (32-days). Stoma ports were used to secure ureteral stent lanyards in the stoma lumen to prevent external exposure of ureteral stent lanyards and to minimize environmental debris contamination of the post-operative urostomy site.

### Phase B animals (nos. 4, 5, and 6).

*Hernia patch:* The utilization of a larger abdominal wall defect and hernia patch resulted in a flush stoma that remained patent until necropsy. The intra-abdominal hernia patch appeared to integrate with the ventral wall of the NUC and may be unsuitable. The subcutaneous hernia patch was sufficient to prevent herniation and was acceptable for use.

**Immunohistochemistry.** Immunohistochemistry (IHC) analysis was performed only with animal no. 3 at 32 days post-implant to characterize the conduit neo-tissue, particularly at the stoma. The same time point was previously analyzed in canine studies evaluating the composition of neo-bladder tissue following Neo-Bladder Augment™ implantation (Jayo II 2008 *supra),* which was seeded with two cell types: bladder-derived urothelial cells and SMC; whereas the Neo-Urinary Conduit test article in this study was seeded with adipose-derived SMC only.

Cytokeratin 7 (CK-7) - The epithelium covering the luminal surfaces of the cranial and mid aspect of the conduit (section 5 - Table 5.4), stained negative for CK-7. However, the luminal epithelium section 6 (caudal aspect of the conduit) stained positive for CK-7.

Pancytokeratin (AE1/AE3) - The luminal epithelium present in Section 5 (Table 5.4), cranial and mid aspects of the conduit) stained negative for AE1/AE3, except for the epithelium at the skin-stoma interface. The epithelium covering the luminal surfaces in section 6 (from the caudal aspect near the ureteral anastomosis)stained positive for AE1/AE3.

Anti α-Smooth Muscle Actin (SMA) - The conduit wall components from section 5 (Table 5.4) at the cranial and mid conduit stained negative for SMA. The wall components of section 6 (Table 5.4), at the caudal region of the conduit, stained weak-positive for SMA. Calponin (CLP) - The conduit wall components from section 5 at the cranial and mid conduit of section 6 (Table 5.4), at the caudal region of the conduit, stained weak positive for CLP. These findings are consistent with those previously reported (Jayo II (2008) *supra*)*.* In addition, epithelium covered the neo-tissue of the skin-stoma interface and the luminal surfaces of the cranial and mid aspect of the conduit.

### CONCLUSIONS:

The *Adipose, Silicone* and *Shutter* methodologies used in the construction/reconstruction of the stoma for the first three animals (1, 2, and 3) displayed subdermal stomal strictures leading to stenosis. Use of an intra-abdominal hernia patch (between muscle and viscera) in flush stoma formation displayed an excessive inflammatory host response leading to obstruction and subsequent fistula formation. The use of subcutaneous hernia patch (between subcutaneous fat and skeletal muscle) in flush stoma formation induced only mild to moderate inflammation and significantly reduced stoma-skin strictures, resulting in prolonged stomal patency. The prevalence of urinary flow obstruction at the stoma was primarily caused by stomal stenosis or use of an internal (between muscle and viscera) patch that led fistula formation. The incidence of upper urinary tract findings was consistent with the stomal stenosis observed.

Thirty-two days post-implantation, native-like mucocutaneous junction was being formed at the skin-stoma interface and at the luminal mucosal surfaces, as evidenced by the cytokeratin-7 and pancytokeratin positive epithelium observed at the cranial and mid aspect of the conduit.

The neo-tissue composition of the conduit tissue in swine at 32 days postimplantation was consistent with neo-bladder tissue in canines at an equivalent time post-implantation (Jayo II (2008) *supra*), suggesting equivalence of the urinary tissue regeneration outcome elicited by a Neo-Bladder Augment in canine and the Neo-Urinary Conduit in swine.

The equivalent urinary neo-tissue formation elicited by the Neo-Bladder Augment in canines (Jayo II (2008) *supra)* and the Neo-Urinary Conduit in swine further suggest that the regenerative capacity of test articles seeded with two cell types (bladder-derived urothelial cells and smooth muscles cells) or one cell type (adipose-derived smooth muscle cells) is similar.

### Example 6 - Lung tissue cell isolation from adult rat

Briefly, lungs were rinsed with PBS. Tissue was minced on ice to thoroughly break up pleural membrane. The minced tissue was digested with collagenase IV for 20 min at room temperature (RT) with rocking and then titurated and pelleted by gravity, aspirating the supernatant. The digestion and tituration steps were repeated twice. After final digestion, the sample was titurated and filtered with 100uM Steriflip. The sample was then neutralized with DMEM + 10% FBS and cells were pelleted at 300xg for 5 min. Cell pellets were washed 3 times with DMEM + 10% FBS after final wash pellet cells at 500xg for 5 min. Cells were resuspended in 50/50 Media [50% DMEM high glucose (4.5g/L), 50% KSFM containing Hu rEGF1-53, BPE, 5% FBS, 1X Anti-Anti and 1X Insulin Transferrin Selinium (ITS)].

### Example 7 - Gene and protein expression of bronchiolar and alveolar specific markers in isolated lung cells in 3D cultures

Cells isolated as in Example 6, were plated onto Matrigel, Gel foam and poly-lactic-co-glycolic acid (PLGA) foam and maintained in 50/50 media to test the hypothesis that these cells can be isolated and express markers consistent with their lung origin.

Results : Markers tested : CCSP (Clara cells secretory protein) for bronchiolar epithelial cells; proSP-C (pro-surfactant protein) for alveolar epithelial cells.

Figure 16A shows the expression of Clara Cell Secretory Protein from Lung Alveolar Forming Units in rat lung digest grown on Matrigel >14 days and immunostained with Clara C (Millipore) at 1/2000 (green). DAPI was used for nuclear staining (blue).

Figure 16B shows the expression of Prosurfactant Protein C from Lung Alveolar Forming Units in rat lung digest grown on Matrigel >14 day and immunostained with ProSP-C (Millipore) at 1/2000 (green). DAPI was used for nuclear staining (blue).

Figure 16C shows the expression of Prosurfactant Protein C from Lung Alveolar Forming Units in lung alveolar cells 10-11 days on gel foam and stained with ProSP-C 1/2000 (green). DAPI was used for nuclear staining (blue).

Figure 16D shows a close-up view of the expression of Prosurfactant Protein C from Lung Alveolar Forming Units in lung alveolar cells growing on GelFoam (10-11 days), stained with ProSP-C 1/2000 (green). DAPI was used for nuclear staining (blue).

Figure 17 shows the expression of Clara Cell protein from Lung Alveolar Forming Units in lung alveolar cells 10-11 days on PLGA foam and stained with Clara Cell SP 1/2000 (green).

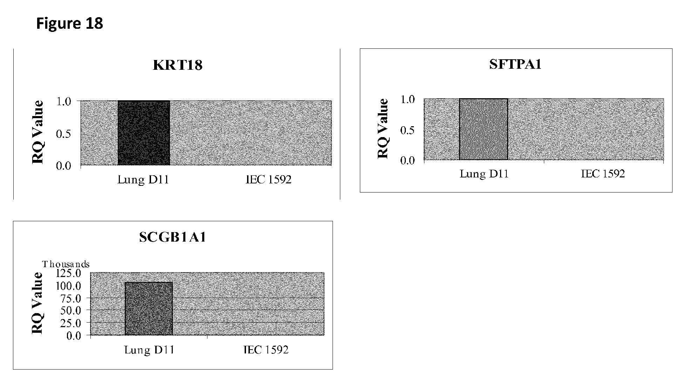

Figures 18 depicts the expression of KRT18 from Lung Alveolar Forming Units. KRT18 is a lung specific cytokeratin and epithelial marker. Lung D11 was used as the calibrator since IEC 1592 was negative, therefore it received a default RQ value of 1.0. Figure 18 also depicts the expression of SCGB1A1 from Lung Alveolar Forming Units. SCGB1A1 (Secretoglobin, Family 1A, Member 1 (Uteroglobin)) is a Clara Cell Marker. Robust expression of SCGB1A1 confirms IF staining for Clara Cells. Figure 18 also shows the expression of SFTPA1 from Lung Alveolar Forming Units. SFTPA1 (Surfactant Protein A1) is an alveolar epithelial marker. Lung D11 was used as the calibrator since IEC 1592 was negative, therefore it received a default RQ value of 1.0.

Gene and protein expression of bronchiolar and alveolar specific markers support conclusion that these cell types have been successfully isolated and cultured in 3-dimensions, using 2 different support materials. Spontaneously-forming pulsatile cell bodies have been observed from lung tissue cell isolates. These are tridimensional cell clusters with satellite streaming cells under several cell culture conditions including, but not only, the use of Matrigel. The cells characterized in these clusters are of epithelial, smooth and skeletal muscle, and neural origins, as assessed by immunoreactivity with antibodies to vWF, calponin, and connexin 43, respectively. The clusters have been shown to pulsate at rhythmic rates and show contractility in vitro.

### Example 8 - Lung AFU derived bodies grown on Matrigel

Connexin 43 detects cell-to-cell channels. Clusters of these channels assemble to make gap junctions. Gap junction communication is important in development and regulation of cell growth.

Figure 19 shows a confocal image of rat lung AFU stained with connexin 43. The left panel of Figure 19 depicts 'bunch of grapes' appearance typical of AFU. The right panel depicts apparent hollow cavity within AFU. Pulsatile body formation was observed from lung AFU on Matrigel. Bodies demonstrated spontaneous contractility.

In conclusion, isolated rat lung cells appear able to form AFU structures during culturing in 3-dimensions.

### Example 9 - Lung AFU on Gelfoam and PLGA scaffolds with and without pre-seeding with Ad-SMC

Figure 20 depicts lung AFU on Gelfoam and PLGA scaffolds with and without pre- seeding with Ad-SMC (top left panel - Gelfoam pre-seeded with Ad-SMC, then seeded with isolated lung cells; top right panel - Gelfoam without pre-seeding with Ad-SMC, then seeded with isolated lung cells; bottom left panel - Gelfoam pre-seeded with Ad-SMC, then seeded with isolated lung cells; bottom right panel - Gelfoam without pre-seeding with Ad-SMC. then seeded with isolated lung cells; arrows depict apparent AFU formation on scaffolds pre-seeded with Ad-SMC); not apparent on scaffolds seeded with isolated lung cells only, no pre-seeding with Ad-SMC.

Figure 21 shows Gelfoam (-) Ad-SMC stained with antibody to Clara cell protein in top left panel; top right panel shows Gelfoam (-) Ad-SMC phase image; bottom left panel shows Gelfoam (+) Ad-SMC stained with antibody to Clara cell protein; and bottom right panel shows Gelfoam (+) Ad-SMC phase image. Increased intensity of staining in bottom left panel compared to top left panel suggests that the presence of Ad-SMC supports rat lung cell proliferation.

Figure 22 shows Gelfoam (-) Ad-SMC stained with antibody to Surfactant Protein C in top left panel; top right panel shows Gelfoam (-) Ad-SMC phase image; bottom left panel shows Gelfoam (+) Ad-SMC stained with antibody to Surfactant Protein C; and bottom right panel shows Gelfoam (+) Ad-SMC phase image (arrows in bottom panels depict apparent AFU formation). Increased intensity of staining in bottom left panel compared to top left panel suggests that the presence of Ad-SMC supports rat lung cell AFU formation.

Figure 23 depicts PLGA scaffold (+) Ad-SMC stained with antibody to Clara Cell Protein in top left panel; top right panel - PLGA scaffold (+) Ad-SMC phase image; and bottom left panel shows merging of immunofluorescent and phase images (arrows in top panel depicts apparent AFU formation).

Figure 24 shows Gelfoam scaffold (+) Ad-SMC stained with antibody to Surfactant Protein C; top right panel shows Gelfoam scaffold (+) Ad-SMC phase image; bottom left panel shows merging of immunofluorescent and phase images (arrows in panels depict hollow spaces in the Gelfoam).

In conclusion, Ad-SMC facilitates increased rat lung cell proliferation. Pre-culturing of Gelfoam and PLGA scaffold material with Ad-SMC before seeding with rat lung cells increases apparent rat lung cell AFU formation compared to scaffolds which were not pre-cultured with Ad-SMC. Rat lung cells growing around the edges of the hollow spaces within Gelfoam mimic the appearance of alveolar structures within the intact lung.

### Example 10 - Isolation of smooth muscle cells from adipose for seeding on GI scaffolds

Abdominal adipose samples from 14 male Lewis rats were obtained subcutaneously and washed 3 times with an equal volume of DMEM-HG/antibiotic/antimycotic (Invitrogen-Gibco) (5ug/ml). Adipose was digested with filter-sterilized collagenase I (0.3%, 1 % BSA, in DMEM-HG) at 37°C for 1 hour, then centrifuged for 5 minutes at 300g in 50ml conical tube. The stromal vascular fraction (SVF) was filtered through a 100um Steriflip vacuum filter to remove fat and remnant tissue and then neutralized with DMEM-HG + 10% FBS. The cell population was pelleted and washed again at 300g for 5 minutes and resuspended in DMEM-HG + 10% FBS + antibiotic/antimycotic 5ug/ml.

Primary adipose tissue-derived smooth muscle cells (Ad-SMC) were plated and maintained in SMC growth medium (DMEM-HG/antibiotic/antimycotic (5ug/ml) supplemented with 10% fetal bovine serum (FBS; Invitrogen-Gibco). Cultures were incubated at 37°C in a humidified, 5% CO₂-containing atmosphere. Passaging was performed at 70-90% confluence by removing the cells from the tissue culture plastic by enzyme digestion with trypsin (Invitrogen-Gibco) and re-plating onto fresh culture vessels. The morphology of rat adipose-derived cells was observed (passage 1, 20X) and appeared identical to that of adipose-dervied cells cultured previously from other species, including canine, porcine, and human.

Immunofluorescence. Culture medium was removed from the dish and the adherent cells rinsed three times with phosphate buffered saline (PBS). Cells were fixed with 2% paraformaldehyde/PBS overnight at 4°C and then rinsed three times with PBS. Cells were incubated overnight with calponin and smooth muscle alpha actin primary antibody (3ug/mL final concentration) diluted in permeabilization buffer (PBS containing 0.2% Triton X-100 and 2% normal goat serum. Following three rinses with PBS, secondary antibody was added at a final concentration of 1ug/mL and incubated for 30 min. Cell nuclei (blue) were stained with Hoechst dye and rinsed three times with PBS prior to viewing with a fluorescent microscope (Leica DMI 4000B). Immunostaining was performed to confirm that these cells expressed a subset of smooth muscle cell markers at the protein level. Calponin and smooth muscle alpha-actin were chosen based on our experience that these proteins are the most reliably detected in statically cultured cells compared to the others (unpublished data). The filamentous staining observed in rat cells isolated from adipose was identical to that observed for staining smooth muscle cells isolated from other tissues. Calponin and smooth muscle alpha-actin immunostaining was observed and provide support that the cells being isolated and cultured were rat adipose-derived smooth muscle cells (Ad-SMC). Calponin and smooth muscle alpha-actin protein expression appears identical to that of Ad-SMC cultured previously from other species, including canine, porcine, and human.

TaqMan qRT-PCR. RNA was purified from rat Ad-SMC using the RNeasy Plus Mini Kit (Qiagen) according to the manufacturer's instructions. cDNA was generated from 2 µg of RNA using the SuperScript VILO cDNA Synthesis Kit (Invitrogen) according to the manufacturer's instructions. Following cDNA synthesis, each sample was diluted 1:6. qRT-PCR was setup using 10 µl master mix (2X), 1 µl primer/probe, 9 µl cDNA (diluted 1:6). The following TaqMan primer/probes were used for evaluation of smooth muscle, endothelial and adipogenic gene expression: SmαA (smooth muscle alpha actin), SM22, MYOCD (myocardin), SMMHC (smooth muscle myosin heavy chain), CNN1 (calponin), ADIPOQ (adiponectin), FABP-4 (fatty acid binding protein #4), CDH5/VECAD (vascular endothelial cadherin), vWF (von Willebrand factor), PECAM1 (platelet endothelial cell adhesion molecule #1), and KDR/FLK1 (fetal liver kinase #1). PPIB (Peptidylprolyl Isomerase B) was used as endogenous control and all samples were calibrated against rat adipose tissue cDNA.

| **Rat TaqMan Primers for Alternate Cell Source Markers** | | | |
|---|---|---|---|
| **Gene** | **Abbrv**. | **Marker** | **TaqMan Cat #** |
| Adiponectin | ADIPOQ | Adipogenic | Rn00595250_ml |
| Fatty-Acid Binding Protein 4 | FABP4 | Adipogenic | Rn00670361_ml |
| Cadherin 5 | CDH5/VECAD | Endothelial | Rn01536708 ml |
| vonWillebrand Factor | vWF | Endothelial | Rn01492i94_sl |
| Platelet/Endothelial Cell Adhesion Molecule | PECAM1 | Endothelial | Rn01467259_ml |
| Kinase Insert Domain Receptor | KDR/FLK1 | Endothelial | Rn00564986_ml |
| Smooth Muscle Alpha Actin | ACTA2/SMAA | Smooth Muscle | Rn01759928_gl |
| Transgelin/SM22 | SM22 | Smooth Muscle | Rn00580659_ml |
| Myocardin | MYOCD | Smooth Muscle | Rn01786178_ml |
| Smooth Muscle Myosin Heavy Chain | MYH11/SMMHC | Smooth Muscle | Rn01530339_ml |
| Calponin | CNNI | Smooth Muscle | Rn00582058_ml |
| Peptidylprolyl Isomerase B | PPIB | Endogenous Control | Rn00574762_ml |

All primer/probes were secured from Applied Biosystems. All reactions were carried out in an ABI 7300 real time thermal cycler using default cycling parameters. Analysis of PCR data was performed using the method of Relative Quantitation (RQ) by Comparative Ct.

Total RNA was isolated and quantitative RT-PCR performed to assess expression of committed smooth muscle marker genes myocardin, smooth muscle alpha actin, transgelin, myosin heavy chain, and calponin across multiple passages. These proteins are all involved in the contractile function of smooth muscle cells. Our results indicate that the cells isolated from rat adipose tissue express all of these committed smooth muscle cell markers at the transcript level. Marker expression was observed in cells cultured immediately following isolation (P0) and in the subsequent 3 passages (P1, P2, and P3), after which the cultures were terminated. Overall, expression of SMC markers is present in the original rat culture (P0), and increases upon subsequent passages (P1- P3). This pattern of expression appears identical to that of Ad-SMC cultured previously from other species, including canine, porcine, and human.
Expression of adipocyte markers adiponectin and fatty-acid binding protein-4, and epithelial markers cadherin-5, platelet/epithelial cell adhesion molecule, kinase insertion domain receptor, and von Willebrand factor were observed in the cultures immediately following isolation (P0). This was expected, since these cell types are also present in adipose tissue. Expression of these markers decreases markedly upon the first passage (P1) and becomes barely detectable in subsequent passages (P2, P3). This pattern of loss of adipocyte and endothelial marker expression reflects that of Ad-SMC cultured previously from other species, including canine, porcine, and human.

Using molecular and protein analysis, we have confirmed that we can isolate adipose-derived smooth muscle cells (Ad-SMC) from rat subcutaneous fat. Rat Ad-SMC have identical characteristics to Ad-SMC isolated from canine, porcine, and human adipose tissues.

### Example 11 - GI scaffolds seeded with smooth muscle cells

The compatibility of the smooth muscle cells upon biomaterials was tested. Several scaffold materials were tested including the following: PCL foam: a) 23-53 µm; b) 106-150 µm; c) 150-250 µm; d) 250-300 µm; PLCL foam (150-250 µm); Regular PLGA/PGA felt (3mm thick); Thin PLGA/PGA felt (0.5 mm thick); Gelatin PLGA/PGA felt; Vicryl woven PGA mesh; Woven PGA tube.

Scaffold Material Preparation - Foams were prepared and other additional materials were purchased from commercial vendors. Coating of the PGA felts was performed in house. After preparation of materials, 5 mm punches were obtained from each material (n=3) and kept in a desiccator until ready for use.

Culture of Seeded Scaffolds - Scaffolds were briefly sterilized with 60% Ethanol for approximately 20 minutes and rinsed in 1X PBS for 5 minutes. Smooth muscle cell (SMC) culture medium was used to prewet the scaffolds for 15 minutes. Afterwards, 3 x 10⁵ rat Ad-SMC in 15 µl of SMC media were seeded onto each scaffold using ultra-low cell attachment 24 well plates. The cells were allowed to attach to the scaffold for 3 hours in a 37°C humidified incubator.

MTS Assay - After the cells attached for 2 hours, each well in the plate was filled with PBS and placed on a rocker for 5 minutes, the PBS was aspirated and then replaced with fresh PBS; this was repeated 3 times. This procedure was performed to remove any unattached cells from the scaffold. After the last wash in PBS, an MTS assay (a non-destructive cell proliferation assay; kit obtained from Promega) was performed on the scaffold to determine rapid cell attachment onto the materials. Briefly, an MTS solution was applied to each scaffold at a ratio of 20 µl MTS to 100 µl SMC media. After the addition of the MTS solution, the scaffolds were incubated for 1 hour on a rocker. The solution from each scaffold was placed in a 96 well plate and the absorbance levels read at 490 nm on an Elisa plate reader. The scaffolds were then rinsed with 1X PBS until all MTS solution was removed. Fresh SMC media was added to the scaffolds and culturing at 37°C was continued for an additional 7 days. At this time, another MTS assay was performed to assess cell attachment.

Figure 25A-C shows attachment/proliferation of smooth muscle cells on various biomaterials. Despite the PCL and PLCL scaffolds, having lower cell attachment values 2 hours after seeding (as seen with the MTS assay absorbance readings), the live/dead images seem to show better cell coverage on these materials compared to the PGA scaffolds. The PCL scaffolds with the 23 - 53 and 106 - 150 µm pore sizes seem to have the most non-viable cells in comparison to the other scaffolds. By day 7, the MTS assay absorbance readings showed that the woven meshes and regular PLGA/PGA scaffolds have the highest absorbance readings, thereby having a higher cell proliferation rate. This general trend was seen as well 2 hours after seeding apart from the thin PLGA/PGA felt, where there was a decrease in growth by day 7. This could be attributed to the "thinness" of the felt and the amount of fibers present and the fact that there might not be any more room for the cells to proliferate and grow on.

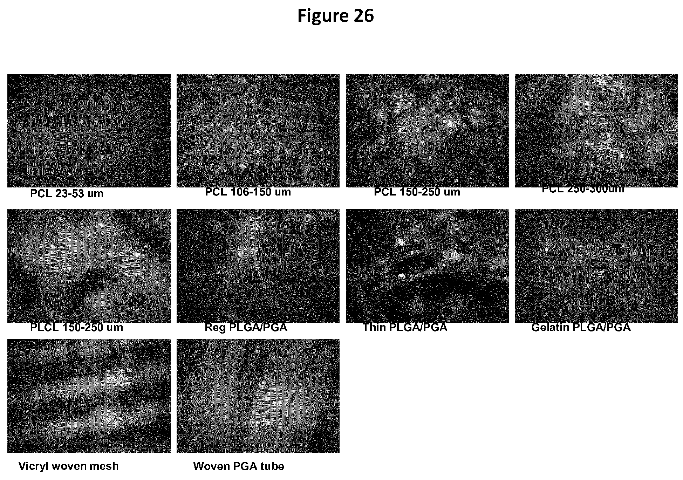

Figure 26 shows the results of a live/dead assay for smooth muscle cells deposited on various biomaterials. A live/dead viability/cytoxicity kit was obtained from Invitrogen. After 2 days in culture, the live/dead assay was performed on the cells on scaffolds to assess viability.

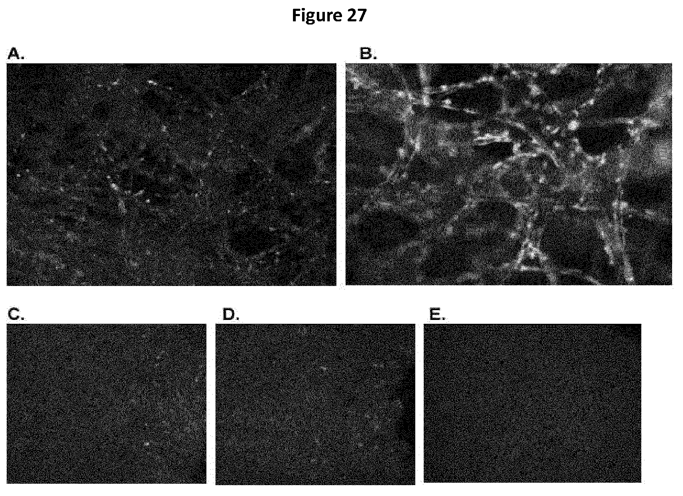

Cell attachment and viability studies using rat Ad-SM and a variety of biomaterials supports moving forward with Regular PLGA/PGA, Thin PLGA/PGA, Vicryl woven mesh, and Woven PGA tube as test articles for the in vivo proof of concept studies. Rat Ad-SMC behaves as Ad-SMC isolated from canine, porcine, and human adipose tissues with respect to cell attachment to biomaterials. PGA/PLGA scaffolds, previously applied successfully for regeneration of bladder (Basu and Ludlow, Trends in Biotechnology, 28: 528-533, 2010; Jayo et al. (2008) Regen Med 3, 671-682) were seeded with Ad-SMC and incubated in culture medium for 7 days. At this time, seeded scaffolds were incubated in Calcein AM (green) and Ethidium Homodimer 1 (red) to highlight live and dead cells, respectively. As shown in Figure 27A, the scaffold was covered with live cells, as indicated by green fluorescence, indicating that the biomaterial used supports rat Ad-SMC attachment and viability. Higher magnification (Figure 27B) reveals details of the attachment along the scaffold fibers.

### Example 12 - Isolation of esophageal cells

Rat esophageal tissue was removed from the shipping container and placed in DMEM+5ug/mL Gentamycin (Wash Solution) and swirled frequently for 5 min. The tissue was then placed into fresh Wash Solution. This process was repeated a total of 3 times before mincing the tissue to a uniform size. The minced tissue was then placed into a 50mL centrifuge tube containing Digest Solution (300U/mL Collagenase TypeIV-Worthington/Dispase-Stem Cell in DMEM; 20mL/1g tissue). Digestion proceeded for 30 min at 37°C. Enzyme neutralization was achieved using 20%FBS in KSFM media. The digested tissue was then mixed, filtered through a 100uM Steriflip filter to ensure that no large tissue fragments were carried over. This material was then centrifuged at 300g for 5 min to pellet the cells. The cell pellet was then washed with KSFM. The cell were then counted and plated in Growth Medium (KSFM+2%FBS or KGM (50:50 of KSFM with Supplements + DMEM 10%FBS containing 1X Anti/Anti, 1X ITS).

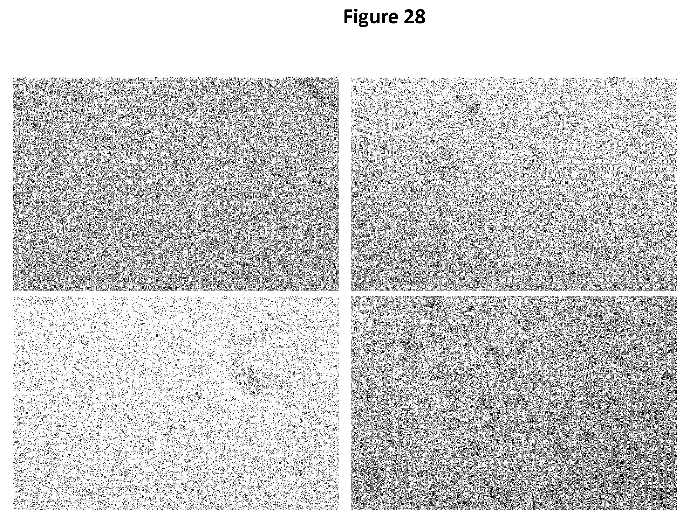

As shown in Figure 28, upon initial attachment (panel 1 top left), there appears to be a mixed population of rounded and elongated cell types. Subsequent passaging (P1; remaining panels 2-4) further reveals a culture comprised of rounded and elongated cell types. TaqMan qRT-PCR. RNA was purified from rat Ad-SMC using the RNeasy Plus Mini Kit (Qiagen) according to the manufacturer's instructions. cDNA was generated from 2 µg of RNA using the SuperScript VILO cDNA Synthesis Kit (Invitrogen) according to the manufacturer's instructions. Following cDNA synthesis, each sample was diluted 1:6. qRT-PCR was setup using 10µl master mix (2X), 1µl primer/probe, 9µl cDNA (diluted 1:6). The following TaqMan primers were used for evaluation of epithelial gene expression: KRT8 (keratin 8), vWF (von Willebrand factor). PPIB (Peptidylprolyl Isomerase B) was used as endogenous control and all samples were calibrated against rat whole esophagus tissue cDNA. All primer/probes were secured from Applied Biosystems. All reactions were carried out in an ABI 7300 real time thermal cycler using default cycling parameters. Analysis of PCR data was performed using the method of Relative Quantitation (RQ) by Comparative Ct.

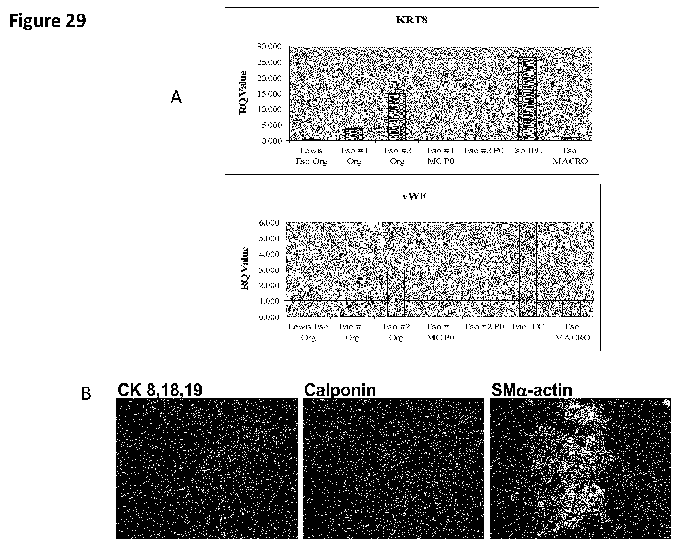

As shown in Figure 29A, gene expression for epithelial cell markers KRT8 and vWF gene was observed for esophageal tissue (Eso #1 org; ESO #2 Org) and cultured esophageal cells (Eso IEC).

Immunofluorescence. Culture medium was removed from the dish and the adherent cells rinsed three times with phosphate buffered saline (PBS). Cells were fixed with 2% paraformaldehyde/PBS overnight at 4°C and then rinsed three times with PBS. Cells were incubated overnight with CNN1 (calponin), SMα-actin (smooth muscle alpha-actin), CK 8,18,19 (cytokeratin 8,18,19) primary antibody (3-5ug/mL final concentration) diluted in permeabilization buffer (PBS containing 0.2% Triton X-100 and 2% normal goat serum. Following three rinses with PBS, secondary antibody was added at a final concentration of 1 µg/mL and incubated for 30 min. Cell nuclei were stained with Hoechst dye and rinsed three times with PBS prior to viewing with a fluorescent microscope. As show in Figure 29B, cytokeratin 8,18,19 staining further supported the qRT-PCR data that these cultures contained epithelial cells. Calponin and smooth muscle alpha-actin staining was performed to determine if these cultures contained smooth muscle cells. These results support the conclusion that rat esophageal cell cultures are minimally composed of a heterogeneous population of epithelial and smooth muscle cells.

### Example 13 - Esophageal cell migration onto GI scaffolds

An in vitro assay was developed to assess the ability of esophageal cells to migrate onto regular PLGA/PGA scaffold material. Briefly, a circular hole of a defined diameter was cut into the scaffold using a sterile biopsy punch (see left panel of figure below). Esophageal tissue was then inserted into this hole (see right panel of figure below) and the entire cassette incubated in Growth Medium KGM (50:50 of KSFM with Supplements + DMEM-HG + 5 %FBS supplemented with antibiotic/antimycotic; 5ug/mL, and 1X insulin/transferring/selenium (Invitrogen)) at 37°C in a humidified 5% CO₂ atmosphere for 8 days.

An intact, native esophagus consists of an inner luminal layer of epithelial cells. To address whether a scaffold seeded with SMCs only could facilitate the migration of esophageal epithelial cells, an experiment was performed whereby surgically removed rodent esophageal tissue explant was inserted into a scaffold cassette, which had been previously unseeded or seeded with Ad-SMC.

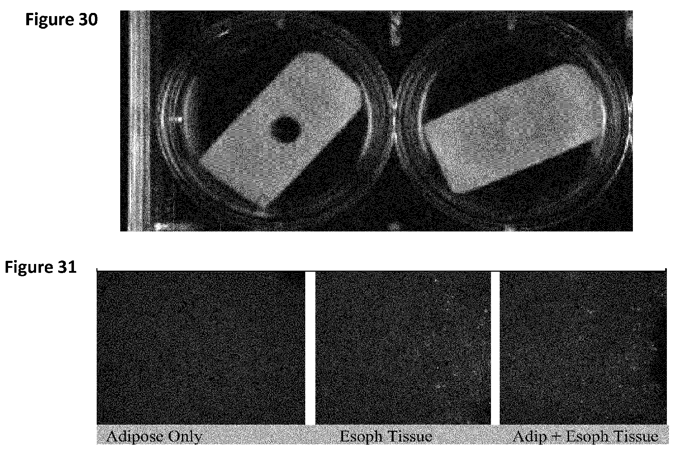

Figure 30 illustrates the design of the experiment whereby a surgically removed rodent esophageal tissue explant was inserted into a scaffold cassette alone (middle panel below) or a scaffold cassette seeded with Ad-SMC (right panel). After incubation for 8 days, the cassette was fixed and stained with antibody to cytokeratin, as a marker for epithelial cells. As demonstrated by the greater distribution of green fluorescence, esophageal epithelial cells migrated a greater distance from the tissue into the scaffold cassette which was previously seeded with rat Ad-SMC (Figure 27D) compared to a scaffold cassette which was not pre-seeded with rat Ad-SMC (Figure 27C). As expected, a cassette seeded with rat Ad-SMC only, without esophageal tissue inserted, was void of detectable epithelial cells (Figure 27E). While quantitating the number of epithelial cells migrating into the scaffold was not performed, the increased density of fluorescence at the leading edge of the scaffold in Figure 27D compared to Figure 27C suggests that pre-seeding the scaffold with Ad-SMC results in a greater number of esophageal cells migrating into the biomaterial.

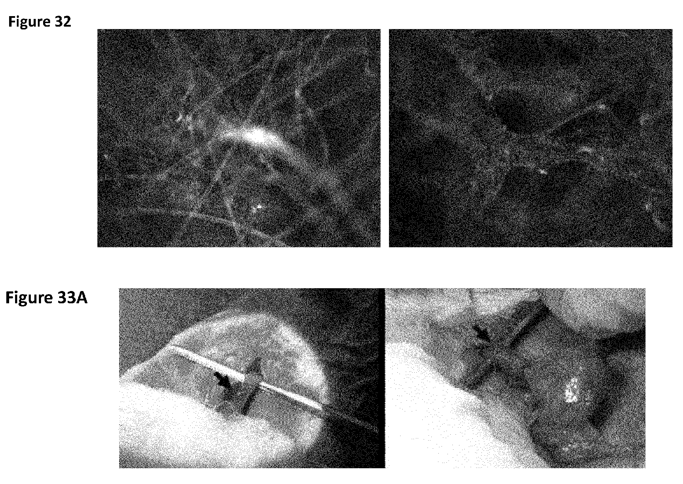

Figure 31 shows increased migration/attachment of cytokeratin + esophageal cells from tissue toward adipose-seeded PGA/PLGA coated scaffold. Figure 31 shows greater distribution of the green fluorescence, indicating more esophageal epithelial cells migrated from the tissue into the scaffold cassette first seeded with rat Ad-SMC (right panel) than onto a scaffold cassette not containing rat Ad-SMC (middle panel). As expected, cassette with rat Ad-SMC only as a control (no esophageal tissue inserted) showed no presence of epithelial cells (left panel). In a variation of this experiment, thin PLGA coated PGA felt was sutured to rat esophagus, cultured 12-14 days, and the scaffold cut and stained with DAPI. As shown in Figure 32 (both panels), esophageal cells have migrated onto the scaffold, as indicated by the white spots (DAPI stain).

### Example 14 - Implantation of esophageal tissue constructs

The following study concerns the isolation and genotypic and phenotypic characterization of smooth muscle cells (SMCs) from rat adipose for the purpose of applying tissue engineering technology to developing esophageal tissue replacements. It was found that the adipose-derived SMCs may be used to seed synthetic, biodegradable scaffold patches and that implantation of these seeded scaffolds into a rat esophageal injury model leads to successful regeneration of the laminarily organized esophagus wall.

**Smooth muscle cell isolation and culture from rat adipose.** SMCs were isolated and cultured according to the protocols of Example 1 and 4 (see also Ludlow et al. U.S. Published Patent Application No. 20100131075).

**In vitro Esophageal Cell Migration Assay.** The assay was performed as described in Example 13.

**Scaffold Production.** The scaffold was comprised of polyglycolic acid (PGA) mesh coated with poly-DL-lactide-co-glycolide (PLGA). PGA is supplied as a non-woven felt in the form of 20 cm x 30 cm x 0.75 mm sheet, with a bulk density of 70 mg/cc (Concordia Medical). PLGA (50:50, Durect Corporation, IV=0.55-0.75) was dissolved in methylene chloride before use. The PGA mesh was coated by dipping into a beaker containing PLGA solution (a liquefied copolymer (poly-DL-lactide co-glycolide 50:50 80 mg/ml methylene chloride). This coating was added in order to achieve adequate mechanical stability. The coated PGA mesh was then cut to size, sterilized with ethylene oxide, and stored in a desiccant chamber until use.

**Construct Implant Manufacture.** Sterile scaffolds (3mm x 5mm) were hydrated in SMC growth medium and seeded with 50,000 cells to make the construct for implantation. The construct was covered with growth medium and incubated at 37°C in a humidified, 5% CO2-containing atmosphere for 5 days prior to implantation.

**Animal Surgery.** Under anesthesia, female Lewis rats (approximately 28 days old) underwent upper midline abdominal laparotomy. The abdominal esophagus was mobilized and a defect measuring approximately 3 mm in width and 5 mm in length was created in the abdominal esophagus 5 mm proximal to the cardia and replaced by the implanted construct using interrupted sutures of non-absorbable 7-0 silk (Ethicon). The construct was then covered with omentum. The abdomen was closed after gentamicin (0.1 mg) was given intraperitoneally. Postoperatively, the rats were returned to their cage and allowed unrestricted oral soft food and water intake for 7 days. After this time, the rats were allowed unrestricted oral hard rat chow and water intake.

**Histological evaluation.** At necropsy, tissue containing the tissue engineered esophagus, as identified and delineated by the non-absorbable sutures, was fixed in 10% buffered formalin (Sigma). Selected sections of the esophageal wall (defect site) were dehydrated in ascending series of ethanol, embedded in paraffin. Sections (5 µm) were cut and stained with hematoxylin & eosin and Masson's Trichrome (Premier Laboratory LLC, Longmont, CO.) to visualize stromal and muscle components.

Figure 33A depicts the surgically-created esophageal defect and subsequent construct implantation. Since the esophagus was distended to permit access to a workable field, surgical resection resulted in the defect being somewhat oval in shape (Figure 33A- left panel). Upon implantation, the non-absorbable sutures used to secure the construct were purposely made visible at the edges of the implant so they could be used to delineate the boundaries of the regenerated esophageal tissue upon necropsy (Figure 33A - right panel). Animals were sacrificed at 3.5, 10, 12, and 16 weeks post-implant. Table 14.1 summarizes the histological outcomes observed for these time points.

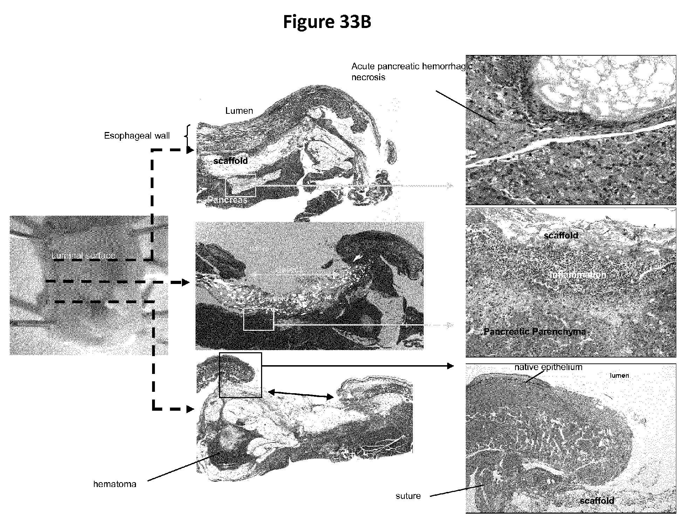

Figure 33B shows the histology at 1 day post implantation. Transverse sections through the defect show adequate apposition of implanted construct to the margins of defect. At the omental wrap side there is organization early tissue regenerative healing responses characterized by the penetration into the construct's spaces of new vessels (neo-vascularization), mild inflammatory infiltrates (neutrophilic and mononuclear), free and intravascular red cells, and eosinophilic fibrin. Multifocal aggregates of bacterial colonies are also present. The luminal surface of the scaffold shows no evidence of epithelialization. Pancreatic tissue is present, with acute hemorrhagic pancreatitis. The surgical site is anatomically near the pancreas and omentum used. It should be noted that iatrogenic or post-surgical inflammation can accidentally entrap the head or tail of the pancreas.

**Table 14.1**

| Animal | Time-course | Histological Outcome |
|---|---|---|
| 1 | 3.5 Weeks | •Nearly complete neoesophagus regeneration characterized by formation of all three wall layers; mucosa, muscularis, and serosa |
| 2 | | •Complete re-epithelialization of mucosal surface and submucosa |
| | 10 weeks | •Partial regeneration of the muscularis externa |
| | | •Minimal scaffold fibers present |
| | | •Minimal inflammation |
| | | •No evidence of calcification, necrosis, or bacterial colonization |
| 3 | 12 weeks | •Complete regeneration of mucosa and submucosa at defect site |
| | | •Incomplete regeneration of muscularis layers |
| | | •Complete re-epithelialization of mucosal surface and submucosa |
| 4 | 16 weeks | •Partial regeneration of the muscularis externa |
| | | •Minimal scaffold fibers present |
| | | •Minimal inflammation |
| | | •No evidence of calcification, necrosis, or bacterial colonization |

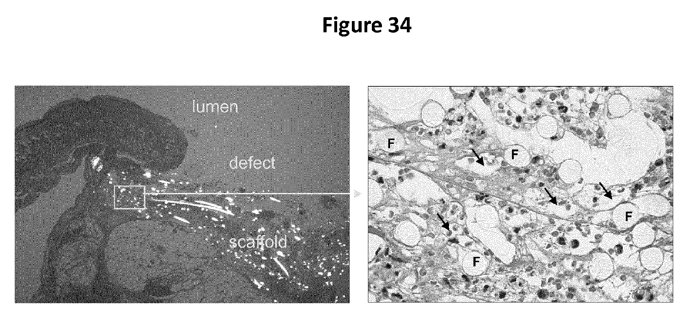

Figure 34 shows neo-vascularization (angiogenesis). The left panel: 40x magnification H&E stained (polarized) section showing the construct's scaffold material within the defect site. The right panel: higher magnification (600x) of the green-boxed area (at left), showing the marked angiogenesis (arrows) within the implanted scaffold fibers of construct (F).

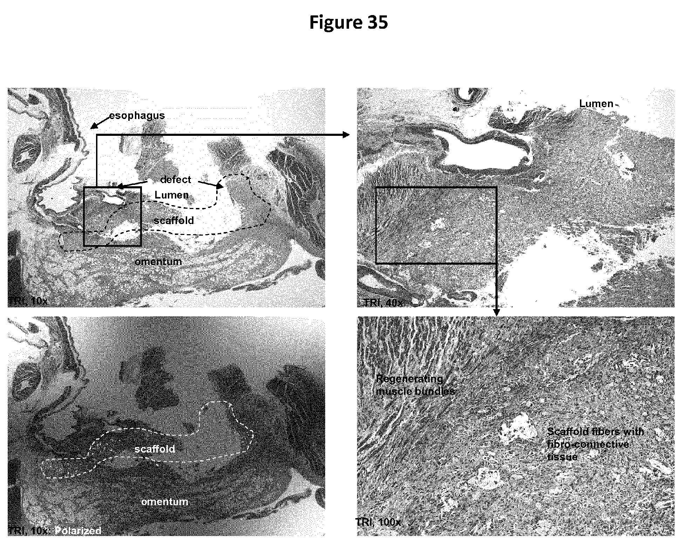

Figure 35 shows the histology at 8 days post implantation. Sections were taken from the center of the implant. Section 2 (longitudinal); the center of the treatment site was captured in this section, which shows adequate apposition of scaffold to the margins of defect. There is no appreciable evidence of epithelialization in the luminal surface. The underlying wall shows early organization consisting granualtion/fibro-connective tissue, neo-vascularization and focal regeneration of smooth muscle bundles near the margins but none observed closer to the center of the implant where most of the scaffold material appeared to be (due to tissue cutting artifact). Overall, there is moderate active chronic inflammation predominantly at the center and mild at the periphery. The omental wrap side shows early organization consisting of fibro-connective tissue and liver appears adhered to omentum without significant injury. There is no evidence of calcifcation, necrosis or bacterial colonization.

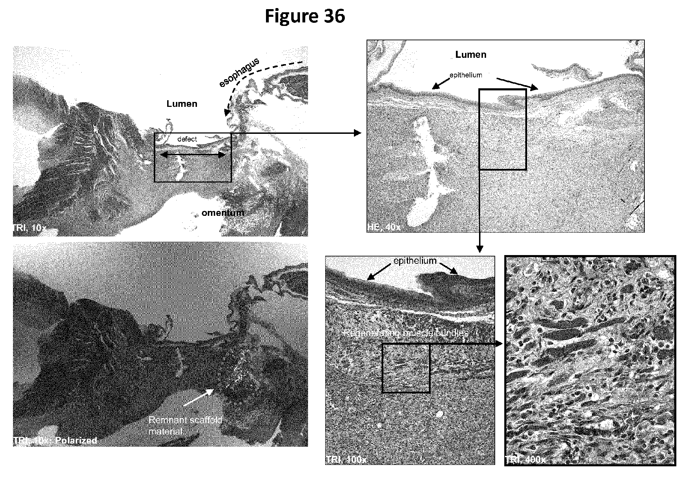

Figure 36 shows the histology at 8 days post implantation. Sections were taken from the periphery of the implant. Section 1 (longitudinal); the peripheral aspect of the treatment site was captured in this section, demonstrating adequate apposition of scaffold patch (construct) to the margins of the defect and complete epithelialization of the luminal surface. There is early regeneration of the esophageal wall characterized by organizing granulation/fibro-connective tissue with presence of smooth muscle bundles, neovascularization and mild inflammatory infiltrate, predominantly macrophages and lymphocytes with some giant cells surrounding suture material. Degradation of the biomaterial (scaffold) is nearly complete (∼90%) as minimal residual fibers were observed when using polarizing light filters. The is no evidence of calcification, necrosis or bacterial colonization. The overlying omentum shows marked active chronic inflammation associated with the adherent lung tissue, which appears to have been included (sutured) to the omentum. Similarly, there are traces of suture material within liver tissue that is adhered to the omentum.

At 8-days post implantation the luminal surface of the defect site (at periphery) is fully covered by epithelium with underlying regenerating muscle bundles and organizing granulation tissue with mild inflammatory response, typically expected in the healing/regenerative process. At 8-days post implantation the center of the defect shows no clear evidence of epithelialization of luminal surface. There is moderate amount of scaffold material that is surrounding by granulation tissue with regenerating muscle bundles at the margins and moderate chronic inflammation.

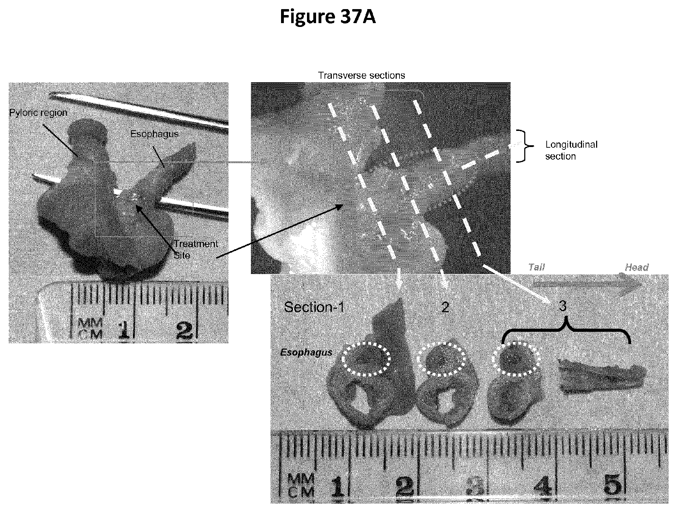

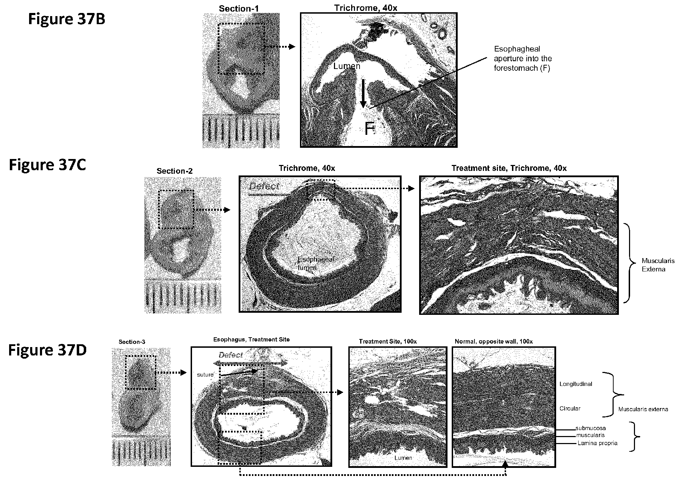

Figure 37A shows the incorporation of an esophagus construct at 10 weeks post implantation. Figure 37B shows Section 1 (transverse) in more detail: native esophagus section with opening (arrow) into the forestomach (F). The defect site treated with an esophagus construct is not present. Figure 37C shows Section 2 (transverse) in more detail: native esophagus section with part of the distal margin of the defect site. There is complete re-epithelialization of luminal mucosal surface with submucosal regeneration (lamina propria and muscularis mucosa). The muscularis externa shows incomplete partial regeneration consisting of fibrovascular connective tissue and smooth muscle cells not completely arranged into circular/longitudinal bundle formation. There is minimal scaffold material (fibers) predominantly within the overlying omentum and muscularis external. There is focal minimal chronic inflammation (macrophages/lymphocytes) without evidence of calcification, necrosis or bacterial colonization.

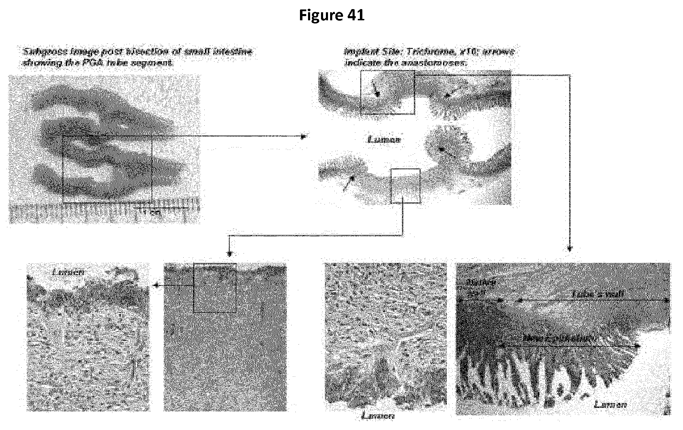

Figure 37D shows Section 3 (transverse) in more detail: neo-esophagus, native esophagus, and treatment site. The 10-weeks post implant animal was sacrificed and a continuous section of the esophagus, containing intact native tissue positioned anterior and posterior to the implant site, was removed and processed for histological assessment (Figure 41D - first panel). A cross section of the implant site and the opposing native intact tissue were stained with H&E (red) and Masson's Trichrome (blue) (Figure 37D - second panel). Higher magnification of the implant site (Figure 37D - third panel) and opposing wall of the intact native esophagus (Figure 37D - fourth panel) revealed complete re-epithelialization of luminal mucosal surface and submucosa in the implant site. The muscularis externa of this site shows evidence of regeneration consisting of fibrovascular connective tissue and smooth muscle cells. However, the smooth muscle cells are not completely arranged into circular/longitudinal bundle formation, as is evident in the native tissue. There is minimal scaffold material (fibers) observed, and where it is present, it appears restricted to the overlying omentum and muscularis external. The implant site appeared to contain minimal focal chronic inflammation, with no evidence of calcification, necrosis or bacterial colonization. After 10 weeks, the implantation of an esophagus construct lead to neo-esophagus regeneration, characterized by the formation of all three wall layers: mucosa, muscularis, and serosa.

### Example 15 - Implantation of small intestine tissue constructs

Tissue engineering principles have been successfully used in developing implantable cell/biomaterial composites for reconstructing luminal organs with laminar wall architecture (e.g., bladder), where *de novo* organogenesis is catalyzed following implantation of the composite (aka, construct) and results in the regeneration of a functional organ. The Organ Regeneration Technology Platform™ (Tengion, Inc.) has successfully applied these principles to develop autologous regenerative products for urinary diversion and other urologic applications as alternative to using gastrointestinal tract segments (the current standard of care). Small intestine (SI) represents a specialized iterative variation of a tubular organ with laminar wall architecture. The results presented here demonstrate the potential to extend a foundational organ regeneration platform beyond the urinary tract to other tubular organs, specifically SI. Smooth muscle cells (Ad-SMC) were expanded *ex vivo* from rat visceral adipose and used to populate biomaterials for SI implantation (Basu J, et al. 7th Annual ISSCR Meeting, 2009). (see also, protocol of Example 4).

**Scaffold Production.** Scaffold was comprised of polyglycolic acid (PGA) mesh coated with poly-DL-lactide-co-glycolide (PLGA). PGA is supplied as a non-woven felt in the form of 20 cm x 30 cm x 0.75 mm sheet, with a bulk density of 70 mg/cc (Concordia Medical). PLGA (50:50, Durect Corporation, IV=0.55-0.75) was dissolved in methylene chloride before use. The PGA mesh was coated by dipping into a beaker containing PLGA solution (a liquefied copolymer (poly-DL-lactide co-glycolide 50:50 80 mg/ml methylene chloride). This coating was added in order to achieve adequate mechanical stability. The coated PGA mesh was then cut to size, sterilized with ethylene oxide, and stored in a desiccant chamber until use. Alternatively, tubular SI scaffold was made by electrospinning a solution of 10% PCL in hexafluoropropanol (Sigma) onto a 4mm diameter mandrel.

**Construct Implant Manufacture.** Sterile scaffolds (patch or tube) were hydrated in SMC growth medium and seeded with 50,000 cells to make constructs for implantation. The construct was covered with growth medium and incubated at 37°C in a humidified, 5% CO²-containing atmosphere for 5 days prior to implantation. Smooth muscle cells (Ad-SMC) were expanded ex vivo from rat visceral adipose and used to populate biomaterials for SI implantation. Ad-SMC were seeded onto woven PGA meshes or PGA or PCL tubes to assemble SI patch or tube constructs (Figure 38). SI patch constructs were sutured with non-resorbable suture over a rectangular defect (5mm x 4 mm) that was cut into the SI wall to completely expose the lumen. Tubular SI scaffolds (1cm length with a 4mm I.D.) were used to connect anterior and distal portions of the SI after transverse dissection and removal of 1 cm portion of native SI.

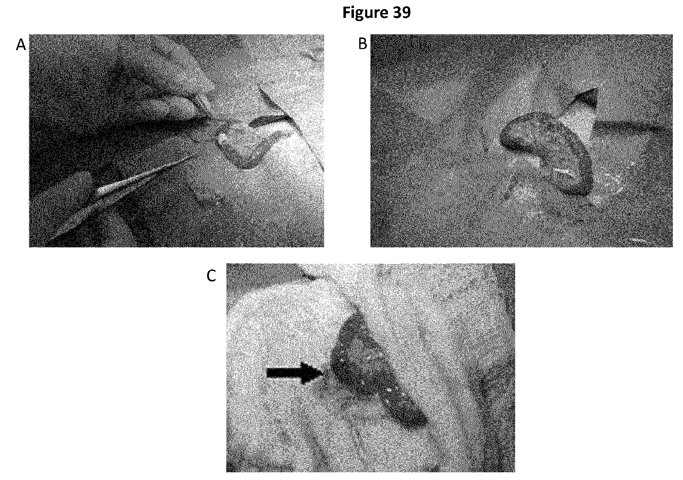

Animal Surgery. Under anesthesia, female Lewis rats (approximately 28 days old) underwent lower midline abdominal laparotomy. Small intestine was mobilized and a defect measuring approximately 3 mm in width and 5 mm in length was created and replaced by the implanted construct using interrupted sutures of non-absorbable 7-0 silk (Ethicon). Alternatively, a 1 cm tubular section of small intestine was completely resected from the host, and end-to-end anastamosis of a 1cm tubular construct achieved using continuous sutures of non-absorbable 7-0 silk (Figure 39A-B). Figure 39C shows an example of a patch construct implanted into the small intestine, with the dark colored non-absorbable suture material being visible around the periphery of the patch. The construct was then covered with omentum. The abdomen was closed after gentamicin (0.1 mg) was given intraperitoneally. Postoperatively, the rats were returned to their cage and allowed unrestricted oral soft food and water intake for 7 days. After this time, the rats were allowed unrestricted oral hard rat chow and water intake. Rats were harvested for histological evaluation of regeneration at times ranging from 4 days to 20 weeks post-implantation.

**Histological evaluation.** At necropsy, tissue containing the regenerated small intestine, as identified and delineated by the non-absorbable sutures, was fixed in 10% buffered formalin (Sigma). Selected sections of the small intestine wall (defect site) were dehydrated in ascending series of ethanol, embedded in paraffin. Sections (5µm) were cut and stained with hematoxylin & eosin and Masson's Trichrome (Premier Laboratory LLC, Longmont, CO) to visualize stromal and muscle components. The non-resorbable suture marking the defect site allowed comparison of native SI and neo-SI tissues to evaluate the regeneration of the laminar layers of mucosa and muscle that comprise the native SI wall.

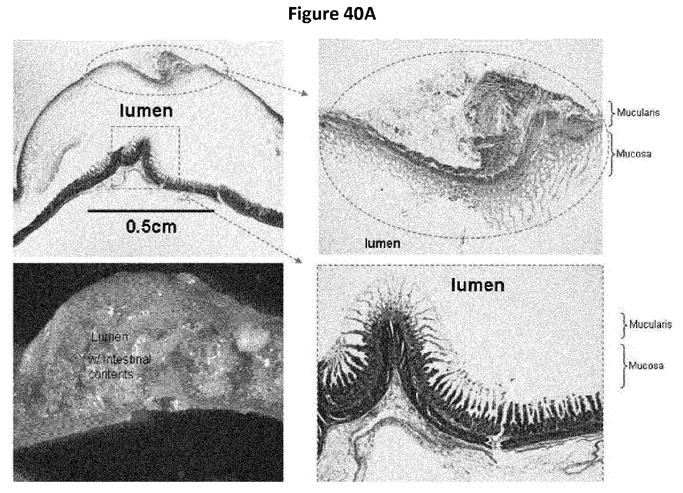

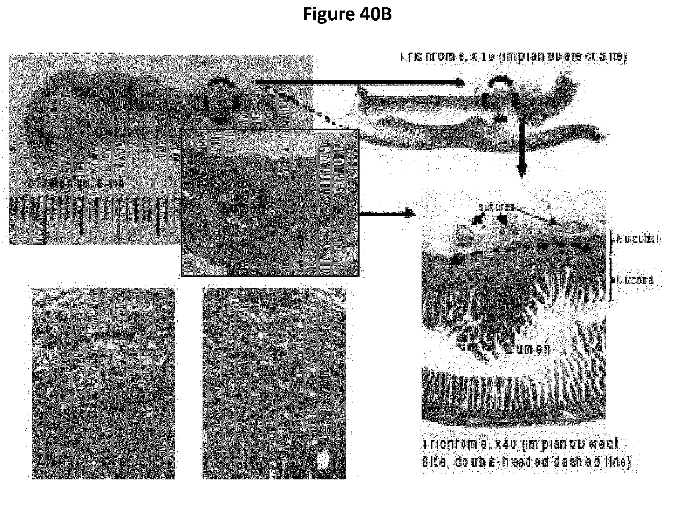

**Complete regeneration of intestinal wall from patch based SI constructs.** To demonstrate regeneration of the small intestine, patch type constructs composed of rectangular 4X5mm vicryl polymer were used to correct surgically induced defects in the SI wall of female Lewis rats (n=4). Test animals were allowed to recover for 8-20 weeks prior to euthanasia and recovery of the area of regeneration as defined by non-absorbable sutures. The extent of regeneration was evaluated by histological analysis in the test animals (summary not shown). Re-epithelialization of the luminal mucosal surface was observed by 8 weeks post-implantation. Near complete degradation of the biomaterial was noted by this time-point. No chronic inflammation was evident. Importantly, as shown in Figure 40A, complete regeneration of all three layers of the small intestinal wall including the muscularis was observable by 8 weeks post-implantation. Figure 40A upper left panel (Trichrome, 10x longitudinal, intestine); upper right panel (Trichrome, 40x implant/defect site); lower left (lumen with intestinal contents shown; subgross, small intestine (longitudinally opened), to expose luminal (intestinal contents) and defect site (dashed line); lower right (Trichrome, 40x normal intestine). Longitudinal sectioning of an SI patch-treated site at 16 weeks post-implant (Figure 40B-C) showed complete regeneration of the intestinal mucosa and submucosa and partial regeneration of muscular layers with no evidence of remnant scaffold fibers, calcification, necrosis, or bacterial colonization. The top left panel of Figure 40B shows the subgross, small intestine and the defect site (dashed elliptical area). The top right panel shows Trichrome, x10 of the implant/defect site. The two lower left panels show Trichrome, x400 of the implant/defect site and regenerated muscle bundles are visible - margin (left) and center (right) of defect. The lower right panel shows Trichrome, x40 of the implant/defect site (double-headed dashed lines). Figure 40C provides a larger version of this panel.

**Partial regeneration of intestinal wall from tubular SI constructs.** Subsequent to surgical excision of approximately 1 cm of native rat SI, tubular SI constructs were implanted by direct end to end anastamosis (n=6) **(****Figure 41****).** Tubular constructs were composed of Ad-SMC seeded biopolymers composed of PGA or PCL. Host rodents were allowed to recover post-implantation for periods ranging from 4 days to 5 months (summary not shown). Efforts to mediate regeneration of SI wall within tubular construct in a manner recapitulating that observed for SI patch were complicated by difficulties associated with development of the surgical procedure, including incomplete anastamosis, strictures, excess suture material, extrusion of biomaterial from construct, poor or incomplete vascularization of construct and adhesions and associated bowel obstructions (summary not shown). These difficulties notwithstanding, preliminary indicators of regenerative outcomes were observable. Partial re-epithelialization of the luminal mucosal surface was noted at 14 days post-implantation of construct. No associated regeneration of muscularis layers was observed, and only minimal degradation of biomaterial was noted. Preliminary regeneration of the intestinal wall was associated with formation of a fibro-vascular layer. Fibro-vascular tissue in-growth with concomitant neo-vascularization was also observable by 14 days post-implantation.

By 10 weeks post-implantation, complete degradation of the biomaterial (PGA) was observed. Significant re-epithelialization of the construct's luminal mucosal surface was noted, with associated fibro-vascular tissue infiltration. Lack of re-epithelialization, where observed, was likely due to significant bacterial colonization. Importantly, regeneration of the tube wall was accompanied by formation of multifocal aggregates of muscle fibers, primarily located at the anastamotic margins (Figure 41). The top left panel of Figure 41 shows subgross image post bisection of small intestine showing the PGA tube segment. The top right panel shows the implant site (Trichrome, x10, arrows indicate anastomoses). The first lower panel on the left shows HE, x400 showing luminal surface, covered by bacterial colonies (staining basophilic). The tube's wall is highly vascularized. The second lower panel shows Trichrome, x10 showing a longitudinal section of the tube's wall (mid). The third lower panel shows the lumen (HE, x400) showing luminal surface, covered by bacterial colonies (staining basophilic). The fourth lower panel on the right shows Trichrome, x40 showing the anastomotic site (proximal) and extent of reepithelialilzation on the luminal surface of the PGA tube.

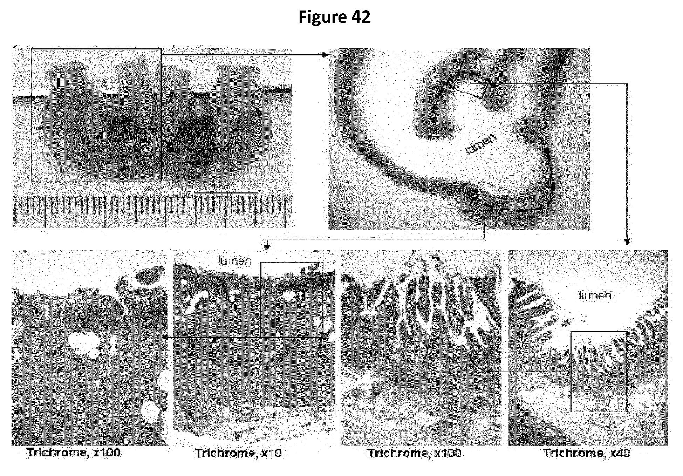

At 5 months post-implantation, biomaterial (PGA) was degraded completely, and near complete regeneration of the epithelial layer of the luminal mucosal surface was noted. Mucosal epithelium with underlying fibro-vascular connective tissue was infiltrated by multi-focal aggregates of muscle fibers and bundles, predominantly near the margins of the defect **(****Figure 42****).** The top left panel of Figure 42 shows a subgross image of post bisection of small intestine showing the PGA tube segment indicated by double-headed black arrows. The upper right panel shows the implant site, Trichrome, x10. The 4 lower panels show Trichrome x100, Trichrome x10, Trichrome x100, and Trichrome x40 (from left to right).

### Example 16 - Evaluation of biomaterials for esophagus or small intestine constructs

Current treatment strategies for patients needing esophageal or small intestine (SI) tissue replacements are often associated with adverse effects, which negatively affect quality of life. To address this issue, this study seeks to apply tissue engineering principles to the regeneration of these organs. Previously, these principles have been successfully used to develop implantable cell/biomaterial composites for reconstructing bladder, another tubular organ with laminar wall architecture. In these cases, de novo organogenesis was catalyzed following implantation of the composite (aka, construct) and resulted in the regeneration of a functional organ (Basu and Ludlow. Trends Biotechnol. 2010;28(10):526-33; Basu J. 7th Annual ISSCR Meeting, 2009; Jayo MJ. Regen Med 2008;3:671; Joseph D. J Urol 2009; 181:555).

**Methods:** Biomaterials of different forms and composition were evaluated. Poly-caprolactone (PCL) foams of pore sizes 23-300µm were made by a solvent cast-particulate leached method as well as polyglycolide (PGA) fibers in various forms coated with poly-DL-lactide-co-glycolide (PLGA). These included coated PGA nonwoven mesh (PGAnw), woven mesh (PGAw) and braided tube (PGAb). Smooth muscle cells were expanded *ex vivo* from rat visceral adipose (Ad-SMC) and used to seed biomaterials for *in vitro* and *in vivo* evaluation (Basu 2009 *supra*)*.* Assessment of this cell-biomaterial *interaction in vitro* was by live/dead staining, cell attachment/proliferation assay (MTS) and scanning electron microscopy (SEM). For evaluation of esophageal and SI regeneration *in vivo,* PGAw and PGAnw were trimmed to 5mm x 4mm rectangular patches and seeded with Ad-SMC to make constructs. PGAb with Ad-SMC was used to make tubular SI constructs. Patch constructs for both esophagus and SI were sutured with non-resorbable suture over a rectangular defect of approximately 5mm x 4mm that was cut into the tissue wall to expose the lumen in adult rats. Tubular SI constructs (10mm length, 4mm I.D.) were used to connect anterior and distal portions of the SI after transverse dissection. Omentum was sutured over the constructs to provide a source of vascularization. Animals were euthanized at time points ranging from 6 days to 20 weeks post-implantation. At necropsy, tissues were harvested, fixed in formalin and paraffin embedded for sectioning and staining with Trichome. The non-resorbable suture marking the defect site allowed comparison of the native and the regenerated tissue.

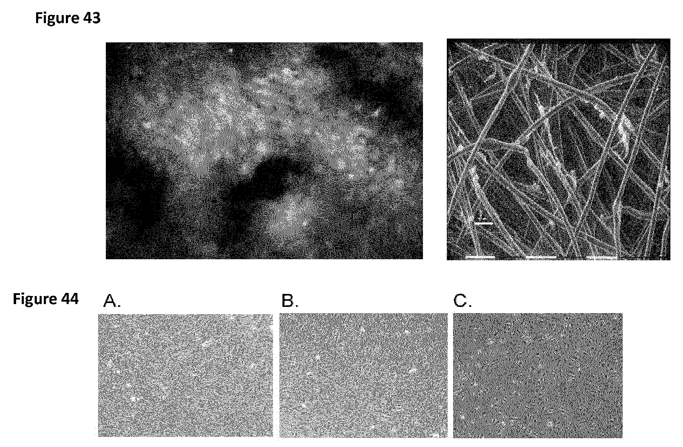

**Results:** *In vitro* assays showed all materials had acceptable cell viability, proliferation, and morphology. Figure 43 - Left: Live/Dead staining of rat Ad-SMC on PCL foam, 150-250µm pore size, 10X. Right: SEM images of rat Ad-SMC on PGAnw, 170X. Lower cell viability and proliferation were seen on the smaller-pore PCL foams.

*In vivo,* analysis was also performed. Figure 37D shows sectioning through the defect sites of the PGAnw esophagus patch construct at 10 weeks post-implant. Figure 40 shows the PGAw SI patch construct at 8 weeks (Figure 40A) and 16 weeks post-implant (Figure 40B-C). Figure 41 shows the PGAb SI tubular construct at 10 weeks. In Figure 42, the PGAb SI tubular construct at 20 weeks post-implant showed complete re-epithelialization of the luminal mucosal surface and a submucosa with partial regeneration of the muscularis externa. There was no evidence of remnant scaffold fibers, calcification, necrosis or bacterial colonization.

PGA and PCL biomaterials showed biocompatibility with Ad-SMC *in vitro.* PGA materials were suitable for producing esophageal and SI patches and SI tubular constructs. *In vivo* implantation of PGA patch constructs resulted in esophageal and SI tissue regeneration within 10 and 16 weeks, respectively. *In vivo* implantation of PGAb tubular constructs resulted in SI tissue regeneration within 20 weeks.

### Example 17 - Endothelial Cell Isolation and Expansion from Peripheral Blood and Adipose Tissue

Endothelial cells have been successfully isolated from peripheral blood and adipose sources (Daiju et al., 2005. Circulation 111: 926-931; Shepherd et al., 2006. The FASEB J. 20: E1124-E1132; melero-Martin JM et al., 2007. Blood 109 (11): 4761-4768; Kern at. Al., 1983. J. Clin. Invest. 71: 1822-1829; Planat-benard V et al., 2004. Circulation 109: 656-663). It has also been reported that culturing of endothelial cells from peripheral blood sources is facilitated by supplementing the culture medium with vascular endothelial growth factor (VEGF).

*Blood-derived cells - Cell Isolation and Culture:* Briefly, peripheral blood was obtained from healthy human volunteers following venapuncture and collection under aseptic conditions. Heparinized blood or leukocyte preps (fresh to 24 hours old) are diluted two to four-fold with DPBS and layered onto an equal volume of Histopaque 1077. Gradients are then spun at 400-800 x g for 30 to 45 minutes. Cells in the single isolated band are retrieved and washed with DPBS. Resuspended in media of choice (α-MEM supplemented with 10 ng/ml VEGF; α-MEM containing 10% FBS, without VEGF supplementation), cells are then plated onto Collagen I, fibronectin or Collagen IV (or combination thereof) coated plates such that the equivalent from 5-20 ml of original whole blood volume is added per P100. Plated cells are placed at 37 C/5% CO2 for 24 - 96 hours prior to media exchange and feeding is continued every 2-4 days with fresh medium. Colony outgrowth occurs within 7-21 days. Cells are passaged following trypsinization and seeding at 4000-8000 cells/cm2 for up to 2 passages. EGM-2 media with or without VEGF can also be used to culture the cells.

*Cell morphology of cultured peripheral blood cells:* Following peripheral blood cell isolation, as described above, cells were cultured for 2-3 weeks in this medium and their morphology examined (see **Figure 44****,** panel B). Control cultures of peripheral blood cells were maintained in medium containing 10% FBS without VEGF supplementation (panel A). Morphology of peripheral blood cells maintained in these two mediums was compared to HuAEC, which served as a positive control

(panel C). Peripheral blood cells maintained in 10% serum-containing medium, without VEGF supplementation, exhibit a fibroblast-like cell morphology (panel A). Following culturing of these cells in medium supplemented with 10ng/ml VEGF (panel B), cells exhibit an endothelial-like morphology of shortened, rounded or cuboidal shape, identical to that seen in control endothelial cell cultures (panel C). Thus, endothelial-like appearing cells isolated from peripheral blood can be successfully cultured in medium supplemented with VEGF

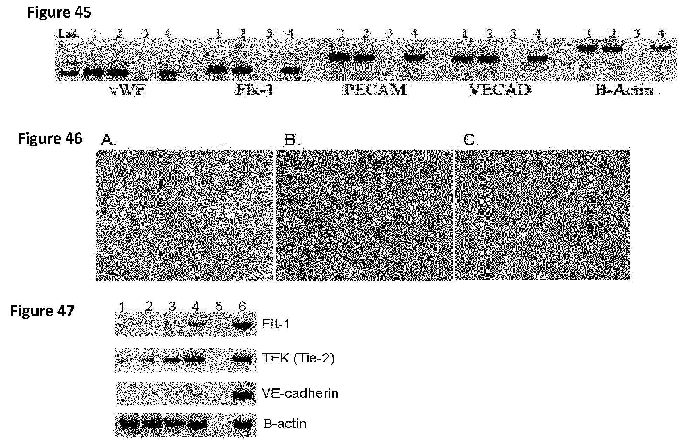

*Endothelial cell gene expression analysis of cultured peripheral blood cells:* Cell samples for RTPCR analysis were taken from cultures maintained in 10% serum-containing medium, without VEGF supplementation **(****Figure 45****,** lanes 1), and cultures maintained in medium supplemented with 10ng/ml VEGF (lanes 2). Distilled water without any PCR primers was used as a negative control (lanes 3), while HuAEC were used a positive control for endothelial cell gene marker expression (lanes 4). **Table 17.1** provides a list of endothelial gene markers used in the RT-PCR analysis. B-actin was used as a loading control for the gel. Expression of these endothelial cell markers is observed when culturing in medium with or without supplementation with VEGF. Thus, confirming that endothelial cells can be isolated and cultured from peripheral blood.

**Table 17.1. Endothelial gene markers chosen for analysis by RTPCR**

| Marker | Gene | Abbreviation |
|---|---|---|
| Endothelial | Cadherin 5 | CDH5/VECAD |
| Endothelial | vonWillebrand Factor | vWF |
| Endothelial | Platelet/Endothelial Cell Adhesion Molecule | PECAM1 |
| Endothelial | FMS-related Tyrosine Kinase I | FLT1/VEGFR |
| Endothelial | Kinase Insert Domain Receptor | KDR/FLK1 |
| Endothelial | Tyrosine Kinase | TEK |

*Adipose-derived cells - Cell Isolation and Culture:* Human adipose samples were obtained either subcutaneously or through lipoaspiration from a commercial vendor and washed 3-5 times with an equal volume of PBS/gentamicin (Gibco) (5µg/ml). Adipose was digested with filter-sterilized collagenase I (Worthington)(0.1%, 1% BSA in DMEM-HG(Gibco)) at 37°C for 1 hour, then centrifuged for 5 minutes at 300g in 50ml conical tubes. The stromal vascular fraction was resuspended in PBS/1% BSA and filtered through a 100 µm Steriflip vacuum filter. The cell population was pelleted again at 300g for 5 minutes, resuspended and cultured in α-MEM supplemented with 10 ng/ml VEGF, α-MEM containing 10% FBS, without VEGF supplementation, or DMEM containing 10% FBS. EGM-2 media with or without VEGF may also be used to culture the cells.

*Cell morphology of cultured adipose-derived cells:* Following adipose cell isolation, as described above, cells were cultured for 2-3 weeks in this medium and their morphology examined (see **Figure 46****,** panel B). Control cultures of adipose-derived cells were maintained in medium containing 10% FBS without VEGF supplementation (panel A). Morphology of adipose-derived cells maintained in these two mediums was compared to HuAEC, which served as a positive control (panel C). Adipose-derived cells maintained in 10% serum-containing medium, without VEGF supplementation, maintained a smooth muscle cell-like morphology, comprised of elongated, whirling cells (panel A). Following culturing of these cells in medium supplemented with 10ng/ml VEGF (panel B), cells exhibit an endothelial-like morphology of shortened, rounded or cuboidal shape, identical to that seen in control endothelial cell cultures (panel C) Thus, endothelial-like appearing cells isolated from adipose tissue can be successfully cultured in medium supplemented with VEGF.

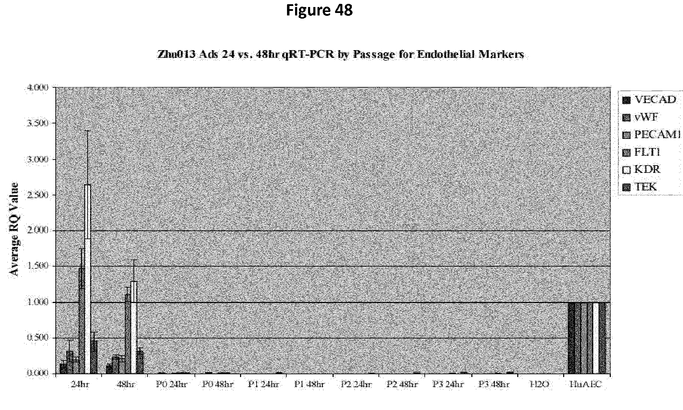

*Endothelial cell gene marker expression of adipose-derived cells:* Adipose-derived cells were isolated and cultured in DMEM + 10% FBS for the purpose of endothelial gene expression analysis by RTPCR (see **Table 17.1.** for list of markers). Cell samples were taken 24- and 48-hr after initial plating, and also at subsequent passages (P0 through P3). Human aortic endothelial cells (HuAEC) were used as a positive control for marker expression. **Figure 48** shows the RTPCR results of the endothelial cell gene expression analysis of adipose-derived cells cultured in DMEM containing 10% FBS. Following initial isolation, adipose-derived cells contain a population of endothelial cells, as evidenced by gene expression of 6 endothelial cell markers (24hr and 48hr after initial plating). Expression of these genes is lost upon culturing and passaging in DMEM 10% FCS, as indicated by loss of endothelial gene expression (P0-P3). Thus, a population of endothelial cells is present in adipose tissue and can be identified following initial cell isolation.

Cell samples for RTPCR analysis were also taken from cultures maintained in 10% serum-containing medium, without VEGF supplementation **(****Figure 47****,** lanes 1 and 3), and cultures maintained in medium supplemented with 10ng/ml VEGF (lanes 2 and 4). Distilled water without any PCR primers was used as a negative control (lane 5), while HuAEC were used a positive control for endothelial cell gene marker expression (lane 6). B-actin was used as a loading control for the gel. Enhanced expression of one or more of these endothelial cell markers is observed when culturing in medium supplemented with VEGF. Thus confirming that endothelial cells can be isolated and cultured from adipose tissue. EGM-2 media with or without VEGF may also be used to culture the cells.

### Example 18 - Canine Study to Assess Neo-Bladder and Neo-Urinary Conduit Implantation

An *in-vivo* study was conducted to determine the functional outcome of implanting a Neo-Bladder Construct (NBR) or Neo-Urinary Conduit (NUC) for tissue regeneration and replacement. Ten animals were enlisted into the study. Prior to the definitive implantation surgery, three animals underwent a bladder biopsy procedure for autologous cell donation to be used in the seeding of the scaffold. These animals were recovered for several days prior to the definitive surgery. All animals underwent a urethral-sparing cystectomy and were implanted with Tengion's Neo-Bladder Constructs. Two of the eleven animals were implanted with constructs seeded with autologous SMC while the remaining eight animals were implanted with Constructs seeded with allogeneic SMC. Animals survived from 43 to 187 days. During the survival period the animals' neo-organs were cycled for a total of 0-162 hours. Neo-organ development was monitored through urodynamic evaluation, cystograms at designated time points, and occasional cystoscopy. Kidney health was monitored using ultrasound and analyses of urine and blood. At the completion of the survival period the uritogenital system tissues were collected and histologically analyzed. Ten animals were successfully implanted and survived from 43 to 188 days. Four of the 11 animals on study were euthanized at elective time points due to terminal medical conditions (i.e., neo-bladder perforation, kidney infection, or hydronephrosis) and one was euthanized for histopathology assessment purposes at week 13. The remaining 6 animals survived to complete scheduled survival period.

**Study design.** A total of 10 canines (7 Females, 3 Males-castrated) were evaluated for complete bladder replacement in healthy animals. Prior to and during the recovery period, blood and urine samples were collected at designated time points, analyzed and recorded. Approximately one week after implantation, the animal's developing neo-organ was cycled through blockage of the urethra opening via a balloon catheter to allow the bladder to fill with urine and then removal of blockage at scheduled time points until the animal demonstrated continence, allowing for "natural cycling" to begin. Fluoroscopic imaging, leak point pressure monitoring (LPP), ultrasounds, and cystoscopic evaluation of the neoorgans were performed at scheduled and unscheduled time points to measure neo-organ capacity, assess neo-organ wall thickness, and monitor the condition of neo-organ, ureters, and kidney throughout the duration of the study. Table 18.1 provides information on each animal. Surgeries. Animals were sedated prior to animal handling, conducting technical procedures, and surgical preparation.

**Table 18.1**

| Animal | Gender | Source | Cell density | Size | Outcome | Cycling (hrs) | Stent removal |
|---|---|---|---|---|---|---|---|
| 1 | F | Autologous | 1.06 x 10⁸ | 67 | Bladder | 0 | 7 days |
| 2 | F | Allogeneic | 1.06 x 10⁸ | 55 | Bladder | 58 | 14 days |
| 4 | F | Autologous | 9.00 x 10⁶ | 47.5 | Bladder | 162 | 2 days |
| 5 | M* | Allogeneic | 2.10 x 10⁷ | 48 | Bladder | 57 | 7 days |
| 6 | F | Allogeneic | 2.10 x 10⁷ | 49 | Conduit | 64 | 8 days |
| 7 | M* | Allogeneic | 4.00 x 10⁶ | 48.5 | Bladder | 144 | 7 days |
| 8 | F | Allogeneic | 4.00 x 10⁶ | 49 | Bladder | 141 | 7 days |
| 9 | F | Allogeneic | 9.00 x 10⁶ | 49.5 | Bladder | 133 | 6 days |
| 10 | F | Allogeneic | 1.06 x 10⁸ | 53 | Bladder | 32 | 5 days |
| 11 | M* | Allogeneic | 1.06 x 10⁸ | 50 | Conduit | 33 | 1/5 days |

| | | | | | | | |
|---|---|---|---|---|---|---|---|
| *males are castrated | | | | | | | |

Biopsy. A midline incision was made in the abdomen beginning immediately caudal to the umbilicus for both animals (Nos. 1 & 4) scheduled to be implanted with autologous Constructs. The urinary bladder was exposed and emptied of urine. One 2 cm x 2 cm apical dome piece of urinary bladder tissue was excised from the bladder. The urinary bladder tissue was immediately preserved aseptically in the tissue culture media for isolation of cells. The defect in the bladder was then closed in at least two layers, using absorbable suture material. The skin was closed in a subcuticular fashion, again using an appropriate size of absorbable suture material. Constructs were prepared according to protocols described herein.

Construct Implantation. A sterile Foley balloon catheter was placed into the bladder and a midline incision was made in the abdomen beginning immediately caudal to the umbilicus. The omentum was identified, kept as two layers, and divided into cranial and caudal halves. The urinary bladder was then exposed and the bladder emptied of urine. A midline longitudinal incision was made into the bladder to assist the surgeon in identifying the trigone area including where the ureters entered on the dorsal aspect and ureteral orifices emptied at the trigone. Vicryl suture of appropriate size was threaded at approximately five points around the urethral neck at the pelvic inlet below the ureters within the trigone area. The ureters were then excised from the bladder leaving a portion of the bladder wall and trigone area intact at the ureter opening. Six of the eleven animals (Animal Nos. 4-9) had a ureter transection to the right or left side that removed the bladder wall/trigone remnant at the ureter opening. A ureteral stent was placed into one or both ureters in ascending fashion from the ureter opening toward the kidney. The entire bladder was then excised proximal to the sutures secured around the urethral neck. The Construct was removed aseptically from the media and maintained moist during the procedure by gently infusing with sterile physiological pH saline using a sterile syringe or moistened gauze. The caudal opening of the Construct was attached to the urethra via the Vicryl suture previously secured to the urethral neck. Following anastomosis, ureter openings for Animal no. 1 was created at approximately the 8 and 4 o'clock positions midpoint on the ventral side of the Construct using a sterile 8mm hole-punch. In Animal Nos. 4-11 the positioning of ureter openings on the dorsal side were adjusted to approximately the 10 and 2 o'clock positions, or closer to the trigone/natural location, to facilitate urine drainage into the Construct. Each ureteral orifice was then sutured onto the appropriate opening on the Construct. Prolene suture was attached to the distal end of each ureter stent to facilitate postsurgical removal. The cranial and caudal omental halves were then pulled over the Construct and approximately 2-4mL of Tisseel® surgical adhesive was used to establish hemostasis in the omental tissue. The Construct wrapped in omentum was visually checked for complete coverage of the Construct to assure adequate closure and water tightness. The abdominal incision was closed in layers and the skin closed in a subcuticular fashion with absorbable suture material. For Animals 1 and 3-9, the Foley balloon catheter was secured to the animal and remained in place to facilitate postoperative urine evacuation, collection and/or cycling. For animals 10 and 11, see "Urinary Catheter & Port System Implantation" below. The ureteral non-degradable stents were secured to all animals. Table 18.1 lists the days after implantation when stents were removed for each animal.

Urinary Catheter & Port System Implantation: During the survival period of Animal Nos. 1, 2 and 4-9 it was noted that the Foley catheter was being removed prematurely by the animals despite efforts to prevent the animals from accessing the catheter (e.g., Elizabethan collar and suturing catheter to animal). To address these issues, Animal Nos. 10 and 11 were each implanted with two independent, indwelling urinary port and catheter systems which were surgically implanted to replace the single Foley balloon catheter system used in Animal Nos. 1, 2, and 4-9. An 8-9Fr. balloon catheter was implanted within the urethra at the bladder neck using a purse-string suture technique during the implantation procedure. Once the balloon end of the catheter was secured to the urethra, the catheter was tunneled to the flank of the animal where a port was attached and implanted in a subcutaneous pocket. The system was then tested to ensure functionality by inflating the balloon with ∼3mL of saline solution and visually verifying the urethra was blocked. The catheter insertion point was on the dorsal side at the apex of the Construct on the side of ureter anastomosis. Once secured to the Construct, the catheter was tunneled through the flank of the animal where a port was attached and implanted in a subcutaneous pocket separate from the balloon catheter port.

Cycling: The indwelling Foley balloon catheter in Animals 1, 2, and 4-9 was used to control filling and emptying of the neo-organ with the animal's own urine. At the start of a cycle period, the distal end of the catheter was blocked to prevent urine from leaking out of the catheter and the catheter balloon was inflated at the urethral opening to block urine from evacuating the neo-organ. The neo-organ was then allowed to naturally fill with urine for approximately 4 hours twice per day. At the end of each 4 hour cycling session, the urine was siphoned from the neo-organ using the Foley catheter and the amount of urine collected was recorded. Animal Nos. 10 and 11 were cycled using the implanted port and catheter system. Via the subcutaneous port, the implanted balloon was filled with saline to obstruct urine flow for approximately 4 hours. The neo-organ was then emptied through the urinary port and catheter system. Cycling performed varied (Table 4) from animal to animal based on animal's ability to tolerate the cycling process or achievement of continence.

Compliance Monitoring - Urodynamics. Leak point pressure (LPP) testing was performed at scheduled time points while the animal was sedated. For Animal Nos. 1, 2, and 4-9, without the indwelling ports and catheter, the area was cleaned and the indwelling Foley balloon catheter was inserted and inflated with ~3 mL of saline solution to block the urethral opening. Residual urine was then removed from the neo-organ using the Foley catheter and a syringe. A physiological pressure transducer (MEMScAP® SP844) monitoring device connected to a calibrated infusion pump containing sterile saline solution was attached to the lumen of the Foley catheter. The saline solution was infused into the neo-organ at a rate of 20 mL/min and the pressure continuously monitored and recorded electronically until saline solution began leaking around the catheter (a.k.a., leak point). Once leakage was identified, the infusion was stopped and the volume (LPP capacity) and pressure (leak point intravesical pressure) were recorded. A confirmation of saline volume within the neo-organ was measured by aspirating neo-organ contents into a container and recording the volume collected. Animal Nos. 10-11, which had the indwelling ports and catheter, were scrubbed with iodine and alcohol and then sprayed with povidone at the port site. The indwelling Foley balloon catheter was inflated with approximately 3 mL of saline solution to block the urethral opening. Residual urine was removed from the neo-organ using the indwelling urinary catheter and syringe. A physiological pressure transducer (MEMScAP® SP844) monitoring device connected to a calibrated infusion pump containing sterile saline solution was attached to the lumen of the indwelling urinary catheter and the leak point volume and pressure were measured and volume confirmed by aspiration of neo-organ contents as described for Animal Nos. 1, 2, and 4-9.

Imaging - Cystograms: Fluoroscopic imaging was performed at scheduled time points while the animal was sedated. For Animal Nos. 1, 2, and 4-9, without the indwelling ports and catheter, the area was cleaned and the indwelling Foley balloon catheter was inserted and inflated with ~3 mL of saline solution to block the urethral opening. Residual urine was then removed from the neo-organ using the Foley catheter and a syringe. Animal Nos. 10-11, which had the indwelling ports and catheter, were scrubbed with iodine and alcohol and then sprayed with povidone at the port site. The indwelling Foley balloon catheter was inflated with approximately 3 mL of saline solution to block the urethral opening. Residual urine was removed from the neo-organ using the indwelling urinary catheter and syringe. A 3:1 saline/contrast solution was injected into the neo-organ via the catheter. Once the neo-organ was filled to capacity based on LPP, an anterior/posterior (AP) and lateral fluoroscopic image were produced to evaluate the characteristics and condition of the neo-organ. The instilled volume was recorded (CYG capacity). All images were electronically archived. The saline/contrast solution was then aspirated from the neo-organ using the indwelling catheter and syringe.

Ultrasounds: Ultrasound imaging of the neo-organ and kidneys was performed at scheduled time points while the animal was sedated. The wall thickness of an empty and full neoorgan was measured and recorded. Left and right kidney length and width were measured and recorded.

Constructs were successfully implanted into mongrel dogs. Seven of eleven dogs survived until scheduled or elective sacrifice, six of the eleven survived for 170-187 days post-implantation. By 43 days, when the first construct was examined, construct healing had achieved steady state, and the resulting neo-organ was comprised of urothelium, submucosa, muscle and serosa/omentum layers.

All neo-organs had adequate to excellent epithelialization, smooth muscle formation and innervation. This was independent of clinical outcome or capacitybased classification (neo-bladder or neo-bladder conduit) at the time of necropsy. Smooth muscle cells from the construct were re-modeled to become a muscle layer (tunica muscularis) in the typical position in which it resides in normal bladder. The tunica muscularis was observed as three separate layers (as is typical in the dog) and as one or two layers of bundled myofibers (as is typical in humans). All presentations were presumed functional.

The successful replacement of an entire canine bladder with both autologous and allogeneic Construct formulations was demonstrated. Both animals implanted with an autologous Construct, survived to or beyond the scheduled 24 Week study duration. 3 animals implanted with allogeneic Constructs also survived to or beyond the study duration. Four allogeneic Construct recipients did not survive to their scheduled necropsy but all 4 appear to be nonproduct related. Positive trends were observed for several of the neo-organ monitoring parameters including: incremental increases in animal weight, neo-organ volume capacity and urodynamic compliance for the majority of the animals throughout the duration of the study. Additionally, the neo-organ wall thickness was found to be slightly thinner but comparable to the general thickness of a canine bladder. Cycling was performed for all animals except Animal No. 1, which was not cycled but was able to regain capacity of its native bladder and complete study (≥24 week survival period). Animals No. 5, 6, 10, and 11 were cycled the least number of hours ranging from 32-64. Of these animals 6 and 11 survived the shortest period of time prior to clinical complications requiring animal euthanasia.

Six out of 10 animals survived a period of≥24 weeks and 5/6 were cycled an average of 146 hours. The quality and quantity of post-surgical neo-organ cycling influenced the outcome of neo-organ regeneration (e.g., neo-bladder or neo-bladder conduit) and increased survivability was noted in this group of animals. Cell density varied over a range of 4.0E+06 to 106E+06 SMC. For functionality purposes, total bladder regeneration was defined as an outcome that at necropsy was ≥50% of the native bladder and/or construct's volume if necropsy occurred ≤120 days post-implantation and ≥65% of the native bladder and/or construct's volume if necropsy occurred >120 days post implantation. Of the 10 animals, 8 achieved a neo-bladder outcome while 2 achieved a neo-bladder conduit outcome.

The functional outcome of implanting an autologous or allogeneic Construct for tissue regeneration and replacement was demonstrated. Ten animals were successfully implanted and survived from 43 to 188 days. The study established that a Neo-Bladder Replacement product may be tested in a canine animal model of total cystectomy and ureteral reimplantation. The quality and quantity of post-surgical neo-organ cycling influenced the outcome of neo-organ regeneration (e.g., neo-bladder or neo-bladder conduit). For functionality purposes, bladder regeneration was observed due to the achievement of a volumetric capacity of≥50% of the native bladder and/or construct's volume if necropsy occurred ≤120 days post-implantation and ≥65% of the native bladder and/or construct's volume if necropsy occurred >120 days post implantation.

## Claims

1. An implantable cell-scaffold construct for treating a gastrointestinal disorder in a subject in need comprising
a) a scaffold comprising a biocompatible matrix having a first surface, wherein the matrix is shaped to conform to at least a part of a native gastrointestinal organ or tissue structure in the subject; and
b) a smooth muscle cell (SMC) population derived from adipose tissue or peripheral blood deposited on or in the first surface, said scaffold and said SMC population forming an implantable construct.

2. The construct of claim 1, wherein the gastrointestinal organ or tissue structure is selected from the group consisting of esophagus, small intestine, large intestine, stomach, colon, and anal sphincter tissue.

3. The construct of claim 1 or 2, wherein the SMC population is derived from adipose tissue or peripheral blood that is autologous to the subject.

4. The construct of claim 1 or claim 2, wherein the SMC population is derived from adipose tissue or peripheral blood that is non-autologous to the subject.

5. The construct of any one of claims 1-4, wherein the matrix is a polymeric matrix selected from the group consisting of a patch and a strip.

6. The construct of claim 5, wherein the patch has a form selected from the group consisting of a disc, a square, an ellipsoid, and a pre-folded form.

7. The construct of any preceding claim, which is free of fibroblast cells.

8. The construct of any preceding claim, which is free of any other gastrointestinal cell population.

9. The construct of any one of claims 1-6, which further comprises a gastrointestinal (GI) cell population.

10. The construct of claim 9, wherein the GI cell population is derived from an esophagus, small intestine, large intestine, stomach, colon, or anal sphincter.

11. The construct of any preceding claim, wherein the gastrointestinal disorder is:
a) an esophagus-related disorder selected from the group consisting of Barrett's esophagus, esophageal atresia, long-gap esophageal atresia, tracheoesophageal fistula, atresia with tracheoesophageal distal fistula, atresia with tracheoesophageal proximal fistula, and atresia with tracheoesophageal double fistula; or
b) a small intestine-related disorder resulting from small bowel resection; or
c) a cancer selected from the group consisting of esophageal cancer, stomach cancer, intestinal cancer, cancer of the sphincter, and colon cancer.

12. The construct of claim 11, part b), wherein the resection was performed in response to a condition selected from the group consisting of inflammatory bowel disease, trauma, mesenteric vascular disease, volvulus, congenital atresias, and neonatal necrotizing enterocolitis.

13. The construct of claim 11, part b) or 12, wherein the small intestine-related disorder is Short Bowel Syndrome (SBS).

14. The construct of any preceding claim, which is for use in a method of treating said gastrointestinal disorder.

15. A method of preparing an implantable cell-scaffold construct for treating a gastrointestinal disorder in a subject according to any preceding claim, the method comprising
a) providing a biocompatible polymeric matrix shaped to conform to at least part of the gastrointestinal organ or tissue structure; and
b) depositing the SMC population on or in the first surface of the matrix and optionally culturing the cell population on the polymeric matrix.

16. Use of an implantable cell-scaffold construct according to any one of claims 1-13 comprising
a) a biocompatible matrix shaped to conform to at least a part of a native gastrointestinal organ or tissue structure in a subject; and
b) a smooth muscle cell (SMC) population derived from adipose tissue or peripheral blood for the manufacture of a medicament for the treatment of said gastrointestinal disorder in a subject.

## Patentansprüche

1. Implantierbares Zellgerüstkonstrukt zur Behandlung einer gastrointestinalen Störung bei einem betroffenen Individuum, umfassend:
a) ein Gerüst, das eine biokompatible Matrix mit einer ersten Oberfläche umfasst, wobei die Matrix so geformt ist, dass sie an zumindest einen Teil einer nativen gastrointestinalen Organ- oder Gewebestruktur im Individuum angepasst ist; und
b) eine Glattmuskelzell(GMZ)-Population, die von adipösem Gewebe oder Peripherblut stammt und auf der oder in der ersten Oberfläche abgelagert ist, wobei das Gerüst und die GMZ-Population ein implantierbares Konstrukt bilden.

2. Konstrukt nach Anspruch 1, wobei die gastrointestinale Organ- oder Gewebestruktur aus der aus Speiseröhre, Dünndarm, Dickdarm, Magen, Kolon und Afterschließmuskelgewebe ausgewählt ist.

3. Konstrukt nach Anspruch 1 oder 2, wobei die GMZ-Population von adipösem Gewebe oder Peripherblut stammt, das bezüglich des Individuums autolog ist.

4. Konstrukt nach Anspruch 1 oder Anspruch 2, wobei die GMZ-Population von adipösem Gewebe oder Peripherblut stammt, das in Bezug auf das Individuum nicht autolog ist.

5. Konstrukt nach einem der Ansprüche 1 bis 4, wobei die Matrix eine Polymermatrix ist, die aus der aus einem Patch und einem Streifen bestehenden Gruppe ausgewählt ist.

6. Konstrukt nach Anspruch 5, wobei das Patch eine Form aufweist, die aus der aus einer Scheibe, einem Viereck, einem Ellipsoid und einer vorgefalteten Form bestehenden Gruppe ausgewählt ist.

7. Konstrukt nach einem der vorangegangenen Ansprüche, das frei von Fibroblastenzellen ist.

8. Konstrukt nach einem der vorangegangenen Ansprüche, das frei von jeglichen anderen gastrointestinalen Zellpopulationen ist.

9. Konstrukt nach einem der Ansprüche 1 bis 6, das weiters eine gastrointestinale (GI-) Zellpopulation umfasst.

10. Konstrukt nach Anspruch 9, wobei die GI-Zellpopulation von einer Speiseröhre, einem Dünndarm, einem Dickdarm, einem Magen, einem Kolon oder einem Afterschließmuskelgewebe stammt.

11. Konstrukt nach einem der vorangegangenen Ansprüche, wobei die gastrointestinale Störung:
a) aus der aus Barrett-Ösophagus, Ösophagusatresie, Ösophagusatresie Typ 2, ösophagotrachealer Fistel, Atresie mit distaler ösophagotrachealer Fistel, Atresie mit proximaler ösophagotrachealer Fistel und Atresie mit ösophagotrachealer Doppelfistel bestehenden Gruppe ausgewählt ist; oder
b) eine von einer Dünndarmresektion herrührende dünndarmbezogene Störung ist; oder
c) ein aus der aus Speiseröhrenkrebs, Magenkrebs, Darmkrebs, Afterschließmuskelkrebs und Kolonkrebs bestehenden Gruppe ausgewählter Krebs ist.

12. Konstrukt nach Anspruch 11, Abschnitt b), wobei die Resektion als Reaktion auf ein Leiden durchgeführt wurde, das aus der aus chronisch-entzündlicher Darmerkrankung, Traumata, Mesenterialgefäßerkrankungen, Volvulus, kongenitalen Atresien und nekrotisierender Enterokolitis des Neugeborenen bestehenden Gruppe ausgewählt ist.

13. Konstrukt nach Anspruch 11, Teil b) oder 12, wobei die dünndarmbezogene Störung das Kurzdarmsyndrom (SBS) ist.

14. Konstrukt nach einem der vorangegangenen Ansprüche, das zur Verwendung in einem Verfahren zur Behandlung der gastrointestinalen Störung dient.

15. Verfahren zur Herstellung eines implantierbaren Zellgerüstkonstrukts zur Behandlung einer gastrointestinalen Störung bei einem Individuum nach einem der vorangegangenen Ansprüche, wobei das Verfahren Folgendes umfasst:
a) Bereitstellen einer biokompatiblen Polymermatrix, die so geformt ist, dass sie an zumindest einen Teil einer gastrointestinalen Organ- oder Gewebestruktur angepasst ist; und
b) Ablagern der GMZ-Population auf der oder in der ersten Oberfläche der Matrix und gegebenenfalls Kultivieren der Zellpopulation auf der Polymermatrix.

16. Verwendung eines implantierbaren Zellgerüstkonstrukts nach einem der Ansprüche 1 bis 13, umfassend:
a) eine biokompatible Matrix, die so geformt ist, dass sie an zumindest einen Teil der gastrointestinalen Organ- oder Gewebestruktur angepasst ist; und
b) eine Glattmuskelzell(GMZ)-Population, die von adipösem Gewebe oder Peripherblut stammt,
zur Herstellung eines Medikaments zur Behandlung der gastrointestinalen Störung bei einem Individuum.

## Revendications

1. Produit de recombinaison à base de structure cellulaire implantable destiné à traiter un trouble gastrointestinal chez un sujet qui en a besoin, qui comprend
a) une structure qui comprend une matrice biocompatible qui possède une première surface, la matrice étant formée afin de se conformer à au moins une partie d'un organe gastro-intestinal natif ou d'une structure tissulaire chez le sujet ; et
b) une population de cellules musculaires lisses (SMC) dérivées d'un tissu adipeux ou du sang périphérique déposé sur ou au sein de la première surface, ladite structure et ladite population de SMC formant un produit de recombinaison implantable.

2. Produit de recombinaison selon la revendication 1, dans lequel l'organe gastro-intestinal ou la structure de tissu est choisi(e) parmi le groupe qui consiste en l'oesophage, l'intestin grêle, le gros intestin, l'estomac, le côlon, et le tissu de sphincter anal.

3. Produit de recombinaison selon la revendication 1 ou 2, dans lequel la population de SMC est dérivée d'un tissu adipeux ou du sang périphérique qui est autologue au sujet.

4. Produit de recombinaison selon la revendication 1 ou 2, dans lequel la population de SMC est dérivée d'un tissu adipeux ou du sang périphérique qui est non autologue au sujet.

5. Produit de recombinaison selon l'une quelconque des revendications 1 à 4, dans lequel la matrice est une matrice polymérique choisie parmi le groupe qui consiste en une retouche et une bande.

6. Produit de recombinaison selon la revendication 5, dans lequel la retouche possède une forme choisie parmi le groupe qui consiste en un disque, un carré, un ellipsoïde, et une forme pré-pliée.

7. Produit de recombinaison selon l'une quelconque des revendications précédentes, qui est exempt de cellules de fibroblaste.

8. Produit de recombinaison selon l'une quelconque des revendications précédentes, qui est exempt de toute autre population de cellules gastro-intestinales.

9. Produit de recombinaison selon l'une quelconque des revendications 1 à 6, qui comprend en outre une population de cellules gastro-intestinales (GI).

10. Produit de recombinaison selon la revendication 9, dans lequel la population de cellules GI est dérivée de l'oesophage, de l'intestin grêle, du gros intestin, de l'estomac, du côlon, et du tissu de sphincter anal.

11. Produit de recombinaison selon l'une quelconque des revendications précédentes, dans lequel le trouble gastro-intestinal est :
a) un trouble lié à l'oesophage choisi parmi le groupe qui consiste en l'oesophage de Barrett, une atrésie oesophagienne, une atrésie oesophagienne à espace long, une fistule trachéo-oesophagienne, une atrésie avec fistule distale trachéo-oesophagienne, une atrésie avec fistule proximale trachéo-oesophagienne, et une atrésie avec fistule double trachéo-oesophagienne ; ou
b) un trouble lié à l'intestin grêle et qui résulte d'une résection de l'intestin grêle ; ou
c) un cancer choisi parmi le groupe qui consiste en un cancer de l'oesophage, un cancer de l'estomac, un cancer intestinal, un cancer des sphincters, et un cancer du côlon.

12. Produit de recombinaison selon la revendication 11, partie b), dans lequel la résection a été effectuée en réponse à un état choisi parmi le groupe qui consiste en une maladie inflammatoire de l'intestin, un traumatisme, une maladie vasculaire mésentérique, un volvulus, des atrésies congénitales, et une entérocolite nécrosante néonatale.

13. Produit de recombinaison selon la revendication 11, partie b) ou 12, dans lequel le trouble lié à l'intestin grêle est un syndrome de l'intestin court (SBS).

14. Produit de recombinaison selon l'une quelconque des revendications précédentes, qui est destiné à être utilisé avec un procédé de traitement dudit trouble gastrointestinal.

15. Procédé de préparation d'un produit de recombinaison à structure cellulaire implantable destiné à traiter un trouble gastro-intestinal chez un sujet selon l'une quelconque des revendications précédentes, le procédé comprenant
a) le fait de prévoir une matrice polymérique biocompatible formée pour se conformer à au moins une partie de l'organe gastro-intestinal ou de la structure tissulaire ; et
b) le dépôt de la population de SMC sur ou au sein de la première surface de la matrice, et, en option, la culture de la population cellulaire sur la matrice polymérique.

16. Utilisation d'un produit de recombinaison à structure cellulaire implantable selon l'une quelconque des revendications 1 à 13, qui comprend
a) une matrice polymérique biocompatible formée pour se conformer à au moins une partie d'un organe gastrointestinal ou d'une structure tissulaire chez un sujet ; et
b) une population de cellules musculaires lisses (SMC) dérivées d'un tissu adipeux ou du sang périphérique pour la fabrication d'un médicament destiné à traiter ledit trouble gastro-intestinal chez un sujet.
